# EUROPEAN PATENT SPECIFICATION

(11) **EP 3 285 778 B1**
(45) Date of publication and mention of the grant of the patent: **10.06.2026**
(21) Application number: 16724150.4
(22) Date of filing: 23.04.2016
(51) Int. Cl.: A61K 31/702, A61K 31/716, A61K 31/733, A61K 45/06, A61P 1/00, A61K 31/19, A61K 31/192, A61K 31/197, A61K 31/198, A61K 31/7016

(54) **MICROBIOME REGULATORS AND RELATED USES THEREOF**
MIKROBIOMREGLER UND ZUGEHÖRIGE VERFAHREN DAVON
RÉGULATEURS DU MICROBIOME ET LEURS UTILISATIONS ASSOCIÉES

(30) Priority: 23.04.2015 US 201562152005 P; 23.04.2015 US 201562152007 P; 23.04.2015 US 201562152017 P; 23.04.2015 US 201562152016 P; 23.04.2015 US 201562152011 P; 13.11.2015 US 201562255366 P; 13.11.2015 US 201562255365 P
(43) Date of publication of application: 28.02.2018
(73) Proprietor: DSM Nutritional Products, LLC, Parsippany, NJ 07054 (US)
(72) Inventor: VON MALTZAHN, Geoffrey, A., Somerville, MA 02145 (US); GEREMIA, John, M., Watertown, MA (US)
(74) Representative: dsm-firmenich IP
(86) International application number: PCT/US2016/029083
(87) International publication number: WO 2016/172658

(56) References cited:
- WO-A1-2004/052121
- WO-A1-2009/082214
- WO-A1-2010/105207
- US-A1- 2004 235 789
- US-A1- 2005 004 070
- KOEN VENEMA ET AL: "The Effect of Lactulose on the Composition of the Intestinal Microbiota and Short-chain Fatty Acid Production in Human Volunteers and a Computer-controlled Model of the Proximal Large Intestine", MICROBIAL ECOLOGY IN HEALTH AND DISEASE, vol. 23, no. 2-3, 1 January 2003 (2003-01-01), pages 16 - 105, XP055209794, ISSN: 0891-060X, DOI: 10.1080/08910600310019895
- RAZA M. A. ET AL: "Effect of rectal lactulose administration with oral therapy on time to recovery from hepatic encephalopathy: a randomized study", ANN. SAUDI MED, vol. 24, no. 5, 1 September 2004 (2004-09-01), pages 374 - 377, XP055965997

## Description

### BACKGROUND

The microbiota of humans is complex. The microbiota performs many activities and may influence the physiology of the host. Changing the numbers and species of gut microbiota can alter community function and interaction with the host. A limited number of probiotic bacteria is known in the art, and some association with health benefits have been documented when ingested by humans. Certain 'prebiotic' foods contain substances that promote the growth of particular bacterial strains that are thought to be beneficial to the human host. The results of clinical tests with these substances are conflicted with respect to their efficacy, and their influence on human health is generally described as being modest. WO 2004/052121 relates to the use of FOS, GPS and other oligosacchardies for modulating the growth of bacteria in the mammalian intestine. US 2004/235789 relates to isomaltoolisaccharaides having a DP less than or eqal to 7 for modifying the abundance of bacterial taxa in animal's intestine. WO 2009/082214 relates to oligosaccharides for modulating the growth of bacteria in the intestine. US 2005/004070 relates to oligosaccharides for reducing and preventing invasion and infection by pathogens in mammalian cells. WO 2010/105207 relates to GOS for stimulating the growth of Bifidobacterium microflora in a patient gut. Venema K. et al. "The Effect of Lactulose on the Composition of the Intestinal Microbiota and Short-chain Fatty Acid Production in Human Volunteers and a Computer-controlled Model of the Proximal Large Intestine" discloses the effect of lactulose on the composition of intestinal microbiota. Thus,
there is a need for novel therapeutics that can stimulate beneficial microbiota shifts and improve human health.

### SUMMARY OF THE INVENTION

The subject matter of the invention is as defined in the appended claims.

The present invention features dosage forms for use in promoting growth of commensal bacteria . In one aspect, the present invention provides a dosage form comprising a composition for use in promoting growth of commensal bacteria, said composition comprising a microbiome regulator capable of modulating the microbiome of a subject, wherein the dosage form targets the release of the composition substantially in the large intestine, wherein the microbiome regulator comprises a sugar or a sugar alcohol, wherein the sugar or sugar alcohol is selected from a monosaccharide and a disaccharide, wherein the sugar or sugar alcohol is selected from the group consisting of D-fructose, D-galactose, L-arabinose, D-mannose, D-xylose, D-arabinose, ribose, L-fucose, L-rhamnose, lactose, D-maltose, sucrose, and glucose; and wherein the composition comprises more than about 50% (w/w) of the microbiome regulator. In some embodiments, the microbiome regulator comprises at least two microbiome regulators (e.g., a sugar, a sugar alcohol, an amino acid, a peptide, a micronutrient, a fatty acid, or a polyphenol). In some embodiments, the microbiome regulator comprises at least three microbiome regulators (e.g., a sugar, a sugar alcohol, an amino acid, a peptide, a micronutrient, a fatty acid, or a polyphenol).

In some embodiments, the microbiome regulator comprises a sugar or sugar alcohol. In some embodiments, the microbiome regulator comprises a sugar or sugar alcohol that is metabolizable by the host. In some embodiments, the microbiome regulator comprises a sugar or sugar alcohol that is non-metabolizable by the host. In some embodiments, the sugar or sugar alcohol comprises a monosaccharide or disaccharide. In some embodiments, the sugar or sugar alcohol comprises a monosaccharide. In some embodiments, the sugar or sugar alcohol comprises a disaccharide. In some embodiments, the sugar or sugar alcohol comprises a disaccharide, and at least one of the glycosidic bonds independently comprise a 1->2 glycosidic bond, a 1->3 glycosidic bond, a 1->4 glycosidic bond, or a 1->6 glycosidic bond. In some embodiments, the sugar or sugar alcohol comprises a disaccharide, and at least one of the glycosidic bonds are present in the alpha or beta configuration.

In some embodiments, the sugar or sugar alcohol comprises of D-fructose, D-galactose, L-arabinose, D-mannose, D-xylose, D-arabinose, ribose, L-fucose, L-rhamnose, lactose, D-maltose, sucrose, or glucose. In some embodiments, the the sugar or sugar alcohol does not comprise glucose.

In some embodiments, the sugar or sugar alcohol comprises D-fructose, D-galactose, L-arabinose, D-mannose, D-xylose, D-arabinose, ribose, L-fucose, L-rhamnose, lactose, D-maltose, sucrose, or glucose. In some embodiments, the sugar or sugar alcohol is metabolizable by the host and comprises D-fructose, D-galactose, L-arabinose, D-mannose, D-xylose, D-arabinose, ribose, L-fucose, L-rhamnose, lactose, D-maltose, sucrose, or glucose.

In some embodiments, the composition comprises more than about 50% (w/w) of a sugar or sugar alcohol (e.g., more than about 55%, about 60%, about 65%, about 70%, about 75%, about 80%, about 85%, about 90%, about 95%, about 96%, about 97%, about 98%, about 99%, or more). **In** some embodiments, the composition comprises more than about 50% (w/w) of a sugar or sugar alcohol (e.g., more than about 55%, about 60%, about 65%, about 70%, about 75%, about 80%, about 85%, about 90%, about 95%, about 96%, about 97%, about 98%, about 99%, or more) that is metabolizable by the host.

**In** some embodiments, the microbiome regulator comprises glucose. **In** some embodiments, the microbiome regulator comprises galactose. **In** some embodiments, the microbiome regulator comprises fucose. **In** some embodiments, the microbiome regulator comprises fructose. **In** some embodiments, the microbiome regulator comprises mannose. **In** some embodiments, the microbiome regulator comprises xylose. **In** some embodiments, the microbiome regulator comprises arabinose. **In** some embodiments, the microbiome regulator comprises rhamnose. **In** some embodiments, the microbiome regulator comprises sucrose. **In** some embodiments, the microbiome regulator comprises lactose. **In** some embodiments, the microbiome regulator comprises maltose.

**In** some embodiments, the microbiome regulator comprises a molecule with a molecular weight less than about 1000 g/mol (e.g., less than about 950 g/mol, about 900 g/mol, about 850 g/mol, about 800 g/mol, about 750 g/mol, about 700 g/mol, about 650 g/mol, about 600 g/mol, about 500 g/mol, about 450 g/mol, about 400 g/mol, about 350 g/mol, about 300 g/mol, about 250 g/mol, about 200 g/mol, or less). **In** some embodiments, the microbiome regulator comprises a molecule with less than about 30 carbon atoms (e.g., less than about 25 carbon atoms, about 20 carbon atoms, about 18 carbon atoms, about 15 carbon atoms, about 12 carbon atoms, about 10 carbon atoms, about 9 carbon atoms, about 8 carbon atoms, about 7 carbon atoms, about 6 carbon atoms, or about 5 carbon atoms). **In** some embodiments, the microbiome regulator comprises a molecule with less than about 30 carbon atoms (e.g., less than about 25 carbon atoms, about 20 carbon atoms, about 18 carbon atoms, about 15 carbon atoms, about 12 carbon atoms, about 10 carbon atoms, about 9 carbon atoms, about 8 carbon atoms, about 7 carbon atoms, about 6 carbon atoms, or about 5 carbon atoms) and less than about 30 heteroatoms (e.g., less than about 25 heteroatoms, about 20 heteroatoms, about 18 heteroatoms, less than about 15 heteroatoms, less than about 12 heteroatoms, less than about 10 heteroatoms, less than about 9 heteroatoms, less than about 8 heteroatoms, less than about 7 heteroatoms, less than about 6 heteroatoms, or less than about 5 heteroatoms). In some embodiments, the microbiome regulator comprises a molecule with less than about 30 carbon atoms (e.g., less than about 25 carbon atoms, about 20 carbon atoms, about 18 carbon atoms, about 15 carbon atoms, about 12 carbon atoms, about 10 carbon atoms, about 9 carbon atoms, about 8 carbon atoms, about 7 carbon atoms, about 6 carbon atoms, or about 5 carbon atoms) and less than about 30 oxygen atoms (e.g., less than about 25 oxygen atoms, about 20 oxygen atoms, about 18 oxygen atoms, less than about 15 oxygen atoms, less than about 12 oxygen atoms, less than about 10 oxygen atoms, less than about 9 oxygen atoms, less than about 8 oxygen atoms, less than about 7 oxygen atoms, less than about 6 oxygen atoms, or less than about 5 oxygen atoms).

In some embodiments, the microbiome regulator does not comprise an alkenyl, alkynyl, aryl, heteroaryl, cycloalkyl, halogen, ester, carboxyl, acyl, thiol, amino, amido, cyano, nitro, sulfonyl, sulfate, or phosphate moiety. In some embodiments, the microbiome regulator does not comprise an alkenyl, alkynyl, aryl, heteroaryl, cycloalkyl, halogen, acyl, thiol, cyano, nitro, or sulfonyl moiety. In some embodiments, the microbiome regulator is an FDA approved molecule.

In some embodiments, the sugar or sugar alcohol has a low degree of sweetness relative to sucrose. In some embodiments, the sugar or sugar alcohol has a degree of sweetness less than about 1 relative to sucrose (e.g., less than about 0.95, about 0.9, about 0.85, about 0.8, about 0.75, about 0.7, about 0.65, about 0.6, about 0.55, about 0.5, or less). In some embodiments, the sugar or sugar alcohol has a degree of sweetness less than about 2 relative to sucrose (e.g., less than about 3, about 4, about 5, about 10, about 20, about 25, about 50, about 75, about 100, about 250, about 500, about 1000, or more). In some embodiments, the sugar or sugar alcohol has a degree of sweetness less than about 5 relative to sucrose (e.g., less than about 0.95, about 0.9, about 0.85, about 0.8, about 0.75, about 0.7, about 0.65, about 0.6, about 0.55, about 0.5, or less) and is metabolizable by the host.

In some embodiments, the sugar or sugar alcohol has a low absorption coefficient relative to glucose. In some embodiments, the sugar or sugar alcohol has an absorption coefficient less than 0.15 (e.g., less than about 0.14, about 0.13, about 0.12, about 0.11, about 0.10, about 0.09, about 0.08, about 0.07, about 0.06, about 0.05, about 0.04, about 0.03, about 0.02, about 0.01, or less). In some embodiments, the sugar or sugar alcohol has an absorption coefficient less than 0.15 (e.g., less than about 0.14, about 0.13, about 0.12, about 0.11, about 0.10, about 0.09, about 0.08, about 0.07, about 0.06, about 0.05, about 0.04, about 0.03, about 0.02, about 0.01, or less) and is metabolizable by the host.

In some embodiments, a substantial portion of the sugar or sugar alcohol that is metabolized by the host is metabolized in the lower GI tract (e.g., the small intestine or large intestine). In some embodiments, more than about 50% (w/w) of the sugar or sugar alcohol that is metabolized by the host is metabolized in the lower GI tract of the host (e.g., more than about 55%, about 60%, about 65%, about 70%, about 75%, about 80%, about 85%, about 90%, about 95%, about 99%, about 99.9%, or more).

In some embodiments, a substantial portion of the sugar or sugar alcohol that is metabolized by the host is metabolized in the small intestine, e.g., the duodenum, jejunum, or ileum. In some embodiments, more than about 50% (w/w) of the sugar or sugar alcohol that is metabolized by the host is metabolized in small intestine, e.g., the duodenum, jejunum, or ileum, of the host (e.g., more than about 55%, about 60%, about 65%, about 70%, about 75%, about 80%, about 85%, about 90%, about 95%, about 99%, about 99.9%, or more).

In some embodiments, a substantial portion of the sugar or sugar alcohol that is metabolized by the host is metabolized in the large intestine, e.g., cecum, colon, or rectum. In some embodiments, more than about 50% (w/w) of the sugar or sugar alcohol that is metabolized by the host is metabolized in large intestine, e.g., the cecum, colon, or rectum, of the host (e.g., more than about 55%, about 60%, about 65%, about 70%, about 75%, about 80%, about 85%, about 90%, about 95%, about 99%, about 99.9%, or more).

In some embodiments, the composition comprises less than about 50% of a sweetener (e.g., less than about 45%, about 40%, about 35%, about 30%, about 25%, about 20%, about 15%, about 10%, about 5%, about 3%, about 2%, about 1%, about 0.5%) that is non-metabolizable by the host. In some embodiments, the ratio (w/w) of a microbiome regulator to a sweetener that is non-metabolizable by the host is greater than about 1:1 (e.g., about 1.1:1, about 1.2:1, about 1.3:1, about 1.4:1, about 1.5:1, about 1.75:1, about 2:1, about 2.25:1, about 2.5:1, about 2.75:1, about 3:1, about 4:1, about 5:1, about 6:1, about 7:1, about 8:1, about 9:1, about 10:1, about 12.5:1, about 15:1, about 20:1, about 30:1, about 40:1, about 50:1, about 100:1, about 500:1, about 1000:1, or more). In some embodiments, the composition is substantially free of a sweetener that is non-metabolizable by the host.

In some embodiments, the sweetener that is non-metabolizable by the host comprises an alkyl, alkenyl, alkynyl, aryl, heteroaryl, cycloalkyl, heterocyclyl, halogen, ester, carboxyl, acyl, thiol, amino, amido, cyano, nitro, sulfonyl, sulfate, or phosphate moiety.

In some embodiments, the sweetener that is non-metabolizable by the host has a high degree of sweetness relative to sucrose. In some embodiments, the sweetener that is non-metabolizable by the host has a degree of sweetness greater than about 5 times that of sucrose (e.g., greater than about 10 times that of sucrose, about 15, about 20, about 25, about 50, about 75, about 100, about 150, about 200, about 250, about 300, about 350, about 400, about 450, about 500, about 550, about 600, about 650, about 700, about 750, about 800, about 850, about 900, about 950, about 1000, about 1250, about 1500, about 2000, about 3000, about 4000, about 5000, about 6000, about 7000, about 8000, about 9000, about 10,000, about 11,000, about 12,000, about 13,000, about 14,000, about 15,000, or more). In some embodiments, the sweetener that is non-metabolizable by the host has a degree of sweetness greater than about 100 times that of sucrose (e.g., greater than about 150 times that of sucrose, about 200, about 250, about 300, about 350, about 400, about 450, about 500, about 550, about 600, about 650, about 700, about 750, about 800, about 850, about 900, about 950, about 1000, about 1250, about 1500, about 2000, about 3000, about 4000, about 5000, about 6000, about 7000, about 8000, about 9000, about 10,000, about 11,000, about 12,000, about 13,000, about 14,000, about 15,000, or more). In some embodiments, the sweetener that is non-metabolizable by the host has a degree of sweetness between about 100-20,000 times greater than that of sucrose (e.g., between about 100-15,000, about 100-10,000, about 100-9,000, about 100-8,000, about 100-7,000, about 100-6,000, about 100-5,000, about 100-4,000, about 100-3,000, about 100-2,000, about 100-1,000, about 100-750, about 100-500, about 100-400, about 100-300, about 100-250, about 100-200 times).

In some embodiments, the sweetener that is non-metabolizable by the host is a sugar or sugar alcohol. In some embodiments, the sweetener that is non-metabolizable by the host is sucralose, aspartame, aspartame-acesulfame salt, advantame, stevioside, neotame, saccharin, acesulfame-K, alitame, cyclamate, neohesperidine, or rebaudioside.

In some embodiments, the microbiome regulator is a sugar or sugar alcohol that is slowly metabolized by the host (e.g., metabolized by the host more slowly than glucose). In some embodiments, the sugar or sugar alcohol is substantially not metabolized by the host. In some embodiments, more than about 5% (w/w) of the sugar or sugar alcohol is not metabolized by the host (e.g., more than about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 99%, about 99.9%, or more). In some embodiments, the sugar or sugar alcohol is substantially not metabolized by the host but is metabolized by the microbiota. In some embodiments, more than about 5% (w/w) of the sugar or sugar alcohol is metabolized by the microbiota (e.g., more than about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 99%, about 99.9%, or more) but is substantially not metabolized by the host.

In some embodiments, the microbiome regulator is a sugar or sugar alcohol and is recognized by, has specificity for, or binds to a protein. In some embodiments, the protein is an enzyme or a lectin. In some embodiments, the enzyme is a glycosidase, a phosphatase, a kinase, a transferase, or a transporter. In some embodiments, the glycosidase is a glycoside hydrolase classified in one of the glycoside hydrolase families 1-128. In some embodiments, the glycosidase is a hydrolase (e.g., amylase, sucrose, lactase, or maltase). In some embodiments, the enzyme is a transferase (e.g., a glycosyltransferase, e.g., a glycosyltransferase classified in one of the glycosyltransferase families 1-98).

In some embodiments, the microbiome regulator comprises an amino acid. In some embodiments, the amino acid is an L-amino acid or a D-amino acid. In some embodiments, the amino acid is naturally occurring. In some embodiments, the amino acid is selected from alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, and valine. In some embodiments, the amino acid is selected from cysteine or leucine. In some embodiments, the composition comprises at least about 1% (w/w) of an amino acid (e.g., at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 96%, about 97%, about 98%, about 99%, or more).

In some embodiments, the microbiome regulator may further comprise a peptide (e.g., a dipeptide, a tripeptide, a tetrapeptide, or a pentapeptide). In some embodiments, the peptide comprises an L- amino acid or D- amino acid.

In some embodiments, the microbiome regulator may further comprise a micronutrient. In some embodiments, the micronutrient comprises a vitamin, an element, or a mineral. In some embodiments, the vitamin comprises pantothenate, thiamine, riboflavin, niacin, pyridoxol, biotin, folate, 4-aminobenzoate, cobinamide, a cobamide (e.g., phenyolyl cobamide, 5-methylbenzimidazolyl cobamide), or cobalamin, or salts or derivatives thereof. In some embodiments, the element or mineral comprises chloride, sodium, calcium, magnesium, nitrogen, potassium, manganese, iron (e.g., Fe²⁺ or Fe³⁺), zinc, nickel, copper, or cobalt. In some embodiments, the composition comprises at least about 0.1% (w/w) of a micronutrient, e.g., a vitamin, element, or mineral (e.g., at least about 0.5%, about 1%, about 1.5%, about 2%, about 3%, about 4%, about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 96%, about 97%, about 98%, about 99%, or more).

In some embodiments, the microbiome regulator may further comprise a fatty acid. In some embodiments, the fatty acid comprises a short-chain fatty acid (SCFA), a medium-chain fatty acid (MCFA), a long-chain fatty acid (LCFA), or a very long chain fatty acid (VLCFA). In some embodiments, the fatty acid comprises a saturated or unsaturated fatty acid. In some embodiments, the fatty acid comprises a molecule containing at least 2 carbon atoms (e.g., at least 3 carbon atoms, 4 carbon atoms, 5 carbon atoms, 6 carbon atoms, 7 carbon atoms, 8 carbon atoms, 9 carbon atoms, 10 carbon atoms, 12 carbon atoms, 14 carbon atoms, 16 carbon atoms, 18 carbon atoms, 20 carbon atoms, 22 carbon atoms, 24 carbon atoms, 26 carbon atoms, 28 carbon atoms, or more). In some embodiments, the short chain fatty acid comprises acetic acid, propionic acid, butryic acid, isobutyric acid, valeric acid, isovaleric acid, hexanoic acid, or octanoic acid. In some embodiments, the composition comprises at least about 0.1% (w/w) of a fatty acid, e.g., a short chain fatty acid (e.g., at least about 0.5%, about 1%, about 1.5%, about 2%, about 3%, about 4%, about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 96%, about 97%, about 98%, about 99%, or more).

In some embodiments, the microbiome regulator may further comprise a polyphenol. In some embodiments, the polyphenol comprises a plant polyphenol isolated from a plant source material. In some embodiments, the plant source material comprises blueberry, cranberry, grape, peach, plum, pomegranate, soy, red wine, black tea, or green tea. In some embodiments, the polyphenol comprises a catechin, ellagitannin, isoflavone, flavonol, flavanone, anthocyanin, or lignin. In some embodiments, the composition comprises at least about 1% (w/w) of a polyphenol (e.g., at least about 2%, about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 96%, about 97%, about 98%, about 99%, or more). In some embodiments, the composition further comprises a probiotic or prebiotic.

In some embodiments, the composition is formulated as a unit dosage form. In some embodiments, the unit dosage form comprises a liquid, a gel, a cream, an ointment, a powder, a tablet, a pill, a capsule, a depository, a single-use applicator, or medical device (e.g. a syringe). In some embodiments, the unit dosage form comprises a liquid dosage form or solid dosage form. In some embodiments, the unit dosage form is formulated for oral administration. In some embodiments, the unit dosage form is a liquid dosage form formulated for oral administration. In some embodiments, the liquid dosage form for oral administration comprises a solution, syrup, a suspension, an emulsion, a tincture, or an elixir. In some embodiments, the unit dosage form is a solid dosage form formulated for oral administration. In some embodiments, the solid dosage form for oral administration comprises a pill, tablet, or capsule. In some embodiments, the solid dosage form for oral administration is enterically coated, coated for timed release, or coated for controlled release.

In some embodiments, the unit dosage form is formulated for enteral administration. In some embodiments, the unit dosage form is a liquid dosage form formulated for enteral administration. In some embodiments, the liquid dosage form for enteral administration comprises a solution, a syrup, a suspension, an emulsion, a tincture, or an elixir. In some embodiments, the unit dosage form is a solid dosage form formulated for enteral administration. In some embodiments, the solid dosage form for enteral administration comprises a pill, tablet, capsule, ointment, suppository, or enema. In some embodiments, the dosage form is targeted to the small intestine, e.g., the duodenum, jejunum, or ileum. In some embodiments, the dosage form is targeted to the large intestine, e.g., cecum, colon, or rectum.

In some embodiments, the composition comprises less than about 50% (w/w) of an agent other than the microbiome regulator (e.g., less than about 40%, about 30%, about 25%, about 20%, about 15%, about 10%, about 5%, about 2.5%, about 2%, about 1%, about 0.5%, about 0.1%, about 0.05%, or less). In some embodiments, the ratio (w/w) of a microbiome regulator to an agent other than a microbiome regulator is greater than about 1:1 (e.g., about 1.1:1, about 1.2:1, about 1.3:1, about 1.4:1, about 1.5:1, about 1.75:1, about 2:1, about 2.25:1, about 2.5:1, about 2.75:1, about 3:1, about 4:1, about 5:1, about 6:1, about 7:1, about 8:1, about 9:1, about 10:1, about 12.5:1, about 15:1, about 20:1, about 30:1, about 40:1, about 50:1, about 100:1, about 500:1, about 1000:1, or more). In some embodiments, the composition is substantially free of an agent other than a microbiome regulator.

In some embodiments, the agent other than the microbiome regulator is a therapeutic agent. In some embodiments, the composition comprises less than about 50% (w/w) of a therapeutic agent (e.g., less than about 40%, about 30%, about 25%, about 20%, about 15%, about 10%, about 5%, about 2.5%, about 2%, about 1%, about 0.5%, about 0.1%, about 0.05%, or less). In some embodiments, the ratio (w/w) of a microbiome regulator to a therapeutic agent is greater than about 1:1 (e.g., about 1.1:1, about 1.2:1, about 1.3:1, about 1.4:1, about 1.5:1, about 1.75:1, about 2:1, about 2.25:1, about 2.5:1, about 2.75:1, about 3:1, about 4:1, about 5:1, about 6:1, about 7:1, about 8:1, about 9:1, about 10:1, about 12.5:1, about 15:1, about 20:1, about 30:1, about 40:1, about 50:1, about 100:1, about 500:1, about 1000:1, or more). In some embodiments, the composition is substantially free of a therapeutic agent.

In some embodiments, the therapeutic agent comprises a peptide, nucleic acid, oligosaccharide, polysaccharide, protein, non-peptide small molecule, or a prodrug or metabolite thereof. In some embodiments, the therapeutic agent is a secondary metabolite (e.g., an alkaloid, glycoside, lipid, nonribosomal peptide, ribosomal peptide, phenazine, phenol, polyketide, terpene, or tetrapyrrole). In some embodiments, the therapeutic agent comprises a molecule with a molecular weight greater than about 200 g/mol (e.g., greater than about 250 g/mol, about 300 g/mol, about 350 g/mol, about 400 g/mol, about 500 g/mol, about 600 g/mol, about 700 g/mol, about 800 g/mol, about 900 g/mol, about 1000 g/mol, about 1100 g/mol, about 1200 g/mol, about 1300 g/mol, about 1400 g/mol, about 1500 g/mol, about 2000 g/mol, or more). In some embodiments, the therapeutic agent comprises a molecule having more than about 6 carbon atoms (e.g., about 7 carbon atoms, about 8 carbon atoms, about 9 carbon atoms, about 10 carbon atoms, about 12 carbon atoms, about 15 carbon atoms, about 20 carbon atoms, about 24 carbon atoms, about 30 carbon atoms, or more). In some embodiments, the therapeutic agent comprises a molecule having more than about 6 carbon atoms (e.g., about 7 carbon atoms, about 8 carbon atoms, about 9 carbon atoms, about 10 carbon atoms, about 12 carbon atoms, about 15 carbon atoms, about 20 carbon atoms, about 24 carbon atoms, or about 30 carbon atoms, or more) and more than about 6 heteroatoms (e.g., about 7 carbon atoms, about 8 carbon atoms, about 9 carbon atoms, about 10 carbon atoms, about 12 carbon atoms, about 15 carbon atoms, about 20 carbon atoms, about 24 carbon atoms, about 30 carbon atoms, or more). In some embodiments, the therapeutic agent comprises a molecule having more than about 6 carbon atoms (e.g., about 7 carbon atoms, about 8 carbon atoms, about 9 carbon atoms, about 10 carbon atoms, about 12 carbon atoms, about 15 carbon atoms, about 20 carbon atoms, about 24 carbon atoms, or about 30 carbon atoms, or more) and more than about 6 oxygen atoms (e.g., about 7 oxygen atoms, about 8 oxygen atoms, about 9 oxygen atoms, about 10 oxygen atoms, about 12 oxygen atoms, about 15 oxygen atoms, about 20 oxygen atoms, about 24 oxygen atoms, about 30 oxygen atoms, or more).

In some embodiments, the therapeutic agent has a specificity for a cell surface receptor, an ion channel, a transporter, an enzyme, an antibody, or other biological target. In some embodiments, the therapeutic agent is an agent used in the treatment of a disease, disorder, or condition. In some embodiments, the therapeutic agent is an agent used in the treatment of an inflammatory disease, infectious disease, metabolic disease, or neurodegenerative disease. In some embodiments, the therapeutic agent is an agent used in the treatment of cancer, diabetes, cardiovascular disease, a fibrotic disease, or a microbial infection (e.g., a bacterial, fungal, or viral infection). In some embodiments, the therapeutic agent is a microbiocide (e.g., an antibiotic, antifungal, or antiviral agent). In some embodiments, the therapeutic agent is an FDA approved drug substance. In some embodiments, the therapeutic agent does not naturally occur in nature.

In some embodiments, the agent other than the microbiome regulator is a polymer, carrier, filler, or excipient. In some embodiments, the composition comprises less than about 50% (w/w) of at least one of a polymer, carrier, filler, or excipient (e.g., less than about 40%, about 30%, about 25%, about 20%, about 15%, about 10%, about 5%, about 2.5%, about 2%, about 1%, about 0.5%, about 0.1%, about 0.05%, or less). In some embodiments, the ratio (w/w) of a microbiome regulator to at least one of a polymer, carrier, filler, or excipient is greater than about 1:1 (e.g., about 1.1:1, about 1.2:1, about 1.3:1, about 1.4:1, about 1.5:1, about 1.75:1, about 2:1, about 2.25:1, about 2.5:1, about 2.75:1, about 3:1, about 4:1, about 5:1, about 6:1, about 7:1, about 8:1, about 9:1, about 10:1, about 12.5:1, about 15:1, about 20:1, about 30:1, about 40:1, about 50:1, about 100:1, about 500:1, about 1000:1, or more). In some embodiments, the composition is substantially free of at least one of a polymer, carrier, filler, or excipient. In some embodiments, the composition comprises less than about 50% (w/w) of a polymer (e.g., less than about 40%, about 30%, about 25%, about 20%, about 15%, about 10%, about 5%, about 2.5%, about 2%, about 1%, about 0.5%, about 0.1%, about 0.05%, or less). In some embodiments, the ratio (w/w) of a microbiome regulator to a polymer is greater than about 1:1 (e.g., about 1.1:1, about 1.2:1, about 1.3:1, about 1.4:1, about 1.5:1, about 1.75:1, about 2:1, about 2.25:1, about 2.5:1, about 2.75:1, about 3:1, about 4:1, about 5:1, about 6:1, about 7:1, about 8:1, about 9:1, about 10:1, about 12.5:1, about 15:1, about 20:1, about 30:1, about 40:1, about 50:1, about 100:1, about 500:1, about 1000:1, or more). In some embodiments, the composition is substantially free of a polymer. In some embodiments, the polymer is synthetic or naturally occurring. In some embodiments, the polymer is polyethylene glycol (PEG), polypropylene glycol (PPG), polyvinyl pyrrolidine (PVG), polyvinyl alcohol (PVA), polyacrylic acid (PAA), polyacrylamide, N-(2-hydroxypropyl) methylacrylamide (HMPA), divinyl ether-maleic anhydride (DIVEMA), polyoxazolines, polyphosphates, xanthan gum, pectin, chitin, chitosan, dextran, carrageenan, guar gum, cellulose (e.g., hydroxypropylmethyl cellulose (HPMC), hydroxypropyl cellulose (HPC), hydroxyethylcellulose (HEC), sodium carboxymethyl cellulose (NaCMC)), hyaluronic acid, hyaluronan, albumin, heparin, chondroitin, starch, or derivatives thereof.

In some embodiments, the agent other than the microbiome regulator is a binder, film foaming agent, solubilizing agent, tastant, lyophilizing agent, stabilizer, hydrophilizer, emulsifier, adhesive, or toxicity reducer.

In some embodiments, the microbiome regulator is a sugar or sugar alcohol comprising one or more of: i) a monosaccharide or disaccharidethat is metabolized by the host; wherein if the sugar or sugar alcohol is a disaccharide: a) at least one glycosidic bonds comprise a 1->2 glycosidic bond, a 1->3 glycosidic bond, a 1->4 glycosidic bond, or a 1->6 glycosidic bond; and b) at least one of the glycosidic bonds are present in the alpha or beta configuration; ii) a molecular weight less than about 1000 g/mol (e.g., less than about 950 g/mol, about 900 g/mol, about 850 g/mol, about 800 g/mol, about 750 g/mol, about 700 g/mol, about 650 g/mol, about 600 g/mol, about 500 g/mol, about 450 g/mol, about 400 g/mol, about 350 g/mol, about 300 g/mol, about 250 g/mol, about 200 g/mol, or less); iii) less than about 12 carbon atoms, about 10 carbon atoms, about 9 carbon atoms, about 8 carbon atoms, about 7 carbon atoms, about 6 carbon atoms, or about 5 carbon atoms); iv) less than about about 12 heteroatoms, less than about 10 heteroatoms, less than about 9 heteroatoms, less than about 8 heteroatoms, less than about 7 heteroatoms, less than about 6 heteroatoms, or less than about 5 heteroatoms); or v) does not comprise an alkenyl, alkynyl, aryl, heteroaryl, cycloalkyl, halogen, acyl, thiol, cyano, nitro, or sulfonyl moiety.

In some embodiments, the microbiome regulator comprises at least two of i), ii), iii), or iv). In some embodiments, the microbiome regulator comprises at least three of i), ii), iii), or iv). In some embodiments, the microbiome regulator consists of i), ii), iii), and iv).

In some embodiments, the composition comprises a sugar or sugar alcohol and i) is substantially free of a sugar or sugar alcohol that is not metabolized by the host; or ii) is substantially free of an agent other than a microbiome regulator, e.g., a therapeutic agent (e.g., peptide, nucleic acid, oligosaccharide, polysaccharide, protein, non-peptide small molecule, or a prodrug or metabolite thereof), or a polymer (e.g. is polyethylene glycol (PEG), polypropylene glycol (PPG), polyvinyl pyrrolidine (PVG), polyvinyl alcohol (PVA), polyacrylic acid (PAA), polyacrylamide, N-(2-hydroxypropyl) methylacrylamide (HMPA), divinyl ether-maleic anhydride (DIVEMA), polyoxazolines, polyphosphates, xanthan gum, pectin, chitin, chitosan, dextran, carrageenan, guar gum, cellulose (e.g., hydroxypropylmethyl cellulose (HPMC), hydroxypropyl cellulose (HPC), hydroxyethylcellulose (HEC), sodium carboxymethyl cellulose (NaCMC)), hyaluronic acid, hyaluronan, albumin, heparin, chondroitin, starch, or derivatives thereof). In some embodiments, the composition consists of i) and ii).

In some embodimens, the composition is substantially free of a therapeutic agent, wherein the therapeutic agent comprises one or more of: i) a peptide, nucleic acid, oligosaccharide, polysaccharide, protein, non-peptide small molecule, or a prodrug or metabolite thereof; ii) a molecular weight greater than about 500 g/mol; iii) more than about 6 carbon atoms; iv) a specificity for a cell surface receptor, an ion channel, a transporter, an enzyme, an antibody, or other biological target; or v) an agent used in the treatment of a disease, disorder, or condition. In some embodiments, the composition comprises at least two of i), ii), iii), iv), or v). In some embodiments, the composition comprises at least three of i), ii), iii), iv) or v). In some embodiments, the composition consists of i), ii), iii), iv) and v).

In some embodiments, the composition further comprises a polymer (e.g., a polysaccharide) to target the composition to a specific site in the gastrointestinal tract, e.g., the small intestine (e.g., the duodenum, jejunum, or ileum) or large intestine (e.g., cecum, colon, or rectum). In some embodiments, the composition comprises more than about 1% (w/w) of a polymer (e.g., more than about 2%, about 5%, about 10%, about 20%, about 30%, about 40%, about 50%, or more). In some embodiments, the ratio (w/w) of a microbiome regulator to a polymer is about than about 1:1 (e.g., about 1.1:1, about 1.2:1, about 1.3:1, about 1.4:1, about 1.5:1, about 1.75:1, about 2:1, about 2.25:1, about 2.5:1, about 2.75:1, about 3:1, about 4:1, about 5:1, about 6:1, about 7:1, about 8:1, about 9:1, about 10:1, about 12.5:1, about 15:1, about 20:1, about 30:1, about 40:1, about 50:1, about 100:1, about 500:1, about 1000:1, or more). In some embodiments, the polymer is a polysaccharide. In some embodiments, the polymer is amylose, arabinogalactan, carrageenan, chitosan, chondroitin sulfate, dextran, furcelleran, galactomannan, glucomannan, gellan gum, hyaluronic acid, Karaya gum (sterculia gum), locust bean gum, scleroglucan, pullalan, xylan, or a derivative thereof.

In some embodiments, the composition further comprises a bacterial taxa. In some embodiments, the composition comprises at least two (e.g., at least three, at least four) microbiome regulators and a bacterial taxa (e.g., a commensal bacterial taxa). In some embodiments, the bacterial taxa (e.g., the commensal bacterial taxa) comprises the genus Methanosarcina, Pyrococcus, Methanothermobacter, Actinomyces, Nacardiopsis, Propionibacterium, Bifidobacterium, Mycobacterium, Gordonia, Nocardia, Rhodococcus, Corynebacterium, Arthrobacter, Micrococcus, Kocuria, Microbacterium, Psueodonocardia, Saccharomonospora, Amycolatopsis, Streptomyces, Micromonospora, Collinsella, Alicyclobacillus, Laceyella, Sporosarcina, Halobacillus, Staphylococcus, Sporolactobacillus, Listeria, Paenibacillus, Leuconostoc, Weissella, Streptococcus, Enterococcus, Moorella, Thermoanaerobacter, Thermoanaerobacterium, Caldicellulosiruptor, Desulfitobacterium, Desulfotomaculum, Blautia, Lachnoclostridium, Butyrivibrio, Eubacterium, Ruminiclostridium, Clostridium, Veillonella, Selenomonoas, Deinococcus, Thermus, Meiothermus, Fusobacterium, Spirochaeta, Mycoplasma, Campylobacter, Helicobacter, Desulfovibrio, Cystobacter, Sorangium, Myxococcus, Corrallococcus, Anaeromyxobacter, Geobacter, Achromobacter, Bordetella, Acidovorax, Delftia, Variovorax, Comamonas, Cupriavidus, Burkholderia, Neisseria, Acidithiobacillus, Marinobacter, Shewanella, Halomonas, Acinebacter, Psuedomonas, Vibrio, Xanthomonas, Thiomicrospira, Actinobacillus, Escherichia, Salmonella, Photorhabdus, Sphingobium, Sphingomonas, Paracoccus, Acetobacter, Komagataeibacter, Azospirillum, Rhizobium, Methylobacterium, Ancylobacter, Xanthobacter, Ochrobactrum, Leptospirillum, Spirosoma, Flavobacterium, Capnocytophaga, Porphyromonas, Prevotella, Bacteroides, Chlorobium, Sporomusa, Dehalococcoides, Butirivibrio, Methanobrevibacter, or Methanosphaera.

In another aspect, the present invention features a method to engraft or improve colonization of a bacterial taxa in the gastrointestinal microbiota of a subject, the method comprising administering a dosage form formulated to substantially release a composition in the gastrointestinal tract (e.g., the stomach, small intestine or large intestine), wherein the dosage form comprises a composition comprising: i) a microbiome regulator and ii) a bacterial taxa for which either engraftment or an improvement of colonization is sought. In some embodiments, the dosage form is targeted to the small intestine, e.g., the duodenum, jejunum, or ileum. In some embodiments, the dosage form is targeted to the large intestine, e.g., cecum, colon, or rectum. In some embodiments, the microbiome regulator comprises a sugar, a sugar alcohol, an amino acid, a peptide, a micronutrient, a fatty acid, or a polyphenol. In some embodiments, the microbiome regulator comprises a sugar or sugar alcohol.

In some embodiments, the sugar or sugar alcohol comprises D-fructose, D-galactose, L-arabinose, D-mannose, D-xylose, D-arabinose, ribose, L-fucose, L-rhamnose, lactose, D-maltose, sucrose, or glucose.. In some embodiments, the the sugar or sugar alcohol does not comprise glucose.

In some embodiments, the bacterial taxa is a probiotic. In some embodiments, the subject does not host the bacterial taxa (e.g., the subject is substantially devoid of the bacterial taxa). In some embodiments, the microbiome regulator substantially promotes the growth of the bacterial taxa. In some embodiments, the probiotic provides a health or treatment effect to the subject.

The application further features a method of modulating a bacterial taxa in the gastrointestinal microbiota of a subject, the method comprising administering to the subject an effective amount of a composition comprising a microbiome regulator formulated in a dosage form or administered in a dosage regimen for substantial release of the composition in the gastrointestinal tract of the subject to thereby modulate the bacterial taxa. In some embodiments, the microbiome regulator is administered in an effective amount to modulate a bacterial taxa. In some embodiments, the microbiome regulator is administered in an effective amount to modulate a first and a second bacterial taxa.

In some disclosures, modulating a bacterial taxa comprises an increase or decrease in the abundance of the taxa. In some disclosures, modulating a bacterial taxa comprises an increase or decrease in the abundance of the taxa relative to the abundance of said bacterial taxa in the absence of the composition. In some disclosures, modulating a bacterial taxa comprises an increase or decrease in the abundance of the taxa relative to the abundance of a second bacterial taxa. In some disclosures, the abundance of the bacterial taxa in the microbiota of a subject is increased by at least about 5%, about 10%, about 25% about 50%, about 75%, about 100%, about 250%, about 500%, about 750%, about 1000%, or more. In some disclosures, the abundance of the bacterial taxa in the microbiota of a subject is decreased by at least about 5%, about 10%, about 25% about 50%, about 75%, about 85%, about 90%, about 95%, about 96%, about 97%, about 98%, about 99%, about 99.9%, or less.

In some embodiments, the bacterial taxa is a commensal bacterial taxa. In some disclosures, the bacterial taxa is a pathogenic bacterial taxa. In some embodiments, at least one of the first or second bacterial taxa comprises the genus Akkermansia, Alistipes, Anaerofilum, Bacteroides, Bilophila, Blautia, Bifidobacterium, Butyrivibrio, Campylobacter, Candidatus, Citrobacter, Clostridium, Collinsella, Coprococcus , Desulfovibrio, Dialister, Dorea, , Enterobacter, Enterococcus, Escherichia, Eubacterium, Faecalibacterium, Fusobacterium, Haemophilus, Klebsiella, Lachnospira, Lactobacillus, Odoribacter, Oscillospira, Parabacteroides, Peptococcus, Peptostreptococcus, Phascolarctobacterium, Porphyromonas, Portiera, Prevotella, Providencia, Pseudomonas, Roseburia, Ruminococcus, Salmonella, Shigella, Staphylococcus, Streptococcus, Subdoligranulum, Vibrio, or Yersinia.

In some embodiments, the bacterial taxa comprises the genus Prevotella, Akkermansia, Bacteroides, Clostridium (Erysipelotrichaceae), Clostridium (Clostridiaceae), Bifidobacterium, Aggregatibacter, Clostridium (Peptostreptococcaveae), Parabacteroides, Lactobacillus, or Enterococcus. In some embodiments, the bacterial taxa comprises the genus Akkermansia, Bacteroides, Bifidobacterium, Lactobacillus, or Parabacteroides. In some embodiments, the bacterial taxa comprises the genus Akkermansia or Blautia.

In some embodiments, the bacterial taxa comprises a taxa predominant in the small intestine or large intestine. In some embodiments, the bacterial taxa predominant in the small intestine comprises one or more of the genus Achromobacter, Agrobacterium, Blautia, Burkholderia, Coprococcus, Cryocola, Enterococcus, Eubacterium, Holdemania, Lactococcus, Mycobacterium, Pseudoramibacter, Ralstonia, Sphingomonas, Streptococcus, or Turicibacter. In some embodiments, the bacterial taxa predominant in the large intestine comprises the genus Anaerotruncus, Akkermansia, Bacteroides, Bilophila, Butyricimonas, Odoribacter, Parabacteroides, Phascolarctobacterium, Prevotella, or Ruminococcus. In some embodiments, the bacterial taxa comprises the genus Methanosarcina, Pyrococcus, Methanothermobacter, Actinomyces, Nacardiopsis, Propionibacterium, Bifidobacterium, Mycobacterium, Gordonia, Nocardia, Rhodococcus, Corynebacterium, Arthrobacter, Micrococcus, Kocuria, Microbacterium, Psueodonocardia, Saccharomonospora, Amycolatopsis, Streptomyces, Micromonospora, Collinsella, Alicyclobacillus, Laceyella, Sporosarcina, Halobacillus, Staphylococcus, Sporolactobacillus, Listeria, Paenibacillus, Leuconostoc, Weissella, Streptococcus, Enterococcus, Moorella, Thermoanaerobacter, Thermoanaerobacterium, Caldicellulosiruptor, Desulfitobacterium, Desulfotomaculum, Blautia, Lachnoclostridium, Butyrivibrio, Eubacterium, Ruminiclostridium, Clostridium, Veillonella, Selenomonoas, Deinococcus, Thermus, Meiothermus, Fusobacterium, Spirochaeta, Mycoplasma, Campylobacter, Helicobacter, Desulfovibrio, Cystobacter, Sorangium, Myxococcus, Corrallococcus, Anaeromyxobacter, Geobacter, Achromobacter, Bordetella, Acidovorax, Delftia, Variovorax, Comamonas, Cupriavidus, Burkholderia, Neisseria, Acidithiobacillus, Marinobacter, Shewanella, Halomonas, Acinebacter, Psuedomonas, Vibrio, Xanthomonas, Thiomicrospira, Actinobacillus, Escherichia, Salmonella, Photorhabdus, Sphingobium, Sphingomonas, Paracoccus, Acetobacter, Komagataeibacter, Azospirillum, Rhizobium, Methylobacterium, Ancylobacter, Xanthobacter, Ochrobactrum, Leptospirillum, Spirosoma, Flavobacterium, Capnocytophaga, Porphyromonas, Prevotella, Bacteroides, Chlorobium, Sporomusa, Dehalococcoides, Butirivibrio, Methanobrevibacter, or Methanosphaera.

In some embodiments, the administration of the composition to the subject modulates microbial diversity in the subject. In some embodiments, the microbial diversity comprises bacterial diversity. In some embodiments, the Shannon diversity of the bacterial taxa is increased or decreased by at least about 5% (e.g., at least about 10%, about 15%, about 20%, about 25%, about 30%, about 35%, about 40%, about 45%, about 50%, or more). In some embodiments, the Shannon diversity of the bacterial taxa is increased or decreased by at least about 0.1 log-fold (e.g., about 0.2 log-fold, about 0.3 log-fold, about 0.4 log-fold, about 0.5 log-fold, about 0.6 log-fold, about 0.7-log-fold, about 0.8 log-fold, about 0.9 log-fold, about 1 log-fold, about 1.5 log-fold about 2 log-fold, or more).

In some embodiments, the administration of the composition to the subject modulates a function of the microbiota. In some embodiments, modulating a bacterial taxa comprises modulating (e.g., stimulation or downregulation) a metabolic pathway. In some embodiments, the modulation of a metabolic pathway comprises a stimulation or downregulation of the metabolic pathway by at least about 5% (e.g., at least about 10%, about 15%, about 20%, about 25%, about 30%, about 35%, about 40%, about 45%, about 50%, or more). In some embodiments, the modulation of a metabolic pathway comprises a stimulation or downregulation of the metabolic pathway by at least about 0.1 log-fold (e.g., about 0.2 log-fold, about 0.3 log-fold, about 0.4 log-fold, about 0.5 log-fold, about 0.6 log-fold, about 0.7-log-fold, about 0.8 log-fold, about 0.9 log-fold, about 1 log-fold, about 1.5 log-fold about 2 log-fold, or more).

In some embodiments, the modulating a metabolic pathway comprises an increase or decrease in the level of an anti-microbial agent, a secondary bile acid, a short-chain fatty acid, a siderophore, or a metabolite listed in Table 2 by the microbiota. In some embodiments, the antimicrobial agent comprises a bacteriocin or hydrogen peroxide. In some embodiments, the metabolite comprises 2-hydroxyisobutyrate, 3-hydroxyisovalerate, 3-methylcrotonylglycine, 3-methylcrotonylglycine, allantoin, betaine, formate, mannitol, p-cresol glucuronide, phenylacetylglycine, sarcosine, taurine, acetic acid, acetylaldehyde, ascorbic acid, butanedione, butyric acid, deoxycholic acid, ethylphenyl sulfate, formic acid, indole, isobutyric acid, isovaleric acid, propionic acid, serotonin, succinic acid, succinate, TMAO, tryptophan, valeric acid, ursodeoxycholic acid, lactate, lactic acid, or hydrogen peroxide.

In some embodiments, the modulation of a metabolic pathway comprises an increase or decrease in the level of an inflammatory or immunomodulatory cytokine in the human subject. In some embodiments, the inflammatory and immunomodulatory cytokine comprises interleukin-1α (IL-1α), IL-1β, IL-2, IL-4, IL-6, IL-8, IL-10, IL-12, IL-13, IL-17A, IL-17F, IL-22, IL-23, tumor necrosis factor (TNF), chemokine (C-C motif) ligand 5 (CCL5, also known as RANTES), transforming growth factor beta (TGF-β), or interferon gamma (IFN-γ).

In some embodiments, the modulation of a metabolic pathway comprises an increase or decrease in the level of a short-chain fatty acid in the subject. In some embodiments, the increase in the short-chain fatty acid induces the generation of regulatory T (Treg) cells by the subject. In some embodiments, the increase in the short-chain fatty acid reduces the permeability of the intestinal or plasma endotoxin level in the subject. In some embodiments, the increase of a short-chain fatty acid reduces the inflammatory response of the subject. In some embodiments, the short-chain fatty acid is produced by at least one bacterial species of the *Ruminocaccaceae* and/or *Lachnospiraceae* family. In some embodiments, the short-chain fatty acid comprises acetic acid, propionic acid, butryic acid, isobutyric acid, valeric acid, isovaleric acid, hexanoic acid, or octanoic acid. In some embodiments, the level of a short chain fatty acid is increased or decreased by at least about 5% (e.g., at least about 10%, about 15%, about 20%, about 25%, about 30%, about 35%, about 40%, about 45%, about 50%, or more). In some embodiments, the level of a short chain fatty acid is increased or decreased by at least about 0.1 log-fold (e.g., about 0.2 log-fold, about 0.3 log-fold, about 0.4 log-fold, about 0.5 log-fold, about 0.6 log-fold, about 0.7-log-fold, about 0.8 log-fold, about 0.9 log-fold, about 1 log-fold, about 1.5 log-fold about 2 log-fold, or more).

In some embodiments, the microbiome regulator is a sugar, or a sugar alcohol. In some embodiments, the microbiome regulator comprises a sugar or sugar alcohol. In some embodiments, the microbiome regulator comprises a sugar or sugar alcohol that is metabolizable by the host. In some embodiments, the microbiome regulator comprises a sugar or sugar alcohol that is non-metabolizable by the host. In some embodiments, the sugar or sugar alcohol comprises D-fructose, D-galactose, L-arabinose, D-mannose, D-xylose, D-arabinose, ribose, **L-**fucose, L-rhamnose, lactose, D-maltose, sucrose, and glucose.

In some embodiments, the composition comprises more than about 50% (w/w) of a sugar or sugar alcohol (e.g., more than about 55%, about 60%, about 65%, about 70%, about 75%, about 80%, about 85%, about 90%, about 95%, about 96%, about 97%, about 98%, about 99%, or more). In some embodiments, the microbiome regulator comprises a molecule with less than about 12 carbon atoms (e.g., less than about 10 carbon atoms, about 9 carbon atoms, about 8 carbon atoms, about 7 carbon atoms, about 6 carbon atoms, or about 5 carbon atoms) and less than about12 heteroatoms (e.g., less than about 10 heteratoms, less than about 9 heteroatoms, less than about 8 heteroatoms, less than about 7 heteroatoms, less than about 6 heteroatoms, or less than about 5 heteroatoms), wherein the heteroatom is selected from oxygen, nitrogen, sulfur, or phosphorus. In some embodiments, the microbiome regulator does not comprise an alkenyl, alkynyl, aryl, heteroaryl, cycloalkyl, halogen, acyl, thiol, cyano, nitro, or sulfonyl moiety.

In some embodiments, the sugar or sugar alcohol has a low degree of sweetness relative to sucrose. In some embodiments, the sugar or sugar alcohol has a degree of sweetness less than about 1 relative to sucrose (e.g., less than about 0.95, about 0.9, about 0.85, about 0.8, about 0.75, about 0.7, about 0.65, about 0.6, about 0.55, about 0.5, or less). In some embodiments, the sugar or sugar alcohol has a degree of sweetness that can be no more than about 1 relative to sucrose (e.g., no more than about 2, about 3, about 4, about 5, about 10, about 20, about 25, about 50, about 75, about 100, about 250, about 500, about 1000, or more).

In some embodiments, the sugar or sugar alcohol has a low absorption coefficient relative to glucose. In some embodiments, the sugar or sugar alcohol has a an absorption coefficient less than 0.15 (e.g., less than about 0.14, about 0.13, about 0.12, about 0.11, about 0.10, about 0.09, about 0.08, about 0.07, about 0.06, about 0.05, about 0.04, about 0.03, about 0.02, about 0.01, or less).

In some embodiments, a substantial portion of the sugar or sugar alcohol that is metabolized by the host is metabolized in the lower GI tract (e.g., the small intestine or large intestine). In some embodiments, a substantial portion of the sugar or sugar alcohol that is metabolized by the host is metabolized in the small intestine, e.g., the duodenum, jejunum, or ileum. In some embodiments, a substantial portion of the sugar or sugar alcohol that is metabolized by the host is metabolized in the large intestine, e.g., cecum, colon, or rectum.

In some embodiments, the microbiome regulator comprises an amino acid. In some embodiments, the amino acid is an L-amino acid or a D-amino acid. In some embodiments, the microbiome regulator comprises a micronutrient. In some embodiments, the micronutrient comprises a vitamin, an element, or a mineral. In some embodiments, the microbiome regulator comprises a fatty acid. In some embodiments, the fatty acid comprises a short-chain fatty acid (SCFA), a medium-chain fatty acid (MCFA), a long-chain fatty acid (LCFA), or a very long chain fatty acid (VLCFA). In some embodiments, the microbiome regulator comprises a polyphenol. In some embodiments, the polyphenol comprises a catechin, ellagitannin, isoflavone, flavonol, flavanone, anthocyanin, or lignin.

In another aspect, the present invention features a method of treating a subject having a dysbiosis of the gastrointestinal microbiota, the method comprising administering to the subject a composition comprising a microbiome regulator formulated in a dosage form or administered in a dosage regimen for substantial release of the composition in the gastrointestinal tract to thereby treat the subject. In some embodiments, the dysbiosis is idiopathic (e.g., the subject has no observable cause of a dysbiosis, or the cause of the dysbiosis is unclear or unknown). In some embodiments, the dysbiosis is associated with a disease, disorder, or condition in the subject. In some embodiments, the disease, disorder, or condition comprises an infectious disease, an inflammatory disease, a metabolic disease, an autoimmune disease, a neurological disease, or a cancer.

In some embodiments, infectious disease comprises *Clostridium difficile* infection (CDI); Vancomycin-resistant enterococci (VRE) infection, infectious colitis, C. *difficile* colitis, a mycosis (e.g., *Candida albicans* infection, *Campylobacter jejuni* infection, or *Helicobacter pylori* infection), *Clostridium difficile* associated diarrhea (CDAD), antibiotic-associated diarrhea (AAD), antibiotic-induced diarrhea, travelers' diarrhea (TD), pediatric diarrhea, or (acute) infectious diarrhea.

In some embodiments, the inflammatory disease comprises inflammatory bowel disease (IBD), ulcerative colitis (UC), Crohn's disease (CD), idiopathic inflammation of the small bowel, indeterminatal colitis, pouchitis, irritable bowel syndrome (IBS), necrotizing enterocolitis (NEC), intestinal inflammation, constipation, microscopic colitis, diarrhea, graft versus host disease (GVHD), allergies (e.g., food allergies), pseudomembranous colitis, indigestion, non-ulcer dyspepsia, diverticulosis, diverticulitis, ischemic colitis, radiation colitis, radiation enteritis, collagenous colitis, gastroenteritis, or polyps.

In some embodiments, the metabolic disease comprises obesity, (insulin resistance) pre-diabetes, type II diabetes, high fasting blood sugar (hyperglycemia), metabolic syndrome, or a cardiovascular risk factor (e.g., high blood cholesterol, high LDL, high blood pressure (hypertension), high triglyceride levels, low HDL).

In some embodiments, the autoimmune disease comprises autoimmune arthritis, type I diabetes, multiple sclerosis, psoriasis, an allergy, asthma or atopic dermatitis.

In some embodiments, the neurological disease comprises autism, hyperammonemia, or hepatic encephalopathy.

In some embodiments, the cancer comprises a cancer of the brain, skin, blood, bone, eye, breast, lung, prostate, liver, or gastrointestinal tract.

In some embodiments, the dysbiosis is associated with a gastrointestinal disease.

In some embodiments, the microbiome regulator is a sugar, a sugar alcohol, an amino acid, a peptide, a micronutrient, a fatty acid, or a polyphenol. In some embodiments, the microbiome regulator is a sugar, a sugar alcohol, an amino acid, a peptide, a micronutrient, a fatty acid, or a polyphenol. In some embodiments, the microbiome regulator comprises a sugar or sugar alcohol. In some embodiments, the microbiome regulator comprises a sugar or sugar alcohol that is metabolizable by the host. In some embodiments, the microbiome regulator comprises a sugar or sugar alcohol that is non-metabolizable by the host. In some embodiments, the sugar or sugar alcohol comprises D-fructose, D-galactose, L-arabinose, D-mannose, D-xylose, D-arabinose, ribose, L-fucose, L-rhamnose, lactose, D-maltose, sucrose, or glucose.

In some embodiments, the composition comprises more than about 50% (w/w) of a sugar or sugar alcohol (e.g., more than about 55%, about 60%, about 65%, about 70%, about 75%, about 80%, about 85%, about 90%, about 95%, about 96%, about 97%, about 98%, about 99%, or more). In some embodiments, the microbiome regulator comprises a molecule with less than about 12 carbon atoms (e.g., less than about 10 carbon atoms, about 9 carbon atoms, about 8 carbon atoms, about 7 carbon atoms, about 6 carbon atoms, or about 5 carbon atoms) and less than about12 heteroatoms (e.g., less than about 10 heteratoms, less than about 9 heteroatoms, less than about 8 heteroatoms, less than about 7 heteroatoms, less than about 6 heteroatoms, or less than about 5 heteroatoms), wherein the heteroatom is selected from oxygen, nitrogen, sulfur, or phosphorus. In some embodiments, the microbiome regulator does not comprise an alkenyl, alkynyl, aryl, heteroaryl, cycloalkyl, halogen, acyl, thiol, cyano, nitro, or sulfonyl moiety.

In some embodiments, the sugar or sugar alcohol has a low degree of sweetness relative to sucrose. In some embodiments, the sugar or sugar alcohol has a degree of sweetness less than about 1 relative to sucrose (e.g., less than about 0.95, about 0.9, about 0.85, about 0.8, about 0.75, about 0.7, about 0.65, about 0.6, about 0.55, about 0.5, or less). In some embodiments, the sugar or sugar alcohol has a degree of sweetness that can be no more than about 1 relative to sucrose (e.g., no more than about 2, about 3, about 4, about 5, about 10, about 20, about 25, about 50, about 75, about 100, about 250, about 500, about 1000, or more).

In some embodiments, the sugar or sugar alcohol has a low absorption coefficient relative to glucose. In some embodiments, the sugar or sugar alcohol has a an absorption coefficient less than 0.15 (e.g., less than about 0.14, about 0.13, about 0.12, about 0.11, about 0.10, about 0.09, about 0.08, about 0.07, about 0.06, about 0.05, about 0.04, about 0.03, about 0.02, about 0.01, or less).

In some embodiments, a substantial portion of the sugar or sugar alcohol that is metabolized by the host is metabolized in the lower GI tract (e.g., the small intestine or large intestine). In some embodiments, a substantial portion of the sugar or sugar alcohol that is metabolized by the host is metabolized in the small intestine, e.g., the duodenum, jejunum, or ileum. In some embodiments, a substantial portion of the sugar or sugar alcohol that is metabolized by the host is metabolized in the large intestine, e.g., cecum, colon, or rectum.

In some embodiments, the microbiome regulator comprises an amino acid. In some embodiments, the amino acid is an L-amino acid or a D-amino acid. In some embodiments, the microbiome regulator may furhter comprise a micronutrient. In some embodiments, the micronutrient may further comprise a vitamin, an element, or a mineral. In some embodiments, the microbiome regulator may further comprise a fatty acid. In some embodiments, the fatty acid comprises a short-chain fatty acid (SCFA), a medium-chain fatty acid (MCFA), a long-chain fatty acid (LCFA), or a very long chain fatty acid (VLCFA). In some embodiments, the microbiome regulator may further comprise comprises a polyphenol. In some embodiments, the polyphenol comprises a catechin, ellagitannin, isoflavone, flavonol, flavanone, anthocyanin, or lignin.

In some embodiments, the composition further comprises a polymer (e.g., a polysaccharide) to target the composition to a specific site in the gastrointestinal tract, e.g., the small intestine (e.g., the duodenum, jejunum, or ileum) or large intestine (e.g., cecum, colon, or rectum). In some embodiments, the composition comprises more than about 1% (w/w) of a polymer (e.g., more than about 2%, about 5%, about 10%, about 20%, about 30%, about 40%, about 50%, or more). In some embodiments, the ratio (w/w) of a microbiome regulator to a polymer is about than about 1:1 (e.g., about 1.1:1, about 1.2:1, about 1.3:1, about 1.4:1, about 1.5:1, about 1.75:1, about 2:1, about 2.25:1, about 2.5:1, about 2.75:1, about 3:1, about 4:1, about 5:1, about 6:1, about 7:1, about 8:1, about 9:1, about 10:1, about 12.5:1, about 15:1, about 20:1, about 30:1, about 40:1, about 50:1, about 100:1, about 500:1, about 1000:1, or more). In some embodiments, the polymer is a polysaccharide. In some embodiments, the polymer is amylose, arabinogalactan, carrageenan, chitosan, chondroitin sulfate, dextran, furcelleran, galactomannan, glucomannan, gellan gum, hyaluronic acid, Karaya gum (sterculia gum), locust bean gum, scleroglucan, pullalan, xylan, or a derivative thereof.

**In** some embodiments, the composition further comprises a bacterial taxa. **In** some embodiments, the composition comprises at least two (e.g., at least three, at least four) microbiome regulators and a bacterial taxa (e.g., a commensal bacterial taxa).

**In** another aspect, the present invention features a method for reducing a drug- or treatment-induced symptom in a subject, the method comprising administering to the subject a composition comprising a microbiome regulator formulated in a dosage form or administered in a dosage regimen for substantial release of the composition in the gastrointestinal tract to thereby reduce the symptom in the subject. **In** some embodiments, the method comprises one or more of: i) a microbiome regulator comprising a sugar, a sugar alcohol, an amino acid, a peptide, a micronutrient, a fatty acid, or a polyphenol; ii) a microbiome regulator that does not comprise an alkenyl, alkynyl, aryl, heteroaryl, cycloalkyl, halogen, acyl, thiol, cyano, nitro, or sulfonyl moiety; iii) a microbiome regulator comprising a molecule with less than about 12 carbon atoms (e.g., less than about 10 carbon atoms, about 9 carbon atoms, about 8 carbon atoms, about 7 carbon atoms, about 6 carbon atoms, or about 5 carbon atoms) and less than about12 heteroatoms (e.g., less than about 10 heteratoms, less than about 9 heteroatoms, less than about 8 heteroatoms, less than about 7 heteroatoms, less than about 6 heteroatoms, or less than about 5 heteroatoms), wherein the heteroatom is selected from oxygen, nitrogen, sulfur, or phosphorus; and iv) a composition comprising more than about 50% (w/w) of a microbiome regulator (e.g., more than about 55%, about 60%, about 65%, about 70%, about 75%, about 80%, about 85%, about 90%, about 95%, about 96%, about 97%, about 98%, about 99%, or more).

In some embodiments, the method comprises at least two of i), ii), iii), or iv). In some embodiments, the method comprises at least three of i), ii), iii), or iv). In some embodiments, the method consists of i), ii), iii), and iv).

In some embodiments, the drug- or treatment-induced symptom is bloating, diarrhea, vomiting, nausea, and constipation. In some embodiments, the drug- or treatment-induced is diarrhea. In some embodiments, the drug- or treatment-induced symptom is constipation. In some embodiments, the composition is administered prior to, concomitant with, or after administration of the drug.

In some embodiments, the microbiome regulator comprises a sugar or sugar alcohol. In some embodiments, the microbiome regulator comprises a sugar or sugar alcohol that is metabolizable by the host. In some embodiments, the microbiome regulator comprises a sugar or sugar alcohol that is non-metabolizable by the host. In some embodiments, the sugar or sugar alcohol comprises D-fructose, D-galactose, L-arabinose, D-mannose, D-xylose, D-arabinose, ribose, L-fucose, L-rhamnose, lactose, D-maltose, sucrose, or glucose. In some embodiments, the the sugar or sugar alcohol does not comprise glucose.

In some embodiments, the composition comprises more than about 50% (w/w) of a sugar or sugar alcohol (e.g., more than about 55%, about 60%, about 65%, about 70%, about 75%, about 80%, about 85%, about 90%, about 95%, about 96%, about 97%, about 98%, about 99%, or more). In some embodiments, the microbiome regulator comprises a molecule with less than about 12 carbon atoms (e.g., less than about 10 carbon atoms, about 9 carbon atoms, about 8 carbon atoms, about 7 carbon atoms, about 6 carbon atoms, or about 5 carbon atoms) and less than about12 heteroatoms (e.g., less than about 10 heteratoms, less than about 9 heteroatoms, less than about 8 heteroatoms, less than about 7 heteroatoms, less than about 6 heteroatoms, or less than about 5 heteroatoms), wherein the heteroatom is selected from oxygen, nitrogen, sulfur, or phosphorus. In some embodiments, the microbiome regulator does not comprise an alkenyl, alkynyl, aryl, heteroaryl, cycloalkyl, halogen, acyl, thiol, cyano, nitro, or sulfonyl moiety.

In some embodiments, the sugar or sugar alcohol has a low degree of sweetness relative to sucrose. In some embodiments, the sugar or sugar alcohol has a degree of sweetness less than about 1 relative to sucrose (e.g., less than about 0.95, about 0.9, about 0.85, about 0.8, about 0.75, about 0.7, about 0.65, about 0.6, about 0.55, about 0.5, or less). In some embodiments, the sugar or sugar alcohol has a degree of sweetness that can be no more than about 1 relative to sucrose (e.g., no more than about 2, about 3, about 4, about 5, about 10, about 20, about 25, about 50, about 75, about 100, about 250, about 500, about 1000, or more).

In some embodiments, the sugar or sugar alcohol has a low absorption coefficient relative to glucose. In some embodiments, the sugar or sugar alcohol has a an absorption coefficient less than 0.15 (e.g., less than about 0.14, about 0.13, about 0.12, about 0.11, about 0.10, about 0.09, about 0.08, about 0.07, about 0.06, about 0.05, about 0.04, about 0.03, about 0.02, about 0.01, or less).

In some embodiments, a substantial portion of the sugar or sugar alcohol that is metabolized by the host is metabolized in the lower GI tract (e.g., the small intestine or large intestine). In some embodiments, a substantial portion of the sugar or sugar alcohol that is metabolized by the host is metabolized in the small intestine, e.g., the duodenum, jejunum, or ileum. In some embodiments, a substantial portion of the sugar or sugar alcohol that is metabolized by the host is metabolized in the large intestine, e.g., cecum, colon, or rectum.

In some embodiments, the microbiome regulator comprises an amino acid. In some embodiments, the amino acid is an L-amino acid or a D-amino acid. In some embodiments, the microbiome regulator comprises a micronutrient. In some embodiments, the micronutrient comprises a vitamin, an element, or a mineral. In some embodiments, the microbiome regulator comprises a fatty acid. In some embodiments, the fatty acid comprises a short-chain fatty acid (SCFA), a medium-chain fatty acid (MCFA), a long-chain fatty acid (LCFA), or a very long chain fatty acid (VLCFA). In some embodiments, the microbiome regulator comprises a polyphenol. In some embodiments, the polyphenol comprises a catechin, ellagitannin, isoflavone, flavonol, flavanone, anthocyanin, or lignin.

In some embodiments, the composition further comprises a polymer (e.g., a polysaccharide) to target the composition to a specific site in the gastrointestinal tract, e.g., the small intestine (e.g., the duodenum, jejunum, or ileum) or large intestine (e.g., cecum, colon, or rectum). In some embodiments, the composition comprises more than about 1% (w/w) of a polymer (e.g., more than about 2%, about 5%, about 10%, about 20%, about 30%, about 40%, about 50%, or more). In some embodiments, the ratio (w/w) of a microbiome regulator to a polymer is about than about 1:1 (e.g., about 1.1:1, about 1.2:1, about 1.3:1, about 1.4:1, about 1.5:1, about 1.75:1, about 2:1, about 2.25:1, about 2.5:1, about 2.75:1, about 3:1, about 4:1, about 5:1, about 6:1, about 7:1, about 8:1, about 9:1, about 10:1, about 12.5:1, about 15:1, about 20:1, about 30:1, about 40:1, about 50:1, about 100:1, about 500:1, about 1000:1, or more). In some embodiments, the polymer is a polysaccharide. In some embodiments, the polymer is amylose, arabinogalactan, carrageenan, chitosan, chondroitin sulfate, dextran, furcelleran, galactomannan, glucomannan, gellan gum, hyaluronic acid, Karaya gum (sterculia gum), locust bean gum, scleroglucan, pullalan, xylan, or a derivative thereof.

In some embodiments, the composition further comprises a bacterial taxa. In some embodiments, the composition comprises at least two (e.g., at least three, at least four) microbiome regulators and a bacterial taxa (e.g., a commensal bacterial taxa).

In another aspect, the present invention features a method of treating a subject having a disease, disorder, or condition requiring control of the blood sugar level (e.g., blood glucose level) of the subject, and wherein the subject would benefit from treatment with a composition comprising a microbiome regulator formulated in a dosage form or administered in a dosage regimen for substantial release in the gastrointestinal tract (e.g., the small intestine or large intestine), thereby substantially limiting systemic exposure to the microbiome regulator. In some embodiments, the composition is formulated for substantial relase in the small intestine, e.g., the duodenum, jejunum, or ileum. In some embodiments, the composition is formulated for substantial relase in the large intestine, e.g., cecum, colon, or rectum.

In some embodiments, the microbiome regulator comprises a sugar, or a sugar alcohol. In some embodiments, the microbiome regulator comprises a sugar or sugar alcohol. In some embodiments, the microbiome regulator comprises a sugar or sugar alcohol that is metabolizable by the host. In some embodiments, the microbiome regulator comprises a sugar or sugar alcohol that is non-metabolizable by the host. In some embodiments, the sugar or sugar alcohol comprises D-fructose, D-galactose, L-arabinose, D-mannose, D-xylose, D-arabinose, ribose, L-fucose, L-rhamnose, lactose, D-maltose, sucrose, or glucose. In some embodiments, the the sugar or sugar alcohol comprises glucose.

In some embodiments, the composition comprises more than about 50% (w/w) of a sugar or sugar alcohol (e.g., more than about 55%, about 60%, about 65%, about 70%, about 75%, about 80%, about 85%, about 90%, about 95%, about 96%, about 97%, about 98%, about 99%, or more). In some embodiments, the microbiome regulator comprises a molecule with less than about 12 carbon atoms (e.g., less than about 10 carbon atoms, about 9 carbon atoms, about 8 carbon atoms, about 7 carbon atoms, about 6 carbon atoms, or about 5 carbon atoms) and less than about12 heteroatoms (e.g., less than about 10 heteratoms, less than about 9 heteroatoms, less than about 8 heteroatoms, less than about 7 heteroatoms, less than about 6 heteroatoms, or less than about 5 heteroatoms), wherein the heteroatom is selected from oxygen, nitrogen, sulfur, or phosphorus. In some embodiments, the microbiome regulator does not comprise an alkenyl, alkynyl, aryl, heteroaryl, cycloalkyl, halogen, acyl, thiol, cyano, nitro, or sulfonyl moiety.

In some embodiments, the sugar or sugar alcohol has a low degree of sweetness relative to sucrose. In some embodiments, the sugar or sugar alcohol has a degree of sweetness less than about 1 relative to sucrose (e.g., less than about 0.95, about 0.9, about 0.85, about 0.8, about 0.75, about 0.7, about 0.65, about 0.6, about 0.55, about 0.5, or less). In some embodiments, the sugar or sugar alcohol has a degree of sweetness that can be no more than about 1 relative to sucrose (e.g., no more than about 2, about 3, about 4, about 5, about 10, about 20, about 25, about 50, about 75, about 100, about 250, about 500, about 1000, or more).

In some embodiments, the sugar or sugar alcohol has a low absorption coefficient relative to glucose. In some embodiments, the sugar or sugar alcohol has a an absorption coefficient less than 0.15 (e.g., less than about 0.14, about 0.13, about 0.12, about 0.11, about 0.10, about 0.09, about 0.08, about 0.07, about 0.06, about 0.05, about 0.04, about 0.03, about 0.02, about 0.01, or less).

In some disclosures, the microbiome regulator comprises an amino acid. In some disclosures, the amino acid is an L-amino acid or a D-amino acid. In some disclosures, the microbiome regulator comprises a micronutrient. In some disclosures, the micronutrient comprises a vitamin, an element, or a mineral. In some disclosures, the microbiome regulator comprises a fatty acid. In some disclosures, the fatty acid comprises a short-chain fatty acid (SCFA), a medium-chain fatty acid (MCFA), a long-chain fatty acid (LCFA), or a very long chain fatty acid (VLCFA). In some disclosures, the microbiome regulator comprises a polyphenol. In some disclosures, the polyphenol comprises a catechin, ellagitannin, isoflavone, flavonol, flavanone, anthocyanin, or lignin.

In some embodiments, the disease, disorder, or condition comprises cancer. In some embodiments, the disease, disorder, or condition is a metabolic disease. In some embodiments, the metabolic disease, disorder, or condition comprises diabetes.

In some embodiments, the systemic exposure of the composition in the host is less than about 95% (w/w) of the total composition (e.g., less than about 90%, about 85%, about 80%, about 75%, about 70%, about 65%, about 60%, about 55%, about 50%, about 45%, about 40%, about 35%, about 30%, about 25%, about 20%, about 15%, about 10%, about 5%, about 4%, about 3%, about 2%, about 1%, about 0.5%, or less. In some embodiments, the systemic exposure of the composition in the host is less than about 50% (w/w) of the total composition. In some embodiments, the systemic exposure of the composition in the host is less than about 25% (w/w) of the total composition. In some embodiments, the systemic exposure of the composition in the host is less than about 10% (w/w) of the total composition. In some embodiments, the systemic exposure of the composition in the host is less than about 5% (w/w) of the total composition. In some embodiments, the systemic exposure of the composition in the host is less than about 1% (w/w) of the total composition. In some embodiments, the composition (e.g., the microbiome regulator) is not systemically exposed to the host.

In some embodiments, the composition further comprises a polymer (e.g., a polysaccharide) to target the composition to a specific site in the gastrointestinal tract, e.g., the small intestine (e.g., the duodenum, jejunum, or ileum) or large intestine (e.g., cecum, colon, or rectum). In some embodiments, the composition comprises more than about 1% (w/w) of a polymer (e.g., more than about 2%, about 5%, about 10%, about 20%, about 30%, about 40%, about 50%, or more). In some embodiments, the ratio (w/w) of a microbiome regulator to a polymer is about than about 1:1 (e.g., about 1.1:1, about 1.2:1, about 1.3:1, about 1.4:1, about 1.5:1, about 1.75:1, about 2:1, about 2.25:1, about 2.5:1, about 2.75:1, about 3:1, about 4:1, about 5:1, about 6:1, about 7:1, about 8:1, about 9:1, about 10:1, about 12.5:1, about 15:1, about 20:1, about 30:1, about 40:1, about 50:1, about 100:1, about 500:1, about 1000:1, or more). In some embodiments, the polymer is a polysaccharide. In some embodiments, the polymer is amylose, arabinogalactan, carrageenan, chitosan, chondroitin sulfate, dextran, furcelleran, galactomannan, glucomannan, gellan gum, hyaluronic acid, Karaya gum (sterculia gum), locust bean gum, scleroglucan, pullalan, xylan, or a derivative thereof.

In some embodiments, the composition further comprises a bacterial taxa. In some embodiments, the composition comprises at least two (e.g., at least three, at least four) microbiome regulators and a bacterial taxa (e.g., a commensal bacterial taxa).

In some embodiments, the benefiting from treatment with a composition comprising a microbiome regulator comprises one or more of: i) treating a dysbiosis; ii) treating a drug-induced or treatment-induced side effect; or iii) modulating a bacterial taxa to provide a health benefit. In some embodiments, the method comprises at least two of i), ii), or iii). In some embodiments, the method consists of i), ii), and iii).

In another aspect, the present invention features a method of modulating microbial diversity in the gastrointestinal microbiota of a subject, the method comprising administering to the subject a composition comprising a microbiome regulator formulated in a dosage form or administered in a dosage regimen for substantial release of the composition to the gastrointestinal tract, to thereby modulate microbial diversity in the subject. In some embodiments, the microbial diversity comprises bacterial diversity. In some embodiments, the Shannon diversity of the microbiota is increased or decreased by at least about 5% (e.g., at least about 10%, about 15%, about 20%, about 25%, about 30%, about 35%, about 40%, about 45%, about 50%, or more). In some embodiments, the Shannon diversity of the microbiota is increased or decreased by at least about 0.1 log-fold (e.g., about 0.2 log-fold, about 0.3 log-fold, about 0.4 log-fold, about 0.5 log-fold, about 0.6 log-fold, about 0.7-log-fold, about 0.8 log-fold, about 0.9 log-fold, about 1 log-fold, about 1.5 log-fold about 2 log-fold, or more).

In some embodiments, the microbiome regulator comprises a sugar, or a sugar alcohol. In some embodiments, the microbiome regulator comprises a sugar or sugar alcohol. In some embodiments, the microbiome regulator comprises a sugar or sugar alcohol that is metabolizable by the host. In some embodiments, the microbiome regulator comprises a sugar or sugar alcohol that is non-metabolizable by the host. In some embodiments, the sugar or sugar alcohol comprises D-fructose, D-galactose, L-arabinose, D-mannose, D-xylose, D-arabinose, ribose, L-fucose, L-rhamnose, lactose, D-maltose, sucrose, or glucose. In some embodiments, the the sugar or sugar alcohol does not comprise glucose.

In some embodiments, the composition comprises more than about 50% (w/w) of a sugar or sugar alcohol (e.g., more than about 55%, about 60%, about 65%, about 70%, about 75%, about 80%, about 85%, about 90%, about 95%, about 96%, about 97%, about 98%, about 99%, or more). In some embodiments, the microbiome regulator comprises a molecule with less than about 12 carbon atoms (e.g., less than about 10 carbon atoms, about 9 carbon atoms, about 8 carbon atoms, about 7 carbon atoms, about 6 carbon atoms, or about 5 carbon atoms) and less than about12 heteroatoms (e.g., less than about 10 heteratoms, less than about 9 heteroatoms, less than about 8 heteroatoms, less than about 7 heteroatoms, less than about 6 heteroatoms, or less than about 5 heteroatoms), wherein the heteroatom is selected from oxygen, nitrogen, sulfur, or phosphorus. In some embodiments, the microbiome regulator does not comprise an alkenyl, alkynyl, aryl, heteroaryl, cycloalkyl, halogen, acyl, thiol, cyano, nitro, or sulfonyl moiety.

In some embodiments, the sugar or sugar alcohol has a low degree of sweetness relative to sucrose. In some embodiments, the sugar or sugar alcohol has a degree of sweetness less than about 1 relative to sucrose (e.g., less than about 0.95, about 0.9, about 0.85, about 0.8, about 0.75, about 0.7, about 0.65, about 0.6, about 0.55, about 0.5, or less). In some embodiments, the sugar or sugar alcohol has a degree of sweetness that can be no more than about 1 relative to sucrose (e.g., no more than about 2, about 3, about 4, about 5, about 10, about 20, about 25, about 50, about 75, about 100, about 250, about 500, about 1000, or more).

In some embodiments, the sugar or sugar alcohol has a low absorption coefficient relative to glucose. In some embodiments, the sugar or sugar alcohol has a an absorption coefficient less than 0.15 (e.g., less than about 0.14, about 0.13, about 0.12, about 0.11, about 0.10, about 0.09, about 0.08, about 0.07, about 0.06, about 0.05, about 0.04, about 0.03, about 0.02, about 0.01, or less).

In some embodiments, a substantial portion of the sugar or sugar alcohol that is metabolized by the host is metabolized in the lower GI tract (e.g., the small intestine or large intestine). In some embodiments, a substantial portion of the sugar or sugar alcohol that is metabolized by the host is metabolized in the small intestine, e.g., the duodenum, jejunum, or ileum. In some embodiments, a substantial portion of the sugar or sugar alcohol that is metabolized by the host is metabolized in the large intestine, e.g., cecum, colon, or rectum.

In some disclosures, the microbiome regulator comprises an amino acid. In some disclosures, the amino acid is an L-amino acid or a D-amino acid. In some disclosures, the microbiome regulator comprises a micronutrient. In some disclosures, the micronutrient comprises a vitamin, an element, or a mineral. In some disclosures, the microbiome regulator comprises a fatty acid. In some disclosures, the fatty acid comprises a short-chain fatty acid (SCFA), a medium-chain fatty acid (MCFA), a long-chain fatty acid (LCFA), or a very long chain fatty acid (VLCFA). In some disclosures, the microbiome regulator comprises a polyphenol. In some disclosures, the polyphenol comprises a catechin, ellagitannin, isoflavone, flavonol, flavanone, anthocyanin, or lignin.

In some embodiments, the composition further comprises a polymer (e.g., a polysaccharide) to target the composition to a specific site in the gastrointestinal tract, e.g., the small intestine (e.g., the duodenum, jejunum, or ileum) or large intestine (e.g., cecum, colon, or rectum). In some embodiments, the composition comprises more than about 1% (w/w) of a polymer (e.g., more than about 2%, about 5%, about 10%, about 20%, about 30%, about 40%, about 50%, or more). In some embodiments, the ratio (w/w) of a microbiome regulator to a polymer is about than about 1:1 (e.g., about 1.1:1, about 1.2:1, about 1.3:1, about 1.4:1, about 1.5:1, about 1.75:1, about 2:1, about 2.25:1, about 2.5:1, about 2.75:1, about 3:1, about 4:1, about 5:1, about 6:1, about 7:1, about 8:1, about 9:1, about 10:1, about 12.5:1, about 15:1, about 20:1, about 30:1, about 40:1, about 50:1, about 100:1, about 500:1, about 1000:1, or more). In some embodiments, the polymer is a polysaccharide. In some embodiments, the polymer is amylose, arabinogalactan, carrageenan, chitosan, chondroitin sulfate, dextran, furcelleran, galactomannan, glucomannan, gellan gum, hyaluronic acid, Karaya gum (sterculia gum), locust bean gum, scleroglucan, pullalan, xylan, or a derivative thereof.

In some embodiments, the composition further comprises a bacterial taxa. In some embodiments, the composition comprises at least two (e.g., at least three, at least four) microbiome regulators and a bacterial taxa (e.g., a commensal bacterial taxa).

In another aspect, the present invention features a method of treating a subject having a gastrointestinal disease, the method comprising administering to the subject a composition comprising a microbiome regulator formulated in a dosage form or administered in a dosage regimen for substantial release of the composition in the gastrointestinal tract of the subject, provided that if the microbiome regulator comprises glucose, the microbiome regulator is provided in a dosage form that is enterically coated.

In some embodiments, the microbiome regulator is a sugar or sugar alcohol comprising one or more of: i) a monosaccharide, or disaccharide that is metabolized by the host; wherein if the sugar or sugar alcohol is a disaccharide, trisaccharide, tetrasaccharide, or pentasaccharide: a) at least one glycosidic bonds comprise a 1->2 glycosidic bond, a 1->3 glycosidic bond, a 1->4 glycosidic bond, or a 1->6 glycosidic bond; and b) at least one of the glycosidic bonds are present in the alpha or beta configuration; ii) a molecular weight less than about 1000 g/mol (e.g., less than about 950 g/mol, about 900 g/mol, about 850 g/mol, about 800 g/mol, about 750 g/mol, about 700 g/mol, about 650 g/mol, about 600 g/mol, about 500 g/mol, about 450 g/mol, about 400 g/mol, about 350 g/mol, about 300 g/mol, about 250 g/mol, about 200 g/mol, or less); iii) less than about 12 carbon atoms, about 10 carbon atoms, about 9 carbon atoms, about 8 carbon atoms, about 7 carbon atoms, about 6 carbon atoms, or about 5 carbon atoms); iv) less than about 12 heteroatoms, less than about 10 heteroatoms, less than about 9 heteroatoms, less than about 8 heteroatoms, less than about 7 heteroatoms, less than about 6 heteroatoms, or less than about 5 heteroatoms); or v) does not comprise an alkenyl, alkynyl, aryl, heteroaryl, cycloalkyl, halogen, acyl, thiol, cyano, nitro, or sulfonyl moiety.

In some embodiments, the method comprises at least two of i), ii), iii), iv), or v). In some embodiments, the method comprises at least three of i), ii), iii), iv), or v). In some embodiments, the method comprises at least four of i), ii), iii), iv), or v). In some embodiments, the method consists of i), ii), iii), iv), and v).

In some embodiments, the composition comprises a sugar or sugar alcohol and one or more of the following: i) is substantially free of a sugar or sugar alcohol that is not metabolized by the host; and ii) is substantially free of an agent other than a microbiome regulator, e.g., a therapeutic agent (e.g., peptide, nucleic acid, oligosaccharide, polysaccharide, protein, non-peptide small molecule, or a prodrug or metabolite thereof), or a polymer (e.g. is polyethylene glycol (PEG), polypropylene glycol (PPG), polyvinyl pyrrolidine (PVG), polyvinyl alcohol (PVA), polyacrylic acid (PAA), polyacrylamide, N-(2-hydroxypropyl) methylacrylamide (HMPA), divinyl ether-maleic anhydride (DIVEMA), polyoxazolines, polyphosphates, xanthan gum, pectin, chitin, chitosan, dextran, carrageenan, guar gum, cellulose (e.g., hydroxypropylmethyl cellulose (HPMC), hydroxypropyl cellulose (HPC), hydroxyethylcellulose (HEC), sodium carboxymethyl cellulose (NaCMC)), hyaluronic acid, hyaluronan, albumin, heparin, chondroitin, starch, or derivatives thereof). In some embodiments, the method consists of i) and ii).

In some embodiments, the agent other than a microbiome regulator is a therapeutic agent and comprises one or more of: i) a peptide, nucleic acid, oligosaccharide, polysaccharide, protein, non-peptide small molecule, or a prodrug or metabolite thereof; ii) a molecular weight greater than about 500 g/mol; iii) more than about 6 carbon atoms; iv) a specificity for a cell surface receptor, an ion channel, a transporter, an enzyme, an antibody, or other biological target; or v) an agent used in the treatment of a disease, disorder, or condition. In some embodiments, the method comprises at least two of i), ii), iii), iv), or v). In some embodiments, wherein the method comprises at least three of i), ii), iii), iv), or v). In some embodiments, the method comprises at least four of i), ii), iii), iv), or v). In some embodiments, the method consists of i), ii), iii), iv), and v).

In some embodiments, the agent other than a microbiome regulator is a polymer.

In some embodiments, the composition further comprises a polymer (e.g., a polysaccharide) to target the composition to a specific site in the gastrointestinal tract, e.g., the small intestine (e.g., the duodenum, jejunum, or ileum) or large intestine (e.g., cecum, colon, or rectum). In some embodiments, the composition comprises more than about 1% (w/w) of a polymer (e.g., more than about 2%, about 5%, about 10%, about 20%, about 30%, about 40%, about 50%, or more). In some embodiments, the ratio (w/w) of a microbiome regulator to a polymer is about than about 1:1 (e.g., about 1.1:1, about 1.2:1, about 1.3:1, about 1.4:1, about 1.5:1, about 1.75:1, about 2:1, about 2.25:1, about 2.5:1, about 2.75:1, about 3:1, about 4:1, about 5:1, about 6:1, about 7:1, about 8:1, about 9:1, about 10:1, about 12.5:1, about 15:1, about 20:1, about 30:1, about 40:1, about 50:1, about 100:1, about 500:1, about 1000:1, or more). In some embodiments, the polymer is a polysaccharide. In some embodiments, the polymer is amylose, arabinogalactan, carrageenan, chitosan, chondroitin sulfate, dextran, furcelleran, galactomannan, glucomannan, gellan gum, hyaluronic acid, Karaya gum (sterculia gum), locust bean gum, scleroglucan, pullalan, xylan, or a derivative thereof.

In some embodiments, the composition further comprises a bacterial taxa. In some embodiments, the composition comprises at least two (e.g., at least three, at least four) microbiome regulators and a bacterial taxa (e.g., a commensal bacterial taxa).

In some embodiments, the present invention further comprisies identifying a subject in need of treatment of a gastrointestinal disease. In some embodiments, the subject in need of treatment of a gastrointestinal disease is identified based on assessing of the state of the microbiota of the subject. In some embodiments, the assessing comprises acquiring (e.g., directly or indirectly) knowledge of either the specific OTU or the microbial diversity of the gastrointestinal microbiota of the subject. In some embodiments, the identifying comprises acquiring (e.g., directly or indirectly) a sample from the subject (e.g., a fecal sample). In some embodiments, an effective amount of a composition comprising a microbiome regulator is administered based on the results of the assessing. In some embodiments, wherein the method further comprises identifying a subject having a dysbiosis.

In another disclosure, the application provides a pharmaceutical composition comprising a microbiome regulator comprising a sugar, a sugar alcohol, an amino acid, a peptide, a micronutrient, a fatty acid, or a polyphenol, formulated in a dosage form or administered in a dosage regimen that targets the release of the composition substantially to the gastrointestinal tract.

In some embodiments, the microbiome regulator comprises a sugar, a sugar alcohol, wherein the sugar or sugar alcohol is selected from a monosaccharide and a disaccharide, wherein the sugar or sugar alcohol is selected from the group consisting of D-fructose, D-galactose, L-arabinose, D-mannose, D-xylose, D-arabinose, ribose, L-fucose, L-rhamnose, lactose, D-maltose, sucrose, and glucose. In a further disclosure, the microiome regulator comprises an amino acid, a peptide, a micronutrient, a fatty acid, or a polyphenol. In some embodiments, the microbiome regulator comprises a sugar or sugar alcohol. In some embodiments, the microbiome regulator comprises a sugar or sugar alcohol that is metabolizable by the host. In some embodiments, the microbiome regulator comprises a sugar or sugar alcohol that is non-metabolizable by the host. In some embodiments, the sugar or sugar alcohol comprises is selected from the group consisting of D-fructose, D-galactose, L-arabinose, D-mannose, D-xylose, D-arabinose, ribose, L-fucose, L-rhamnose, lactose, D-maltose, sucrose, and glucoseIn some embodiments, the the sugar or sugar alcohol does not comprise glucose.

In some embodiments, the composition comprises more than about 50% (w/w) of a sugar or sugar alcohol (e.g., more than about 55%, about 60%, about 65%, about 70%, about 75%, about 80%, about 85%, about 90%, about 95%, about 96%, about 97%, about 98%, about 99%, or more). In some embodiments, the microbiome regulator comprises a molecule with less than about 12 carbon atoms (e.g., less than about 10 carbon atoms, about 9 carbon atoms, about 8 carbon atoms, about 7 carbon atoms, about 6 carbon atoms, or about 5 carbon atoms) and less than about12 heteroatoms (e.g., less than about 10 heteratoms, less than about 9 heteroatoms, less than about 8 heteroatoms, less than about 7 heteroatoms, less than about 6 heteroatoms, or less than about 5 heteroatoms), wherein the heteroatom is selected from oxygen, nitrogen, sulfur, or phosphorus. In some embodiments, the microbiome regulator does not comprise an alkenyl, alkynyl, aryl, heteroaryl, cycloalkyl, halogen, acyl, thiol, cyano, nitro, or sulfonyl moiety.

In some embodiments, the sugar or sugar alcohol has a low degree of sweetness relative to sucrose. In some embodiments, the sugar or sugar alcohol has a degree of sweetness less than about 1 relative to sucrose (e.g., less than about 0.95, about 0.9, about 0.85, about 0.8, about 0.75, about 0.7, about 0.65, about 0.6, about 0.55, about 0.5, or less). In some embodiments, the sugar or sugar alcohol has a degree of sweetness that can be no more than about 1 relative to sucrose (e.g., no more than about 2, about 3, about 4, about 5, about 10, about 20, about 25, about 50, about 75, about 100, about 250, about 500, about 1000, or more).

In some embodiments, the sugar or sugar alcohol has a low absorption coefficient relative to glucose. In some embodiments, the sugar or sugar alcohol has a an absorption coefficient less than 0.15 (e.g., less than about 0.14, about 0.13, about 0.12, about 0.11, about 0.10, about 0.09, about 0.08, about 0.07, about 0.06, about 0.05, about 0.04, about 0.03, about 0.02, about 0.01, or less).

In some embodiments, a substantial portion of the sugar or sugar alcohol that is metabolized by the host is metabolized in the lower GI tract (e.g., the small intestine or large intestine). In some embodiments, a substantial portion of the sugar or sugar alcohol that is metabolized by the host is metabolized in the small intestine, e.g., the duodenum, jejunum, or ileum. In some embodiments, a substantial portion of the sugar or sugar alcohol that is metabolized by the host is metabolized in the large intestine, e.g., cecum, colon, or rectum.

In some disclosures, the microbiome regulator comprises an amino acid. In some disclosures, the amino acid is an L-amino acid or a D-amino acid. In some disclosures, the microbiome regulator comprises a micronutrient. In some disclosures, the micronutrient comprises a vitamin, an element, or a mineral. In some disclosures, the microbiome regulator comprises a fatty acid. In some disclosures, the fatty acid comprises a short-chain fatty acid (SCFA), a medium-chain fatty acid (MCFA), a long-chain fatty acid (LCFA), or a very long chain fatty acid (VLCFA). In some embodiments, the microbiome regulator comprises a polyphenol. In some disclosures, the polyphenol comprises a catechin, ellagitannin, isoflavone, flavonol, flavanone, anthocyanin, or lignin.

In some embodiments, the composition further comprises a polymer (e.g., a polysaccharide) to target the composition to a specific site in the gastrointestinal tract, e.g., the small intestine (e.g., the duodenum, jejunum, or ileum) or large intestine (e.g., cecum, colon, or rectum). In some embodiments, the composition comprises more than about 1% (w/w) of a polymer (e.g., more than about 2%, about 5%, about 10%, about 20%, about 30%, about 40%, about 50%, or more). In some embodiments, the ratio (w/w) of a microbiome regulator to a polymer is about than about 1:1 (e.g., about 1.1:1, about 1.2:1, about 1.3:1, about 1.4:1, about 1.5:1, about 1.75:1, about 2:1, about 2.25:1, about 2.5:1, about 2.75:1, about 3:1, about 4:1, about 5:1, about 6:1, about 7:1, about 8:1, about 9:1, about 10:1, about 12.5:1, about 15:1, about 20:1, about 30:1, about 40:1, about 50:1, about 100:1, about 500:1, about 1000:1, or more). In some embodiments, the polymer is a polysaccharide. In some embodiments, the polymer is amylose, arabinogalactan, carrageenan, chitosan, chondroitin sulfate, dextran, furcelleran, galactomannan, glucomannan, gellan gum, hyaluronic acid, Karaya gum (sterculia gum), locust bean gum, scleroglucan, pullalan, xylan, or a derivative thereof.

In some embodiments, the composition further comprises a bacterial taxa. In some embodiments, the composition comprises at least two (e.g., at least three, at least four) microbiome regulators and a bacterial taxa (e.g., a commensal bacterial taxa).

In any and all of the foregoing aspects, in some embodiments, the invention features dosage forms for use in promoting growth of commensal bacteria.

### BRIEF DESCRIPTION OF THE DRAWINGS

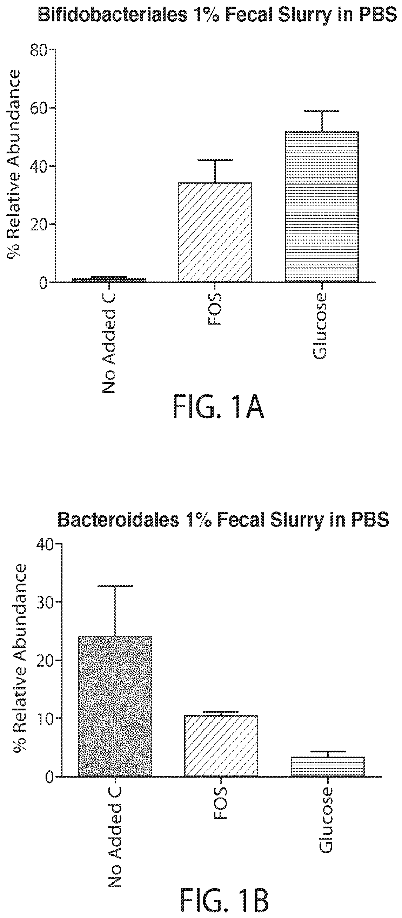

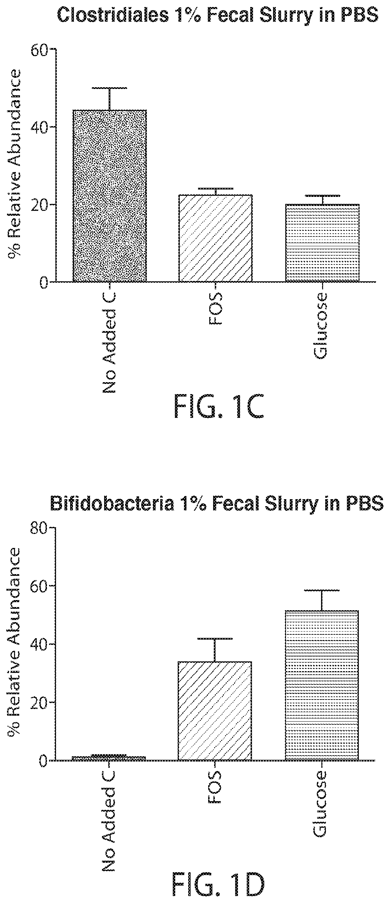

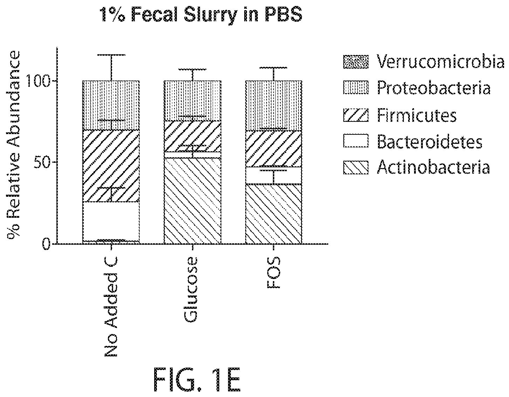

**Figures 1A-1E****.** Graphs showing the relative abundance of selected bacterial taxa from a human fecal slurry grown in glucose monomer and commercially available FOS as the carbon source as described in Example 6. Selected bacterial taxa examined include Bifidobacteriales **(****Fig. 1A****),** Bacteroidales **(****Fig. 1B****),** Clostridiales **(****Fig. 1C****),** Bifidobacteria **(****Fig. 1D****),** and Verrucomicrobia, Proteobacteria, Firmicutes, Bacteroidetes, and Actinobacteria **(****Fig. 1E**). For each, the percent (%) relative abundance in 1% fecal slurry grown in FOS, glucose, and no added carbon is depicted.
**Figure 2****.** A chart depicting short chain fatty acid (SCFA) concentrations in supernatants of BUN.80 and DLO.76 grown with either FOS of glucose monomer as described in Example 7.
**Figure 3****:** A table depicting exemplary combinations of microbial genera, microbiome regulators, and selected media components.

### DETAILED DESCRIPTION OF THE INVENTION

In humans, the gastrointestinal microbiota is largely stable when the host is in good health; however, the ecosystem of the gastrointestinal microbiota varies depending on host age, disease, including infections with pathogens, stress, diet, and pharmaceutical treatments and can enter a state of dysbiosis. The present invention features compounds, compositions, and methods comprising one or more microbime regulators modulation of human microbiota and for the treatment of disease. Though not bound by theory, a microbiome regulator may be digested by certain microbial species to thereby induce changes in the GI tract to confer benefits upon host well-being and health. The microbiome regulators can act as tailored, finely tuned modulators for the resident or acquired microbiota, e.g., by enhancing the growth and/or function of beneficial bacteria and/or suppressing the growth and/or function of pathogenic microbes, such as those associated with a disease or condition.

The microbiome regulators and methods of use described herein can mediate a surprising array of shifts in the abundance of important taxa of the gastrointestinal microbiota. In some embodiments, the microbial shifts allow for particular tuning of microbial properties, such as i) ecosystem resilience to disturbance, ii) microbiota diversity, iii) metabolite production, iv) pathobiont and pathogen colonization, and v) altered effects on host metabolic, immune, and other functions or any combination thereof.

Described herein are compositions, methods, and kits useful for the treatment and prevention of diseases associated with a dysbiosis of the gastrointestinal microbiota, reduction of symptoms thereof in a subject in need, and for improving overall health of the host. Further described herein are dosage forms for microbiome regulators. In some embodiments, the dosage forms are formulated for targeted delivery to specific regions of the GI tract, such as, e.g., the small or large intestine (e.g., the colon). Administration of the pharmaceutical compositions, medical foods, or dietary supplements comprising microbiome regulators may treat or prevent conditions in which a microbiota is disturbed and in which the subject may exhibit a dybiosis. In some embodiments, the disturbance can be ameliorated by the use of the microbiome regulators described herein so that improved physiological growth and function of both the beneficial microbiota and the host can be achieved. Such treatment or prevention may occur directly, e.g., a microbiome regulator or composition thereof described herein may cause displacement of a pathogenic microbe with a non-pathogenic one or increase the growth of beneficial or commensal microbes, or it may occur indirectly, e.g., a microbiome regulator or composition thereof described herein may affect metabolism or other functions of the microbiota, thus modulating host physiology, e.g., through the effect of one or more downstream metabolic products. Administration of a microbiome regulator or composition thereof described herein may improve the overall health of the host and may restore a healthy equilibrium in a selected niche, such as the GI tract, by influencing one or more members of the microbial community.

### Definitions

As used herein, the term "abundance" as it relates to a microbial taxa refers to the presence of one microbial taxa as compared to another microbial taxa in a defined microbial niche, such as the GI tract, or in the entire host organism (e.g. a human or a laboratory animal model of disease).

"Acquire" or "acquiring" as the terms are used herein, refer to obtaining possession of a value, e.g., a numerical value, or image, or a physical entity (e.g., a sample), by "directly acquiring" or "indirectly acquiring" the value or physical entity. "Directly acquiring" means performing a process (e.g., performing a synthetic or analytical method or protocol) to obtain the value or physical entity. "Indirectly acquiring" refers to receiving the value or physical entity from another party or source (e.g., a third party laboratory that directly acquired the physical entity or value). Directly acquiring a value or physical entity includes performing a process that includes a physical change in a physical substance or the use of a machine or device. Examples of directly acquiring a value include obtaining a sample from a human subject. Directly acquiring a value includes performing a process that uses a machine or device, e.g., an NMR spectrometer to obtain an NMR spectrum.

As used herein, "colonization" of a host organism refers to the non-transitory residence of a bacterium or other microbial organism in a niche.

"Diversity of a microbial community" or "microbial diversity" as used herein refers to the diversity found in the microbiota within a given niche or host subject. Diversity can relate to the number of distinct microbial taxa and/or richness of the microbial taxa within the niche or host and can be expressed, e.g. using the Shannon Diversity index (Shannon entropy), alpha-beta diversity, total number of observed OTUs, or Chao1 index, as described herein. In some embodiments, a microbiome regulator described herein modulates diversity within a microbial community, which may be expressed using Shannon entropy as a measure. For example, the more unequal the abundances of the bacterial taxa, the larger the weighted geometric mean of the pᵢ values in Shannon's formula, and the smaller the corresponding Shannon entropy. If practically all abundance is concentrated to one taxa, and the other taxa are very rare (even if there are many of them), Shannon entropy approaches zero. When there is only one taxa Shannon entropy exactly equals zero.

As used herein, a "dosage regimen", "dosing regimen", or "treatment regimen" is a modality of drug administration that achieves a therapeutic objective. A dosage regimen includes definition of one, two, three, or four of: a route of administration, a unit dose, a frequency of dosage, or a length of treatment.

As used herein, a "dysbiosis" refers to the state of the microbiota under conditions of host disease, predisposition to host disease, or other unwanted condition or symptom of the host. In an embodiment, dysbiosis refers to the state of the microbiota under conditions of disease. Dysbiosis can be contrasted with eubiosis, which refers to the state of the microbiota under healthy conditions of the host. The state of the microbiota may include the characteristics relating to either the structure or function of the microbiota. In an embodiment, a dysbiosis includes an imbalance in the state of the microbiota, wherein the normal diversity or relative abundance of a microbial taxa is affected, e.g., relative to a second bacterial taxa or relative to the abundance of said taxa under conditions of health. In an embodiment, a dysbiosis comprises an imbalance in the function of the microbiota, e.g., a change in level of gene expression, level of a gene product, or metabolic output (e.g., an immune function such as immune surveillance or the inflammation response). In some embodiments, a dysbiosis is an an undesired, e.g., unhealthy, state associated with unwanted symptoms in the host and that no longer promotes health.

A"dysbiosis of the gastrointestinal microbiota" refers to an imbalanced state of the microbiota of the GI tract (e.g., in the stomach, small intestine, or large intestine).

As used herein, "ecological niche" or simply "niche" refers to the ecological space in which an organism or group of organisms occupies (such as the GI tract or one or more subsection of the GI-tract, such as, e.g., the stomach, the large and small intestine, the rectum, etc.). In some embodiments, niche specifically refers to a space that microorganisms occupy. Niche may describe how an organism or population of organisms responds to the distribution of resources, physical parameters (e.g., host tissue space) and competitors (e.g., by growing when resources are abundant, and when predators, parasites and pathogens are scarce) and how it in turn alters those same factors (e.g., limiting access to resources by other organisms, acting as a food source for predators and a consumer of prey).

An "effective amount" or "therapeutically effective amount" as used herein refers to an amount of a pharmaceutical composition or a drug agent that is sufficient to provide a desired effect. In some embodiments, a physician or other health professional decides the appropriate amount and dosage regimen. An effective amount also refers to an amount of a pharmaceutical composition or a drug agent that prevents the development or relapse of a medical condition.

As used herein, an "isolated" or "purified" preparation of a microbiome regulator is substantially pure and free of cellular material or other chemicals. In some embodiments, pure or isolated compounds, compositions or preparations may contain traces of solvents and/or salts. A purified compounds is at least about 60% (by w/w, w/v, v/v or molar %), at least about 75%, at least about 90%, at least about 95%, at least about 97%, at least about 98%, or at least about 99% by w/w, w/v, v/v or molar % the compound of interest. For example, a purified (substantially pure) or isolated preparation of a microbiome regulator is one that is at least 90%, 91%, 92%, 93%, 94%, 95%, 98%, 99%, 99.5%, 99.8%, 99.9% or 100% (w/w) of the desired microbiome regulators by w/w, w/v, v/v or molar % and separated from the components that accompany it, e.g. during manufacture, extraction/purification and/or processing (e.g. free from an undesired compound). Purity may be measured by any appropriate standard method, for example, by column chromatography, thin layer chromatography, or high-performance liquid chromatography (HPLC) analysis.

As used herein, the term "metabolizable" refers to a substance (e.g., a microbiome regulator, e.g., a sugar or sugar alcohol) that is digested or absorbed either by the host, the microbiota, or both. In an embodiment, a substance may be substantially metabolizable only by the host. In an embodiment, a substance may be substantially metabolizable only by the microbiota. A "nutritive" metabolizable substance is one wherein the substance or byproducts of the substance are harnessed for energy or other purposes by the the host. A non-nutritive metabolizable substance is substantially non-nutritive to the host, but may be broken down by microbiota. Exemplary metabolizable substances include sugars and sugar alcohols, such as glucose, galactose, mannose, fructose, and fucose.

As used herein, "microbiome" refers to the genetic content of the communities of microbes that live in and on a subject (e.g. a human subject), both sustainably and transiently, including eukaryotes, archaea, bacteria, and viruses (including bacterial viruses (e.g., phage)), wherein "genetic content" includes genomic DNA, RNA such as ribosomal RNA and messenger RNA, the epigenome, plasmids, and all other types of genetic information. In some embodiments, microbiome specifically refers to genetic content of the communities of microorganisms in a niche.

As used herein, the term "microbiome regulator" refers to a sugar, a sugar alcohol, which is capable of modulating the microbiome of a subject. In some embodiments, modulation of the microbiome comprises increasing or decreasing the abundance of at least one microbial taxa, increasing or decreasing the diversity of at least one microbial taxa, or altering a metabolic pathway of at least one microbial taxa. In some embodiments, a microbiome regulator has a therapeutic effect, such as a treatment or preventative effect, and may improve the health of a microbial niche (e.g., the GI tract of a subject).

An "agent other than a microbiome regulator" as used herein refers to an agent that does not comprise a microbiome regulator as described herein. In some embodiments, an agent other than a microbiome regulator does not comprise a sugar, sugar alcohol, amino acid, a peptide, a fatty acid, a micronutrient, or a polyphenol. An exemplary agent other than a microbiome regulator may include a therapeutic agent (e.g., a nucleic acid, an oligosaccharide or polysaccharide longer than a pentasaccharide, a protein, or a non-peptide small molecule) or an non-therapeutic agent such as a polymer, carrier, filler, coating, or excipient.

"Microbiota" as used herein refers to the community of microorganisms that occur (sustainably or transiently) in and on a subject (e.g. a human subject), including eukaryotes, archaea, bacteria, and viruses (including bacterial viruses, e.g. phage). In some embodiments, microbiota specifically refers to the microbial community in a niche.

"Modulate the microbiota" or "modulating the microbiota" as used herein refers to changing the state of the microbiota. Changing the state of the microbiota may include changing the structure and/or function of the microbiota. A change in the structure of the microbiota is, e.g., a change in the relative composition of a taxa, e.g., in one or more regions of the GI tract such as the cecum, ascending colon, transverse colon, descending colon, sigmoid colon, and/or rectum. In an embodiment, a change in the structure of the microbiota comprises a change in the abundance of a taxa, e.g., relative to another taxa or relative to what would be observed in the absence of the modulation. Modulation of the microbiota may also include a change in a function of the microbiota, such as a change in microbiota gene expression, level of a gene product (e.g., RNA or protein), and/or metabolic output of the microbiota. Functions of the microbiota may also include pathogen protection, nutrition, host metabolism, and immune modulation. Modulation of the structure or function of the microbiota may additionally induce a change in one or more functional pathway of the host (e.g., a change in gene expression, level of a gene product, and/or metabolic output of a host cell or host process) as a result of a change in the microbiota structure or its function.

As used herein, the term "pathogenic"(e.g. "pathogenic bacteria") refers to a substance, microorganism or condition that has the capability to cause a disease. In certain contexts, pathogens also include microbes (e.g. bacteria) that are associated with a disease or condition but for which a causative relationship (e.g., a direct causative relationship)has not been established or has yet to be established.

As used herein, a "pharmaceutical composition" or "pharmaceutical preparation" is a composition or preparationhaving pharmacological activity or other direct effect in the mitigation, treatment, or prevention of disease, and/or a finished dosage form or formulation thereof and is for human use. A pharmaceutical composition or pharmaceutical preparation is typically produced under good manufacturing practices (GMP) conditions. Pharmaceutical compositions or preparations may be sterile or non-sterile. If non-sterile, such pharmaceutical compositions meet the microbiological specifications and criteria for non-sterile pharmaceutical products as described in the U.S. Pharmacopeia (USP) or European Pharmacopoeia (EP). Pharmaceutical compositions may further comprise or may be co-administered with additional active agents, such as, e.g. additional therapeutic agents. Pharmaceutical compositions may also comprise pharmaceutically acceptable excipients, solvents, carriers, fillers, or any combination thereof.

The term "phenotype" refers to a set of observable characteristics of an individual entity. For example, an individual subject may have a phenotype of "healthy" or "diseased." A phenotype may describe the state of an entity, wherein all entities within a phenotype share the same set of characteristics that describe the phenotype. The phenotype of an individual results in part, or in whole, from the interaction of the entities genome and/or microbiome with the environment.

As used herein, the term "subject" or "patient" generally refers to any human subject. The term does not denote a particular age or gender. Subjects may include pregnant women. Subjects may include a newborn (e.g., a preterm newborn, a full term newborn), an infant up to one year of age, young children (e.g., 1 yr to 12 yrs), teenagers (e.g., 13-19 yrs), adults (e.g., 20-64 yrs), and elderly adults (65 yrs and older). A subject does not include an agricultural animal, e.g., farm animals or livestock, e.g., cattle, horses, sheep, swine, chickens, etc. In general, a subject comprises a host and its corresponding microbiota.

A "substantial decrease" as used herein is a decrease of 5%, 10%, 20%, 30%, 40%, 50%, 60%, 70%, 80%, 90%, 95%, 97%, 98%, 99%, 99.9%, or 100%.

A "substantial increase" as used herein is an increase of 10%, 20%, 30%, 40%, 50%, 60%, 70%, 80%, 90%, 100%, 150%, 200%, 250%, 300%, 350%, 400%, 450%, 500%, 550%, 600%, 650%, 700%, 750%, 800%, 850%, 900%, 950%, 1000%, or more than 1000%.

The terms "treating" and "treatment" as used herein refer to the administration of an agent or composition to a subject (e.g., a symptomatic subject afflicted with an adverse condition, disorder, or disease) so as to affect a reduction in severity and/or frequency of a symptom, eliminate a symptom and/or its underlying cause, and/or facilitate improvement or remediation of damage, and/or preventing an adverse condition, disorder, or disease in an asymptomatic subject who is susceptible to a particular adverse condition, disorder, or disease, or who is suspected of developing or at risk of developing the condition, disorder, or disease.

### Microbiome Regulators: Sugars and Sugar Alcohols

Microbiome regulators in some embodiments are compounds, preparations, pharmaceutical compositions or dosage forms (and kits comprising same) that comprise a simple sugar (such as a monosaccharide, a disaccharide, a trisaccharide, a tetrasacchaaride or a pentasaccharide), a sugar alcohol, an amino acid (such as a single amino acid, a dipeptide, tripeptide, tetrapeptide, or a pentapeptide), a peptide (such as a dipeptide, tripeptide, tetrapeptide, or pentapeptide), a lipid or fatty acid (e.g., a C1, C2, C3, etc. fatty acid), a micronutrient (e.g., a vitamin, element, or mineral), a polyphenol, or any combination thereof. In some embodiments, the microbiome regulator comprises a metabolizable sugar or metabolizable sugar alcohol, wherein the sugar or sugar alcohol is metabolized in the gastrointestinal tract of the host.

In some embodiments, the preparations, pharmaceutical compositions or dosage forms (and kits comprising same) of microbiome regulators are not synbiotics. In some embodiments, the microbiome regulator preparations are not nutritional supplements that comprise a probiotics and a prebiotic. In some embodiments, the microbiome regulator preparations do not contain a probiotic bacterium. In some embodiments, the microbiome regulator preparations do not contain a dietary fiber (DF), e.g. non-starch polysaccharides (NSP) or lignin. In some embodiments, the microbiome regulator preparations do not contain oligosaccharides (e.g. saccharides that are larger than a disaccharide). In some embodiments, the microbiome regulator preparations do not contain one or more of: glucooligosaccharide, mannanoligosaccharide, inulin, lychnose, maltotretraose, nigerotetraose, nystose, sesemose, stachyose, isomaltotriose, nigerotriose, maltotriose, melezitose, maltotriulose, raffinose, kestose, fructooligosaccharide, 2'-fucosyllactose, galactooligosaccharide, idraparinux, isomaltooligosaccharide, maltodextrin and xylooligosaccharide. In some embodiments, the microbiome regulator is not glucose.

In some embodiments, preparations, pharmaceutical compositions or dosage forms (and kits comprising same) of microbiome regulators comprise one or more (or a plurality of) of a simple sugar or sugar alcohol (e.g., a sugar or sugar alcohol metabolizable by the host). In one embodiment, the sugar is selected from the group consisting of a monosaccharide and a disaccharide.

In some embodiments, the one or more sugar is a monosaccharide, including, but not limited to D-fructose, D-galactose, L-arabinose, D-mannose, D-xylose, D-arabinose, ribose, L-fucose, L-rhamnose, and glucose.

In other embodiments, the one or more sugar or sugar alcohol (e.g., a sugar or sugar alcohol metabolizable by the host) is a monosaccharide comprising D-fructose, D-galactose, L-arabinose, D-mannose, D-xylose, D-arabinose, ribose, L-fucose, L-rhamnose, and glucose.

In some embodiments, the one or more sugar (e.g., a sugar metabolizable by the host) is a disaccharide, wherein the disaccharide is one of lactose, maltose, orsucrose.

In other embodiments, the one or more sugar or sugar alcohol (e.g., a metabolizable sugar or metabolizable sugar alcohol) is a disaccharide, wherein the disaccharide is sucrose, lactose, or mannose.

In some embodiments, preparations, pharmaceutical compositions or dosage forms (and kits comprising same) of microbiome regulators comprise one or more sugar alcohol. In some embodiments, the sugar alcohol is methanol, ethylene glycol, glycerol, erythritol, threitol, arabitol, ribitol, xylitol, mannitol, sorbitol, galactitol, iditol, volemitol, fucitol, inositol, maltotritol, maltotetraitol, or polyglycitol.

In cases in which the microbiome regulator comprises a disaccharide,, each terminus of the sugar or sugar alcohol may have a reducing end and a non-reducing end depending upon whether the sugar at the reducing end is in fact a reducing sugar. In accordance with accepted nomenclature, most disaccharides are depicted herein with the non-reducing end on the left and the reducing end on the right. Additionally, these structures are typically described herein are described with the name or abbreviation for the non-reducing saccharide (e.g., Gal or D-Gal), preceded or followed by the configuration of the glycosidic bond (alpha or beta), the ring bond, the ring position of the reducing saccharide involved in the bond, and then the name or abbreviation of the reducing sugar (e.g., Glc or D-Glc). The linkage (e.g., glycosidic linkage, galactosidic linkage, glucosidic linkage, etc.) between two sugar units can be expressed, for example, as 1,4, 1->4, or (1-4), used interchangeably herein. Each sugar may be in a cyclic form (e.g. pyranose or furanose form). For example, lactose is a disaccharide composed of cyclic forms of galactose and glucose joined by a beta (1-4) linkage where the acetal oxygen bridge is in the beta orientation.

Linkages between the individual sugar units comprising a disaccharide, trisaccharide, tetrasaccharide or pentasaccharide may include alpha 1->2, alpha 1->3, alpha 1->4, alpha 1->6, alpha 2->1, alpha 2->3, alpha 2->4, alpha 2->6, beta 1->2, beta 1->3, beta 1->4, beta 1->6, beta 2->1, beta 2->3, beta 2->4, and beta 2->6.

In some embodiments, a sugar and sugar alcohol described herein comprises only alpha linkages. In some embodiments, a sugar and sugar alcohol described herein comprises only beta linkages. In some embodiments, a sugar and sugar alcohol described herein comprises a mixture of alpha and beta linkages. In some embodiments, the alpha:beta glycosidic bond ratio in a particular sugar or sugar alcohol described herein is about 0.1:1, 0.2:1, 0.3:1, 0.4:1, 0.5:1, 0.6:1, 0.7:1, 0.8:1, 0.9:1, 1:1, 1.2:1, 1.5:1, 1.7:1, 2:1, 2.2:1, 2.5:1, 2.7:1, 3:1, 4:1, 5:1, 6:1, 7:1, 8:1, 9:1, or about 10:1.

In some embodiments, a sugar and sugar alcohol described herein comprises at least 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 97%, 98%, 99%, at least 99.9% or even 100% alpha glycosidic bonds. In some embodiments, a sugar and sugar alcohol described herein comprises at least 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 97%, 98%, 99%, at least 99.9% or even 100% beta glycosidic bonds.

In some embodiments, a sugar and sugar alcohol described herein comprises at least two glycosidic bonds selected from the group consisting of alpha 1->2 and alpha 1->3, alpha 1->2 and alpha 1->4, alpha 1->2 and alpha 1->6, alpha 1->2 and beta 1->2, alpha 1->2 and beta 1->3, alpha 1->2 and beta 1->4, alpha 1->2 and beta 1->6, alpha 1->3 and alpha 1->4, alpha 1->3 and alpha 1->6, alpha 1->3 and beta 1->2, alpha 1->3 and beta 1->3, alpha 1->3 and beta 1->4, alpha 1->3 and beta 1->6, alpha 1->4 and alpha 1->6, alpha 1->4 and beta 1->2, alpha 1->4 and beta 1->3, alpha 1->4 and beta 1->4, alpha 1->4 and beta 1->6, alpha 1->6 and beta 1->2, alpha 1->6 and beta 1->3, alpha 1->6 and beta 1->4, alpha 1->6 and beta 1->6, beta 1->2 and beta 1->3, beta 1->2 and beta 1->4, beta 1->2 and beta 1->6, beta 1->3 and beta 1->4, beta 1->3 and beta 1->6, and beta 1->4 and beta 1->6.

In some embodiments, a sugar and sugar alcohol described herein comprises at least one sugar or sugar alcohol in L-form. In some embodiments, a sugar and sugar alcohol described herein comprises least one sugar or sugar alcohol in D-form.

In some embodiments, a sugar and sugar alcohol described herein comprise a desired mixture of L- and D-forms, e.g. of a desired ratio, such as: 1:1, 1:2, 1:3, 1:4, 1:5, 1:6, 1:7, 1:8, 1:9, 1:10, 1:12, 1:14, 1:16, 1:18, 1:20, 1:25, 1:30, 1:35, 1:40, 1:45, 1:50, 1:55, 1:60, 1:65, 1:70, 1:75, 1:80, 1:85, 1:90, 1:100, 1:150 L- to D-forms or D- to L-forms.

In some embodiments, a sugar and sugar alcohol described herein comprise at least one tetrose, a pentose, a hexose, or a heptose. Examples of monosaccharides include hexoses, such as glucose, galactose, and fructose, and pentoses, such as xylose. Monosaccharides generally have the chemical formula: Cₓ(H₂O)_{y}, where conventionally x ≥ 3. Monosaccharides can be classified by the number x of carbon atoms they contain, for example: diose (2) triose (3) tetrose (4), pentose (5), hexose (6), and heptose (7). A monosaccharide may exist in an acyclic (open-chain) form, or may exist as two or more stereoisomers. A monosaccharide may also exist as a cyclic form, e.g., a ring with 5 (furanoses) or 6 atoms (pyranoses).

In some embodiments, a sugar or sugar alcohol described herein (e.g., a sugar or sugar alcohol metabolizable by the host) is present in a modified form, such as an ester, acetyl, carboxylate, amino, amido, or other derivative form thereof. In some embodiments, a sugar or sugar alcohol described herein (e.g., a sugar or sugar alcohol metabolizable by the host) is present as a salt form, such as a sulfate, phosphate, or other salt form thereof.

In some embodiments, a sugar or sugar alcohol described herein (e.g., a sugar or sugar alcohol metabolizable by the host) has a molecular weight less than about 1000 g/mol (e.g., less than about 950 g/mol, about 900 g/mol, about 850 g/mol, about 800 g/mol, about 750 g/mol, about 700 g/mol, about 650 g/mol, about 600 g/mol, about 500 g/mol, about 450 g/mol, about 400 g/mol, about 350 g/mol, about 300 g/mol, about 250 g/mol, about 200 g/mol, or less). In some embodiments, a sugar or sugar alcohol described herein (e.g., a sugar or sugar alcohol metabolizable by the host) has molecular weight less than about 500 g/mol (e.g., less than about 450 g/mol, about 400 g/mol, about 350 g/mol, about 300 g/mol, about 250 g/mol, about 200 g/mol, or less).

In some embodiments, a sugar or sugar alcohol described herein (e.g., a sugar or sugar alcohol metabolizable by the host) comprises a molecule with less than about 30 carbon atoms (e.g., less than about 25 carbon atoms, about 20 carbon atoms, about 18 carbon atoms, about 15 carbon atoms, about 12 carbon atoms, about 10 carbon atoms, about 9 carbon atoms, about 8 carbon atoms, about 7 carbon atoms, about 6 carbon atoms, or about 5 carbon atoms). In some embodiments, a sugar or sugar alcohol described herein (e.g., a sugar or sugar alcohol metabolizable by the host) comprises a molecule with less than about 12 carbon atoms (e.g., less than about 10 carbon atoms, about 9 carbon atoms, about 8 carbon atoms, about 7 carbon atoms, about 6 carbon atoms, or about 5 carbon atoms).

In some embodiments, a sugar or sugar alcohol described herein (e.g., a sugar or sugar alcohol metabolizable by the host) comprises a molecule with less than about 30 carbon atoms (e.g., less than about 25 carbon atoms, about 20 carbon atoms, about 18 carbon atoms, about 15 carbon atoms, about 12 carbon atoms, about 10 carbon atoms, about 9 carbon atoms, about 8 carbon atoms, about 7 carbon atoms, about 6 carbon atoms, or about 5 carbon atoms) and less than about 30 heteroatoms (e.g., less than about 25 heteroatoms, about 20 heteroatoms, about 18 heteroatoms, less than about 15 heteroheteroatomsatoms, less than about 12 heteroatoms, less than about 10 heteroatoms, less than about 9 heteroatoms, less than about 8 heteroatoms, less than about 7 heteroatoms, less than about 6 heteroatoms, or less than about 5 heteroatoms), wherein the heteroatom is selected from oxygen, sulfur, nitrogen, or phosphorus. In some embodiments, a sugar or sugar alcohol described herein (e.g., a sugar or sugar alcohol metabolizable by the host) comprises a molecule with less than about 12 carbon atoms (e.g., less than about 10 carbon atoms, about 9 carbon atoms, about 8 carbon atoms, about 7 carbon atoms, about 6 carbon atoms, or about 5 carbon atoms) and less than about 12 heteroatoms (e.g., less than about 10 heteroatoms, less than about 9 heteroatoms, less than about 8 heteroatoms, less than about 7 heteroatoms, less than about 6 heteroatoms, or less than about 5 heteroatoms), wherein the heteroatom is selected from oxygen, sulfur, nitrogen, or phosphorus.

In some embodiments, the relative sweetness of a sugar or sugar alcohol may be determined compared to a reference standard. For example, the relative sweetness of several sugar and sugar alcohols has been determined relative to sucrose (see, e.g., http://owlsoft.com/pdf_docs/WhitePaper/Rel_Sweet.pdf). Naturally occurring sugars and sugar alcohols (e.g., sugars and sugar alcohols metabolizable by the host) such as glucose, fructose, galacrose, lactose, maltose, xylose, and sorbitol were all found to roughly as sweet or, in many cases, less sweet than sucrose. However, artificial sugars, sugar alcohols, and other sweeteners including acesulfame K and aspartame were determined to have a much higher relative sweetness compared to sucrose (e.g., between 150-200 times as sweet as sucrose). In some embodiments, the relative sweetness of a sugar or sugar alcohol is 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 80, 90, 100, 150, 200, 250, 300, 350, 400, 450, 500, 550, 600, 650, 700, 750, 800, 850, 900, 950, 1000, 2000, 3000, 4000, 5000, 6000, 7000, 8000, 9000, 10000, 25000, 50000, 75000, 100000, 150000, 200000, 250000, 300000, 350000, 40000, 450000, 500000, or more than 500,000 relative to sucrose (with sucrose scored as one). In some embodiments, a sugar or sugar alcohol metabolizable by the host has a degree of sweetness less than about 1 relative to sucrose (e.g., less than about 0.95, about 0.9, about 0.85, about 0.8, about 0.75, about 0.7, about 0.65, about 0.6, about 0.55, about 0.5, or less). In some embodiments, the preparation a sugar or sugar alcohol metabolizable by the host is mildly sweet, or both sweet and bitter.

In other embodiments, the rate of absorption of a sugar or sugar alcohol in the gastrointestinal tract of a subject may vary depending on the chemical structure of the sugar or sugar alcohol. For example, an absorption coefficient of a sugar or sugar alcohol may be determined, e.g., relative to glucose, to describe how readily the substance is absorbed by the subject (see, e.g., Cori, C.F. J Biol Chem (1925) 66:691-715). In some embodiments, a sugar or sugar alcohol metabolizable by the host has a low absorption coefficient relative to glucose. In some embodiments, a sugar or sugar alcohol metabolizable by the host has an absorption coefficient less than 0.15 (e.g., less than about 0.14, about 0.13, about 0.12, about 0.11, about 0.10, about 0.09, about 0.08, about 0.07, about 0.06, about 0.05, about 0.04, about 0.03, about 0.02, about 0.01, or less).

In some embodiments, the sugar or sugar alcohol may not be readily metabolizable by the subject to which it is administered (e.g., a human subject), or may be more readily absorbed in one region of the gastrointestinal tract compared with another. For example, studies by McCance et al (J Biol Chem (1930) 24:795-804) indicate that certain sugars, e.g., glucose and galactose, are readily absorbed by the gastrointestinal tract, while others, e.g., arabinose and rhamnose, are not. Sugars with prolonged or delayed absorption profiles may be termed "slowly metabolized." In some embodiments, a sugar or sugar alcohol metabolizable by the host is slowly metabolized (e.g., is metabolized more slowly than glucose). In some embodiments, the slowly metabolized sugar or slowly metabolized sugar alcohol is mannose, xylose, arabinose, xylose, rhamnose, or ribose.

In some embodiments, a substantial portion of the sugar or sugar alcohol metabolized by the host is metabolized in the lower GI tract (e.g., the small intestine or large intestine). In some embodiments, more than about 50% (w/w) of the the sugar or sugar alcohol metabolized by the host is metabolized in the lower GI tract (e.g., more than about 55%, about 60%, about 65%, about 70%, about 75%, about 80%, about 85%, about 90%, about 95%, about 99%, about 99.9%, or more). In some embodiments, the sugar or sugar alcohol metabolized by the host (e.g., a slowly metabolizable sugar or slowly metabolizable sugar alcohol) is metabolized more readily by the microbiota than the subject.

In some embodiments, the sugar or sugar alcohol described herein does not comprise a non-metabolizable sweetener. A non-metabolizable sweetener may have little to no caloric value to a subject and may be non-nutritive. In some embodiments, the non-metabolizable sweetener comprises an alkyl, alkenyl, alkynyl, aryl, heteroaryl, cycloalkyl, heterocyclyl, halogen, ester, carboxyl, acyl, thiol, amino, amido, cyano, nitro, sulfonyl, sulfate, or phosphate moiety. In some embodiments, the non-metabolizable sweetener has a high degree of sweetness relative to sucrose. In some embodiments, the non-metabolize sweetener comprises sucralose, aspartame, aspartame-acesulfame salt, advantame, stevioside, neotame, saccharin, acesulfame-K, alitame, cyclamate, neohesperidine, or rebaudioside.

In some embodiments, the microbiome regulator is a metabolizable sugar or metabolizable sugar alcohol and is recognized by a protein (e.g., an an enzyme, antibody, or a lectin (e.g., a C-type, P-type, or I-type lectin)).

In some embodiments, the enzyme comprises a glycosidase, a phosphatase, a kinase, a transferase, or a transporter. In some embodiments, the glycosidase is a glycoside hydrolase classified in one of the glycoside hydrolase families 1-128. In some embodiments, the glycosidase is a hydrolase (e.g., amylase, sucrose, lactase, or maltase). In some embodiments, the enzyme is a transferase (e.g., a glycosyltransferase, e.g., a glycosyltransferase classified in one of the glycosyltransferase families 1-98).

### Preparation of Sugar and Sugar Alcohols

In some embodiments, a microbiome regulator comprising a monosaccharide, disaccharide, trisaccharide, tetrasaccharide, or pentasaccharide is naturally occurring. In other embodiments, a microbiome regulator comprising a disaccharide, trisaccharide, tetrasaccharide, or pentasaccharide is generated using a non-enzymatic catalyst, e.g., the polymeric catalyst described in U.S. Patent No. 8,466,242, "POLYMERIC ACID CATALYSTS AND USES THEREOF" or by other suitable methods. Methods to prepare the polymeric and solid-supported catalysts described herein can be found in WO 2014/031956, "POLYMERIC AND SOLID-SUPPORTED CATALYSTS, AND METHODS OF DIGESTING CELLULOSIC MATERIALS USING SUCH CATALYSTS." Microbiome regulator comprising a disaccharide, trisaccharide, tetrasaccharide, or pentasaccharide may be generated, e.g., by using the catalyst, for example as described in WO 2016/007778, "OLIGOSACCHARIDE COMPOSITIONS AND METHODS FOR PRODUCING THEREOF."

A microbiome regulator generated through the use of a non-enzymatic catalyst (e.g., the polymeric catalyst described in U.S. Patent No. 8,466,242) may be prepared through the following steps: a) providing one or more mono- or disaccharides or a combination thereof; b) contacting the mono- or disaccharides with a polymeric catalyst (e.g., the polymeric catalyst described in U.S. Patent No. 8,466,242) and a suitable solvent (e.g. water or a non-aqueous solvent) for a period of time sufficient to a desired microbiome regulator; and c) isolating and/or recovering at least a portion of the desired microbiome regulator.

In some embodiments, a microbiome regulator prepared with a polymeric catalyst (e.g., the polymeric catalyst described in U.S. Patent No. 8,466,242) is polymolecular. In some embodiments, a microbiome regulator prepared with a polymeric catalyst (e.g., the polymeric catalyst described in U.S. Patent No. 8,466,242) is polydisperse. For example, a microbiome regulator prepared with a polymeric catalyst (e.g., the polymeric catalyst described in U.S. Patent No. 8,466,242) may comprise a mixture of distinct species (e.g. of different degree of polymerization and degree of branching and different alpha-to-beta glycosidic bond ratios). In some embodiments, the microbiome regulator prepared with a polymeric catalyst (e.g., the polymeric catalyst described in U.S. Patent No. 8,466,242) comprises a plurality of distinct species and may consist of 1x10³, 1x10⁴, 1x10⁵, 1x10⁶, 1x10⁷, 1x10⁸, 1x10⁹, 1x10¹⁰, 1x10¹¹, 1x10¹², 1x10¹³, 1x10¹⁴, or more species in various proportions to each other.

In some embodiments of the method, the starting material for the polymerization reaction is one or more monosaccharides, one or more disaccharides, or a combination thereof. In some embodiments of the method, the starting material for the polymerization reaction is one or more mono- or disaccharides selected from a tetrose, a pentose, a hexose, or a heptose. In some embodiments of the method, the starting material for the polymerization reaction is one or more of glucose, galactose, arabinose, mannose, fructose, xylose, fucose, and rhamnose, all optionally in either their L- or D-form, in alpha or beta configuration (for dimers), and/or a deoxy-form, where applicable, and any combination thereof. In some embodiments, the sugars or sugar alcohols are substituted or derivatized with one or more of an acetate ester, sulfate half-ester, phosphate ester, or a pyruvyl cyclic acetal group, or have been otherwise derivatized at, e.g., at one or more hydroxyl groups. In some embodiments, the the sugars or sugar alcohols may exist as a salt (e.g., a pharmaceutically acceptable salt), such as, e.g., a hydrochlorate, hydroiodate, hydrobromate, phosphate, sulfate, methanesulfate, acetate, formate, tartrate, malate, citrate, succinate, lactate, gluconate, pyruvate, fumarate, propionate, aspartate, glutamate, benzoate, ascorbate salt.

The sugars or sugar alcohols used in the methods described herein may be obtained from any commercially known sources, or produced according to any methods known in the art.

Generally, the polymeric catalyst and the starting materials are introduced into an interior chamber of a reactor, either concurrently or sequentially. Synthesis of a desired microbiome regulator (e.g., as described herein) can be performed in a batch process or a continuous process. For example, in one embodiment, synthesis of sugars or sugar alcohols is performed in a batch process, where the contents of the reactor are continuously mixed or blended, and all or a substantial amount of the products of the reaction are removed (e.g. isolated and/or recovered). In other embodiments, sugar or sugar alcohol synthesis is performed in a continuous process, where the contents flow through the reactor with an average continuous flow rate but with no explicit mixing.

In some embodiments, the sugar or sugar alcohol and catalyst (e.g., polymeric catalyst or solid-supported catalyst) are allowed to react for at least 1 hour, at least 2 hours, at least 3 hours, at least 4 hours, at least 6 hours, at least 8 hours, at least 16 hours, at least 24 hours, at least 36 hours, or at least 48 hours; or between 1-24 hours, between 2-12 hours, between 3-6 hours, between 1-96 hours, between 12-72 hours, or between 12-48 hours.

In some embodiments, the reaction temperature is maintained in the range of about 25°C to about 150°C. In certain embodiments, the temperature is from about 30°C to about 125°C, about 60°C to about 120°C, about 80°C to about 115°C, about 90°C to about 110°C, about 95°C to about 105°C, or about 100°C to 110°C.

The amount of the starting materials used in the methods described herein relative to the amount solvent used may affect the rate of reaction and yield. The amount of the starting material used may be characterized by the dry solids content. In certain embodiments, dry solids content refers to the total solids of a slurry as a percentage on a dry weight basis. In some embodiments, the dry solids content of the sugar or sugar alcohol is between about 5 wt% to about 95 wt %, between about 10 wt% to about 80 wt %, between about 15 wt %, to about 75 wt %, or between about 15 wt %, to about 50 wt %.

The amount of the catalyst used in the methods described herein may depend on several factors including, for example, the type and concentration of mono- or disaccharide starting material, and the reaction conditions (e.g., temperature, time, and pH).

In certain embodiments, the methods of using the catalyst are carried out in an aqueous environment, e.g., water. In some embodiments where the aqueous solvent is water, the water has less than 10% of ionic species (e.g., salts of sodium, phosphorous, ammonium, magnesium).

Moreover, as the dehydration reaction of the methods progresses, water is produced with each coupling of the one or more sugars or sugar alcohols. In certain embodiments, the methods described herein may further include monitoring the amount of water present in the reaction mixture and/or the ratio of water to monomer or catalyst over a period of time. In some embodiments, the method further includes removing at least a portion of water produced in the reaction mixture (e.g., by removing at least about any of 10%, 20%, 30%, 40%, 50%, 60%, 70%, 80%, 90%, 95%, 97%, 99%, or 100%, such as by vacuum filtration). It should be understood, however, that the amount of water to monomer may be adjusted based on the reaction conditions and specific catalyst used.

Any method known in the art may be used to remove water in the reaction mixture, including, for example, by vacuum filtration, vacuum distillation, heating, and/or evaporation. In some embodiments, the method comprises including water in the reaction mixture. Optionally, the preparation may undergo additional processing steps. Additional processing steps may include, for example, purification steps. Purification steps may include, for example, separation, dilution, concentration, filtration, desalting or ion-exchange, chromatographic separation, or decolorization, or any combination thereof.

### Microbiome Regulators: Amino Acids, Lipids, Fatty Acids, and Micronutrients

In some embodiments, preparations, pharmaceutical compositions or dosage forms (and kits comprising same) of a microbiome regulator comprise one or more (or a plurality of) amino acid and short peptides (e.g. 1-5 amino acids) thereof. In one embodiment, the amino acid is selected from the group consisting of a single amino acid, a dipeptide, a tripeptide, a tetrapeptide, and a pentapaptide.

In some embodiments, the amino acid is a D- or L-amino acid selected from the group consisting of: alanine, cysteine, aspartic acid, glutamic acid, phenylalanine, glycine, histidine, isoleucine, lysine, leucine, methionine, asparagine, proline, glutamine, arginine, serine, threonine, valine, tryptophan, and tyrosine. In some embodiments, the amino acid is taurine, L-lysine, L-proline, L-arginine, L-carnitine, or L-cysteine.

In some embodiments, the amino acid is pyrrolysine, selenocysteine, or n-formylmethionine. In some embodiments, the amino acid is α-amino-n-butyric acid, norvaline, norleucine, alloisoleucine, t-leucine, α-amino-n-heptanoic acid, pipecolic acid, α,β-diaminopropionic acid, α,γ-diaminobutyric acid, ornithine, allothreonine, homocysteine, homoserine, β-alanine, β-amino-n-butyric acid, β-aminoisobutyric acid, γ-aminobutyric acid, α-aminoisobutyric acid, isovaline, sarcosine, N-ethyl glycine, N-propyl glycine, N-isopropyl glycine, N-methyl alanine, N-ethyl alanine, N-methyl β-alanine, N-ethyl β-alanine, isoserine, or α-hydroxy-y-aminobutyric acid.

In some embodiments, the amino acid is a dipeptide, including, but not limited to carnosine (beta-alanyl-L-histidine), anserine (beta-alanyl-N-methyl histidine), homoanserine (N-(4-aminobutyryl)-L-histidine), kyotorphin (L-tyrosyl-L-arginine), balenine (beta-alanyl-N tau-methyl histidine), aspartame (N-L-α-aspartyl-L-phenylalanine 1-methyl ester), glorin (N-propionyl-γ-L-glutamyl-L-ornithine-δ-lac ethyl ester), barettin (cyclo-[(6-bromo-8-en-tryptophan)-arginine]), and glycylglycine.

In some embodiments, the amino acid is a tripeptide, including, but not limited to eisenin (pGlu-Gln-Ala-OH), GHK-Cu (glycyl-L-histidyl-L-lysine), glutathione (γ-L-Glutamyl-L-cysteinylglycine), isoleucine-proline-proline (IPP), leupeptin (N-acetyl-L-leucyl-L-leucyl-L-argininal), melanostatin (prolyl-leucyl-glycinamide), ophthalmic acid (L-γ-glutamyl-L-α-aminobutyryl-glycine), norophthalmic acid (y-glutamyl-alanyl-glycine), thyrotropin-releasing hormone (TRH, L-pyroglutamyl-L-histidinyl-L-prolinamide), and ACV (δ-(L-α-aminoadipyl)-L-Cys-D-Val).

In some embodiments, the amino acid is a tetrapeptide, including, but not limited to tuftsin (L-threonyl-L-lysyl-L-prolyl-L-arginine), rigin (glycyl-L-glutaminyl-L-prolyl-L-arginine), postin (Lys-Pro-Pro-Arg), endomorphin-1 (H-Tyr-Pro-Trp-Phe-NH2), endomorphin-2 (H-Tyr-Pro-Phe-Phe-NH2), morphiceptin (H-Tyr-Pro-Phe-Pro-NH2), gluten exorphine A4 (H-Gly-Tyr-Tyr-Pro-OH), gluten exorphine B4 (H-Tyr-Gly-Gly-Trp-OH), tyrosine-MIF-1 (H-Tyr-Pro-Leu-Gly-NH2), tetragastrin (N-((phenylmethoxy)carbonyl)-L-tryptophyl-L-methionyl-L-aspartyl-L-phenylalaninamide), kentsin (H-Thr-Pro-Arg-Lys-OH), achatin-I (glycyl-phenylalanyl-alanyl-aspartic acid), tentoxin (cyclo(N-methyl-L-alanyl-L-leucyl-N-methyl-trans-dehydrophenyl-alanyl-glycyl)), rapastinel (H-Thr-Pro-Pro-Thr-NH2), and HC-toxin, cyclo(D-Pro-L-Ala-D-Ala-L-Aeo (Aeo=2-amino-8-oxo-9,10-epoxidecanoic acid)).

In some embodiments, the amino acid is a pentapeptide.

In some embodiments, the microbiome regulator comprises at least about 1% (w/w) of an amino acid or a peptide (e.g., at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 96%, about 97%, about 98%, about 99%, or more).

In some embodiments, preparations, pharmaceutical compositions or dosage forms (and kits comprising same) of microbiome regulators comprise one or more (or a plurality of) a lipid or fatty acid. In some embodiments, the lipid is selected from the group consisting of a C1, C2, C3, C4, C5, C6, C7, C8, C9, C10, and a C18 fatty acid.

In some embodiments, the fatty acid is a short-chain fatty acid (SCFA), a medium-chain fatty acid (MCFA), a long-chain fatty acid (LCFA), or a very long chain fatty acid (VLCFA). In some embodiments, the fatty acids are saturated. In other embodiments, the fatty acids are unsaturated.

Short-chain fatty acids (SCFA) may include, e.g., acetic acid, propionic acid, butryic acid, isobutyric acid, valeric acid, and isovaleric acid. Saturated fatty acids may include, but are not limited to, propionic acid, butyric acid, valeric acid, caproic acid, enanthic acid, caprylic acid, pelargonic acid, capric acid, undecylic acid, lauric acid, tridecylic acid, myristic acid, pentadecylic acid, palmitic acid, margaric acid, stearic acid, nonadecylic acid, arachidic acid, heneicosylic acid, behenic acid, tricosylic acid, lignoceric acid, pentacosylic acid, cerotic acid, heptacosylic acid, montanic acid, nonacosylic acid, melissic acid, henatriacontylic acid, lacceroic acid, psyllic acid, geddic acid, ceroplastic acid, and hexatriacontylic acid. Unsaturated fatty acids include, but are not limited to, a) mono-unsaturated fatty acids, such as, e.g., crotonic acid, myristoleic acid, palmitoleic acid, sapienic acid, oleic acid, elaidic acid, vaccenic acid, gadoleic, eicosenoic acid, erucic acid, and nervonic acid; b) di-unsaturated fatty acids, such as, e.g., linoleic acid, eicosadienoic acid, and docosadienoic acid; c) tri-unsaturated fatty acids, such as, e.g., linolenic acid, pinolenic acid, eleostearic acid, mead acid, dihomo-γ-linolenic acid, and eicosatrienoic acid; d) tetra-unsaturated fatty acids, such as, e.g., stearidonic acid, arachidonic acid, eicosatetraenoic acid, and adrenic acid; e) pentaunsaturated fatty acids, such as, e.g., bosseopentaenoic acid, eicosapentaenoic acid, ozubondo acid, sardine acid, and tetracosanolpentaenoic acid; f) hexa-unsaturated fatty acids, such as, e.g., docosahexaenoic acid and herring acid.

In some embodiments, the fatty acid is a C1, C2, C3, C4, C5, C6, C7, C8, C9, C10, or a C18 fatty acid. Examples include: C1 acids, such as formic acid; C2 acids, such as acetic acid, oxalic acid, glyoxylic acid, glycolic acid; C3 acids, such as propionic acid, acrylic acid, malonic acid, pyruvic acid, lactic acid; C4 acids such as butyric acid, isobutyric acid, succinic acid, acetoacetic acid, fumaric acid, maleic acid, oxaloacetic acid, malic acid, tartaric acid, crotonic acid; C5 acids, such as valeric acid, glutaric acid, alpha-ketoglutaric acid; C6 acids, such as caproic acid, adipic acid, citric acid, aconitic acid, isocitric acid, sorbic acid; C7 acids, such as enanthic acid, pimelic acid, benzoic acid, salicylic acid; C8 acids, such as caprylic acid, phthalic acid; C9 acids, such as pelargonic acid, trimesic acid, cinnamic acid; C10 acids, such as capric acid, sebacic acid; C18 acids, such as stearic acid, oleic acid, linoleic acid, α-linolenic acid (ALA), γ-linolenic acid (GLA), and stearidonic acid (SDA).

In some embodiments, the microbiome regulator is a short chain fatty acid comprising acetic acid, propionic acid, butryic acid, isobutyric acid, valeric acid, isovaleric acid, hexanoic acid, or octanoic acid.

In some embodiments, the microbiome regulator comprises at least about 0.1% (w/w) of a fatty acid, e.g., a short chain fatty acid (e.g., at least about 0.5%, about 1%, about 1.5%, about 2%, about 3%, about 4%, about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 96%, about 97%, about 98%, about 99%, or more).

In some embodiments, preparations, pharmaceutical compositions or dosage forms (and kits comprising same) of microbiome regulators comprise one or more (or a plurality of) a micronutrient. In some embodiments, the micronutrient comprises a vitamin, element, or mineral (e.g., a trace mineral). In some embodiments, the micronutrient is selected from the group consisting of a trace mineral, element, choline, or a vitamin.

In some embodiments, the micronutrient is a vitamin. Vitamins suitable as a micronutrient include, but are not limited to, Vitamin B complex, Vitamin B1 (thiamin), Vitamin B2 (riboflavin), Vitamin B3 (niacin), Vitamin B5 (pantothenic acid), Vitamin B6 group (pyridoxine, pyridoxal, pyridoxamine), Vitamin B7 (biotin), Vitamin B8 (ergadenylic acid), Vitamin B9 (folic acid), Vitamin B12 (cyanocobalamin), Choline, Vitamin A (retinol), Vitamin C (ascorbic acid), Vitamin D, Vitamin E (tocopherol), Vitamin K, carotenoids (alpha carotene, beta carotene, cryptoxanthin, lutein, lycopene) and zeaxanthin.

In some embodiments, the micronutrient is an element or a trace mineral. Exemplary elements or trace minerals include, but are not limited to, boron, chloride, fluoride, sodium, calcium, magnesium, nitrogen, potassium, selenium, manganese, iron (e.g., Fe²⁺ or Fe³⁺), zinc, nickel, copper, and cobalt.

In some embodiments, the microbiome regulator comprises at least about 0.1% (w/w) of a micronutrient, e.g., a vitamin, element, or mineral (e.g., at least about 0.5%, about 1%, about 1.5%, about 2%, about 3%, about 4%, about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 96%, about 97%, about 98%, about 99%, or more).

In some embodiments, the microbiome regulator comprises a polyphenol. Polyphenols are chemical compounds or molecules that are characterized by having at least one aromatic ring with one or more hydroxyl groups. In some embodiments, the polyphenol is a flavonoid or catechin. In some embodiments, the flavonoid or catechin is selected from anthocyanins, chalcones, dihydrochalcones, dihydroflavonols, flavanols, flavanones, flavones, flavonols and isoflavonoids. In some embodiments, the polyphenol is a lignan. In some embodiments, the polyphenol is selected from alkylmethoxyphenols, alkylphenols, curcuminoids, furanocoumarins, hydroxybenzaldehydes, hydroxybenzoketones, hydroxycinnamaldehydes, hydroxycoumarins, hydroxyphenylpropenes, methoxyphenols, naphtoquinones, phenolic terpenes, and tyrosols. In some embodiments, the polyphenol is a tannin or tannic acid. In some embodiments, the polyphenol is selected from hydroxybenzoic acids, hydroxycinnamic acids, hydroxyphenylacetic acids, hydroxyphenylpropanoic acids, and hydroxyphenylpentanoic acids. In some embodiments, the polyphenol comprises a catechin, ellagitannin, isoflavone, flavonol, flavanone, anthocyanin, or lignin. In some embodiments, the polyphenol is a stilbene.

In some embodiments, the polyphenol comprises a plant polyphenol isolated from a plant source material. In some embodiments, the plant source material comprises blueberry, cranberry, grape, peach, plum, pomegranate, soy, red wine, black tea, or green tea.

In some embodiments, the micrbiome regulator comprises at least about 1% (w/w) of a polyphenol (e.g., at least about 2%, about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 96%, about 97%, about 98%, about 99%, or more).

Further, if desired, preparations, pharmaceutical compositions or dosage forms of the microbiome regulators may comprise therapeutically active agents, prebiotic substances and/or probiotic bacteria. Alternatively or in addition, therapeutically active agents, prebiotic substances and/or probiotic bacteria may be administered separately (e.g. prior to, concurrent with or after administration of the microbiome regulators) and not as a part of the preparation, pharmaceutical composition or dosage form (e.g. as a co-formulation) of microbiome regulators.

In some embodiments, microbiome regulators are administered in combination with a recommended or prescribed diet, e.g. a diet that is rich in probiotic and/or prebiotic-containing foods, such as it may be determined by a physician or other healthcare professional. Therapeutically active agents, prebiotic substances and/or probiotic bacteria may be administered to modulate the gut microbiome of the subject. In some embodiments, the combined effect (e.g. on the number or intensity of the microbial shifts) is additive. In other embodiments, the combined effect (e.g. on the number or intensity of the microbial shifts) is synergistic.

In some embodiments, the preparations, pharmaceutical compositions or dosage forms (and kits comprising same) of microbiome regulators further comprise a prebiotic substance or preparation thereof. In some embodiments, prebiotics may be administered to a subject receiving the microbiome regulators described herein. Prebiotics are substantially non-digestible substances by the host that when consumed may provide a beneficial physiological effect on the host by selectively stimulating the favorable growth or activity of a limited number of indigenous bacteria in the gut (Gibson G R, Roberfroid M B. J Nutr. (1995) 125:1401-12.). A prebiotic such as a dietary fiber or prebiotic oligosaccharide (e.g. crystalline cellulose, wheat bran, oat bran, cone fiber, soy fiber, beet fiber and the like) may further encourage the growth of probiotic and/or commensal bacteria in the gut by providing a fermentable dose of carbohydrates to the bacteria and increase the levels of those microbial populations (e.g. lactobacilli and bifidobacteria) in the gastrointestinal tract.

Prebiotics may include, but are not limited to, various galactans and carbohydrate based gums, such as psyllium, guar, carrageen, gellan, lactulose, and konjac. In some embodiments, the prebiotic is one or more of galactooligosaccharides (GOS), lactulose, raffinose, stachyose, lactosucrose, fructo-oligosaccharides (FOS, e.g. oligofructose or oligofructan), inulin, isomaltooligosaccharide, xylo-oligosaccharides (XOS), paratinose oligosaccharide, isomaltose oligosaccharides (IMOS), transgalactosylated oligosaccharides (e.g. transgalactooligosaccharides), transgalactosylate disaccharides, soybean oligosaccharides (e.g. soyoligosaccharides), chitosan oligosaccharide (chioses), gentiooligosaccharides, soy- and pectin-oligosaccharides, glucooligosaccharides, pecticoligosaccharides, palatinose polycondensates, difructose anhydride III, sorbitol, maltitol, lactitol, polyols, polydextrose, linear and branched dextrans, pullalan, hemicelluloses, reduced paratinose, cellulose, beta-glucose, beta-galactose, beta-fructose, verbascose, galactinol, xylan, inulin, chitosan, beta-glucan, guar gum, gum arabic, pectin, high sodium alginate, and lambda carrageenan, or mixtures thereof.

Prebiotics can be found in certain foods, e.g. chicory root, Jerusalem artichoke, Dandelion greens, garlic, leek, onion, asparagus, wheat bran, wheat flour, banana, milk, yogurt, sorghum, burdock, broccoli, Brussels sprouts, cabbage, cauliflower, collard greens, kale, radish and rutabaga, and miso. In some embodiments, the microbiome regulators described herein are administered to a subject in conjunction with a diet that includes foods rich in prebiotics. Suitable sources of soluble and insoluble fibers are commercially available.

In some embodiments, the composition comprising a microbiome regulator further comprises at least about 1% (w/w) of a prebiotic substance (e.g., at least about 2%, about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 96%, about 97%, about 98%, about 99%, or more).

In some embodiments, the preparations, pharmaceutical compositions or dosage forms (and kits comprising same) of a microbiome regulator further comprise a probiotic bacterium or preparation thereof, e.g., derived from bacterial cultures that are generally recognized as safe (GRAS) or known commensal or probiotic microbes. In some embodiments, to maximize the beneficial effect of endogenous commensal microbes or exogenously administered probiotic microorganisms, microbiome regulators described herein are administered to stimulate the growth and/or activity of advantageous bacteria in the GI tract. Examples of suitable probiotics include, but are not limited to, organisms classified as genera Bacteroides, Blautia, Clostridium, Fusobacterium, Eubacterium, Ruminococcus, Peptococcus, Peptostreptococcus, Akkermansia, Faecalibacterium, Roseburia, Prevotella, Bifidobacterium, Lactobacillus, Bacillus, Enterococcus, Escherichia, Streptococcus, Saccharomyces, Streptomyces, and family Christensenellaceae. Non-exclusive examples of probiotic bacteria that can be used in the methods and compositions described herein include L. acidophilus, Lactobacillus species, such as L. crispatus, L. casei, L. rhamnosus, L. reuteri, L. fermentum, L. plantarum, L. sporogenes, and L. bulgaricus, as well as Bifidobacterum species, such as B. lactis, B. animalis, B. bifidum, B. longum, B. adolescentis, and B. infantis. Yeasts, such as Saccharomyces boulardii, are also suitable as probiotics for administration to the gut, e.g. via oral dosage forms or foods. For example, yogurt is a product which already contains bacteria species, such as Lactobacillus bulgaricus and Streptococcus thermophilus.

Beneficial bacteria for the modulation of the gastrointestinal microbiota may include bacteria that produce organic acids (lactic & acetic acids) or that produce cytotoxic or cytostatic agents (to inhibit pathogenic growth), such as, e.g., hydrogen peroxide (H₂O₂) and bacteriocins. Bacteriocins are small antimicrobial peptides which can kill both closely-related bacteria, or exhibit a broader spectrum of activity (e.g., nisin). Beneficial bacteria may include one or more of the genus Akkermansia, Anaerofilum, Bacteroides, Blautia, Bifidobacterium, Butyrivibrio, Clostridium, Coprococcus, Dialister, Dorea, Fusobacterium, Eubacterium, Faecalibacterium, Lachnospira, Lactobacillus, Phascolarctobacterium, Peptococcus, Peptostreptococcus, Prevotella, Roseburia, Ruminococcus, and Streptococcus, and/or one or more of the species Akkermansia municiphilia, Christensenella minuta, Clostridium coccoides, Clostridium leptum, Clostridium scindens, Dialister invisus, Eubacterium rectal, Eubacterium eligens, Faecalibacterium prausnitzii, Streptococcus salivarius, and Streptococcus thermophilus. In some embodiments, the probiotic or commensal bacteria include one or more of the bacteria listed in Table 1.

In some embodiments, the composition comprising a microbiome regulator further comprises at least about 1% (w/w) of a probiotic bacterium or a combination thereof (e.g., at least about 2%, about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 96%, about 97%, about 98%, about 99%, or more).

The prebiotic substances and probiotic strains that may be combined with microbiome regulators to produce a composition or kit may be isolated at any level of purity by standard methods and purification can be achieved by conventional means known to those skilled in the art, such as distillation, recrystallization and chromatography. The cultivated bacteria to be used in the composition are separated from the culture broth with any method including, without limitations, centrifuging, filtration or decantation. The cells separated from the fermentation broth are optionally washed by water, saline (0.9% NaCl) or with any suitable buffer. The wet cell mass obtained may be dried by any suitable method and preferably by lyophilization.

In one embodiment, a microbiome regulator composition comprises a prebiotic and probiotic. The prebiotic substances and probiotic strains of the microbiome regulator composition described herein may be administered alone or in combination with pharmaceutically acceptable carriers or diluents, and such administration may be carried out in single or multiple doses.

In one embodiment, the composition comprises probiotics whose viability has been partially attenuated, or probiotics consisting solely of non-viable microbes. If desired, the probiotic organism can be incorporated into the microbiome regulator composition as a culture in water or another liquid or semisolid medium in which the probiotic remains viable. In another technique, a freeze-dried powder containing the probiotic organism may be incorporated into a particulate material or liquid or semisolid material by mixing or blending.

In some embodiments, the preparations, pharmaceutical compositions or dosage forms (and kits comprising same) of a microbiome regulator or combination thereof further comprise a pharmaceutically active agent or preparation thereof. In some embodiments, the pharmaceutically active agent is an antibiotic, an antifungal agent, an antiviral agent, or an anti-inflammatory agent (e.g. a cytokine, hormone, etc.). Antibiotics may include an aminoglycoside, such as amikacin, gentamicin, kanamycin, neomycin, streptomycin, and tobramycin; cephalosporins, such as cefamandole, cefazolin, cephalexin, cephaloglycin, cephaloridine, cephalothin, cephapirin, and cephradine; a macrolide, such as erythromycin and troleandomycin; penicillins, such as penicillin G, amoxicillin, ampicillin, carbenicillin, cloxacillin, dicloxacillin, methicillin, nafcillin, oxacillin, phenethicillin, and ticarcillin; a polypeptide antibiotic, such as bacitracin, colistimethate, colistin, polymyxin B; tetracyclines, such as chlortetracycline, demeclocycline, doxycycline, methacycline, minocycline, tetracycline, and oxytetracycline; a miscellaneous antibiotic such as chloramphenicol, clindamycin, cycloserine, lincomycin, rifampin, spectinomycin, vancomycin, viomycin and metronidazole; or any combination thereof.

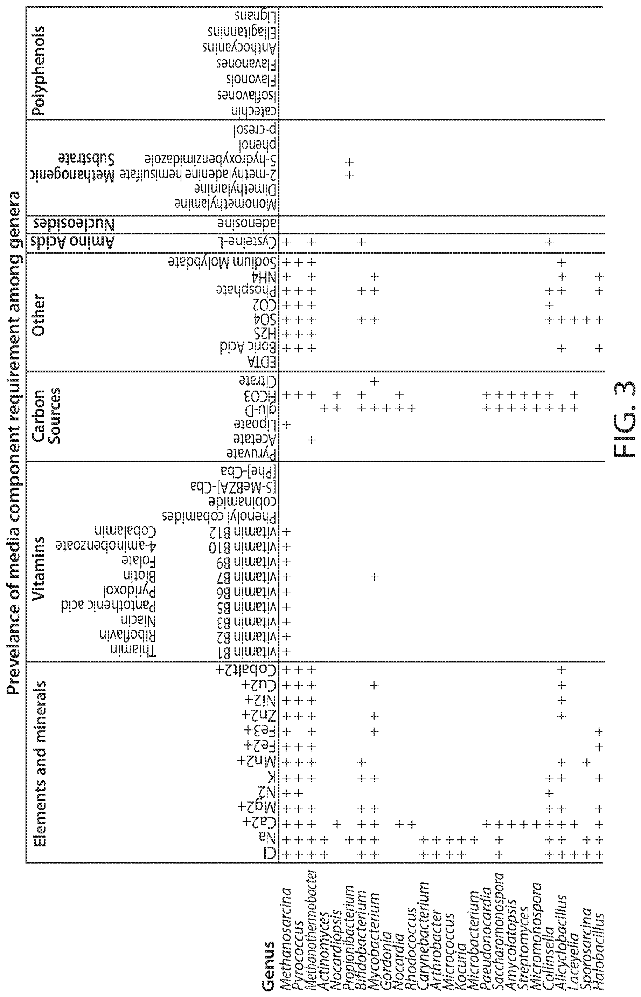

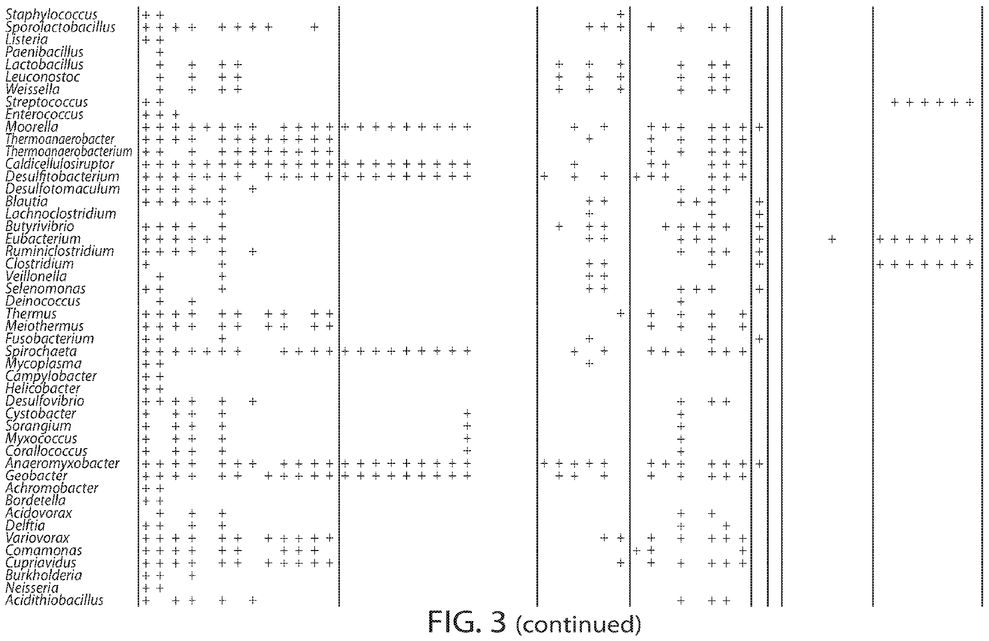

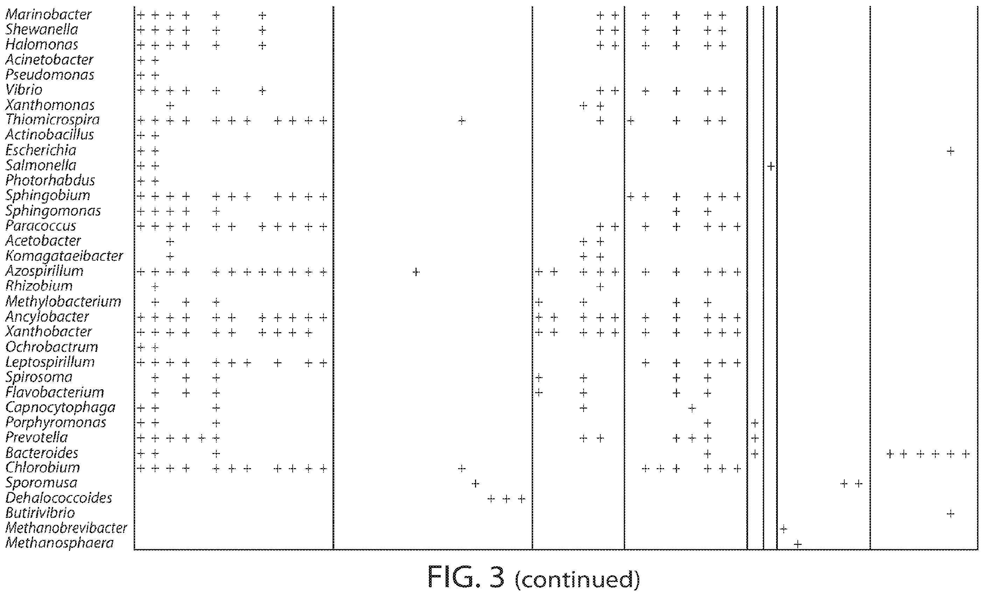

In some embodiments, the composition comprises a microbiome regulator in combination with a selected bacterial taxa, wherein the particular microbiome regulator is selected to enhance the growth or colonization of said taxa in the gastrointestinal microbiota of the host. In some embodiments, the selected bacterial taxa is a new taxa relative to the bacterial taxa present in the microbiota. In some embodiments, an additional agent is provided in combination with the microbiome regulator and bacterial taxa to enhance the growth or colonization of said taxa in the gastrointestinal microbiota of the host. In some embodiments, a plurality of microbiome regulators (e.g., at least two, at least three, or at least four) are combined with a selected bacterial taxa and another agent to enhance the growth or colonization of said taxa in the gastrointestinal microbiota of the host. Exemplary bacterial taxa may include one or more of the genus Methanosarcina, Pyrococcus, Methanothermobacter, Actinomyces, Nacardiopsis, Propionibacterium, Bifidobacterium, Mycobacterium, Gordonia, Nocardia, Rhodococcus, Corynebacterium, Arthrobacter, Micrococcus, Kocuria, Microbacterium, Psueodonocardia, Saccharomonospora, Amycolatopsis, Streptomyces, Micromonospora, Collinsella, Alicyclobacillus, Laceyella, Sporosarcina, Halobacillus, Staphylococcus, Sporolactobacillus, Listeria, Paenibacillus, Leuconostoc, Weissella, Streptococcus, Enterococcus, Moorella, Thermoanaerobacter, Thermoanaerobacterium, Caldicellulosiruptor, Desulfitobacterium, Desulfotomaculum, Blautia, Lachnoclostridium, Butyrivibrio, Eubacterium, Ruminiclostridium, Clostridium, Veillonella, Selenomonoas, Deinococcus, Thermus, Meiothermus, Fusobacterium, Spirochaeta, Mycoplasma, Campylobacter, Helicobacter, Desulfovibrio, Cystobacter, Sorangium, Myxococcus, Corrallococcus, Anaeromyxobacter, Geobacter, Achromobacter, Bordetella, Acidovorax, Delftia, Variovorax, Comamonas, Cupriavidus, Burkholderia, Neisseria, Acidithiobacillus, Marinobacter, Shewanella, Halomonas, Acinebacter, Psuedomonas, Vibrio, Xanthomonas, Thiomicrospira, Actinobacillus, Escherichia, Salmonella, Photorhabdus, Sphingobium, Sphingomonas, Paracoccus, Acetobacter, Komagataeibacter, Azospirillum, Rhizobium, Methylobacterium, Ancylobacter, Xanthobacter, Ochrobactrum, Leptospirillum, Spirosoma, Flavobacterium, Capnocytophaga, Porphyromonas, Prevotella, Bacteroides, Chlorobium, Sporomusa, Dehalococcoides, Butirivibrio, Methanobrevibacter, or Methanosphaera. Exemplary microbiome regulators may include a sugar (e.g., glucose), a vitamin (e.g., pantothenate, thiamine, riboflavin, niacin, pyridoxol, biotin, folate, 4-aminobenzoate, cobinamide, a cobamide (e.g., phenyolyl cobamide, 5-methylbenzimidazolyl cobamide), or cobalamin, or salts or derivatives thereof), an amino acid (e.g., cysteine), an element or mineral (e.g., chloride, sodium, calcium, magnesium, nitrogen, potassium, manganese, iron (e.g., Fe²⁺ or Fe³⁺), zinc, nickel, copper, or cobalt) or a polyphenol (e.g., catechin, ellagitannin, isoflavone, flavonol, flavanone, anthocyanin, or lignin). A combination of a microbiome regulator and a selected bacterial taxa may be further supplemented with a nucleoside (e.g., adenosine), a carbon source (e.g., pyruvate, acetate, lipoate, HCO₃, or citrate), ethylenediaminetetraacetic acid, boric acid, H₂S, SO₄, CO₂, phosphate, NH₄, sodium molybdate, monomethylamine, dimethylamine, 2-methyladenine hemisulfate, 5-hydroxybenzimidazole, phenol, or p-cresol. Exemplary combinations of specific microbiome regulators, bacterial taxa, and other agents are depicted in Figure 3; see also Oberhardt, M.A. at al, Nat Commun (2015) 6: 8493(http://komodo.modelseed.org); Richards, M.A. et al, PLoS One (2014) 9:e103548 (http://medialab.systemsbiology.net); Mok, K.C. and Taga, M.E. (2013) J. Bacteriol 195:1902-1911; Ti, S. et al (2012) Appl Environ Microbiol 78:7745-7752; Chaudhary, P. P. et al, (2015) Appl Microbiol Biotechnol 99:5801-5815; Anderson, P.J. et al, J Bacteriol (2008) 190:1160-1171; Tzounis, X et al, Br J Nutr (2008) 99:782-792; Selma, M.V. (2009) J Agric Food Chem 57:6485-6501; and Lagier, J.C. et al, Clin Microbiol Infect (2012) 18:1185-1193.

In some embodiments, the composition described herein comprises a microbiome regulator and is coated with a substance (e.g., a polysaccharide) that targets delivery of the composition to a specific site within the gastrointestinal tract. In some embodiments, a particular coating (e.g., a polysaccharide) may be selected to target one or more particular microbial taxa found within a certain gastrointestinal niche or region. Exemplary polysaccharides that may target delivery to a specific site within the gastrointestinal tract include amylose, arabinogalactan, carrageenan, chitosan, chondroitin sulfate, dextran, furcelleran, galactomannan, glucomannan, gellan gum, hyaluronic acid, Karaya gum (sterculia gum), locust bean gum, scleroglucan, pullalan, or xylan. Further details regarding targeting mechanisms and selected bacterial taxa that may be applied to a composition comprising a microbiome regulator described herein can be found in Jain, A. et al (J Pharm Pharmaceut Sci (2007) 10:86-128).

In some embodiments, the composition described herein does not comprise an active agent other than a microbiome regulator. Exemplary active agents other than a microbiome regulator may include, but are not limited to, a therapeutic agent (e.g., an agent with pharmaceutical activity not directed to regulation of the microbiome), a carrier, a filler, an excipient, a binder, a film foaming agent, a solubilizing agent, a tastant, a lyophilizing agent, a stabilizer, a hydrophilizer, an emulsifier, an adhesive, or a toxicity reducer. In some embodiments, if included in the composition, these agents are not considered active agents, do not comprise a microbiome regulator, and are outside the scope of the present invention. In some embodiments, a composition described herein (e.g., a composition comprising a microbiome regulator) comprises less than about 50% (w/w) of an agent other than the microbiome regulator (e.g., less than about 40%, about 30%, about 25%, about 20%, about 15%, about 10%, about 5%, about 2.5%, about 2%, about 1%, about 0.5%, about 0.1%, about 0.05%, or less). In some embodiments, the ratio (w/w) of a microbiome regulator to an agent other than a microbiome regulator is greater than about 1: 1 (e.g., about 1.1:1, about 1.2:1, about 1.3:1, about 1.4:1, about 1.5:1, about 1.75:1, about 2:1, about 2.25:1, about 2.5:1, about 2.75:1, about 3:1, about 4:1, about 5:1, about 6:1, about 7:1, about 8:1, about 9:1, about 10:1, about 12.5:1, about 15:1, about 20:1, about 30:1, about 40:1, about 50:1, about 100:1, about 500:1, about 1000:1, or more). In some embodiments, the composition is substantially free of an agent other than a microbiome regulator.

In some embodiments, the agent other than the microbiome regulator is a therapeutic agent. An exemplary agent may comprise a peptide, nucleic acid, oligosaccharide, polysaccharide, protein, non-peptide small molecule, a secondary metabolite, or a prodrug or metabolite thereof. In some embodiments, the composition described herein is substantially free of a therapeutic agent.

In some embodiments, the therapeutic agent comprises a molecule with a molecular weight greater than about 200 g/mol (e.g., greater than about 250 g/mol, about 300 g/mol, about 350 g/mol, about 400 g/mol, about 500 g/mol, about 600 g/mol, about 700 g/mol, about 800 g/mol, about 900 g/mol, about 1000 g/mol, about 1100 g/mol, about 1200 g/mol, about 1300 g/mol, about 1400 g/mol, about 1500 g/mol, about 2000 g/mol, or more).

In some embodiments, the therapeutic agent comprises a molecule having more than about 6 carbon atoms (e.g., about 7 carbon atoms, about 8 carbon atoms, about 9 carbon atoms, about 10 carbon atoms, about 12 carbon atoms, about 15 carbon atoms, about 20 carbon atoms, about 24 carbon atoms, about 30 carbon atoms, or more). **In** other embodiments, the therapeutic agent comprises a molecule having more than about 6 carbon atoms (e.g., about 7 carbon atoms, about 8 carbon atoms, about 9 carbon atoms, about 10 carbon atoms, about 12 carbon atoms, about 15 carbon atoms, about 20 carbon atoms, about 24 carbon atoms, or about 30 carbon atoms, or more) and more than about 6 heteroatoms (e.g., about 7 carbon atoms, about 8 carbon atoms, about 9 carbon atoms, about 10 carbon atoms, about 12 carbon atoms, about 15 carbon atoms, about 20 carbon atoms, about 24 carbon atoms, about 30 carbon atoms, or more), wherein the heteroatom is selected from oxygen, nitrogen, sulfur, or phosphorus. **In** still other embodiments, the therapeutic agent comprises a molecule having more than about 6 carbon atoms (e.g., about 7 carbon atoms, about 8 carbon atoms, about 9 carbon atoms, about 10 carbon atoms, about 12 carbon atoms, about 15 carbon atoms, about 20 carbon atoms, about 24 carbon atoms, or about 30 carbon atoms, or more) and more than about 6 oxygen atoms (e.g., about 7 oxygen atoms, about 8 oxygen atoms, about 9 oxygen atoms, about 10 oxygen atoms, about 12 oxygen atoms, about 15 oxygen atoms, about 20 oxygen atoms, about 24 oxygen atoms, about 30 oxygen atoms, or more).

In some embodiments, the therapeutic agent is characterized by the specific target in the subject (e.g., human subject). In some embodiments, the therapeutic agent has a specificity for a cell surface receptor, an ion channel, a transporter, an enzyme, an antibody, or other biological target. In other embodiments, the therapeutic agent is used in the treatment or prevention of a disease, disorder, or condition, e.g., an inflammatory disease, infectious disease, metabolic disease, or neurodegenerative disease. Exemplary diseases, disorders, or conditions that the therapeutic agent may be used to treat or prevent include, but are not limited to, cancer, diabetes, cardiovascular disease, a fibrotic disease, or a microbial infection (e.g., a bacterial, fungal, or viral infection).

In some embodiments, the therapeutic agent is a microbiocide (e.g., an antibiotic, antifungal, or antiviral agent). In some embodiments, the therapeutic agent is an FDA approved drug substance.

In other embodiments, the agent other than the microbiome regulator is a carrier, filler, or excipient (e.g., a polymer). In some embodiments, the polymer is synthetic or naturally occurring. In some embodiments, the polymer comprises polyethylene glycol (PEG), polypropylene glycol (PPG), polyvinyl pyrrolidine (PVG), polyvinyl alcohol (PVA), polyacrylic acid (PAA), polyacrylamide, N-(2-hydroxypropyl) methylacrylamide (HMPA), divinyl ether-maleic anhydride (DIVEMA), polyoxazolines, polyphosphates, xanthan gum, pectin, chitin, chitosan, dextran, carrageenan, guar gum, cellulose (e.g., hydroxypropylmethyl cellulose (HPMC), hydroxypropyl cellulose (HPC), hydroxyethylcellulose (HEC), sodium carboxymethyl cellulose (NaCMC)), hyaluronic acid, hyaluronan, albumin, heparin, chondroitin, starch, or derivatives thereof. In some embodiments, the composition is substantially free of a polymer (e.g., a polymer described herein).

In some embodiments, the agent other than the microbiome regulator is a binder, film foaming agent, solubilizing agent, tastant, lyophilizing agent, stabilizer, hydrophilizer, emulsifier, adhesive, or toxicity reducer.

Tables 1-4 below summarize exemplary microbial taxa and metabolites thereof that may be targeted or modulated by features of the invention.

**Table 1: Genus level microbial constituents of the GI tract.**

| Phylum | Class | Genus |
|---|---|---|
| Actinobacteria | Actinobacteria | Actinomyces, Adlercreutzia, Atopobium, Bifidobacterium, Collinsella, Corynebacterium, Eggerthella, Mobiluncus, Propionibacterium, Rothia, Slackia |
| Bacteroidetes | Bacteroidia | Alistipes, Bacteroides, Dysgonomonas, Odoribacter, Parabacteroides, Porphyromonas, Prevotella, Tannerella |
| | Flavobacteria | Capnocytophaga |
| Firmicutes | Bacilli | Bacillus, Enterococcus, Gemella, Granulicatella, Lactobacillus, Lactococcus, Staphylococcus, Streptococcus, Turicibacter, Weissella |
| | Clostridia | Acidaminococcus, Anaerococcus, Anaerofilum, Anaerofustis, Anaerostipes, Anaerotruncus, Anaerovorax, Bacteroides, Bacteroides, Blautia, Clostridium, Coprococcus, Dehalobacterium, Dialister, Dorea, Eubacterium, Faecalibacterium, Finegoldia, Lachnobacterium, Lachnospira, Megamonas, Megasphaera, Mitsuokella, Moryella, Oribacterium, Oscillospira, Peptococcus, Peptoniphilus, Peptostreptococcus, Phascolarctobacterium, Pseudobutyrivibrio, Roseburia, Ruminococcus, Ruminococcus, Selenomonas, Subdoligranulum, Veillonella |
| Fusobacteria | Fusobacteria | Fusobacterium, Leptotrichia |
| | Betaproteobacteria | Comamonas, Herbaspirillum, Lautropia, Neisseria, Oxalobacter, Sutterella |
| | Deltaproteobacteria | Bilophila, Desulfovibrio, LE30 |
| | Epsilonproteobacteria | Campylobacter, Helicobacter |
| | Gammaproteobacteria | Actinobacillus, Aggregatibacter, Citrobacter, Escherichia, Haemophilus, Klebsiella, Moraxella, Pseudomonas, Raoultella |
| Spirochaetes | Spirochaetes | Treponema |
| Synergistetes | Synergistetia | Cloacibacillus, Synergistes |
| Teneri cute s | Erysipelotrichi | Bulleidia, Catenibacterium, Clostridium, Coprobacillus, Holdemania, RFN20 |
| | Mollicutes | Asteroleplasma, Mycoplasma |
| Verrucomicrobia | Verrucomicrobiae | Akkermansia |
| Euryarchaeota | Methanobacteria | Methanobrevibacter |

**Table 2: Microbial metabolites**

| |
|---|
| 2-hydroxyisobutyrate, 3-hydroxyisovalerate, 3-methyl- crotonylglycine, 3-methylcrotonylglycine, allantoin, betaine, formate, mannitol, p-cresol glucuronide, phenylacetylglycine, sarcosine, taurine, acetic acid, acetylaldehyde, ascorbic acid, butanedione, butyric acid, deoxycholic acid, ethylphenyl sulfate, formic acid / formate, indole, isobutyric acid, isovaleric acid, propionic acid, serotonin, succinic acid / succinate, TMAO, tryptophan, valeric acid, ursodeoxycholic acid, lactate, lactic acid, hydrogen peroxide |

**Table 3: Genus level microbial constituents predominant in the large intestine (compared to small intestine) in healthy humans.**

| Phylum | Class | Genus |
|---|---|---|
| Bacteroidetes | Bacteroidia | Bacteroides, Butyricimonas, Odoribacter, Parabacteroides, Prevotella |
| Firmicutes | Clostridia | Anaerotruncus, Phascolarctobacterium, Ruminococcus, |
| Proteobacteria | Deltaproteobacteria | Bilophila |
| Verrucomicrobia | Verrucomicrobiae | Akkermansia |

**Table 4: Genus level microbial constituents predominant in the small intestine (compared to large intestine) in healthy humans.**

| Phylum | Class | Genus |
|---|---|---|
| Actinobacteria | Actinobacteria | Cryocola, Mycobacterium |
| Firmicutes | Bacilli | Enterococcus, Lactococcus, Streptococcus, Turicibacter |
| | Clostridia | Blautia, Coprococcus, Holdemania, Pseudoramibacter Eubacterium |
| Proteobacteria | Alphaproteobacteria | Agrobacterium, Sphingomonas |
| | Betaproteobacteria | Achromobacter, Burkholderia, Ralstonia |

Tables 5-6 below summarize exemplary microbiome regulators and selected characteristics thereof.

**Table 5. Exemplary Microbiome Regulators**

| Microbiome Regulator | Compound |
|---|---|
| Sugar | glucose (5-1), galactose (5-2), N-acetylglucosamine (5-3), N-acetylgalactosamine (5-4), fructose (5-5), fucose (5-6), mannose (5-7), N-acetylmannosamine (5-8), glucuronic acid (5-9), N-acetylglucuronic acid (5-10), galactosuronic acid (5-11), N-acetylgalactosuronic acid (5-12), xylose (5-13), arabinose (5-14), rhamnose (5-15), ribose (5-16), sucrose (5-17), sorbose (5-18), lactose (5-19), maltose (5-20), lactulose (5-21), tagatose (5-22), kojibiose (5-23), nigerose (5-24), isomaltose (5-25), trehalose (5-26), sophorose (5-27), laminaribiose (5-28), gentiobiose (5-29), turanose (5-30), maltulose (5-31), palatinose (5-32), gentiobiulose (5-33), mannobiose (5-34), melibiulose (5-35), rutinulose (5-36), xylobiose (5-37) |
| Sugar Alcohol | sorbitol (5-40), mannitol (5-41), lactitol (5-42), erythritol (5-43), glycerol (5-44), arabitol (5-45), maltitol (5-46), xylitol (5-47), ribitol (5-48), threitol (5-49), galactitol (5-50), fucitol (5-51), iditol (5-52), inositol (5-53) |
| Amino Acid | alanine (5-60), arginine (5-61), asparagine (5-62), aspartic acid (5-63), cysteine (5-64), glutamic acid (5-65), glutamine (5-66), glycine (5-67), histidine (5-68), isoleucine (5-69), leucine (5-70), lysine (5-71), methionine (5-72), phenylalanine (5-73), proline (5-74), serine (5-75), threonine (5-76), tryptophan (5-77), tyrosine (5-78), valine (5-79) |
| Vitamin | pantothenate (5-80), thiamine (5-81), riboflavin (5-82), niacin (5-83), pyridoxol (5-84), biotin (5-85), folate (5-86), 4-aminobenzoate (5-87), cobinamide (5-88), phenyolyl cobamide (5-89), 5-methylbenzimidazolyl cobamide (5-90), cobalamin (5-91), pyridoxine (5-92), pyridoxamine (5-93), ergadenylic acid (5-94), cyanocobalamin (5-95), choline (5-96), retinol (5-97), a carotenoid (5-98), zeaxanthin (5-99) |
| Element/Mineral | chloride (5-100) , sodium (5-101), calcium (5-102), magnesium (5-103), nitrogen (5-104), potassium (5-105), manganese (5-106), iron (5-107), zinc (5-108), nickel (5-109), copper (5-110), cobalt (5-111) |
| Fatty Acid | acetic acid (5-120), propionic acid (5-121), butryic acid (5-122), isobutyric acid (5-123), valeric acid (5-124), isovaleric acid (5-125), hexanoic acid (5-126), octanoic acid (5-126), formic acid (5-127), oxalic acid (5-128), glyoxylic acid (5-129), glycolic acid (5-130), acrylic acid (5-131), malonic acid (5-132), pyruvic acid (5-133), lactic acid (5-134), succinic acid (5-135), acetoacetic acid (5-136), fumaric acid (5-137), maleic acid (5-138), oxaloacetic acid (5-139), malic acid (5-140), tartaric acid (5-141), crotonic acid (5-142), glutaric acid (5-143), alpha-ketoglutaric acid (5-144), caproic acid (5-145), adipic acid (5-146), citric acid (5-147), aconitic acid (5-148), isocitric acid (5-149), sorbic acid (5-150), enanthic acid (5-151), pimelic acid (5-152), benzoic acid (5-153), salicylic acid (5-154), caprylic acid (5-155), phthalic acid (5-156), pelargonic acid (5-157), trimesic acid (5-158), cinnamic acid (5-159), capric acid (5-160), sebacic acid (5-161), stearic acid (5-162), oleic acid (5-163), linoleic acid (5-164), α-linolenic acid (5-165), γ-linolenic acid (5-166), stearidonic acid (5-167) |
| Polyphenol | catechin (5-170), ellagitannin (5-171), isoflavone (5-172), flavonol (5-173), flavanone (5-174), anthocyanin (5-175), lignin (5-176), alkylmethoxyphenol (5-177), alkylphenol (5-178), curcuminoid (5-179), furanocoumarin (5-180), hydroxybenzaldehyde (5-181), hydroxybenzoketone (5-182), hydroxycinnamaldehyde (5-183), hydroxycoumarin (5-184), hydroxyphenylpropene (5-185), methoxyphenol (5-186), naphtoquinone (5-187), phenolic terpenes (5-188), tyrosols (5-189) |

**Table 6. Exemplary Characteristics of Microbiome Regulators and Compositions Thereof**

| Characteristic of Microbiome Regulator | Compound |
|---|---|
| Molecular Weight | less than about 2000 g/mol (6-1), less than about 1750 g/mol (6-2), less than about 1500 g/mol (6-3), less than about 1250 g/mol (6-4), less than about 1000 g/mol (6-5), less than about 950 g/mol (6-6), less than about 900 g/mol (6-7), less than about 850 g/mol (6-8), less than about 800 g/mol (6-9), less than about 750 g/mol (6-10), less than about 700 g/mol (6-11), less than about 650 g/mol (6-12), less than about 600 g/mol (6-13), less than about 500 g/mol (6-14), less than about 450 g/mol (6-15), less than about 400 g/mol (6-16), less than about 350 g/mol (6-17), less than about 300 g/mol (6-18), less than about 250 g/mol (6-19), less than about 200 g/mol (6-20) |
| Number of Carbon Atoms | less than 30 carbon atoms, (6-21), less than 25 carbon atoms (6-22), less than 20 carbon atoms (6-23), less than 18 carbon atoms (6-24), less than 15 carbon atoms (6-25), less than 12 carbon atoms (6-26), less than 10 carbon atoms (6-27), less than 9 carbon atoms (6-28), less than 8 carbon atoms (6-29), less than 7 carbon atoms (6-30), less than 6 carbon atoms (6-31), less than 5 carbon atoms (6-32), less than 4 carbon atoms (6-33), less than 3 carbon atoms (6-34), less than 2 carbon atoms (6-35) |
| Number of Heteroatoms¹ | less than 30 heteroatoms, (6-40), less than 25 heteroatoms (6-41), less than 20 heteroatoms (6-42), less than 18 heteroatoms (6-43), less than 15 heteroatoms (6-44), less than 12 heteroatoms (6-45), less than 10 heteroatoms (6-46), less than 9 heteroatoms (6-47), less than 8 heteroatoms (6-48), less than 7 heteroatoms (6-49), less than 6 heteroatoms (6-50), less than 5 heteroatoms (6-51), less than 4 heteroatoms (6-52), less than 3 heteroatoms (6-53), less than 2 heteroatoms (6-54), less than 1 heteroatom (6-55), 0 heteroatoms (6-56) |
| Chemical Moiety | alkyl (6-60), alkenyl (6-61), alkynyl (6-62), aryl (6-63), heteroaryl (6-64), cycloalkyl (6-65), heterocyclyl (6-66), halogen (6-67), ester (6-68), amino (6-69), amido (6-70), thiol (6-71), cyano (6-72), nitro (6-73), sulfonyl (6-74), |
| Percent Weight in Composition (% w/w) | less than about 99% (6-80), less than about 95% (6-81), less than about 90% (6-82), less than about 85% (6-83), less than about 80% (6-84), less than about 75% (6-85), less than about 70% (6-86), less than about 65% (6-87), less than about 60% (6-88), less than about 55% (6-89), less than about 50% (6-90), less than about 45% (6-91), less than about 40% (6-92), less than about 35% (6-93), less than about 30% (6-94), less than about 25% (6-95), less than about 20% (6-96), less than about 15% (6-97), less than about 10% (6-98), less than about 5% (6-99), less than about 2.5% (6-100), less than about 2% (6-101), less than about 1% (6-102), less than about 0.5% (6-103), less than about 0.1% (6-104), less than about 0.05% (6-105) |
| Relative Degree of Sweetness² | less than about 30,000 (6-110), less than about 20,000 (6-111), less than about 15,000 (6-112), less than about 10,000 (6-113), less than about 7,500 (6-114), less than about 5,000 (6-115), less than about 2,500 (6-116), less than about 1,000 (6-117), less than about 750 (6-118), less than about 500 (6-119), less than about 250 (6-120), less than about 100 (6-121), less than about 75 (6-122), less than about 50 (6-123), less than about 25 (6-124), less than about 10 (6-125), less than about 5 (6-126), less than about 2.5 (6-127), less than about 1 (6-128), less than about 0.95 (6-129), less than about 0.9 (6-130), less than about 0.85 (6-131), less than about 0.8 (6-132), less than about 0.75 (6-133), less than about 0.7 (6-134), less than about 0.65 (6-135), less than about 0.6 (6-136), less than about 0.55 (6-137), less than about 0.5 (6-138) |
| Primary Location of Metabolism by Host | stomach (6-140), duodenum (6-141), jejunum (6-142), ileum (6-143), cecum (6-144), ascending colon (6-145), traverse colon (6-146), descending colon (6-147), sigmoid colon (6-148), rectum (6-149) |
| Therapeutic Agent | a peptide (6-150), nucleic acid (6-151), oligosaccharide (6-152), polysaccharide (6-153), protein (6-154), non-peptide small molecule (6-155) |
| Polymer | polyethylene glycol (6-160), polypropylene glycol (6-161), polyvinyl pyrrolidine (6-162), polyvinyl alcohol (6-163), polyacrylic acid (6-164), polyacrylamide, N-(2-hydroxypropyl) methylacrylamide (6-165), divinyl ether-maleic anhydride (6-166), polyoxazolines (6-167), polyphosphates (6-168), xanthan gum (6-169), pectin (6-170), chitin (6-171), chitosan (6-172), dextran (6-173), carrageenan (6-174), guar gum (6-175), cellulose (6-176), hydroxypropylmethyl cellulose (6-177), hydroxypropyl cellulose (6-178), hydroxyethylcellulose (6-179), sodium carboxymethyl cellulose (6-180), hyaluronic acid (6-181), hyaluronan (6-182), albumin (6-183), heparin (6-184), chondroitin (6-185), starch (6-186) |
| Target Specificity | a cell surface receptor (6-190), an ion channel (6-191), a transporter (6-192), an enzyme (6-193), an antibody (6-194), a metabolite (6-195), an amino acid (6-196), a peptide (6-197), a nucleic acid (6-198) |

| | |
|---|---|
| ¹Heteroatoms include oxygen, sulfur, nitrogen, and phosphorus ²Relative degree of sweetness is compared to sucrose, which has a degree of sweetness equal to 1 | |

In some embodiments, a composition described herein comprises at least one of a microbiome regulator recited in Table 5, e.g., to modulate a bacterial taxa recited in Tables 1, 3, or 4. In some embodiments, a composition described herein comprises at least two, at least three, at least four, at least five, or more of a microbiome regulator recited in Table 5, e.g., to modulate a bacterial taxa recited in Tables 1, 3, or 4.

In some embodiments, a composition described herein comprises at least one of a microbiome regulator recited in Table 5, e.g., to prevent or treat a dysbiosis, e.g., a dysbiosis of the gastrointestinal microbiota of a subject. In some embodiments, a composition described herein comprises at least two, at least three, at least four, at least five, or more of a microbiome regulator recited in Table 5, e.g., to prevent or treat a dysbiosis, e.g., a dysbiosis of the gastrointestinal microbiota of a subject. In some embodiments, the dysbiosis is associated with at least of the bacterial taxa recited in Tables 1, 3, or 4. In some embodiments, the dysbiosis is associated with at least two, at least three, at least four, at least five, or more of a bacterial taxa recited in Tables 1, 3, or 4. In some embodiments, the dysbiosis is associated with a disease, disorder, or condition described herein (e.g., an infectious disease, an inflammatory disease, a metabolic disease, an autoimmune disease, a neurological disease, or cancer).

In some embodiments, a composition described herein comprises a microbiome regulator recited in Table 5. In some embodiments, the microbiome regulator is one or more of a sugar selected from 5-1 through 5-37. In some embodiments, the microbiome regulator is one or more of a sugar alcohol selected from 5-40 through 5-53. In some embodiments, the microbiome regulator is one or more of an amino acid selected from 5-60 through 5-79. In some embodiments, the microbiome regulator is one or more of a vitamin selected from 5-80 through 5-99. In some embodiments, the microbiome regulator is one or more of an element or mineral selected from 5-100 through 5-111. In some embodiments, the microbiome regulator is one or more of a fatty acid selected from 5-120 through 5-167. In some embodiments, the microbiome regulator is one or more of a polyphenol selected from 5-170 through 5-189.

In some embodiments, a composition described herein comprises at least two microbiome regulators recited in Table 5, e.g., at least two, at least three, or at least four microbiome regulators recited in Table 5. In some embodiments, the at least two (e.g., at least three or at least four) microbiome regulators comprise a sugar selected from 5-1 through 5-37. In some embodiments, the at least two (e.g., at least three or at least four) microbiome regulators comprise a sugar alcohol selected from 5-40 through 5-53. In some embodiments, the at least two (e.g., at least three or at least four) microbiome regulators comprise an amino acid selected from 5-60 through 5-79. In some embodiments, the at least two (e.g., at least three or at least four) microbiome regulators comprise a vitamin selected from 5-80 through 5-99. In some embodiments, the at least two (e.g., at least three or at least four) microbiome regulators comprise an element or mineral selected from 5-100 through 5-111. In some embodiments, the at least two (e.g., at least three or at least four) microbiome regulators comprise a fatty acid selected from 5-120 through 5-167. In some embodiments, the at least two (e.g., at least three or at least four) microbiome regulators comprise a polyphenol selected from 5-170 through 5-189.

In some embodiments, the composition comprises one or more of a sugar (e.g., a sugar selected from 5-1 to 5-37) and one or more of a sugar alcohol (e.g., a sugar alcohol selected from 5-40 to 5-53). In some embodiments, the composition comprises one or more of a sugar (e.g., a sugar selected from 5-1 to 5-37) and one or more of an amino acid (e.g., an amino acid selected from 5-60 to 5-79). In some embodiments, the composition comprises one or more of a sugar (e.g., a sugar selected from 5-1 to 5-37) and one or more of a vitamin, element, or mineral (e.g., a vitamin selected from 5-80 to 5-99 or an element or mineral selected from 5-100 to 5-111). In some embodiments, the composition comprises one or more of a sugar (e.g., a sugar selected from 5-1 to 5-37) and one or more of a fatty acid (e.g., a fatty acid selected from 5-120 to 5-167). In some embodiments, the composition comprises one or more of a sugar (e.g., a sugar selected from 5-1 to 5-37) and one or more of a polyphenol (e.g., polyphenol selected from 5-170 to 5-189).

In some embodiments, the microbiome regulator comprises a characteristic or feature recited in Table 6. In some embodiments, the microbiome regulator is a sugar (e.g., a sugar selected from 5-1 to 5-37) and has a molecular weight recited in Table 6 (e.g., a molecular weight of 6-1 to 6-20). In some embodiments, the microbiome regulator is a sugar (e.g., a sugar selected from 5-1 to 5-37) and has a number of carbon atoms recited in Table 6 (e.g., a number of carbon atoms of 6-21 to 6-35). In some embodiments, the microbiome regulator is a sugar (e.g., a sugar selected from 5-1 to 5-37) and has a number of heteroatoms recited in Table 6 (e.g., a number of heteroatoms of 6-40 to 6-56). In some embodiments, the microbiome regulator is a sugar (e.g., a sugar selected from 5-1 to 5-37) and does not feature a chemical moiety recited in Table 6 (e.g., a chemical moiety of 6-60 to 6-74). In some embodiments, the microbiome regulator is a sugar (e.g., a sugar selected from 5-1 to 5-37) and is present in a composition described herein in a percent weight (% w/w) recited in Table 6 (e.g., a percent weight (% w/w) of 6-80 to 6-105). In some embodiments, the microbiome regulator is a sugar (e.g., a sugar selected from 5-1 to 5-37) and has a relative degree of sweetness recited in Table 6 (e.g., a relative degree of sweetness of 6-110 to 6-138). In some embodiments, the microbiome regulator is a sugar (e.g., a sugar selected from 5-1 to 5-37) and is primarily metabolized in one or more specific regions of the gastrointestinal tract of the host recited in Table 6 (e.g., primary locations of metabolism of 6-140 to 6-149).

In some embodiments, the microbiome regulator is a sugar (e.g., a sugar selected from 5-1 to 5-37) and is present in a composition that does not further comprise a therapeutic agent recited in Table 6 (e.g., a therapeutic agent of 6-150 to 6-155). In some embodiments, the microbiome regulator is a sugar (e.g., a sugar selected from 5-1 to 5-37) and is present in a composition that does not further comprise a therapeutic agent recited in Table 6 (e.g., a therapeutic agent of 6-150 to 6-155) in an percent weight (% w/w) recited in Table 6 (e.g., a percent weight (% w/w) of 6-80 to 6-105). In some embodiments, the therapeutic agent (e.g., a therapeutic agent of 6-150 to 6-155) does not feature a chemical moiety recited in Table 6 (e.g., a chemical moiety of 6-60 to 6-74). In some embodiments, the therapeutic agent (e.g., a therapeutic agent of 6-150 to 6-155) does not feature target a compound or substance recited in Table 6 (e.g., a target of 6-190 to 6-198). In some embodiments, the microbiome regulator is a sugar (e.g., a sugar selected from 5-1 to 5-37) and is present in a composition that does not further comprise a polymer recited in Table 6 (e.g., a polymer of 6-160 to 6-186). In some embodiments, the microbiome regulator is a sugar (e.g., a sugar selected from 5-1 to 5-37) and is present in a composition that does not further comprise a polymer recited in Table 6 (e.g., a polymer of 6-160 to 6-186) in an percent weight (% w/w) recited in Table 6 (e.g., a percent weight (% w/w) of 6-80 to 6-105).

In some embodiments, the microbiome regulator is a sugar alcohol (e.g., a sugar alcohol selected from 5-40 to 5-53) and has a molecular weight recited in Table 6 (e.g., a molecular weight of 6-1 to 6-20). In some embodiments, the microbiome regulator a sugar alcohol (e.g., a sugar alcohol selected from 5-40 to 5-53) and has a number of carbon atoms recited in Table 6 (e.g., a number of carbon atoms of 6-21 to 6-35). In some embodiments, the microbiome regulator is a sugar alcohol (e.g., a sugar alcohol selected from 5-40 to 5-53) and has a number of heteroatoms recited in Table 6 (e.g., a number of heteroatoms of 6-40 to 6-56). In some embodiments, the microbiome regulator a sugar alcohol (e.g., a sugar alcohol selected from 5-40 to 5-53) and does not feature a chemical moiety recited in Table 6 (e.g., a chemical moiety of 6-60 to 6-74). In some embodiments, the microbiome regulator is a sugar alcohol (e.g., a sugar alcohol selected from 5-40 to 5-53) and is present in a composition described herein in a percent weight (% w/w) recited in Table 6 (e.g., a percent weight (% w/w) of 6-80 to 6-105). In some embodiments, the microbiome regulator is a sugar alcohol (e.g., a sugar alcohol selected from 5-40 to 5-53) and has a relative degree of sweetness recited in Table 6 (e.g., a relative degree of sweetness of 6-110 to 6-138). In some embodiments, the microbiome regulator is a sugar alcohol (e.g., a sugar alcohol selected from 5-40 to 5-53) and is primarily metabolized in one or more specific regions of the gastrointestinal tract of the host recited in Table 6 (e.g., primary locations of metabolism of 6-140 to 6-149).

In some embodiments, the microbiome regulator is a sugar alcohol (e.g., a sugar alcohol selected from 5-40 to 5-53) and is present in a composition that does not further comprise a therapeutic agent recited in Table 6 (e.g., a therapeutic agent of 6-150 to 6-155). In some embodiments, the microbiome regulator is a sugar alcohol (e.g., a sugar alcohol selected from 5-40 to 5-53) and is present in a composition that does not further comprise a therapeutic agent recited in Table 6 (e.g., a therapeutic agent of 6-150 to 6-155) in an percent weight (% w/w) recited in Table 6 (e.g., a percent weight (% w/w) of 6-80 to 6-105). In some embodiments, the therapeutic agent (e.g., a therapeutic agent of 6-150 to 6-155) does not feature a chemical moiety recited in Table 6 (e.g., a chemical moiety of 6-60 to 6-74). In some embodiments, the therapeutic agent (e.g., a therapeutic agent of 6-150 to 6-155) does not feature target a compound or substance recited in Table 6 (e.g., a target of 6-190 to 6-198). In some embodiments, the microbiome regulator is a sugar alcohol (e.g., a sugar alcohol selected from 5-40 to 5-53) and is present in a composition that does not further comprise a polymer recited in Table 6 (e.g., a polymer of 6-160 to 6-186). In some embodiments, the microbiome regulator is a sugar alcohol (e.g., a sugar alcohol selected from 5-40 to 5-53) and is present in a composition that does not further comprise a polymer recited in Table 6 (e.g., a polymer of 6-160 to 6-186) in an percent weight (% w/w) recited in Table 6 (e.g., a percent weight (% w/w) of 6-80 to 6-105).

In some disclosures, the microbiome regulator is an amino acid (e.g., an amino acid selected from 5-60 to 5-79) and has a molecular weight recited in Table 6 (e.g., a molecular weight of 6-1 to 6-20). In some disclosures, the microbiome regulator is an amino acid (e.g., an amino acid selected from 5-60 to 5-79) and is present in a composition described herein in a percent weight (% w/w) recited in Table 6 (e.g., a percent weight (% w/w) of 6-80 to 6-105). In some disclosures, the microbiome regulator is an amino acid (e.g., an amino acid selected from 5-60 to 5-79) and is present in a composition that does not further comprise a therapeutic agent recited in Table 6 (e.g., a therapeutic agent of 6-150 to 6-155). In some disclosures, the microbiome regulator is an amino acid (e.g., an amino acid selected from 5-60 to 5-79) and is present in a composition that does not further comprise a therapeutic agent recited in Table 6 (e.g., a therapeutic agent of 6-150 to 6-155) in an percent weight (% w/w) recited in Table 6 (e.g., a percent weight (% w/w) of 6-80 to 6-105). In some disclosures, the microbiome regulator is an amino acid (e.g., an amino acid selected from 5-60 to 5-79) and is present in a composition that does not further comprise a polymer recited in Table 6 (e.g., a polymer of 6-160 to 6-186). In some disclosures, the microbiome regulator is an amino acid (e.g., an amino acid selected from 5-60 to 5-79) and is present in a composition that does not further comprise a polymer recited in Table 6 (e.g., a polymer of 6-160 to 6-186) in an percent weight (% w/w) recited in Table 6 (e.g., a percent weight (% w/w) of 6-80 to 6-105).

In some disclosures, the microbiome regulator is a vitamin, element, or mineral (e.g., a vitamin selected from 5-80 to 5-99 or an element or mineral selected from 5-100 to 5-111) and has a molecular weight recited in Table 6 (e.g., a molecular weight of 6-1 to 6-20). In some disclosures, the microbiome regulator is a vitamin, element, or mineral (e.g., a vitamin selected from 5-80 to 5-99 or an element or mineral selected from 5-100 to 5-111) and is present in a composition described herein in a percent weight (% w/w) recited in Table 6 (e.g., a percent weight (% w/w) of 6-80 to 6-105). In some disclosures, the microbiome regulator is a vitamin, element, or mineral (e.g., a vitamin selected from 5-80 to 5-99 or an element or mineral selected from 5-100 to 5-111) and is present in a composition that does not further comprise a therapeutic agent recited in Table 6 (e.g., a therapeutic agent of 6-150 to 6-155). In some disclosures, the microbiome regulator is a vitamin, element, or mineral (e.g., a vitamin selected from 5-80 to 5-99 or an element or mineral selected from 5-100 to 5-111) and is present in a composition that does not further comprise a therapeutic agent recited in Table 6 (e.g., a therapeutic agent of 6-150 to 6-155) in an percent weight (% w/w) recited in Table 6 (e.g., a percent weight (% w/w) of 6-80 to 6-105). In some disclosures, the microbiome regulator is a vitamin, element, or mineral (e.g., a vitamin selected from 5-80 to 5-99 or an element or mineral selected from 5-100 to 5-111) and is present in a composition that does not further comprise a polymer recited in Table 6 (e.g., a polymer of 6-160 to 6-186). In some disclosures, the microbiome regulator is a vitamin, element, or mineral (e.g., a vitamin selected from 5-80 to 5-99 or an element or mineral selected from 5-100 to 5-111) and is present in a composition that does not further comprise a polymer recited in Table 6 (e.g., a polymer of 6-160 to 6-186) in an percent weight (% w/w) recited in Table 6 (e.g., a percent weight (% w/w) of 6-80 to 6-105).

In some disclosures, the microbiome regulator is a fatty acid (e.g., a fatty acid selected from 5-120 to 5-167) and has a molecular weight recited in Table 6 (e.g., a molecular weight of 6-1 to 6-20). In some disclosures, the microbiome regulator is a fatty acid (e.g., a fatty acid selected from 5-120 to 5-167) and is present in a composition described herein in a percent weight (% w/w) recited in Table 6 (e.g., a percent weight (% w/w) of 6-80 to 6-105). In some disclosures, the microbiome regulator is a fatty acid (e.g., a fatty acid selected from 5-120 to 5-167) and is present in a composition that does not further comprise a therapeutic agent recited in Table 6 (e.g., a therapeutic agent of 6-150 to 6-155). In some disclosures, the microbiome regulator is a fatty acid (e.g., a fatty acid selected from 5-120 to 5-167) and is present in a composition that does not further comprise a therapeutic agent recited in Table 6 (e.g., a therapeutic agent of 6-150 to 6-155) in an percent weight (% w/w) recited in Table 6 (e.g., a percent weight (% w/w) of 6-80 to 6-105). In some disclosures, the microbiome regulator is a fatty acid (e.g., a fatty acid selected from 5-120 to 5-167) and is present in a composition that does not further comprise a polymer recited in Table 6 (e.g., a polymer of 6-160 to 6-186). In some disclosures, the microbiome regulator is a fatty acid (e.g., a fatty acid selected from 5-120 to 5-167) and is present in a composition that does not further comprise a polymer recited in Table 6 (e.g., a polymer of 6-160 to 6-186) in an percent weight (% w/w) recited in Table 6 (e.g., a percent weight (% w/w) of 6-80 to 6-105).

In some disclosures, the microbiome regulator is a polyphenol (e.g., a polyphenol selected from 5-170 to 5-189) and has a molecular weight recited in Table 6 (e.g., a molecular weight of 6-1 to 6-20). In some disclosures, the microbiome regulator is a polyphenol (e.g., a polyphenol selected from 5-170 to 5-189) and is present in a composition described herein in a percent weight (% w/w) recited in Table 6 (e.g., a percent weight (% w/w) of 6-80 to 6-105). In some disclosures, the microbiome regulator is a polyphenol (e.g., a polyphenol selected from 5-170 to 5-189) and is present in a composition that does not further comprise a therapeutic agent recited in Table 6 (e.g., a therapeutic agent of 6-150 to 6-155). In some disclosures, the microbiome regulator is a polyphenol (e.g., a polyphenol selected from 5-170 to 5-189) and is present in a composition that does not further comprise a therapeutic agent recited in Table 6 (e.g., a therapeutic agent of 6-150 to 6-155) in an percent weight (% w/w) recited in Table 6 (e.g., a percent weight (% w/w) of 6-80 to 6-105). In some disclosures, the microbiome regulator is a polyphenol (e.g., a polyphenol selected from 5-170 to 5-189) and is present in a composition that does not further comprise a polymer recited in Table 6 (e.g., a polymer of 6-160 to 6-186). In some disclosures, the microbiome regulator is a polyphenol (e.g., a polyphenol selected from 5-170 to 5-189) and is present in a composition that does not further comprise a polymer recited in Table 6 (e.g., a polymer of 6-160 to 6-186) in an percent weight (% w/w) recited in Table 6 (e.g., a percent weight (% w/w) of 6-80 to 6-105).

### Methods of Modulating Microbial Taxa

The compounds and compositions provided herein may be used in methods to modulate a bacterial taxa (e.g. 1, 2, 3, 4, 5 or more taxa) present in the microbiota of a subject. In some embodiments, modulation comprises a change in the structure of the microbiota, such as a change in the relative composition of a taxa or a change in the relative abundance of a taxa, e.g., relative to another taxa or relative to what would be observed in the absence of the modulation. In other embodiments, modulation comprises a change in a function of the microbiota, such as a change in gene expression, level of a gene product (e.g., RNA or protein), or metabolic output of the microbiota, or a change in a functional pathway of the host (e.g, a change in gene expression, level of a gene product, or metabolic output of a host cell or host process).

The methods describe herein include administering to the human subject a microbiome regulator or a pharmaceutical composition thereof in an amount effective to modulate taxa. In some embodiments, the abundance of a bacterial taxa may increase relative to other taxa (or relative from one point in time to another) when the microbiome regulator is administered and the increase can be at least a 5%, 10%, 25% 50%, 75%, 100%, 250%, 500%, 750% increase or at least a 1000% increase. The abundance of a bacterial taxa may also decrease relative to other taxa (or relative from one point in time to another) when the microbiome regulator is administered and the decrease can be at least a 5%, 10%, 25% 50%, 75%, 85%, 90%, 95%, 96%, 97%, 98%, 99% decrease, or at least a 99.9% decrease. In some embodiments, a dysbiosis has shifted the microbiota and has increased one or more non-desired taxa and/or increased one or more desired taxa. Administration of the microbiome regulator can modulate the abundance of the desired and/or non-desired bacterial taxa in the subject's gastrointestinal microbiota, thereby treating the dysbiosis.

In some embodiments, the microbiome regulator is capable of modulating (e.g. increasing or decreasing) the growth of one or more bacterium, such as, e.g., those that belong to genera Bacteroides, Odoribacter, Parabacteroides, Alistipes, Blautia, Clostridium, Coprococcus, Dorea, Eubacterium, Lachnospira, Roseburia, Ruminococcus, Faecalibacterium, Oscillospira, and Subdoligranulum which can be found in the GI tract. In some embodiments, the microbiome regulator is capable of modulating (e.g. increasing or decreasing) the growth of one or more bacterium, such as, e.g., those that are thought to be associated with a healthy gastrointestinal state, such as, for example, one or more of the genus Akkermansia, Anaerofilum, Bacteroides, Blautia, Bifidobacterium, Butyrivibrio, Clostridium, Coprococcus , Dialister, Dorea, Fusobacterium, Eubacterium, Faecalibacterium, Lachnospira, Lactobacillus, Phascolarctobacterium, Peptococcus, Peptostreptococcus, Prevotella, Roseburia, Ruminococcus, and Streptococcus, and/or one or more of the species Akkermansia municiphilia, Christensenella minuta, Clostridium coccoides, Clostridium leptum, Clostridium scindens, Dialister invisus, Eubacterium rectal, Eubacterium eligens, Faecalibacterium prausnitzii, Streptococcus salivarius, and Streptococcus thermophilus.

In some embodiments, the microbiome regulator is capable of modulating (e.g. increasing or decreasing) the growth of at least two bacterial taxa selected from Prevotella, Akkermansia, Bacteroides, Clostridium (Erysipelotrichaceae), Clostridium (Clostridiaceae), Bifidobacterium, Aggregatibacter, Clostridium (Peptostreptococcaveae), Parabacteroides, Lactobacillus, and Enterococcus. In some embodiments, the microbiome regulator is capable of modulating the growth of the two bacterial taxa: Akkermensia and Blautia.

In some embodiments, a microbiome regulator or a composition thereof drives selective changes in both the composition and activity (or function) of the gastrointestinal microbiota, thereby conferring health benefits upon the host. In some embodiments, the microbiome regulator is a selective substrate for one or a limited number of potentially beneficial bacteria that reside in the GI tract, stimulating their growth and/or metabolic activity. In some embodiments, the microbiome regulator is capable of altering the composition of gastrointestinal microbiota to a composition higher or lower in specific bacteria. In some embodiments, the microbiome regulator stimulates the growth and/or selective activity of gastrointestinal bacteria associated with health and well-being. In one example, the microbiome regulator compositions described herein decrease the abundance or relative number or density of a pathogenic bacterium.

The relationship between microbiota and their host is not merely commensal (a non-harmful coexistence), but in many cases a symbiotic relationship. Though subjects can survive without microbiota, the microorganisms perform a variety of useful functions, such as fermenting unused energy substrates, training the immune system, preventing growth of pathogenic bacteria, regulating the development of the gut, producing vitamins for the host (such as biotin and vitamins) (See, e.g., Dominguez-Bello M G and Blaser M J, 2008 Microbes Infect, 10(9): 1072-1076). Common gastrointestinal bacterial taxa include genera Bacteroides, Odoribacter, Parabacteroides, Alistipes, Blautia, Clostridium, Coprococcus, Dorea, Eubacterium, Lachnospira, Roseburia, Ruminococcus, Faecalibacterium, Oscillospira, and Subdoligranulum. Some bacterial genera and species are thought to be associated with a healthy state of the GI tract, such as, e.g., the genus Akkermansia, Anaerofilum, Bacteroides, Blautia, Bifidobacterium, Butyrivibrio, Clostridium, Coprococcus , Dialister, Dorea, Fusobacterium, Eubacterium, Faecalibacterium, Lachnospira, Lactobacillus, Phascolarctobacterium, Peptococcus, Peptostreptococcus, Prevotella, Roseburia, Ruminococcus, and Streptococcus, and/or the species Akkermansia municiphilia, Christensenella minuta, Clostridium coccoides, Clostridium leptum, Clostridium scindens, Dialister invisus, Eubacterium rectal, Eubacterium eligens, Faecalibacterium prausnitzii, Streptococcus salivarius, and Streptococcus thermophilus.

However, in certain conditions, pathogenic species and pathobionts which are capable of causing disease, e.g. by inducing an infection and/or inflammation and/or bacteria associated with a disease state without necessarily being a causative agent, are present in the niche. In some embodiments, disease-associated bacteria, pathobionts or pathogens that may be modulated by the microbiome regulators described herein are selected from the group consisting of the genera Bilophila, Campylobacter, Candidatus, Citrobacter, Clostridium, Collinsella, Desulfovibrio, Enterobacter, Enterococcus, Escherichia, Fusobacterium, Haemophilus, Klebsiella, Lachnospiraceae, Peptostreptococcus, Porphyromonas, Portiera, Providencia, Pseudomonas, Salmonella, Shigella, Staphylococcus, Streptococcus, Vibrio, and Yersinia.

In some embodiments, disease-associated bacteria, pathobionts or pathogens that may be modulated by a microbiome regulator described herein are selected from the group consisting of the species Bilophila wadsworthia, Campylobacter jejuni, Citrobacter farmer, Clostridium difficile, Clostridium perfringens, Clostridium tetani, Collinsella aerofaciens, Enterobacter hormaechei, Enterococcus faecalis, Enterococcus faecium, Escherichia coli, Fusobacterium varium, Fusobacterium nucleatum, Haemophilus parainfluenzae, Klebsiella pneumonia, Peptostreptococcus stomatis, Porphyromonas asaccharolytica, Pseudomonas aeruginosa, Salmonella bongori, Salmonella enteric, Shigella boydii, Shigella dysenteriae, Shigella flexneri, Shigella sonnei, Staphylococcus aureus, Streptococcus infantarius, Vibrio cholera, and Yersinia enterocolitica.

In some embodiments, disease-associated bacteria, pathobionts or pathogens that may be modulated by a microbiome regulator described herein may reside predominantly in one or more specific regions of the GI tract. For example, the following disease-associated bacteria, pathobionts and pathogens reside predominantly in the large intestine (colon): Listeria, Entamoeba histolytica, Balantidium coli, Basidiobolus ranarum, Trypanosoma cruzi, Clostridium botulinum, Fasciola hepatica, Histoplasma capsulatum, Rotavirus, Schistosoma mansoni, Schistosoma japonicum, and Schistosoma mekongi, Shigella, Brachyspira aalborgi, Serpulina pilosicoli, Trichuris trichiura, and Yersinia enterocolitica. The following disease-associated bacteria, pathobionts and pathogens reside predominantly in the small intestine: Vibrio, Yersinia enterocolitica, Yersinia pseudotuberculosis, Clostridium perfringens, Capillaria philippinensis, Cryptosporidium parvum, Cyclospora cayetanensis and CMV virus. The following disease-associated bacteria, pathobionts and pathogens reside predominantly in the large and small intestine: Campylobacter and Salmonella. In another example, the following disease-associated bacteria, pathobionts and pathogens reside predominantly in the stomach: CMV virus, Bacillus anthracis, Candida, Cryptosporidium, EBV (Epstein-Barr virus), Giardia lamblia, Helicobacter pylori, Helicobacter felis, Helicobacter fennelliae, Helicobacter cinaedi, Mycobacterium avium, Herpes varicella zoster, Histoplasma, and Toxoplasma.

A healthy microbial community protects the host, e.g., by enhancing the intestinal barrier, by competitive exclusion of potential pathogens or disease-associated bacteria, and by growth inhibition of bacterial pathogens and disease-associated bacteria. A healthy bacterial community may exert direct antibacterial effects on pathogens and disease-associated bacteria through production of antibacterial substances, including bacteriocins and acid (Cotter P D, et al. 2005 Nat Rev, 3:777-788; Servin A L, 2004 FEMS Microbiol Rev, 28: 405-440). The antibacterial substances exert their effects alone or synergistically to inhibit the growth of pathogens or disease-associated bacteria. A healthy bacterial community may decrease adhesion of both pathogens and their toxins to gastrointestinal lining.

In some embodiments, a microbiome regulator or composition thereof modulates (e.g. increases or decreases) the growth of one or more bacterial taxa residing in the GI tract, such as, e.g., those that belong to genera Bacteroides, Odoribacter, Parabacteroides, Alistipes, Blautia, Clostridium, Coprococcus, Dorea, Eubacterium, Lachnospira, Roseburia, Ruminococcus, Faecalibacterium, Oscillospira, and Subdoligranulum which can be found in the GI tract. In some embodiments, a microbiome regulator or composition thereof modulates (e.g. increases or decreases) the growth of one or more bacterial taxa, such as those that are thought to be associated with a healthy gastrointestinal state, e.g., one or more of the genus Akkermansia, Anaerofilum, Bacteroides, Blautia, Bifidobacterium, Butyrivibrio, Clostridium, Coprococcus , Dialister, Dorea, Fusobacterium, Eubacterium, Faecalibacterium, Lachnospira, Lactobacillus, Phascolarctobacterium, Peptococcus, Peptostreptococcus, Prevotella, Roseburia, Ruminococcus, and Streptococcus, and/or one or more of the species Akkermansia municiphilia, Christensenella minuta, Clostridium coccoides, Clostridium leptum, Clostridium scindens, Dialister invisus, Eubacterium rectal, Eubacterium eligens, Faecalibacterium prausnitzii, Streptococcus salivarius, and Streptococcus thermophilus. In some embodiments, the microbiome regulator modulates (e.g. increases or decreases) the growth of one or more bacterial taxa, such as taxa of the phylum Verrucomicrobia, e.g., those of the genus Akkermansia.

In some embodiments, a microbiome regulator or composition thereof modulates (e.g. increases or decreases) the growth of one or more bacterial taxa predominantly residing in the small intestine. For example, the microbiome regulator modulates one or more (2, 3, 4, 5, 6, 7, 8, 9, 10 or more) bacterial taxa that reside predominantly in the small intestine, such as, e.g. Actinobacteria, Firmicutes (Bacilli, Clostridia), and Proteobacteria (Alphaproteobacteria, Betaproteobacteria). In some embodiments, a microbiome regulator or composition thereof modulates one or more (2, 3, 4, 5, 6, 7, 8, 9, 10 or more) bacterial taxa that reside predominantly in the small intestine selected from the genera: Cryocola, Mycobacterium, Enterococcus, Lactococcus, Streptococcus, Turicibacter, Blautia, Coprococcus, Holdemania, Pseudoramibacter Eubacterium, Agrobacterium, Sphingomonas, Achromobacter, Burkholderia, and Ralstonia.

In some embodiments, a microbiome regulator or composition thereof modulates (e.g. increases or decreases) the growth of one or more bacterial taxa predominantly residing in the large intestine. For example, a microbiome regulator or composition thereof modulates one or more (2, 3, 4, 5, 6, 7, 8, 9, 10 or more) bacterial taxa that reside predominantly in the large intestine, such as, e.g. Bacteroidetes, Firmicutes (Clostridia), Verrucomicrobia, and Proteobacteria (Deltaproteobacteria). In some embodiments, a microbiome regulator or composition thereof modulates one or more (2, 3, 4, 5, 6, 7, 8, 9, 10 or more) bacterial taxa that reside predominantly in the large intestine selected from the genera: Bacteroides, Butyricimonas, Odoribacter, Parabacteroides, Prevotella, Anaerotruncus, Phascolarctobacterium, Ruminococcus, Bilophila, and Akkermansia.

In some embodiments, the microbiome regulator modulates (e.g. increases or decreases) the growth of one or more bacterial taxa predominantly residing in the cecum, such as, e.g. Actinobacteria, Bacteroides, Bacilli, Clostridia, Mollicutes, Alpha Proteobacteria, and Verrucomicrobia.

In some embodiments, a microbiome regulator or composition thereof modulates (e.g. increases or decreases) the growth of one or more bacterial taxa predominantly residing in the ascending colon, such as, e.g. Actinobacteria, Bacteroides, Bacilli, Clostridia, Fusobacteria, Beta Proteobacteria, Delta/Epsilon Proteobacteria, Gamma Proteobacteria, and Verrucomicrobia. In some embodiments, the microbiome regulator modulates (e.g. increases or decreases) the growth of one or more bacterial taxa predominantly residing in the traverse colon, such as, e.g. Actinobacteria, Bacteroides, Clostridia, Mollicutes, Fusobacteria, and Gamma Proteobacteria.

In some embodiments, a microbiome regulator or composition thereof modulates (e.g. increases or decreases) the growth of one or more bacterial taxa predominantly residing in the descending colon, such as, e.g. Bacteroides, Clostridia, Mollicutes, Fusobacteria, Delta/Epsilon Proteobacteria and Verrucomicrobia.

In some embodiments, a microbiome regulator or composition thereof modulates (e.g. increases or decreases) the growth of one or more bacterial taxa predominantly residing in the sigmoid colon, such as, e.g. Actinobacteria, Bacteroides, Bacilli, Clostridia, Mollicutes, Alpha Proteobacteria, Beta Proteobacteria, and Verrucomicrobia.

In some embodiments, a microbiome regulator or composition thereof modulates (e.g. increases or decreases) the growth of one or more bacterial taxa predominantly residing in the rectum, such as, e.g. Bacteroides, Clostridia, Mollicutes, Alpha Proteobacteria, Gamma Proteobacteria, and Verrucomicrobia.

In some embodiments, a microbiome regulator or composition thereof modulates (e.g. stimulate/increase or suppress/decrease) the growth of one or more (e.g. 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 80, 90, 100, 150, 200, or more than 200) endogenous commensal microbial taxa or exogenously administered probiotic bacterial taxa of various genera including, e.g. Alistipes, Akkermansia, Anaerofilum, Bacteroides, Blautia, Bifidobacterium, Butyrivibrio, Clostridium, Coprococcus , Dialister, Dorea, Fusobacterium, Eubacterium, Faecalibacterium, Lachnospira, Lactobacillus, , Odoribacter, Oscillospira, Parabacteroides, Phascolarctobacterium, Peptococcus, Peptostreptococcus, Prevotella, Roseburia, Ruminococcus, and Streptococcus and Subdoligranulum.

In some embodiments, a microbiome regulator or composition thereof modulates (e.g. stimulate/increase or suppress/decrease) the growth of one or more (e.g. 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 80, 90, 100, 150, 200, or more than 200) endogenous commensal or symbiotic microbial taxa or exogenously administered probiotic bacterial taxa of various genera including, but not limited to, bacterial taxa selected from the group consisting of genera Akkermansia, Anaerofilum, Bacteroides, Blautia, Bifidobacterium, Butyrivibrio, Clostridium, Coprococcus , Dialister, Dorea, Fusobacterium, Eubacterium, Faecalibacterium, Lachnospira, Lactobacillus, Phascolarctobacterium, Peptococcus, Peptostreptococcus, Prevotella, Roseburia, Ruminococcus, and Streptococcus and of the species Akkermansia municiphilia, Christensenella minuta, Clostridium coccoides, Clostridium leptum, Clostridium scindens, Dialister invisus, Eubacterium rectal, Eubacterium eligens, Faecalibacterium prausnitzii, Streptococcus salivarius, and Streptococcus thermophilus thought to be associated with gastrointestinal health may be modulated by the microbiome regulators described herein.

In some embodiments, a microbiome regulator or composition thereof modulates (e.g. substantially increase or substantially decrease) the growth (and the total number) of (or substantially increase or substantially decrease the relative representation in the total gastrointestinal community) of one or more of (e.g. 1, 2, 3, 4, 5, 6, 7, 8, 9, 10, 11, 12, 13, 14, 15, 16, 17, 18, 19, 20, 25, 30, 35, 40, 45, 50, or more than 50) the genus, species, or phylogenetic clade listed in Table 1. Table 1 provides a genus level list of microbial constituents of the GI tract.

In some embodiments, a microbiome regulator or composition thereof substantially increases the growth, e.g. the total number or the relative representation in the total gastrointestinal community, the community of the large intestine or the community of the small intestine of one or more of (e.g. 1, 2, 3, 4, 5, 6, 7, 8, 9, 10, 11, 12, 13, 14, 15, 16, 17, 18, 19, 20, 25, 30, 35, 40, 45, 50, or more than 50) of the OTU, genus, species, or phylogenetic clade listed in Table 1, 3, and 4.

In some embodiments, a microbiome regulator or composition thereof substantially decreases the growth, e.g. the total number or the relative representation in the total gastrointestinal community, the community of the large intestine or the community of the small intestine of one or more of (e.g. 1, 2, 3, 4, 5, 6, 7, 8, 9, 10, 11, 12, 13, 14, 15, 16, 17, 18, 19, 20, 25, 30, 35, 40, 45, 50, or more than 50) of the OTU, genus, species, or phylogenetic clade listed in Table 1, 3, and 4.

In some embodiments, a microbiome regulator or composition thereof substantially increases and decreases the growth, e.g. the total number or the relative representation in the total gastrointestinal community, the community of the large intestine or the community of the small intestine of one or more of (e.g. 1, 2, 3, 4, 5, 6, 7, 8, 9, 10, 11, 12, 13, 14, 15, 16, 17, 18, 19, 20, 25, 30, 35, 40, 45, 50, or more than 50) of the OTU, genus, species, or phylogenetic clade listed in Table 1, 3, and 4.

In some embodiments, provided herein are microbiome regulators and compositions thereof that are substrates only for a selected group bacteria that are capable of utilizing the microbiome regulator as a food source. The breakdown of the microbiome regulatorthen exerts beneficial effects on the health of the host. The beneficial health effects are due to a selective stimulation of the growth and/or biological activity of a selected number of microbial genera, species, or strains in the gastrointestinal microbiota that are capable of utilizing the microbiome regulator as a food source and confer health benefits to the host. The effects of the microbiome regulator, in certain embodiments, are due to selective stimulation of the growth of the beneficial bacteria in the GI tract. Such increases and decreases in the abundance of certain taxa may be sufficient to "normalize" the resident microbiota, e.g. to reinstate a healthy state or equilibrium. Increase or decrease is with respect to the ratio present in the human subject prior to ingestion of the pharmaceutical microbiome regulator composition, or to a control group not taking the pharmaceutical microbiome regulator composition. The prebiotic index (PI) can be used as a proxy for effects of the microbiome regulators described herein. PI relates to the sum of: (Bifidobacteria/total bacteria) + (Lactobacilli/total bacteria) - (Bacteroides/total bacteria) - (Clostridia/total bacteria), (see Palframan et al, 2003, Lett Appl Microbiol 37:281-284). In some embodiments, the ratio of Eubacterium rectale/total bacteria may also be considered. Eubacterium rectale produces butyrate which is advantageous for the gut barrier in adults.

For example, the stimulation of growth of certain bacterial taxa may reduce the pH of the colon, increase the production of short chain fatty acids, prevent the proliferation and adhesion of pathogenic microorganisms (barrier effect), increase the metabolism of potentially carcinogenic aminated compounds, and/or increase the production of vitamins.

In some embodiments, provided herein are microbiome regulators and compositions thereof that can be digested by the microbiota (e.g. by carbohydrate fermentation) without certain side effects or with a substantial reduction of symptoms of fermentation, such as increased gas formation that may cause flatulence, discomfort, and/or bloating.

In certain embodiments, the ratio of certain bacterial taxa or their relative abundance may be shifted. Such shifts may be measured with respect to the ratio present in the subject prior to administration of the pharmaceutical microbiome regulator composition, or to a control group not taking the pharmaceutical microbiome regulator composition.

In some embodiments, the microbiome regulator is a selective substrate for one or a limited number of potentially beneficial bacterial taxa that reside in the GI tract, stimulating their growth and/or metabolic activity. In some embodiments, the microbiome regulator is capable of altering the composition of gastrointestinal microbiota to a composition higher or lower in specific bacterial taxa. In some embodiments, the microbiome regulator selectively stimulates the growth and/or selective activity of gastrointestinal bacterial taxa associated with health and well-being.

Methods are provided that comprise administering to a subject in need thereof a pharmaceutical microbiome regulator composition in an amount effective to modulate microbial diversity. In some embodiments, administration of the microbiome regulator modulates (e.g. increases or decreases) microbial diversity in the GI tract (or specifically in the large intestine or the small intestine) of a human subject. The diversity may increase or decrease when an effective amount of the microbiome regulator is administered.

In some embodiments, a microbiome regulator increases diversity. In some embodiments, a microbiome regulator decreases diversity. In some embodiments, a dysbiosis has shifted the microbiota and has increased or decreased the microbial diversity such that a disturbed state is reached. Administration of the microbiome regulator can modulate the microbial diversity, thereby treating the dysbiosis. In some embodiments, the microbial diversity is decreased and the abundance of one or more, two or more, three or more, or four or more bacterial taxa is increased, including Akkermansia, Blautia, Bacteroides, Bifidobacterium Lactobacillus, and Parabacteroides.

Microbial diversity can be measured by any suitable method known in the art, including analysis of 16S rDNA sequences described herein. Diversity can be expressed, e.g. using the Shannon Diversity index (Shannon entropy), number of observed OTUs, Chao1 index, etc. In some embodiments, the microbiome regulator modulates (e.g. increase or decrease) diversity within a microbial community, e.g. that of the GI tract, which may be expressed using Shannon entropy as a measure.

In some embodiments, the microbiome regulator increases microbial diversity and associated Shannon entropy by 0.0001%, 0.0005%, 0.001%, 0.005%, 0.01%, 0.05%, 0.1%, 0.5%, 1%, 5%, 10%, 50%, 100%, 500%, 1000%, 5000%, or 10000%. In some embodiments, the microbiome regulator increases microbial diversity and associated Shannon entropy by (log) 1-fold, 2-fold, 3-fold, 4-fold, 5-fold, 6-fold, 7-fold, 8-fold, 9-fold, 10-fold, 20-fold, 30-fold, 40-fold, 50-fold, 60-fold, 70-fold, 80-fold, 90-fold, 100-fold, or more. In some embodiments, the microbiome regulator decreases microbial diversity and associated Shannon entropy by 0.0001%, 0.0005%, 0.001%, 0.005%, 0.01%, 0.05%, 0.1%, 0.5%, 1%, 5%, 10%, 20%, 30%, 40%, 50%, 60%, 70%, 80%, 90%, or 99% or more. In some embodiments, the microbiome regulator decreases microbial diversity and associated Shannon entropy by (log) 1-fold, 2-fold, 2-fold, 3-fold, 4-fold, 5-fold, 6-fold, 7-fold, 8-fold, 9-fold, 10-fold, 20-fold, 30-fold, 40-fold, 50-fold, 60-fold, 70-fold, 80-fold, 90-fold, 100-fold, or more.

In some embodiments, the microbiome regulator increases microbial diversity and associated Shannon entropy by at least 1%, 2%, 3%, 4%, 5%, 10%, 15% 20%, 25%, 30%, 35%, 40%, 45%, or by at least 50%. In some embodiments, the microbiome regulator increases microbial diversity and associated Shannon entropy by at least (log) 0.2-fold, 0.3-fold, 0.4-fold, 0.5-fold, 0.8-fold, 1-fold, 1.2-fold, 1.5-fold, 1.8-fold, or at least 2-fold.

In some embodiments, the microbiome regulator decreases microbial diversity and associated Shannon entropy by at least 1%, 2%, 3%, 4%, 5%, 10%, 15% 20%, 25%, 30%, 35%, 40%, 45%, 50%, 55%, 60%, 65%, 70%, or by at least 75%. In some embodiments, the microbiome regulator decreases microbial diversity and associated Shannon entropy by at least (log) 0.2-fold, 0.3-fold, 0.4-fold, 0.5-fold, 0.8-fold, 1-fold, 1.2-fold, 1.5-fold, 1.8-fold, 2-fold, 3-fold, 4-fold, or at least 5-fold.

Some methods described herein include the administration of a microbiome regulator or composition thereof to modulate the host's immune functions and intestinal epithelial cell functions. A microbiome regulator may upregulate the immune function, e.g. to improve the ability of the host to fight infections, while downregulation of immune function may prevent the onset of allergy or intestinal inflammation. Modulated beneficial bacteria may stimulate intestinal epithelial cell responses, including restitution of damaged epithelial barrier, production of antibacterial substances and cell-protective proteins, and blocking of cytokine-induced intestinal epithelial cell apoptosis.

Bacteria can elicit both pro- and anti-inflammatory responses from host (mammalian) cells, and different bacterial species can elicit different host responses. In one embodiment, microbiome regulators are used to alter the bacterial population to elicit a desired host response. The host response may be modulated via direct interactions with the bacterial population or via indirect interactions via secreted or shed bacterial products (e.g., short-chain fatty acids). A microbiome regulator may alter the bacterial population such that the bacterial population, upon either direct or indirect interaction with host cells, stimulates the production of antimicrobial peptides (AMPs), or modulates (i.e., increases or decreases the production of) inflammatory and immunomodulatory cytokines including interleukin-1α (IL-1α), IL-1β, IL-2, IL-4, IL-6, IL-8, IL-10, IL-12, IL-13, IL-17A, IL-17F, IL-22, IL-23, tumor necrosis factor (TNF), chemokine (C-C motif) ligand 5 (CCL5, also known as RANTES), transforming growth factor beta (TGF-β), interferon gamma (IFN-γ), or modulates other innate or adaptive immune responses.

In some embodiments, the inflammatory state of the GI tract is modulated by oral administration of a microbiome regulator. In some embodiments, bacterial fermentation of a microbiome regulator in the gut produces short-chain fatty acids (SCFAs). SCFAs produced by the gut microbiota serve as energy sources for colonic epithelial cells and are thought to contribute to the maintenance of gut barrier function, which in turn limits plasma endotoxin levels and prevents systemic inflammation (Cani et al., Gut, 2009, 58:1091). In addition, SCFAs promote the generation of regulatory T (Treg) cells, and are thought to play a role in limiting inflammatory responses (Arpaia et al., Nature, 2013, 504:451). In some embodiments, a microbiome regulator is administered to induce systemic effects, e.g. of SCFAs and other microbially produced immunomodulatory molecules or metabolites to modulate the inflammatory state of distal sites.

A microbiome regulator when administered to a subject in an effective amount may modulate the production of one or more microbial metabolites, such as those listed in Table 2. In some embodiments, a microbiome regulator when administered to a subject in an effective amount may modulate (e.g. increase or decrease) one or more of the following microbial metabolites: formic acid, acetic acid, propionic acid, butryic acid, isobutyric acid, valeric acid, isovaleric acid, ascorbic acid, lactic acid, tryptophan, serotonin, and/or indole. In some embodiments, a microbiome regulator when administered to a subject in an effective amount may modulate (e.g. increase or decrease) one or more of the following microbial metabolites: succinic acid, trimethylamine (TMA), TMAO (trimethylamine N-oxide), deoxy cholic acid, ethyphenyl sulfate, acetylaldehyde, hydrogen peroxide, and/or butanedione. In some embodiments, a substantial increase or decrease in a metabolite may be detected. In some embodiments, a microbiome regulator is digested by the gut microbiota (e.g. Clostridia), resulting, e.g., in the release of short-chain fatty acids such as butyrate, acetate, and propionate, which may act immunomodulatory (e.g. anti-inflammatory) and other metabolites (e.g. bile acids, and lactate) that may confer beneficial health effects on the host.

A microbiome regulator when administered to a subject in an effective amount may modulate one or more host pathways. Short chain fatty acids (SCFAs) are bacterial metabolites produced in the gut by commensal bacteria including members of the families *Ruminocaccaceae* and *Lachnospiraceae* (Vital M, Howe AC, Tiedje JM. 2014. mBio 5(2):e00889-14). SCFAs modulate a number of human immunological factors; for example, treatment with propionate, a SCFA, in mice or in vitro increased expression of Foxp3, a T cell regulatory factor, and IL-10, an anti-inflammatory cytokine, in colonic regulatory T cells. Additionally, exposure to SCFAs has been shown to increase frequency and number of colonic regulatory T cells (cTregs) and CD4+ T cells in germ-free mice (Smith PM et al. 2013. Science; 341(6145). SCFAs promote gut barrier function by affecting mucin production and gastrointestinal peptide LL-37, and SCFAs additionally modulate inflammation by suppressing NF-kB and the production of inflammatory cytokines such as IL-6 and TNF-α (Kim CH et al. 2014. Immune Network 14(6):277-288). In some embodiments, a microbiome regulator when administered in an effective amount modulates bacterial species that produce SCFAs, such as, e.g., those of the *Ruminocacceae* family and/or *Lachnospiraceae* family. In some embodiments, a microbiome regulator modulates host immunity and inflammation.

In some embodiments, methods of modulating a functional pathway of the microbiota of the gastrointestinal tract are provided. The methods include administering to the human subject a pharmaceutical composition comprising a microbiome regulator in an amount effective to modulate the functional pathway. In some embodiments, the functional pathway modulates the production of anti-microbial agent, a secondary bile acid, a short-chain fatty acid, a siderophore or a metabolite listed in Table 2 by the microbiota. In some embodiments, the short chain fatty acid is produced by one or more bacterial member of the Ruminocaccaceae and/or Lachnospiraceae family. In some embodiments, the subject is obese.

In some embodiments, the pharmaceutical microbiome regulator compositions comprise one or more polyphenols. The microbiome regulator preparation and the one or more polyphenols in the pharmaceutical composition can have additive or synergistic effects.

In some embodiments, the polyphenols are capable of modulating one or more bacterial constituents in the GI tract. In some embodiments, the pharmaceutical composition comprising a microbiome regulator and the polyphenol preparation modulates (e.g. increases or decreases) the growth of one or more bacterial taxa, such as bacteria of the phylum Verrucomicrobia, e.g., those of the genus Akkermansia. In some embodiments, the pharmaceutical composition comprising a microbiome regulator and the polyphenol preparation increases the abundance of bacteria of the phylum Verrucomicrobia, including the genus Akkermansia.

In some embodiments, polyphenols in the compositions have antioxidant functions. In some embodiments, polyphenols in the compositions have anti-bacterial functions. In some embodiments, the antioxidant and/or anti-bacterial function of the polyphenols in the composition modulates the abundance of one or more bacteria residing in the GI tract.

In some embodiments, the pharmaceutical microbiome regulator composition comprises polyphenols that act as antimicrobials, e.g., by inhibiting the growth of subsets of species, such as, e.g. pathogens or pathobionts. (Puupponen-Pimiä R et al. 2001. Journal of Applied Microbiology 90: 494-507; Puupponen-Pimiä R et al. 2005. Journal of Applied Microbiology 98: 991-1000). In some embodiments, polyphenols in the composition are a selective substrate for one or more bacterial taxa that reside in the GI tract, (e.g., Selma MV et al. 2009. Journal of Agricultural and Food Chemistry 57: 6485-6501; Déprez S et al).

The microbiome regulators described herein when administered to a subject in an effective amount may modulate one or more host pathways. The microbiome regulator treatment may result in increases or decreases of one or more biomarkers that can be determined by methods well known in the art. An investigator can easily determine at which point or points during treatment the biomarker(s) should be measured, e.g. prior to treatment, at various intervals during treatment and/or after treatment. Any suitable sample, e.g. a gastrointestinalspecific sample such as, e.g. a tissue sample or biopsy, a swab, a gastrointestinal secretion (such as feces/a stool sample), etc. may be drawn from the subject and the sample may be analyzed. In some embodiments, a substantial increase or decrease in a biomarker may be detected.

In some embodiments, the microbiome regulator is digested by the gut microbiota (e.g. Clostridia), resulting, e.g., in the release of short-chain fatty acids such as butyrate, acetate, and propionate, which may act immunomodulatory (e.g. anti-inflammatory) and other metabolites (e.g. bile acids, and lactate) that may confer beneficial health effects on the host.

To evaluate the effect of administered microbiome regulator compositions on SCFA production in the gut, fecal pellets can be collected. SCFA levels, particularly acetate, propionate, and butyrate may be quantified. SCFAs, creatines, and hydroxy-SCFAs can be quantified by alkalinizing stool samples, obtaining fingerprints of the metabolic composition of the sample using, e.g., 1D 1H NMR Spectrometer, and analyzing with supervised multivariate statistical methods. Inulin may serve as a positive control.

In some embodiments, microbial metabolite profiles of patient samples or microbes cultures from subject samples are used to identify risk factors for developing a gastrointestinal infectious and/or inflammatory disease, disorder or condition. Exemplary metabolites for the purposes of diagnosis, prognostic risk assessment, or treatment assessment purposes include those listed in Table 2. In some embodiments, microbial metabolite profiles are taken at different time points during a subject's disease and treatment in order to better evaluate the subject's disease state including recovery or relapse events. Such monitoring is also important to lower the risk of a subject developing a new gastrointestinal disease, disorder or condition. In some embodiments, metabolite profiles inform subsequent treatment.

Further, if determined useful by a treating physician or other healthcare provider, the microbiome regulator compositions described herein can be administered in combination with various other standard of care therapies. The microbiome regulator compositions may be administered prior to, concurrent with, or post treatment with standard of care therapies. In some instances, the therapies disrupt the composition and health of the GI tract's normal microbiota (e.g. use of anti-bacterial, anti-viral or anti-fungal agents), which may lead to the undesirable proliferation of harmful bacteria or pathogens, which may cause one or more of the symptoms described herein. In some embodiments, administration of the microbiome regulator compositions described herein is useful for alleviating those symptoms and improving the composition of the gastrointestinal microbial community.

### Methods of Treatment

Any disclosure in this application relating to methods of treatment of the human or animal body by surgery or therapy, or diagnostic methods practised on the human or animal body, is to be understood as relating to purpose-limited product claims, including first and further medical uses, within the meaning of Articles 54(4) and 54(5) EPC.

Disclosed herein are methods of treating a subject having a disease, disorder or condition with a microbiome regulator or composition thereof. In some embodiments, the methods described herein include one or both of i) identifying a subject having or suspected of having a disease, disorder or condition, and ii) administering to the subject a pharmaceutical composition comprising a microbiome regulator in an amount effective to treat the disease, disorder, or condition.

In some embodiments, a subject having a disease, disorder, or condition may have a dysbiosis, e.g., a dysbiosis of the gastrointestinal microbiota. In some embodiments, the methods described herein include one or both of i) identifying a human subject having or suspected of having a dysbiosis of the gastrointestinal microbiota, and ii) administering to the human subject a pharmaceutical composition comprising a microbiome regulator in an amount effective to treat the dysbiosis. Disturbances in beneficial microbiota can occur due to a variety of factors (e.g. genetic or environmental) including, but not limited to, use of antibiotics, chemotherapeutics and other dysbiosis-inducing drugs or treatments (e.g. radiation treatment), pathogen infection, pathobiont activity, miscalibrated caloric intake (e.g. high-fat, high-sugar), miscalibrated (non-digestible) fiber intake (e.g. low or zero fiber), host factors (e.g. host genetic alterations), and similar. In some embodiments, by treating the dysbiosis the disease, disorder or condition is treated.

Symptoms that may be associated with a dysbiosis of the gastrointestinal microbiota and/or with a gastrointestinal disease, disorder or condition include, but are not limited to gas, heartburn, stomach upset, bloating, flatulence, diarrhea, abdominal pain, cramping, nausea, and vomiting. Minor digestive problems related to the GI also include occasional bloating, diarrhea, constipation, gas, or stomach upset.

In some embodiments, the microbiome regulators and compositions thereof described herein are used to treat a disease comprising an infectious disease, an inflammatory disease, a metabolic disease, an autoimmune disease, a neurological disease, or cancer. Each of these diseases, disorders, or conditions are outlined below.

### Infectious Diseases

In some embodiments, administration of a microbiome regulator or composition described herein reduces infection. In some embodiments, a subject is identified to be suitable for treatment if the subject has or is suspected of having a disease, disorder or condition including: gastrointestinal infectious diseases including Clostridium difficile infection (CDI); Vancomycin-resistant enterococci (VRE) infection, infectious colitis, and C. difficile colitis; mycoses, such as, e.g., Candida albicans infection, Campylobacter jejuni infection, Helicobacter pylori infection; diarrhea, such as, e.g., Clostridium difficile associated diarrhea (CDAD), antibiotic-associated diarrhea (AAD), antibiotic-induced diarrhea, travellers' diarrhea (TD), pediatric diarrhea, (acute) infectious diarrhea, colon and liver cancers, ameboma; necrotizing enterocolitis (NEC), and small intestine bacterial overgrowth (SIBO); indigestion or non-ulcer dyspepsia; anal fissures, perianal abscess and anal fistula; diverticulosis or diverticulitis; peptic ulcers; and gastroenteritis.

In one embodiment, the subject being identified to be suitable for treatment with a microbiome regulator has or is suspected of having a Clostridium difficile infection (CDI); a Vancomycin-resistant enterococci (VRE) infection, infectious colitis, or C. difficile colitis.

In one embodiment, the subject being identified to be suitable for treatment with a microbiome regulator has or is suspected of having mycoses, such as, e.g., Candida albicans infection, Campylobacter jejuni infection, or Helicobacter pylori infection.

In some embodiments, the GI tract infection is a bacterial or viral infection, such as an infection with, e.g., VRE, C. difficile, Escherichia coli, Salmonella, Shigella, Campylobacter, Vibrio cholera, Clostridium perfringes, Bacillus cereus, Vibrio parahemolyticus, Yersinia enterocolitica, Helicobacter pylori, rotavirus, or norovirus.

In some embodiments, the GI tract infection is a fungal infection, such as an infection with, e.g., Candida, Aspergillus, Mucor, Cryptococcus, Histoplasma, or Coccidioides.

In some embodiments, the GI tract infection is a protozoal infection, such as an infection with, e.g., Entamoeba histolytica, Giardia lamblia, Cryptosporidium parvum.

In one embodiment, the subject being identified to be suitable for treatment with a microbiome regulator has or is suspected of having diarrhea, such as, e.g., Clostridium difficile associated diarrhea (CDAD), antibiotic-associated diarrhea (AAD), antibiotic-induced diarrhea, travellers' diarrhea (TD), pediatric diarrhea, or (acute) infectious diarrhea.

In one embodiment, the subject being identified to be suitable for treatment with a microbiome regulator has or is suspected of having necrotizing enterocolitis (NEC); gastroenteritis; small intestine bacterial overgrowth (SIBO) or similar disease, disorder or condition associated with a GI tract infection.

In one embodiment, the subject being identified to be suitable for treatment with a microbiome regulator has or is suspected of having ameboma; indigestion or non-ulcer dyspepsia; anal fissures, perianal abscess and anal fistula; diverticulosis or diverticulitis; peptic ulcer or similar disease, disorder or condition associated with structural alterations of the GI tract.

In some embodiments, subjects with Clostridium difficile infection (CDI)-induced colitis may be treated according to the methods provided herein. Subjects with CDI-induced colitis may present with watery diarrhea, cramping, abdominal pain, anorexia, malaise, fever, dehydration, lower abdominal tenderness, and/or rebound tenderness. The presence of C. difficile in the stool of patients can be tested by stool culture, glutamate dehydrogenase enzyme immunoassay, PCR assay to detect genes for C. difficile toxins, stool cytotoxin assay, or enzyme immunoassay for C. difficile toxins A and B. Patient populations include subjects with primary CDI, subjects with recurrent CDI, subjects with different severities of CDI-associated diarrhea (mild, moderate, severe), and subjects at risk for CDI due to the presence of risk factors such as antibiotics treatment, broad-spectrum antibiotics treatment, residence in a hospital or long-term care facility, gastrointestinal tract surgery, diseases of the colon, a weakened immune system, chemotherapy, advanced age, kidney disease, or use of proton-pump inhibitors. Standard-of-care treatments for CDI include antibiotics such as metronidazole, fidaxomicin, or vancomycin. Treatments may also include probiotics, fecal transplant, and fluids to prevent dehydration. Resolution of disease is measured by abatement of diarrhea (e.g., the absence of a 24 hour period with more than three unformed stools) and resolution of other symptoms described above. Clearance of infection may be verified by the absence of a positive stool test for C. difficile.

In one embodiment, methods are provided to prevent, treat, ameliorate symptoms of, and/or prevent initial colonization or relapse of colonization by pathogens. In some embodiments, the relapse occurs during or after first-line or standard-of-care treatment regimen. In some cases, a pathogen load may initially lighten upon the standard-of-care treatment but then the load begins to increase again, potentially triggering a relapse of the disease. In some embodiments, a microbiome regulator or composition thereof may be administered (e.g. at the beginning, during or after the initial treatment regimen) to prevent the relapse or treat one or more relapse symptoms. In some embodiments, disease-associated bacteria, pathobionts or pathogens are selected from the group consisting of the species Bilophila wadsworthia, Campylobacter jejuni, Citrobacter farmer, Clostridium difficile, Clostridium perfringens, Clostridium tetani, Collinsella aerofaciens, Enterobacter hormaechei, Enterococcus faecalis, Enterococcus faecium, Escherichia coli, Fusobacterium varium, Fusobacterium nucleatum, Haemophilus parainfluenzae, Klebsiella pneumonia, Peptostreptococcus stomatis, Porphyromonas asaccharolytica, Pseudomonas aeruginosa, Salmonella bongori, Salmonella enteric, Shigella boydii, Shigella dysenteriae, Shigella flexneri, Shigella sonnei, Staphylococcus aureus, Streptococcus infantarius, Vibrio cholera, and Yersinia enterocolitica.

In some embodiments, disease-associated bacteria, pathobionts or pathogens include the genera Bilophila, Campylobacter, Candidatus, Citrobacter, Clostridium, Collinsella, Desulfovibrio, Enterobacter, Enterococcus, Escherichia, Fusobacterium, Haemophilus, Klebsiella, Lachnospiraceae, Peptostreptococcus, Porphyromonas, Portiera, Providencia, Pseudomonas, Salmonella, Shigella, Staphylococcus, Streptococcus, Vibrio, and Yersinia.

In one embodiment, provided herein is a method of preventing relapse of C. *difficile* symptoms in a subject having been treated with a first-line drug (e.g. vancomycin, metronidazole, fidaxomicin). The method includes the steps of identifying a subject infected with *C.difficile* and having been administered an antibiotic and administering to the subject a pharmaceutical composition comprising a microbiome regulator in an amount effective to prevent the recurrence of one or more symptoms associated with C. *difficile* infection. In some embodiments, viable C. *difficile* pathogen is retained in the gastrointestinal tract of the subject (e.g. CFU counts are detectable in a sample taken from the subject, e.g. a fecal sample) even post-treatment with the antibiotic but C. *difficile* associated symptoms are significantly reduced. In some embodiments, subjects exhibiting vancomycin-resistant enterococci (VRE) colonization and infection may be treated according to the methods provided herein. Bacteria of the genus Enterococcus are common members of the gut microbiota. Vancomycin-resistant members of this genus, commonly E. faecalis and E. faecium, can cause vancomycin-resistant enterococci (VRE) colonization and infection. Subjects colonized with VRE may present with a VRE-positive stool sample, rectal swab, perirectal swab, or sample from another body site. Vancomycin resistance can be assessed by bacterial culture or by PCR-based assays that detect vancomycin resistance (Van) gene operons. Although colonized subjects may be asymptomatic, this population is at increased risk for infection with VRE. Subjects with VRE infection may present with diarrhea, fever, chills, urinary tract infection (UTI), bacteremia, endocarditis, intra-abdominal and pelvic infection, respiratory infection, or infection at another body site. Patient populations include subjects who are colonized with VRE, subjects suffering from a VRE infection, and subjects who are at risk for colonization or infection with VRE due to the presence of risk factors such as hospitalization, residence in a long-term care facility, long-term antibiotic use, immunosuppression, surgery, open wounds, indwelling devices (e.g., intravenous lines or urinary catheters), or employment as a health care worker. Standard prevention measures for VRE colonization or infection include strict adherence to good hygiene practices (e.g., hand washing) and avoidance of risk factors where possible (e.g., removal of indwelling devices). Subjects colonized with VRE but not suffering from a VRE infection are typically not treated. Standard-of-care treatment options for VRE infections are limited due to resistance to standard antibiotics, but can include combinations of antibiotics and/or antibiotics such as quinupristin-dalfopristin, linezolid, daptomycin, and tigecycline that have been demonstrated to retain activity against many strains of VRE. Treatments may also include probiotics or supportive care. Resolution of disease is measured by clearance of infection and resolution of other symptoms described above. Clearance of infection or colonization may be verified by the absence of a VRE-positive test in a relevant biological sample. Prevention of infection or colonization may be quantified in a similar manner.

### Inflammatory Diseases

In some embodiments, administration of a microbiome regulator or composition thereof reduces inflammation. In some embodiments, a subject is identified to be suitable for treatment if the subject has or is suspected of having a disease, disorder or condition including: gastrointestinal inflammatory diseases including inflammatory bowel disease (IBD), ulcerative colitis (UC), Crohn's disease (CD), idiopathic inflammation of the small bowel, indeterminatal colitis, pouchitis; irritable bowel syndrome (IBS), colon and liver cancers, necrotizing enterocolitis (NEC), intestinal inflammation, constipation, microscopic colitis, diarrhea; graft versus host disease (GVHD); (food) allergies; pseudomembranous colitis; indigestion or non-ulcer dyspepsia; diverticulosis or diverticulitis, ischemic colitis; radiation colitis or enteritis; collagenous colitis; gastroenteritis; and polyps.

In one embodiment, the subject being identified to be suitable for treatment with a microbiome regulator has or is suspected of having inflammatory bowel disease (IBD), ulcerative colitis (UC), Crohn's disease (CD), intestinal inflammation, microscopic colitis or similar disease, disorder or condition that is associated with inflammation of the intestine.

In one embodiment, the subject being identified to be suitable for treatment with a microbiome regulator has or is suspected of having idiopathic inflammation of the small bowel, indeterminatal colitis, pouchitis, pseudomembranous colitis, ischemic colitis, radiation colitis (enteritis), collagenous colitis or similar disease, disorder or condition that is associated with inflammation of the intestine.

In one embodiment, the subject being identified to be suitable for treatment with a microbiome regulator has or is suspected of having gastroenteritis; graft versus host disease (GVHD), or a (food) allergy.

In one embodiment, the subject being identified to be suitable for treatment with a microbiome regulator has or is suspected of having irritable bowel syndrome (IBS), constipation, diarrhea, indigestion, non-ulcer dyspepsia or similar disease, disorder or condition that is associated with an altered intestinal transit.

In one embodiment, the subject being identified to be suitable for treatment with a microbiome regulator has or is suspected of having necrotizing enterocolitis (NEC); diverticulosis or diverticulitis; polyps or similar disease, disorder or condition that is associated with structural alteration of the intestine.

Subjects with inflammatory bowel disease (IBD) may present with abdominal cramps and pain, diarrhea that may be bloody, urgency of bowel movements, constipation, nausea, vomiting, fever, weight loss, loss of appetite, and/or iron deficiency anemia due to blood loss. Symptoms of IBD may occur in flares, with alternating periods of symptomatic and asymptomatic disease. IBD may be diagnosed by a combination of tests, including stool exams (to eliminate the possibility of infectious causes of diarrhea, check for trace amounts of blood in the stool, and quantify biomarkers associated with IBD such as fecal calprotectin), a complete blood count to assess levels of inflammation, blood tests to assess biomarkers including C-reactive protein (CRP) and perinuclear anti-neutrophil cytoplasmic antibody (pANCA), barium X-ray, sigmoidoscopy, colonoscopy, and endoscopy. Patient populations include subjects with ulcerative colitis (UC; limited to the colon or large intestine), subjects with Crohn's disease (CD; affecting any segment of the gastrointestinal tract), and subjects with different disease severities (mild, moderate, severe). Standard-of-care treatments for IBD include aminosalicylates (e.g., sulfasalazine, mesalamine, balsalazide, olsalazine), corticosteroids (e.g., hydrocortisone, prednisone, methylprednisolone, prednisolone, budesonide, dexamethasone), immunosuppressants (e.g., azathioprine, 6-mercaptopurine, methotrexate, cyclosporine), antibiotics (e.g., metronidazole, ciprofloxacin, rifaximin), tumor necrosis factor inhibitors (e.g, infliximab, adalimumab, certolizumab pegol), integrin inhibitors (e.g., natalizumab, vedolizumab), and surgery. Resolution or control of disease may be quantified by endoscopic or sigmoidoscopic assessment of disease severity according to standard scoring metrics, abatement of symptoms described above, reduction in disease severity as determined by composite indexes such as the Crohn's Disease Activity Index (CDAI), or improvement in health-related quality of life as measured by the IBD Questionnaire (IBD-Q).

### Metabolic Diseases

In some embodiments, a subject is identified to be suitable for treatment with a microbiome regulator or a composition thereof if the subject has or is suspected of having a disease, disorder or condition including: obesity, pre-diabetes, type II diabetes, high blood cholesterol, high LDL, high blood pressure, high fasting blood sugar, high triglyceride levels, low HDL non-alcoholic fatty liver disease (NAFLD), nonalcoholic steatohepatitis (NASH); metabolic syndrome; hyperammonemia, essential nutrient deficiency, hemochromatosis, lactose intolerance, gluten intolerance; and acrodermatitis enteropathica.

In one embodiment, the subject being identified to be suitable for treatment with a microbiome regulator has or is suspected of having obesity, (insulin resistance) pre-diabetes, type II diabetes, high fasting blood sugar (hyperglycemia), metabolic syndrome or similar disease, disorder or condition associated with metabolic disease symptoms.

In one embodiment, the subject being identified to be suitable for treatment with a microbiome regulator has or is suspected of having high blood cholesterol, high LDL, high blood pressure (hypertension), high triglyceride levels, low HDL or similar cardiovascular risk factor.

In one embodiment, the subject being identified to be suitable for treatment with microbiome regulator has or is suspected of having non-alcoholic fatty liver disease (NAFLD), nonalcoholic steatohepatitis (NASH), hyperammonemia or similar disease, disorder or condition of the liver.

In one embodiment, the subject being identified to be suitable for treatment with a microbiome regulator has or is suspected of having lactose intolerance, gluten intolerance or similar disease, disorder or condition that is associated with food intolerance.

In one embodiment, the subject being identified to be suitable for treatment with a microbiome regulator has or is suspected of having essential nutrient deficiency, hemochromatosis, acrodermatitis enteropathica or similar disease, disorder or condition that is associated with a nutrient mismanagement.

In one embodiment, provided is a method of treating a metabolic disorder in a human in need thereof, by: administering to the human a pharmaceutical microbiome regulator composition to treat the metabolic disorder. In one embodiment, the metabolic disorder is selected from obesity, adiposity, insulin resistance, diabetes, and fatty liver syndrome.

Metabolic disorders may include disorders, diseases, and conditions that are caused or characterized by abnormal weight gain; energy use or consumption; altered responses to nutrients, energy sources, hormones, or other signaling molecules; or altered metabolism of carbohydrates, lipids, proteins, or nucleic acids, or a combination thereof. Examples of metabolic disorders include insulin resistance, insulin sensitivity, fatty liver syndrome, obesity, adiposity, and diabetes (e.g., type 1 diabetes, type 2 diabetes). In one variation, the methods provided herein treat obesity. Provided herein are methods for treating obesity in a subject in need thereof using a pharmaceutical microbiome regulator composition that can alter gut microbiota of the subject in a way that results in weight loss and/or decreased body fat in the subject.

In one embodiment, provided is a method of reducing adiposity in a subject in need thereof, by: administering to the human a pharmaceutical microbiome regulator composition in an amount effective to reduce adiposity. Adiposity may be determined using any appropriate method known in the art, including, for example, waist circumference, waist to hip ratio, skinfold thickness, bioelectric impedance, underwater weighing, air-displacement plethysmography, or hydrometry.

In one embodiment, provided is a method of improving glucose metabolism in a subject in need thereof, by: administering to the subject a pharmaceutical microbiome regulator composition in an amount effective to improve glucose metabolism. Glucose metabolism may be determined by any appropriate method known in the art, including, for example, fasting blood sugar level, fasting insulin level, postprandial blood sugar test, postprandial insulin test, oral glucose tolerance test, intravenous glucose tolerance test, glycated hemoglobin level, or random blood sugar test.

In one embodiment, provided is a method of increasing insulin sensitivity in a human, by: administering to the subject a pharmaceutical microbiome regulator composition in an amount effective to increase insulin sensitivity, wherein the human has an insulin sensitivity prior to the administration of the microbiome regulator and an insulin sensitivity after the administration of the microbiome regulator, and the insulin sensitivity of the human after the administration of the microbiome regulator is higher than the insulin sensitivity of the human prior to the administration of the microbiome regulator. Insulin sensitivity may be determined by any appropriate method known in the art, including, for example, fasting blood sugar level, fasting insulin level, postprandial blood sugar test, postprandial insulin test, oral glucose tolerance test, intravenous glucose tolerance test, glycated hemoglobin level, or random blood sugar test.

In some embodiments, subjects with type 2 diabetes may be treated according to the methods provided herein. Subjects with type 2 diabetes may present with blurred vision, peripheral neuropathy, increased urination, increased thirst, fatigue, increased hunger, weight loss, or yeast, bladder, kidney, skin, or other infections. Type 2 diabetes is diagnosed by criteria described by the American Diabetes Association (ADA), including the following: fasting plasma glucose (FPG) of 126 mg/dL (7 mM) or higher, or a 2 hour plasma glucose level of 200 mg/dL (11.1 mM) or higher during a 75 g oral glucose tolerance test (OGTT), or a random plasma glucose of 200 mg/dL (11.1 mM) or higher in a patient with classic symptoms of hyperglycemia or hyperglycemic crisis, or a hemoglobin A1c (HbA1c) level of 6.5% or higher. Patient populations include adults and children with type 2 diabetes, subjects at risk for developing type 2 diabetes (e.g., subjects with prediabetes or subjects who are overweight), and subjects with type 2 diabetes in conjunction with conditions of metabolic syndrome including obesity, elevated blood pressure, elevated serum triglycerides, and low high-density lipoprotein (HDL) levels. Standard-of-care treatments for type 2 diabetes include lifestyle management (diet, exercise, and behavioral modifications), alpha-glucosidase inhibitors, biguanides (e.g., metformin), sulfonylureas, dipeptidyl peptidase IV (DPP-4) inhibitors, glucagon-like peptide-1 (GLP-1) analogs, meglitinides, selective sodium-glucose transporter-2 (SGLT2) inhibitors, thiazolidinediones, insulin, and amylinomimetics. Treatment efficacy may be assessed by resolution of the symptoms or diagnostic criteria listed above (e.g., decrease in FPG to healthy levels), or, in subjects at risk for developing type 2 diabetes, by decreased rates of conversion to a type 2 diabetic state.

In some embodiments, subjects exhibiting non-alcoholic fatty liver disease (NAFLD) and/or non-alcoholic steatohepatitis (NASH) may be treated according to the methods provided herein. Non-alcoholic fatty liver disease (NAFLD) is characterized by an abnormal buildup of fat in the liver. NAFLD can progress to non-alcoholic steatohepatitis (NASH), which is characterized by liver inflammation, fibrosis, and cirrhosis. Subjects with NAFLD may be asymptomatic. Subjects with NAFLD or NASH may present with increased liver size (noted during physical exam), fatigue, weight loss, general weakness, and/or ache in the upper right of the belly. Diagnosis of NAFLD/NASH includes elevated blood levels of alanine aminotransferase (ALT) or aspartate aminotransferase (AST), enlarged liver and specific histopathologic markers (e.g. by liver biopsy, abdominal ultrasound, CT scan, or an MRI scan). Patient populations include subjects with NAFLD, subjects with NASH, subjects at risk of developing NAFLD/NASH (e.g., subjects who are overweight or have elevated cholesterol levels), and subjects with NAFLD/NASH in conjunction with conditions of metabolic syndrome including obesity, elevated fasting plasma glucose, elevated blood pressure, elevated serum triglycerides, and low high-density lipoprotein (HDL) levels. Standard-of-care treatments for NAFLD/NASH include lifestyle management (diet, exercise, behavioral modifications, and avoidance of alcohol). Treatments in clinical trials or under development include farnesoid X receptor (FXR) agonists (e.g., obeticholic acid), Takeda G protein-coupled receptor 5 (TGR5) agonists, fatty acid-bile acid conjugates (e.g., aramchol), antioxidants (e.g., vitamin E), antifibrotic agents, peroxisome proliferator-activated receptor (PPAR)-gamma agonists, PPAR alpha/delta agonists, caspase inhibitors (e.g., Emricasan), and/or galectin-3 inhibitors. Treatment efficacy may be assessed by resolution of the symptoms or diagnostic criteria listed above (e.g., decrease in ALT to healthy levels), or, in subjects at risk for developing NAFLD/NASH, by decreased rates of conversion to NAFLD/NASH.

In some embodiments, obese subjects may be treated according to the methods provided herein. Obesity is a significant health concern, and may have a negative effect on health. For example, obesity may lead to reduced life expectancy and/or increased health problems, such as diabetes, high blood pressure, heart disease, stroke, high cholesterol, sleep apnea, and arthritis. Obese subjects present with a body mass index (BMI) of greater than 30 kg/m². Alternatively, obese subjects may be classified based on body fat percentage (greater than 25% for males or greater than 33% for females). Diagnosis may also include an evaluation of fasting lipid levels (cholesterol, triglycerides), liver function, glucose levels, insulin levels, glycosylated hemoglobin (HbA1c), and/or glucose tolerance. Patient populations include subjects with childhood obesity, moderate obesity, morbid/severe obesity, genetic causes of obesity (including Prader-Willi syndrome, Bardet-Biedl syndrome, Cohen syndrome, and MOMO syndrome), and obesity in conjunction with other conditions of metabolic syndrome (elevated blood pressure, elevated fasting plasma glucose, elevated serum triglycerides, and low high-density lipoprotein (HDL) levels). Standard-of-care treatments for obesity include lifestyle management (diet, exercise, and behavioral modifications), bariatric surgery, medications that impair dietary absorption (e.g., tetrahydrolipstatin), medications that impair dietary intake, medications that increase energy expenditure, and medications to treat common comorbidities (e.g., medications for type 2 diabetes or hypertension). Treatment endpoints include change in body weight, fasting lipid levels, liver function, glucose levels, insulin levels, HbA1C, and/or glucose tolerance.

### Cancer

In some embodiments, a subject is identified to be suitable for treatment with a microbiome regulator or a composition thereof if the subject has or is suspected of having a cancer. In some embodiments, the cancer may be any solid or liquid cancer and includes benign or malignant, non-invasive or invasive tumors, hyperplasias, and premalignant lesions. In some embodiments the subject has metastatic cancer. In other embodiments, the subject has non-metastatic cancer. In some embodiments, the subject has a benign tumor. In some embodiments, the subject has a premalignant lesion or a pre-cancerous condition. Examples of premalignant lesions or pre-cancerous conditions include: actinic keratosis, Barrett's esophagus, atrophic gastritis, ductal carcinoma in situ, dyskeratosis congenital, sideropenic dysphagia, lichen planus, oral submucous fibrosis, solar elastosis, cervical dysplasia, leukoplakia, and erythroplakia.

In some embodiments, the cancer is a highly immunogenic cancer, e.g., the cancer has (e.g., as determined by analysis of a cancer biopsy) one or more of the following characteristics: (a) tumor infiltrating lymphocytes (TIL), e.g., 1 TIL per 1000 tumor cells; (b) mutations, e.g., 0.1 or more somatic mutations per megabase of tumor genomic DNA; (c) neoantigens, e.g., 1 or more neoantigen with one or more endogenous T cell receptor and/or one or more idiotype clone that recognizes a processed and presented moiety of the neoantigen; (d) tertiary lymphoid structures; (e) high expression of inflammatory gene expression, e.g., 2-fold increased expression of cytokines above baseline expression in non-cancerous tissue; and (f) immune cells exhibiting immunosuppressive phenotype, e.g. dendritic cells lacking cytokine expression. In some embodiments, the cancer is melanoma, lung cancer, bladder cancer, colorectal cancer, esophageal cancer, cervical cancer, head and neck cancer, stomach cancer, uterine cancer, liver cancer, kidney cancer, ovarian cancer, prostate cancer, myeloma, B cell lymphoma, or glioma. Methods of assessing these characteristics of the cancer are known (see, e.g., Clin Cancer Res. 2000 May; 6(5):1875-81; Nature. 2013 Aug 22;500(7463):415-21).

In some embodiments, the cancer is a primary tumor. In some embodiments, the cancer is a metastasized tumor. In some embodiments, the cancer patient has: had one or more tumors resected, received chemotherapy or other pharmacological treatment for the cancer, received radiation therapy, and/or received other therapy for the cancer.

Exemplary cancers that may be treated with a microbiome regulator or composition thereof (e.g., as described herein) include acoustic neuroma; adenocarcinoma; adrenal gland cancer; anal cancer; angiosarcoma (e.g., lymphangiosarcoma, lymphangioendotheliosarcoma, hemangiosarcoma); appendix cancer; benign monoclonal gammopathy; biliary cancer (e.g., cholangiocarcinoma); bladder cancer; breast cancer (e.g., adenocarcinoma of the breast, papillary carcinoma of the breast, mammary cancer, medullary carcinoma of the breast); brain cancer (e.g., meningioma, glioblastomas, glioma (e.g., astrocytoma, oligodendroglioma), medulloblastoma); bronchus cancer; carcinoid tumor; cervical cancer (e.g., cervical adenocarcinoma); choriocarcinoma; chordoma; craniopharyngioma; colorectal cancer (e.g., colon cancer, rectal cancer, colorectal adenocarcinoma); connective tissue cancer; epithelial carcinoma; ependymoma; endotheliosarcoma (e.g., Kaposi's sarcoma, multiple idiopathic hemorrhagic sarcoma); endometrial cancer (e.g., uterine cancer, uterine sarcoma); esophageal cancer (e.g., adenocarcinoma of the esophagus, Barrett's adenocarcinoma); Ewing's sarcoma; eye cancer (e.g., intraocular melanoma, retinoblastoma); familiar hypereosinophilia; gall bladder cancer; gastric cancer (e.g., stomach adenocarcinoma); gastrointestinal stromal tumor (GIST); germ cell cancer; head and neck cancer (e.g., head and neck squamous cell carcinoma, oral cancer (e.g., oral squamous cell carcinoma), throat cancer (e.g., laryngeal cancer, pharyngeal cancer, nasopharyngeal cancer, oropharyngeal cancer)); hematopoietic cancers (e.g., leukemia such as acute lymphocytic leukemia (ALL) (e.g., B-cell ALL, T-cell ALL), acute myelocytic leukemia (AML) (e.g., B-cell AML, T-cell AML), chronic myelocytic leukemia (CML) (e.g., B-cell CML, T-cell CML), and chronic lymphocytic leukemia (CLL) (e.g., B-cell CLL, T-cell CLL)); lymphoma such as Hodgkin lymphoma (HL) (e.g., B-cell HL, T-cell HL) and non-Hodgkin lymphoma (NHL) (e.g., B-cell NHL such as diffuse large cell lymphoma (DLCL) (e.g., diffuse large B-cell lymphoma), follicular lymphoma, chronic lymphocytic leukemia/small lymphocytic lymphoma (CLL/SLL), mantle cell lymphoma (MCL), marginal zone B-cell lymphomas (e.g., mucosa-associated lymphoid tissue (MALT) lymphomas, nodal marginal zone B-cell lymphoma, splenic marginal zone B-cell lymphoma), primary mediastinal B-cell lymphoma, Burkitt lymphoma, lymphoplasmacytic lymphoma (i.e., Waldenström's macroglobulinemia), hairy cell leukemia (HCL), immunoblastic large cell lymphoma, precursor B-lymphoblastic lymphoma and primary central nervous system (CNS) lymphoma; and T-cell NHL such as precursor T-lymphoblastic lymphoma/leukemia, peripheral T-cell lymphoma (PTCL) (e.g., cutaneous T-cell lymphoma (CTCL) (e.g., mycosis fungoides, Sezary syndrome), angioimmunoblastic T-cell lymphoma, extranodal natural killer T-cell lymphoma, enteropathy type T-cell lymphoma, subcutaneous panniculitis-like T-cell lymphoma, and anaplastic large cell lymphoma); a mixture of one or more leukemia/lymphoma as described above; and multiple myeloma (MM)), heavy chain disease (e.g., alpha chain disease, gamma chain disease, mu chain disease); hemangioblastoma; hypopharynx cancer; inflammatory myofibroblastic tumors; immunocytic amyloidosis; kidney cancer (e.g., nephroblastoma a.k.a. Wilms' tumor, renal cell carcinoma); liver cancer (e.g., hepatocellular cancer (HCC), malignant hepatoma); lung cancer (e.g., bronchogenic carcinoma, small cell lung cancer (SCLC), non-small cell lung cancer (NSCLC), adenocarcinoma of the lung); leiomyosarcoma (LMS); mastocytosis (e.g., systemic mastocytosis); muscle cancer; myelodysplastic syndrome (MDS); mesothelioma; myeloproliferative disorder (MPD) (e.g., polycythemia vera (PV), essential thrombocytosis (ET), agnogenic myeloid metaplasia (AMM) a.k.a. myelofibrosis (MF), chronic idiopathic myelofibrosis, chronic myelocytic leukemia (CML), chronic neutrophilic leukemia (CNL), hypereosinophilic syndrome (HES)); neuroblastoma; neurofibroma (e.g., neurofibromatosis (NF) type 1 or type 2, schwannomatosis); neuroendocrine cancer (e.g., gastroenteropancreatic neuroendocrine tumor (GEP-NET), carcinoid tumor); osteosarcoma (e.g., bone cancer); ovarian cancer (e.g., cystadenocarcinoma, ovarian embryonal carcinoma, ovarian adenocarcinoma); papillary adenocarcinoma; pancreatic cancer (e.g., pancreatic adenocarcinoma, intraductal papillary mucinous neoplasm (IPMN), Islet cell tumors); penile cancer (e.g., Paget's disease of the penis and scrotum); pinealoma; primitive neuroectodermal tumor (PNT); plasma cell neoplasia; paraneoplastic syndromes; intraepithelial neoplasms; prostate cancer (e.g., prostate adenocarcinoma); rectal cancer; rhabdomyosarcoma; salivary gland cancer; skin cancer (e.g., squamous cell carcinoma (SCC), keratoacanthoma (KA), melanoma, basal cell carcinoma (BCC)); small bowel cancer (e.g., appendix cancer); soft tissue sarcoma (e.g., malignant fibrous histiocytoma (MFH), liposarcoma, malignant peripheral nerve sheath tumor (MPNST), chondrosarcoma, fibrosarcoma, myxosarcoma); sebaceous gland carcinoma; small intestine cancer; sweat gland carcinoma; synovioma; testicular cancer (e.g., seminoma, testicular embryonal carcinoma); thyroid cancer (e.g., papillary carcinoma of the thyroid, papillary thyroid carcinoma (PTC), medullary thyroid cancer); urethral cancer; vaginal cancer; and vulvar cancer (e.g., Paget's disease of the vulva).

In some embodiments, a microbiome regulator described herein may be used in combination with other anti-proliferative, anti-neoplastic or anti-tumor drugs or treatments. Such drugs or treatments include chemotherapeutic drugs, e.g., cytotoxic drugs (e.g., alkylating agents, antimetabolites, anti-tumor antibiotics, topoisomerase inhibitors, mitotic inhibitors, corticosteroids); cancer growth blockers such as tyrosine kinase inhibitors and proteasome inhibitors; other chemical drugs such as L-asparaginase and bortezomib (Velcade^{®}). Hormone therapies (or anti-hormone therapies) may be used, e.g., for hormone-sensitive cancers.

In some embodiments, a microbiome regulator described herein may be used in combination with other anti-proliferative, anti-neoplastic or anti-tumor drugs or treatments that include an anti-cancer drug, such as, e.g., checkpoint inhibitors (such as, e.g., anti-PD-1, anti-PD-L1, anti-CTLA4, anti-TIM-3, anti-LAG-3); vaccines (such as, e.g., autologous cancer vaccines, allogeneic cancer vaccines, neoantigen cancer vaccines, shared antigen cancer vaccines (e.g. NY-ESO-1)); targeted kinase inhibitors (such as, e.g., Imatinib mesylate, Ibrutinib, Neratinib, Palpociclib, Erlotinib, Lapatinib); antibodies (such as, e.g., Bevacizumab, Trastuzumab, Rituximab, Cetuximab); chemotherapeutics (such as, e.g., irinotecan, 5-flurouracil, lenalidomide, capecitabine, docetaxel), antibody-drug conjugates (e.g. ado-trastuzumab emtansine).

In some embodiments, a microbiome regulator is administered to minimize systemic exposure to glucose and to control the blood sugar levels in a subject. A cancer patient may have a substantial improvement in prognosis the supply of cancer's preferred fuel, glucose, was controlled. Administration of a microbiome regulator or a composition thereof may slow growth of a cancer in a subject and therefore allow his/her immune systems and medical debulking , e.g., chemotherapy, radiation, and surgery to reduce the bulk of the tumor mass, to catch up to the disease. A study of rats fed diets with equal calories from sugars and starches, for example, found the animals on the high-sugar diet developed more cases of breast cancer. Similarly, a mouse model of human breast cancer demonstrated that tumors are sensitive to blood-glucose levels. In some embodiments, the subject is administered a microbiome regulator or composition thereof in order to control blood sugar levels in conjunction with monitoring diet and other precautions.

### Autoimmune Diseases, Neurological Diseases, and Other Diseases

In some embodiments, a subject is identified to be suitable for treatment with a microbiome regulator or a composition thereof if the subject has or is suspected of having a disease, disorder or condition including: autoimmune arthritis, type I diabetes, atopic dermatitis, autism, asthma, cardiovascular disease, chronic kidney disease, multiple sclerosis, heart disease, psoriasis, hyperammonemia, hepatic encephalopathy, cachexia, Gout, drug intolerance (e.g., to metformin), low oral bioavailability of drugs, fecal incontinence, Hirschsprung's disease, anismus, colic, ileus, hemorrhoids, and intussusceptions.

In one embodiment, the subject being identified to be suitable for treatment with a microbiome regulator has or is suspected of having autoimmune arthritis, type I diabetes, multiple sclerosis, psoriasis or similar autoimmune disease, disorder or condition.

In one embodiment, the subject being identified to be suitable for treatment with a microbiome regulator has or is suspected of having asthma, atopic dermatitis or similar environmental-driven allergy.

In one embodiment, the subject being identified to be suitable for treatment with a microbiome regulator has or is suspected of having chronic kidney disease, heart disease, cardiovascular disease or similar disease, disorder or condition that is associated with organ failure.

In one embodiment, the subject being identified to be suitable for treatment with a microbiome regulator has or is suspected of having autism, hyperammonemia, hepatic encephalopathy or similar disease, disorder or condition that is associated with neurological symptoms.

In one embodiment, the subject being identified to be suitable for treatment with a microbiome regulator has or is suspected of having cachexia, Gout or similar nutritional disorder.

In one embodiment, the subject being identified to be suitable for treatment with a microbiome regulator has or is suspected of having hirschsprung's disease, ileus, anismus, intussusceptions, fecal incontinence, hemorrhoids or similar gastrointestinal disorder.

In some embodiments, subjects with atopic dermatitis (AD) may be treated according to the methods provided herein. Subjects with atopic dermatitis (AD) may present with skin that is dry, itchy, and/or inflamed. Diagnosis and severity of AD may be determined by using the SCORAD index (Oranje, A. P., et al. Brit J Dermatol 157.4 (2007): 645-648) or the Eczema Area and Severity Index (EASI) score (Hanifin et al., Exper Dermat, 2001, 10:11). AD may occur in flares, with alternating periods of symptomatic and asymptomatic disease. Staphylococcus aureus is commonly present on skin sites with AD, and biomarkers including IgE and inflammatory or Th2 cytokines and chemokines may also be elevated in the diseased skin or systemically. Patient populations include infants with early-onset AD, children with pediatric AD, adults with late-onset AD, pregnant women at risk for flares of AD ("atopic eruption of pregnancy"), subjects with mild, moderate, or severe AD flares, or subjects who are at risk of developing AD. Standard-of-care treatments for AD include topically applied moisturizers, topically applied steroid ointments such as hydrocortisone, bleach baths, antibiotics, immunomodulatory agents such as tacrolimus, antihistamines, antibody-based therapies (including antibodies to block IgE, the IL-4 receptor, IL-4, and IL-13), and other anti-inflammatory agents. Treatment may also include probiotics. Resolution or control of disease may be quantified by the standard SCORAD or EASI criteria described above.

In some embodiments, subjects with asthma may be treated according to the methods provided herein. Subjects with asthma may present with wheezing, coughing, shortness of breath, and/or chest tightness or pain. These symptoms are commonly episodic and may be triggered by factors such as exercise or exposure to allergens. Additionally, children with asthma may present with a history of recurrent bronchitis, bronchiolitis, or pneumonia or a persistent cough with colds. Diagnosis of asthma is established by lung function testing with spirometry in the presence and absence of treatment with a bronchodilator. Patient populations include infants with asthma; subjects with childhood asthma; adult-onset asthma; intermittent, mild persistent, moderate persistent, or severe persistent asthma; exercise-induced asthma; allergic asthma; cough-variant asthma; occupational asthma; nocturnal asthma; and subjects who are at risk of developing asthma, for example, due to a family history of atopy. Standard-of-care treatments for asthma include inhaled corticosteroids (e.g., budesonide, fluticasone, beclomethasone, mometasone, and ciclesonide), short-acting bronchodilators (e.g., albuterol), long-acting bronchodilators (e.g., salmeterol), leukotriene modifiers (e.g., montelukast) or other anti-inflammatory agents, anti-cholinergic agents (e.g., ipratropium, tiotropium), anti-IgE (e.g., omalizumab) for allergic asthma, and/or systemic steroids (e.g., prednisone, prednisolone, methylprednisolone, dexamethasone). Treatments may also include probiotics. Treatment efficacy may be assessed by a decrease in the frequency or severity of the symptoms described above, improvement in lung function (assessed by measurements such as peak expiratory flow rate (PEFR) or forced expiratory volume in 1 second (FEV1)), decrease in the need to continue or initiate treatments for asthma, or changes in the levels of biomarkers of airway inflammation (e.g., serum IgE, exhaled nitric oxide, sputum or blood eosinophil counts, inflammatory cytokines, Th2 cytokines, etc.).

In some embodiments, subjects with chronic kidney disease (CKD) may be treated according to the methods provided herein. Subjects with CKD may present with fatigue, trouble concentrating, poor appetite, trouble sleeping, nocturnal muscle cramping, swollen feet and ankles, skin rash/itching, nausea, vomiting, a metallic taste in the mouth, shortness of breath, and/or increased urination. Diagnosis of kidney disease, including CKD, is performed by tests of the glomerular filtration rate (GFR), blood levels of urea and creatinine, urine levels of albumin, kidney biopsy, ultrasound, and/or CT scan. Patient populations include subjects with CKD caused by diabetic nephropathy; subjects with CKD caused by high blood pressure; subjects with polycystic kidney disease, pyelonephritis, or glomerulonephritis; subjects with kidney damage due to long-term use of kidney-damaging medicines; and subjects at risk of developing CKD due to the presence of risk factors such as diabetes, high blood pressure, or family history of kidney disease. Standard-of-care treatments for CKD include medicines to lower blood pressure, control blood glucose, and lower blood cholesterol. Treatments may also include dietary modifications and probiotics. Treatment efficacy may be assessed by resolution of the symptoms or diagnostic criteria listed above (e.g., decrease in urine albumin and serum creatinine), reduction in the need to start dialysis or prolongation of the time before starting dialysis, reduction in blood levels of uremic solutes (e.g., p-cresol sulfate and indoxyl sulfate) or other potentially harmful circulating factors (e.g., trimethylamine N-oxide (TMAO), or, in subjects at risk for developing CKD, by decreased rates of conversion to CKD.

In some embodiments, subjects with hepatic encephalopathy (HE) may be treated according to the methods provided herein. Hepatic encephalopathy includes multiple adverse neurological symptoms that occur when the liver is unable to remove toxic substances such as ammonia from the blood. Subjects with HE may present with confusion, forgetfulness, anxiety or excitation, sudden changes in personality or behavior, changes in sleep patterns, disorientation, sweet or musty smelling breath, slurred speech, and/or difficulty controlling motor functions. Diagnosis of HE is performed by tests of liver function, serum ammonia levels, EEG, and other blood and neurological tests. Patient populations include subjects with mild HE, severe HE, overt HE, subjects who have previously experience one or more episodes of HE, and patients who are at risk for HE due to the presence of risk factors such as liver damage. Standard-of-care treatments for HE include lactulose, lactitol, and antibiotics (e.g., rifaximin or neomycin). Treatments may also include dietary modifications and probiotics. Treatment efficacy may be assessed by resolution of the symptoms or diagnostic criteria listed above (e.g., reduction in serum ammonia levels), decreased incidence of future episodes of HE, or, in subjects at risk of HE, by decreased occurrence of an initial episode of HE.

### Drug- or treatment-induced digestive abnormalities

Provided herein are methods of reducing drug- or treatment-induced symptoms in a human subject with a microbiome regulator or a composition thereof. Such drug- or treatment-induced symptoms include any digestive abnormalities. Exemplary digestive abnormalies include, but are not limited to weight-gain, constipation, heartburn, upset stomach, gas, bloating, flatulence, diarrhea, abdominal pain, cramping, nausea, and vomiting. In some embodiments, the digestive abnormality is diarrhea. The method include administering to the human subject a pharmaceutical composition comprising a microbiome regulator in an amount effective to reduce one or more symptoms induced by a drug or treatment. In one embodiment, the treatment is radiation treatment.

In one embodiment, the subject being identified to be suitable for treatment with a microbiome regulator has or is suspected of having drug-induced diarrhea, drug-induced constipation, drug-induced toxicity, drug-induced intolerance (e.g. to metformin, to chemotherapies), drug-induced microbiome damage, drug-induced microbiome disease, drug-induced gastrointestinal disease, drug-induced enteritis or colitis or similar drug-induced disorder or condition.

In some embodiments, the pharmaceutical composition comprising a microbiome regulator is administered prior to, concomitant with or after administration of the drug (or radiation treatment), administration of which induces the symptoms. Examplary drugs which often are associated with drug- or treatment-induced symptoms include, but are not limited to a cancer drug, an anti-diabetic, an immune-suppressive drug, an antimicrobial drug, a chemotherapeutic, an anti-psychotic, a proton pump inhibitor, and a non-steroid anti-inflammatory drug (NSAID). Administration of these drugs generally is associated with dysbioses that can, e.g., occur during the treatment regimen. In some embodiments, the dysbiosis causes or amplifies the drug- or treatment-induced symptoms, such as digestive abnormalities. In some embodiments, administration of the microbiome regulator modulates the microbiome such that the drug- or treatment-induced symptoms are reduced. In some embodiments, the microbiome regulator promotes the growth of commensal bacteria and/or supports the growth of beneficial microbial communities which would negatively be affected or lost in response to the drug treatment or which can complement commensal bacteria that have been negatively affected or lost in response to the drug treatment.

Specifc examples of drugs associated with digestive abnormalities symptoms of which can be reduced by administration of the microbiome regulator include, but are not limited to ciprofloxacin, clindamycin, amoxicillin-clavulanate, cefixime, ephalosporins, fluoroquinolones, azithromycin, clarithromycin, erythromycin, tetracycline, azithromycin, irinotecan (camptosar), 5-fluorouracil, leucovorin, oxaliplatin, bortezomib, imatinib, lenalidomide, imbruvica, ipilimumab, pertuzumab, capecitabine, docetaxel, lapatinib, erlotinib, carmustine, etoposide, aracytine, melphalan, cytarabine, daunorubicine, amsacrine, mitoxantrone, olanzapine, ranitidine, famotidine, cimetidine, omeprazole, sucralfate, esomeprazole, naproxen, diclofenac, indomethacin, ibuprofen, ketoprofen, piroxicam, celecoxib, nimesulid, aspirin, metformin, paroxetine, valproic acid, or clozapine.

In some embodiments, the digestive abnormalities are associated with treatment of the subject with a chemotherapeutic agent. In one embodiment, the digestive abnormality is diarrhea. In specific embodiments, the chemotherapeutic agent is Irinotecan, 5-fluorouracil, leucovorin, or combinations thereof. In specific emobidments, the chemotherapeutic agent is oxaliplatin, leucovorin, 5-fluorouracil, or combinations thereof. In specific embodiments the chemotherapeutic agent is bortezomib, imatinib, lenalidomide, imbruvica, ipilimumab, pertuzumab, capecitabine, docetaxel, lapatinib, erlotinib, or combinations thereof. In some embodiments, the chemotherapeutic agent is carmustine, etoposide, aracytine, melphalan, or combinations thereof. In specific embodiments the chemotherapeutic agent is cytarabine, daunorubicine, etoposide, or combinations thereof. In specific embodiments the chemotherapeutic agent is amsacrine, cytarabine, etoposide, or combinations thereof. In specific embodiments, the chemotherapeutic agent is mitoxantrone, cytarabine, or combinations thereof.

In some embodiments, the digestive abnormalities are associated with treatment of the subject with an antibiotic. In one embodiment, the digestive abnormality is diarrhea. In specific embodiments, the antibiotic is ciprofloxacin, clindamycin, amoxicillin-clavulanate, cefixime, ephalosporins, fluoroquinolones, azithromycin, clarithromycin, erythromycin, tetracycline, or azithromycin.

In some embodiments, the digestive abnormalities are associated with treatment of the subject with an anti-psychotic drug. In one embodiment, the digestive abnormality is weight gain. In one embodiment, the drug is olanzapine.

In some embodiments, the digestive abnormalities are associated with treatment of the subject with a proton-pump inhibitor drug. In one embodiment, the digestive abnormality is diarrhea. In specific embodiments, the drug is ranitidine, famotidine, cimetidine, omeprazole, sucralfate, or esomeprazole.

In some embodiments, the digestive abnormalities are associated with treatment of the subject with a non-steroidal anti-inflammatory drug (NSAID). In one embodiment, the digestive abnormality is diarrhea. In specific embodiments, the drug is naproxen, diclofenac, indomethacin, ibuprofen, ketoprofen, piroxicam, celecoxib, nimesulid, or aspirin.

In some embodiments, the digestive abnormalities are associated with treatment of the subject with metformin, paroxetine, valproic acid, or clozapine.

In one embodiment, reducing the one or more symptoms increases compliance by the subject to the treatment regimen. In one embodiment, reducing one or more symptom enables the physician to prescribe a higher-dose of the drug to be administered. In such embodiments, treatment of the underlying disease is more effective (e.g. increased reduction of symptoms, shorter period to achieve a disease or symptom-free state, or longer maintainance of a disease or symptom-free state, etc.).

### Other embodiments

In some embodiments, the subject experiences a reduction in at least one symptom of the gastrointestinal disease, disorder or condition following treatment with a microbiome regulator or composition thereof. In some embodiments, a reduction in the severity of a symptom following treatment can be determined (e.g. by measuring a known biomarker) and is in the order of about 3%, 5%, 7%, 10%, 20%, 30%, 40%, 50%, 60%, 70%, 80%, 90%, 95%, or about 100%. In some embodiments, the symptoms, measured as described herein, are decreased by an average of about 10%, 20%, 30%, 40%, 50%, 60%, 70%, 80%, 90%, 95%, or about 100% when compared to symptoms prior to the administration of a pharmaceutical microbiome regulator composition. In some embodiments, the reduction in the severity of the symptom persists for at least about a day, two days, three days, four days, five days, a week, two weeks, three weeks, a month, 3 months, 6 months, 9 months, a year, two years, five years, ten years after treatment or the reduction is permanent.

In one embodiment, a symptom of a gastrointestinal disease, disorder or condition remains partially, substantially, or completely eliminated or decreased in severity in a subject for at least about 1 day, 1 week, 1 month, 2 months, 3 months, 4 months, 5 months, 6 months, 9 months, one year, 18 months, two years, three years, four years, five years, ten years, or more than ten years after the termination of treatment. In another embodiment a symptom of a gastrointestinal disease, disorder or condition is permanently eliminated or decreased in severity in a subject after the termination of treatment.

In some embodiments, administration of the pharmaceutical microbiome regulator compositions improves the overall health of the host and/or the health of a specific niche, such as the GI tract, e.g. by modulating (e.g. increasing or decreasing) the growth or abundance of one or more members of the microbial community in the niche (such as resident commensal bacteria and/or acquired pathogens or pathobionts).

Research from the gut has led to the identification of biomarkers with the potential to demonstrate the health effects of prebiotics, which may also be used to characterize the health effects and treatment efficacies of the pharmaceutical microbiome regulator compositions described herein on the gastrointestinal microbiota and environment. These markers include: i) changes in gastrointestinal microbiota and the overall metabolism of the gastric environment, such as the production of organic acids, ii) modulation of the immune system, assessing inflammatory and immune globulins iii) increase the absorption of minerals in the colon, such as calcium, zinc or magnesium iv) regulation of lipid metabolism, lowering cholesterol, v) induction of other important processes for host homeostasis (see, reviews by Pool-Zobel B L. (2005) Brit J Nutr 93 Suppl 1:S73-90; and Liong M T. (2008). Int J Mol Sci 9(5):854-63).

The pharmaceutical microbiome regulator compositions when administered to a subject in an effective amount may modulate one or more host pathways. The microbiome regulator treatment may result in increases or decreases of one or more biomarkers that can be determined by methods known in the art. An investigator can easily determine at which point or points during treatment the biomarker(s) should be measured, e.g. prior to treatment, at various intervals during treatment and/or after treatment. Any suitable sample, e.g. a gastrointestinal-specific sample such as, e.g. a tissue sample or biopsy, a swab, a gastrointestinal secretion (such as feces/a stool sample), etc. may be drawn from the subject and the sample may be analyzed. In some embodiments, a substantial increase or decrease in a biomarker may be detected.

In some embodiments, the microbiome regulator or a composition thereof is digested by the gut microbiota (e.g. Clostridia), resulting, e.g., in the release of short-chain fatty acids such as butyrate, acetate, and propionate, which may act in an immunomodulatory capacity (e.g. anti-inflammatory) and other metabolites (e.g. bile acids, and lactate) that may confer beneficial health effects on the host.

To evaluate the effect of administered pharmaceutical microbiome regulator compositions on SCFA production in the gut, fecal samples can be collected. SCFA levels, particularly acetate, propionate, and butyrate may be quantified. SCFAs, creatines, and hydroxy-SCFAs can be quantified by alkalinizing stool samples, obtaining fingerprints of the metabolic composition of the sample using, e.g., 1D 1H NMR spectrometer, and analyzing with supervised multivariate statistical methods. Inulin may serve as a positive control.

In some embodiments, microbial metabolite profiles of patient samples or microbes cultures from subject samples are used to identify risk factors for developing a gastrointestinal infectious and/or inflammatory disease, disorder or condition. Exemplary metabolites for the purposes of diagnosis, prognostic risk assessment, or treatment assessment purposes include those listed in Table 2. In some embodiments, microbial metabolite profiles are taken at different time points during a subject's disease and treatment in order to better evaluate the subject's disease state including recovery or relapse events. Such monitoring is also important to lower the risk of a subject developing a new gastrointestinal disease, disorder or condition. In some embodiments, metabolite profiles inform subsequent treatment.

Further, if determined useful by a treating physician or other healthcare provider, the pharmaceutical microbiome regulator compositions described herein can be administered in combination with various other standard of care therapies. In some embodiments, the combination of administration of the microbiome regulator and the standard-of-care therapy agent has additive or synergistic treatment effects. The pharmaceutical microbiome regulator compositions may be administered prior to, concurrent with, or post treatment with standard of care therapies. In some instances, the therapies disrupt the composition and health of the GI tract's normal microbiota (e.g. use of anti-bacterial, anti-viral or anti-fungal agents), which may lead to the undesirable proliferation of harmful bacteria or pathogens, which may cause one or more of the symptoms described herein. In some embodiments, administration of the pharmaceutical microbiome regulator compositions described herein is useful for alleviating those symptoms and improving the composition of the gastrointestinal microbial community.

### Pharmaceutical Compositions, Medical Foods, and Dosage Forms

Provided herein are pharmaceutical compositions, medical foods, and dietary supplements comprising a microbiome regulator (e.g., a microbiome regulator described herein). In some embodiments, the pharmaceutical compositions, medical foods, and dietary supplements comprise one or more of a sugar (e.g., a sugar metabolized by the host), a sugar alcohol (e.g., a sugar alcohol metabolized by the host), an amino acid, a peptide, a fatty acid, a micronutrient, a polyphenol, or any combination thereof. Optionally, the pharmaceutical compositions and preparations of microbiome regulators further comprise a second agent, e.g., a prebiotic substance and/or a probiotic bacterium. In some embodiments, the pharmaceutical compositions, medical foods, and dietary supplements do not contain a prebiotic substance. In some embodiments, the pharmaceutical compositions, medical foods, and dietary supplements do not contain a probiotic bacterium. Further, optionally, the pharmaceutical compositions, medical foods, and dietary supplements comprise one or more excipients or carriers, including diluents, binders, disintegrants, dispersants, lubricants, glidants, stabilizers, surfactants and colorants.

In some embodiments, the pharmaceutical compostions, medical foods, and dietary supplements comprise a sugar or sugar alcohol comprising glucose, galactose, fructose, fucose, mannose, xylose, arabinose, rhamnose, ribose, sucrose, sorbose, lactose, sorbitol, maltose, mannitol, lactulose, lactitol, erythritol, tagatose, kojibiose, nigerose, isomaltose, trehalose, sophorose, laminaribiose, gentiobiose, turanose, maltulose, palatinose, gentiobiulose, mannobiose, melibiulose, rutinulose, or xylobiose. In some embodiments, the pharmaceutical compostions, medical foods, and dietary supplements comprise a sugar or sugar alcohol sugar or sugar alcohol metabolizable by the host comprising glucose, galactose, fructose, fucose, mannose, xylose, ribose, sucrose, lactose, sorbitol, maltose, mannitol, or erythritol.

In some embodiments, the pharmaceutical compostions, medical foods, and dietary supplements do not comprise a sweetener that is non-metabolizable by the host. In some embodiments, the pharmaceutical compostions, medical foods, and dietary supplements do not comprise sucralose, aspartame, aspartame-acesulfame salt, advantame, stevioside, neotame, saccharin, acesulfame-K, alitame, cyclamate, neohesperidine, or rebaudioside. In some embodiments, the pharmaceutical compostions, medical foods, and dietary supplements do not comprise glucose.

In some embodiments, the pharmaceutical compostions, medical foods, and dietary supplements comprise an amino acid comprising alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, or valine. In some embodiments, the pharmaceutical compostions, medical foods, and dietary supplements comprise a micronutrient (e.g., a vitamin, an element, or a mineral). In some embodiments, the pharmaceutical compostions, medical foods, and dietary supplements comprise a vitamin comprising pantothenate, thiamine, riboflavin, niacin, pyridoxol, biotin, folate, 4-aminobenzoate, cobinamide, a cobamide (e.g., phenyolyl cobamide, 5-methylbenzimidazolyl cobamide), or cobalamin, or salts or derivatives thereof. In some embodiments, the pharmaceutical compostions, medical foods, and dietary supplements comprise an element or mineral comprising chloride, sodium, calcium, magnesium, nitrogen, potassium, manganese, iron (e.g., Fe²⁺ or Fe³⁺), zinc, nickel, copper, or cobalt. In some embodiments, the pharmaceutical compostions, medical foods, and dietary supplements comprise a fatty acid (e.g., a short chain fatty acid). In some embodiments, the pharmaceutical compostions, medical foods, and dietary supplements comprise a short chain fatty acid comprising acetic acid, propionic acid, butryic acid, isobutyric acid, valeric acid, isovaleric acid, hexanoic acid, or octanoic acid. In some embodiments, the pharmaceutical compostions, medical foods, and dietary supplements comprise a polyphenol comprising a catechin, ellagitannin, isoflavone, flavonol, flavanone, anthocyanin, or lignin.

The microbiome regulators, prebiotic substances, and probiotics may be comingled or mixed in a single preparation. In other embodiments, they may be contained in separate containers (and/or in various suitable dosage forms) but packaged together in one or more kits. In some embodiments, the preparations are not packaged or placed together. A physician may then administer the preparations together, e.g. prior to, concomitant with, or after one another. In some embodiments, the preparations act synergistically in modulating the microbiota in the GI tract.

In one embodiment, a pharmaceutical composition comprising a microbiome regulator comprises between 0.1% and 100% microbiome regulator by w/w, w/v, v/v or molar %. In another embodiment, a pharmaceutical composition of microbiome regulators comprises about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79% 80%, 81%, 82%, 83%, 84%, 85%, 86%, 87%, 88%, 89%, 90%, 91%, 92%, 93%, 94%, 95%, 96%, 97%, 98%, 99%, 99.5%, or 100% microbiome regulator by w/w, w/v, v/v or molar %. In one embodiment, a pharmaceutical composition of microbiome regulators comprises about 1-90%, about 10-90%, about 20-90%, about 30-90%, about 40-90%, about 40-80%, about 40-70%, about 40-60%, about 40-50%, about 50-90%, about 50-80%, about 50-70%, about 50-60%, about 60-90%, about 60-80%, about 60-70%, about 70-90%, about 70-80%, about 70-90%, about 70-80%, about 80-90%, about 90-96%, about 93-96%, about 93-95%, about 94-98%, about 93-99%, or about 90-100% microbiome regulator by w/w, w/v, v/v or molar %.

Also provided herein are preparations of microbiome regulators formulated as a medical food. Any microbiome regulator described herein may be formulated as a medical food as well as pharmaceutical compositions that comprise a microbiome regulator.

A medical food is defined in section 5(b)(3) of the Orphan Drug Act (21 U.S.C. 360ee(b)(3)). A medical food is formulated to be consumed (oral intake) or administered enterally (e.g. feeding/nasogastric tube) under medical supervision, e.g. by a physician. It is intended for the specific dietary management of a disease or condition, such as, e.g. dysbiosis of the gastrointestinal microbiota or a GI-tract disease described herein. Medical foods as used herein do not include food that is merely recommended by a physician as part of an overall diet to manage the symptoms or reduce the risk of a disease or condition. Medical foods comprising a microbiome regulator are foods that are synthetic (e.g., formulated and/or processed products, such as, being formulated for the partial or exclusive feeding of a patient by oral intake or enteral feeding by tube) and not naturally occurring foodstuff used in a natural state. Medical foods comprising microbiome regulators may represent a major component of the management of a GI tract disease or condition, e.g. the medical food may represent a partial or exclusive source of food for the subject in need of a medical food. In some embodiments, the subject has limited or impaired capacity to ingest, digest, absorb, or metabolize ordinary foodstuffs or certain nutrients. In other embodiments, the subject has other special medically determined nutrient requirements, the dietary management of which cannot be achieved by the modification of the normal diet alone. Medical foods comprising a microbiome regulator are administered to a subject in need thereof under medical supervision (which may be active and ongoing) and usually, the subject receives instructions on the use of the medical food. Medical foods may comprise one or more food additives, color additives, GRAS excipients and other agents or substances suitable for medical foods. Medical foods may further be be nutritionally complete or incomplete formulas.

Any microbiome regulator described herein may be formulated as a dietary supplement, e.g, for use in a method described herein.

Dietary supplements are regulated under the Dietary Supplement Health and Education Act (DSHEA) of 1994. A dietary supplement is a product taken by mouth that contains a "dietary ingredient" intended to supplement the diet. The "dietary ingredients" in these products may include, in addition to a microbiome regulator described herein, one or more of: vitamins, minerals, herbs or other botanicals, amino acids, and substances such as enzymes, organ tissues, glandulars, and metabolites. Dietary supplements can also be extracts or concentrates, and may be found in many forms such as tablets, capsules, softgels, gelcaps, liquids, or powders. They can also be in other forms, such as a bar, but if they are, information on their label must not represent the product as a conventional food or a sole item of a meal or diet. DSHEA requires that every supplement be labeled a dietary supplement and not as a general food.

The dosage form may be a packet, such as any individual container that contains a pharmaceutical microbiome regulator composition in the form of, e.g., a liquid (wash/rinse), a gel, a cream, an ointment, a powder, a tablet, a pill, a capsule, a depository, a single-use applicator or medical device (e.g. a syringe). For example, provided is also an article of manufacture, such as a container comprising a unit dosage form of the pharmaceutical microbiome regulator composition, and a label containing instructions for use of such microbiome regulator.

Forms of the compositions that can be used orally include tablets, push-fit capsules made of gelatin, as well as soft, sealed capsules made of gelatin and a plasticizer, such as glycerol or sorbitol. Tablets can be made by compression or molding, optionally with one or more accessory ingredients. Compressed tablets can be prepared by compressing in a suitable machine the active ingredient in a free-flowing form such as a powder or granules, optionally mixed with binders (e.g., povidone, gelatin, hydroxypropylmethyl cellulose), inert diluents, preservative, antioxidant, disintegrant (e.g., sodium starch glycolate, cross-linked povidone, cross-linked sodium carboxymethyl cellulose) or lubricating, surface active or dispersing agents. Molded tablets can be made by molding in a suitable machine a mixture of the powdered compound moistened with an inert liquid diluent. The tablets can optionally be coated or scored and can be formulated so as to provide slow or controlled release of the active ingredient therein. Tablets can optionally be provided with an enteric coating, to provide release in parts of the gut (e.g., colon, lower intestine) other than the stomach. All formulations for oral administration can be in dosages suitable for such administration. The push-fit capsules can contain the active ingredients in admixture with filler such as lactose, binders such as starches, and/or lubricants such as talc or magnesium stearate and, optionally, stabilizers. In soft capsules, the active compounds and/or other agents (e.g., prebiotics or probiotics) can be dissolved or suspended in suitable liquids, such as fatty oils, liquid paraffin, or liquid polyethylene glycols. In addition, stabilizers can be added. Dragee cores are provided with suitable coatings. For this purpose, concentrated sugar solutions can be used, which can optionally contain gum arabic, talc, polyvinyl pyrrolidone, carbopol gel, polyethylene glycol, or titanium dioxide, lacquer solutions, and suitable organic solvents or solvent mixtures. Dyestuffs or pigments can be added to the tablets or Dragee coatings for identification or to characterize different combinations of active compound doses.

Formulations for oral use can also be presented as hard gelatin capsules wherein the active ingredient is mixed with an inert solid diluent, for example, calcium carbonate, calcium phosphate or kaolin, or as soft gelatin capsules wherein the active ingredient is mixed with water soluble carrier such as polyethylene glycol or an oil medium, for example peanut oil, liquid paraffin, or olive oil.

In one embodiment, a provided microbiome regulator composition includes a softgel formulation. A softgel can contain a gelatin based shell that surrounds a liquid fill. The shell can be made of gelatin, plasticizer (e.g., glycerin and/or sorbitol), modifier, water, color, antioxidant, or flavor. The shell can be made with starch or carrageenan. The outer layer can be enteric coated. In one embodiment, a softgel formulation can include a water or oil soluble fill solution, or suspension of a composition covered by a layer of gelatin.

Solid formulations for oral use may comprise an enteric coating, which may control the location at which a microbiome regulator composition is absorbed in the digestive system. For example, an enteric coating can be designed such that a microbiome regulator composition does not dissolve in the stomach but rather travels to the small intestine, where it dissolves. An enteric coating can be stable at low pH (such as in the stomach) and can dissolve at higher pH (for example, in the small intestine). Material that can be used in enteric coatings includes, for example, alginic acid, cellulose acetate phthalate, plastics, waxes, shellac, and fatty acids (e.g., stearic acid, palmitic acid).

Formulations for oral use may also be presented in a liquid dosage from. Liquid preparations can be in the form of, for example, aqueous or oily suspensions, solutions, emulsions syrups or elixirs, or can be presented as a dry product for reconstitution with water or other suitable vehicle before use. Such liquid preparations can contain conventional additives, such as suspending agents, for example sorbitol, methyl cellulose, glucose syrup, gelatin, hydroxyethyl cellulose, carboxymethyl cellulose, aluminum stearate gel or hydrogenated edible fats, emulsifying agents, for example lecithin, sorbitan monooleate, acacia; nonaqueous vehicles (which can include edible oils), for example almond oil, oily esters such as glycerine, propylene glycol, or ethyl alcohol; preservatives, for example methyl or propyl p-hydoxybenzoate or sorbic acid, and, if desired, conventional flavoring or coloring agents. In some embodiments, liquid formulations can comprise, for example, an agent in water-in-solution and/or suspension form; and a vehicle comprising polyethoxylated castor oil, alcohol, and/or a polyoxyethylated sorbitan mono-oleate with or without flavoring. Each dosage form may comprise an effective amount of a microbiome regulator and can optionally comprise pharmaceutically inert agents, such as conventional excipients, vehicles, fillers, binders, disintegrants, pH adjusting substances, buffer, solvents, solubilizing agents, sweeteners, coloring agents, and any other inactive agents that can be included in pharmaceutical dosage forms for administration. Examples of such vehicles and additives can be found in Remington's Pharmaceutical Sciences, 17th edition (1985).

In another embodiment, an oral dosage form is provided comprising a microbiome regulator composition, wherein the oral dosage form is a syrup. The syrup can comprise about 1%, 5%, 10%, 15%, 20%, 25%, 30%, 35%, 40%, 45%, 50%, 55%, 60%, 65%, 70%, 75%, 80%, or 85% solid. The syrup can comprise about 15%, 20%, 25%, 30%, 35%, 40%, 45%, or 50% liquid, for example, water. The solid can comprise a microbiome regulator composition. The solid can be, for example, about 1-96%, 10-96%, 20-96%, 30-96%, 40-96%, 50-96%, 60-96%, 70-96%, 80-96%, or 90-96% microbiome regulator composition. In another embodiment, a microbiome regulator composition is formulated as a viscous fluid.

In one embodiment, the composition further comprises a foaming component, a neutralizing component, or a water-insoluble dietary fiber. A foaming component can be at least one member selected from the group consisting of sodium hydrogencarbonate, sodium carbonate, and calcium carbonate. In one embodiment a neutralizing component can be at least one member selected from the group consisting of citric acid, L-tartaric acid, fumaric acid, L-ascorbic acid, DL-malic acid, acetic acid, lactic acid, and anhydrous citric acid. In one embodiment a water-insoluble dietary fiber can be at least one member selected from the group consisting of crystalline cellulose, wheat bran, oat bran, cone fiber, soy fiber, and beet fiber. The formulation can contain a sucrose fatty acid ester, powder sugar, fruit juice powder, and/or flavoring material.

The pharmaceutical compositions provided herein can be in unit-dosage forms or multiple-dosage forms. A unit-dosage form, as used herein, refers to physically discrete unit suitable for administration to human in need thereof. In an embodiment, the unit-dosage form is provided in a package. Each unit-dose can contain a predetermined quantity of an active ingredient(s) sufficient to produce the desired therapeutic effect, in association with other pharmaceutical carriers or excipients. Examples of unit-dosage forms include, but are not limited to, ampoules, syringes, and individually packaged tablets and capsules. Unit-dosage forms can be administered in fractions or multiples thereof. A multiple-dosage form is a plurality of identical unit-dosage forms packaged in a single container, which can be administered in segregated unit-dosage form. Examples of multiple-dosage forms include, but are not limited to, vials, bottles of tablets or capsules, or bottles of pints or gallons. In another embodiment the multiple dosage forms comprise different pharmaceutically active agents. For example a multiple dosage form can be provided which comprises a first dosage element comprising a composition comprising a microbiome regulator and a second dosage element comprising a prebiotic, a therapeutic agent and/or a probiotic, which can be in a modified release form. In this example a pair of dosage elements can make a single unit dosage. In one embodiment a kit is provided comprising multiple unit dosages, wherein each unit comprises a first dosage element comprising a composition comprising a microbiome regulator and a second dosage element comprising probiotic, a pharmaceutical agent, a prebiotic or a combination thereof, which can be in a modified release form. **In** another embodiment the kit further comprises a set of instructions.

**In** some embodiments, the unit-dosage form comprises between about 0.001 mg to about 10 g of a microbiome regulator (e.g., a microbiome regulator disclosed herein). For example, the unit-dosage form may comprise about 0.001 mg to about 9.5 g, about 0.005 mg to about 9 g, about 0.01 mg to about 8.5 g, about 0.05 mg to about 8 g, about 0.075 mg to about 7.5 g, about 0.1 mg to about 7 g, about 0.25 mg to about 6.5 g, about 0.5 mg to about 6 g, about 0.75 mg to about 5.5 g, about 1 mg to about 5 g, about 2.5 mg to about 4.5 g, about 5 mg to about 4 g, about 7.5 mg to about 3.5 g, about 10 mg to about 3 g, about 12.5 mg to about 2.5 g, about 15 mg to about 2 g, about 17.5 mg to about 1.5 g, about 20 mg to about 1 g, about 25 mg to about 750 mg, about 50 mg to about 500 g, or about 75 mg to about 250 mg of the microbiome regulator.

**In** certain embodiments, the unit-dosage form comprises about 0.001 mg to about 100 mg, about 0.005 mg to about 75 mg, about 0.01 mg to about 50 mg, about 0.05 mg to about 25 mg, about 0.1 mg to about 10 mg, about 0.5 mg to about 7.5 mg, or about 1 mg to about 5 mg of a microbiome regulator. **In** other embodiments, the unit-dosage form comprises about 1 mg to about 100 mg, about 2.5 mg to about 75 mg, about 5 mg to about 50 mg, or about 10 mg to about 25 mg of the microbiome regulator. **In** other embodiments, the unit-dosage form comprises about 100 mg to about 10 g, about 250 mg to about 7.5 g, about 500 mg to about 5 g, about 750 mg to about 2.5 g, or about 1 g to about 2 g of the microbiome regulator.

**In** other embodiments, the unit-dosage form comprises between about 0.001 mL to about 1000 mL of the microbiome regulator (e.g., a microbiome regulator disclosed herein). For example, the unit-dosage form may comprise about 0.001 mL to about 950 mL, about 0.005 mL to about 900 mL, about 0.01 mL to about 850 mL, about 0.05 mL to about 800 mL, about 0.075 mL to about 750 mL, about 0.1 mL to about 700 mL, about 0.25 mL to about 650 mL, about 0.5 mL to about 600 mL, about 0.75 mL to about 550 mL, about 1 mL to about 500 mL, about 2.5 mL to about 450 mL, about 5 mL to about 400 mL, about 7.5 mL to about 350 mL, about 10 mL to about 300 mL, about 12.5 mL to about 250 mL, about 15 mL to about 200 mL, about 17.5 mL to about 150 mL, about 20 mL to about 100 mL, or about 25 mL to about 75 mL of the microbiome regulator.

In certain embodiments, the unit-dosage form comprises about 0.001 mL to about 10 mL, about 0.005 mL to about 7.5 mL, about 0.01 mL to about 5 mL, about 0.05 mL to about 2.5 mL, about 0.1 mL to about 1 mL, about 0.25 mL to about 1 mL, or about 0.5 mL to about 1 mL of the microbiome regulator. In other embodiments, the unit-dosage form comprises about 0.01 mL to about 10 mL, about 0.025 mL to about 7.5 mL, about 0.05 mL to about 5 mL, or about 0.1 mL to about 2.5 mL of the microbiome regulator. In other embodiments, the unit-dosage form comprises about 0.1 mL to about 10 mL, about 0.25 mL to about 7.5 mL, about 0.5 mL to about 5 mL, about 0.5 mL to about 2.5 mL, or about 0.5 mL to about 1 mL of the microbiome regulator.

In some embodiments, the unit-dosage form, e.g., a tablet, capsule (e.g., a hard capsule, push-fit capsule, or soft capsule), or softgel, has a body length of between about 0.1 inches to about 1.5 inches (e.g., about 0.5 inches and about 1 inch), or about 5 mm to about 50 mm (e.g., about 10 mm to about 25 mm). In some embodiments, the unit-dosage form. e.g., a tablet, capsule (e.g., a hard capsule, push-fit capsule, or soft capsule), or softgel, has an external diameter of about 0.05 inches to about 1 inch (e.g., about 0.1 inches to about 0.5 inches), or about 1 mm to about 25 mm (e.g., about 5 mm to about 10 mm).

Each unit-dosage form comprising a microbiome regulator may have a caloric value of between about 0.01 kcal and about 1000 kcal. For example, the unit-dosage form may have a caloric value of about 0.01 kcal to about 900 kcal, about 0.05 kcal to about 800 kcal, about 0.1 kcal to about 700 kcal, about 0.25 kcal to about 600 kcal, about 0.5 kcal to about 500 kcal, about 0.75 kcal to about 400 kcal, about 1 kcal to 300 kcal, about 5 kcal to about 200 kcal, or about 10 kcal to about 100 kcal. In certain embodiments, the unit-dosage form comprising a microbiome regulator has a caloric value of between 10 kcal to about 500 kcal. In other embodiments, the unit-dosage comprising a microbiome regulator has a caloric value of between 50 kcal to about 500 kcal. In still other embodiments, the unit-dosage form comprising a microbiome regulator may have a caloric value of about 0.001 kcal to about 100 kcal, about 0.005 kcal to about 90 kcal, about 0.01 kcal to about 80 kcal, about 0.025 kcal to about 70 kcal, about 0.05 kcal to about 60 kcal, about 0.075 kcal to about 50 kcal, about 0.1 kcal to 40 kcal, about 0.25 kcal to about 30 kcal, about 0.5 kcal to about 25 kcal, about 0.25 kcal to about 20 kcal, or about 0.1 kcal to about 10 kcal.

The unit-dosage form of the microbiome regulator may be formulated to dissolve in an aqueous solution (e.g., water, milk, juice, and the like) and is orally administered as a beverage, syrup, solution, or suspension. For example, the unit-form dosage comprising a microbiome regulator may comprise a cube, packet, lozenge, pill, tablet, capsule, candy, powder, elixir, or concentrated syrup formulated for dissolving into an aqueous solution prior to oral administration. In other embodiments, the unit-dosage form comprising a microbiome regulator may comprise a cube, packet, lozenge, pill, tablet, capsule, candy, powder, elixir, or concentrated syrup formulated to dissolve in vivo, e.g., in the mouth, stomach, intestine, or colon of the subject upon oral administration.

In some embodiments, the microbiome regulator composition is administered enterically. This preferentially includes oral administration, or by an oral or nasal tube (including nasogastric, nasojejunal, oral gastric, or oral jejunal). In other embodiments, administration includes rectal administration (including enema, suppository, or colonoscopy).

The dosage forms described herein can be manufactured using processes that are known to those of skill in the art. For example, for the manufacture of tablets, an effective amount of a prebiotic can be dispersed uniformly in one or more excipients or additives, for example, using high shear granulation, low shear granulation, fluid bed granulation, or by blending for direct compression. Excipients and additives include diluents, binders, disintegrants, dispersants, lubricants, glidants, stabilizers, surfactants, antiadherents, sorbents, sweeteners, and colorants, or a combination thereof. Diluents, also termed fillers, can be used to increase the bulk of a tablet so that a practical size is provided for compression. Non-limiting examples of diluents include lactose, cellulose, microcrystalline cellulose, mannitol, dry starch, hydrolyzed starches, powdered sugar, talc, sodium chloride, silicon dioxide, titanium oxide, dicalcium phosphate dihydrate, calcium sulfate, calcium carbonate, alumina and kaolin. Binders can impart cohesive qualities to a tablet formulation and can be used to help a tablet remain intact after compression. Non-limiting examples of suitable binders include starch (including corn starch and pregelatinized starch), gelatin, sugars (e.g., glucose, dextrose, sucrose, lactose and sorbitol), celluloses, polyethylene glycol, alginic acid, dextrin, casein, methyl cellulose, waxes, natural and synthetic gums, e.g., acacia, tragacanth, sodium alginate, gum arabic, xantan gum, and synthetic polymers such as polymethacrylates, polyvinyl alcohols, hydroxypropylcellulose, and polyvinylpyrrolidone. Lubricants can also facilitate tablet manufacture; non-limiting examples thereof include magnesium stearate, calcium stearate, stearic acid, glyceryl behenate, and polyethylene glycol. Disintegrants can facilitate tablet disintegration after administration, and non-limiting examples thereof include starches, alginic acid, crosslinked polymers such as, e.g., crosslinked polyvinylpyrrolidone, croscarmellose sodium, potassium or sodium starch glycolate, clays, celluloses (e.g., carboxymethylcelluloses (e.g., carboxymethylcellulose (CMC), CMC-Na, CMC-Ca)), starches, gums and the like. Non-limiting examples of suitable glidants include silicon dioxide, talc, and the like. Stabilizers can inhibit or retard drug decomposition reactions, including oxidative reactions. Surfactants can also include and can be anionic, cationic, amphoteric or nonionic. If desired, the tablets can also comprise nontoxic auxiliary substances such as pH buffering agents, preservatives, e.g., antioxidants, wetting or emulsifying agents, solubilizing agents, coating agents, flavoring agents (e.g., mint, cherry, anise, peach, apricot, licorice, raspberry, vanilla), and the like. Additional excipients and additives may include aluminum acetate, benzyl alcohol, butyl paraben, butylated hydroxy toluene, calcium disodium EDTA, calcium hydrogen phosphate dihydrate, dibasic calcium phosphate, tribasic calcium phosphate, candelilla wax, carnuba wax, castor oil hydrogenated, cetylpyridine chloride, citric acid, colloidal silicone dioxide, copolyvidone, corn starch, cysteine HCl, dimethicone, disodium hydrogen phosphate, erythrosine sodium, ethyl cellulose, gelatin, glycerin, glyceryl monooleate, glyceryl monostearate, glycine, HPMC pthalate, hydroxypropylcellulose, hydroxyl propyl methyl cellulose, hypromellose, iron oxide red or ferric oxide, iron oxide yellow, iron oxide or ferric oxide, magnesium carbonate, magnesium oxide, magnesium stearate, methionine, methacrylic acid copolymer, methyl paraben, silicified microcrystalline cellulose, mineral oil, phosphoric acid, plain calcium phosphate, anhydrous calcium phosphate, polaxamer 407, polaxamer 188, plain polaxamer, polyethylene oxide, polyoxy140 stearate, polysorbate 80, potassium bicarbonate, potassium sorbate, potato starch, povidone, propylene glycol, propylene paraben, propyl paraben, retinyl palmitate, saccharin sodium, selenium, silica, silica gel, fumed silica, sodium benzoate, sodium carbonate, sodium citrate dihydrate, sodium crossmellose, sodium lauryl sulfate, sodium metabisulfite, sodium propionate, sodium starch, sodium starch glycolate, sodium stearyl fumarate, sorbic acid, sorbitol, sorbitan monooleate, pregelatinized starch, succinic acid, triacetin, triethyl citrate, vegetable stearin, vitamin A, vitamin E, vitamin C, or a combination thereof. The amounts of these excipients and additives can be properly selected based on their relation to other components and properties of the preparation and production method.

Immediate-release formulations comprising a microbiome regulator can additionally comprise one or more combinations of excipients that allow for a rapid release of a pharmaceutically active agent (such as from 1 minute to 1 hour after administration). Controlled-release formulations (also referred to as sustained release (SR), extended-release (ER, XR, or XL), time-release or timed-release, controlled-release (CR), or continuous-release) refer to the release of a microbiome regulator from a dosage form at a particular desired point in time after the dosage form is administered to a subject.

In one embodiment a controlled release dosage form begins its release and continues that release over an extended period of time. Release can occur beginning almost immediately or can be sustained. Release can be constant, can increase or decrease over time, can be pulsed, can be continuous or intermittent, and the like. In one embodiment, a controlled release dosage refers to the release of an agent from a composition or dosage form in which the agent is released according to a desired profile over an extended period of time. In one aspect, controlled-release refers to delayed release of an agent from a composition or dosage form in which the agent is released according to a desired profile in which the release occurs after a period of time.

Pharmaceutical carriers or vehicles suitable for administration of the compounds provided herein include all such carriers known to those skilled in the art to be suitable for the particular mode of administration. In addition, the compositions can one or more components that do not impair the desired action, or with components that supplement the desired action, or have another action.

In a further aspect, the dosage form can be an effervescent dosage form. Effervescent means that the dosage form, when mixed with liquid, including water and saliva, evolves a gas. Some effervescent agents (or effervescent couple) evolve gas by means of a chemical reaction which takes place upon exposure of the effervescent disintegration agent to water or to saliva in the mouth. This reaction can be the result of the reaction of a soluble acid source and an alkali monocarbonate or carbonate source. The reaction of these two general compounds produces carbon dioxide gas upon contact with water or saliva. An effervescent couple (or the individual acid and base separately) can be coated with a solvent protective or enteric coating to prevent premature reaction. Such a couple can also be mixed with previously lyophilized particles (such as a microbiome regulator). The acid sources can be any which are safe for human consumption and can generally include food acids, acid and hydrite antacids such as, for example: citric, tartaric, amalic, fumeric, adipic, and succinics. Carbonate sources include dry solid carbonate and bicarbonate salt such as sodium bicarbonate, sodium carbonate, potassium bicarbonate and potassium carbonate, magnesium carbonate and the like. Reactants which evolve oxygen or other gasses and which are safe for human consumption are also included. In one embodiment citric acid and sodium bicarbonate are used.

In another aspect, the dosage form can be in a candy form (e.g., matrix), such as a lollipop or lozenge. In one embodiment an effective amount of a microbiome regulator is dispersed within a candy matrix. In one embodiment the candy matrix comprises one or more sugars (such as dextrose or sucrose). In another embodiment the candy matrix is a sugar-free matrix. The choice of a particular candy matrix is subject to wide variation. Conventional sweeteners (e.g., sucrose), sugar alcohols suitable for use with diabetic patients (e.g., sorbitol or mannitol), or other substances (e.g., described herein) may be employed. The candy base can be very soft and fast dissolving, or can be hard and slower dissolving. Various forms will have advantages in different situations.

A candy mass composition comprising an effective amount of a microbiome regulator can be orally administered to a subject in need thereof so that an effective amount of the microbiome regulator will be released into the subject's mouth as the candy mass dissolves and is swallowed. A subject in need thereof includes a human adult or child.

The dosage forms described herein can also take the form of pharmaceutical particles manufactured by a variety of methods, including but not limited to high-pressure homogenization, wet or dry ball milling, or small particle precipitation (e.g., nGimat's NanoSpray). Other methods useful to make a suitable powder formulation are the preparation of a solution of active ingredients and excipients, followed by precipitation, filtration, and pulverization, or followed by removal of the solvent by freeze-drying, followed by pulverization of the powder to the desired particle size. **In** one embodiment, the pharmaceutical particles have a final size of 3-1000 microns, such as at most 3, 4, 5, 6, 7, 8, 9, 10, 20, 30, 40, 50, 60, 70, 80, 90, 100, 150, 200, 250, 300, 350, 400, 450, 500, 550, 600, 650, 700, 750, 800, 850, 900, 950, 1000 microns. **In** one embodiment, the pharmaceutical particles have a final size of 10-500 microns. **In** another embodiment, the pharmaceutical particles have a final size of 50-600 microns. **In** another embodiment, the pharmaceutical particles have a final size of 100-800 microns.

**In** some embodiments, the pharmaceutical particles have a particular flowability or moisture content. The flowability of a pharmaceutical particle may be measured in a static angle of reponse, and in some embodiments may range between 10° and 50°. **In** one embodiment, the pharmaceutical particles described herein have a static angle of repose of between 10° and 50° , or in other embodiments, between 20° and 50° or 25° and 40°. The moisture content of a pharmaceutical powder may range from 0% to 100%. In some embodiments, the pharmaceutical particles described herein have moisture content of between about 0% and about 100%, or about 0.1%, about 0.25%, about 0.5%, about 0.75%, about 1%, about 2%, about 3%, about 4%, about 5%, about 6%, about 7%, about 8%, about 9%, about 10%, about 15%, about 20%, about 30%, about 40%, about 50%, about 60%, about 70%, about 80%, about 90%, or about 95%. In some embodiments, the pharmaceutical particles described herein have moisture content of between about 0.1% to about 10%, or about 0.1% to about 1%, or about 1% to about 10%.

Further disclosed is a method of making a unit-dosage form described herein, comprising providing a microbiome regulator (e.g., a microbiome regulator described herein); formulating the microbiome regulator into a unit-dosage form (e.g., a unit-dosage form described herein), packaging the unit-dosage form, labelling the packaged unit-dosage form, and/or selling or offering for sale the packaged and labeled unit-dosage form.

The unit-dosage forms described herein may also be processed. In one embodiment, the processing comprises one or more of: processing the dosage form into a pharmaceutical composition, e.g., formulating, combining with a second component, e.g., an excipient or buffer; portioning into smaller or larger aliquots; disposing into a container, e.g., a gas or liquid tight container; packaging; associating with a label; shipping or moving to a different location. In one embodiment, the processing comprises one or more of: classifying, selecting, accepting or discarding, releasing or withholding, processing into a pharmaceutical composition, shipping, moving to a different location, formulating, labeling, packaging, releasing into commerce, or selling or offering for sale, depending on whether the predetermined threshold is met. In some embodiments, the processed dosage forms comprise a microbiome regulator described herein.

In some embodiments, the processing comprises one or more of: processing the dosage form into a pharmaceutical composition, e.g., formulating, combining with a second component, e.g., an excipient or buffer; portioning into smaller or larger aliquots; disposing into a container, e.g., a gas or liquid tight container; packaging; associating with a label; shipping or moving to a different location. In one embodiment, the processing comprises one or more of: classifying, selecting, accepting or discarding, releasing or withholding, processing into a pharmaceutical composition, shipping, moving to a different location, formulating, labeling, packaging, releasing into commerce, or selling or offering for sale, depending on the determination.

The microbiome regulator compositions described herein may be formulated into any suitable dosage form, e.g. for oral or enteral administration. The dosage forms described herein can be manufactured using processes that are well known to those of skill in the art.

In some embodiments, the dosage forms are formulated to release the pharmaceutical compostions or preparations of microbiome regulators in a specific region(s) of the GI tract, such as the small or the large intestine. In some embodiments, the dosage forms are formulated to release the pharmaceutical compostions or preparations of microbiome regulators in a specific region(s) of the GI tract, such as the cecum, ascending colon, transverse colon, descending colon, sigmoid colon, and/or rectum.

In some embodiments, the dosage form for the microbiome regulator compositions described herein is an enzyme-responsive delivery system. For example, trypsin responsive polymers can be made using hydrogels that are crosslinked by peptides that are degraded by trypsin. Trypsin is active in the small intestine. Trypsin-responsive delivery systems can be used to target delivery of the microbiome regulator compositions to the small intestine. In another example, enzyme-digestible hydrogels consisting of poly(vinyl pyrrolidone) crosslinked with albumin are degraded in the presence of pepsin.

In some embodiments, the dosage form for the microbiome regulator compositions described herein is a delivery device that enables prolonged retention at a specific site in the GI tract. For example, a gastroretentive delivery system enables prolonged release of the microbiome regulator compositions to the stomach. Gastroretentive delivery may be used for the microbiome regulator compositions that modulate bacteria in the stomach or in the upper small intestine.

In some embodiments, the dosage form for the microbiome regulator compositions described herein is a mucoadhesive delivery system that adheres to the mucosal surfaces of the stomach. They are typically composed of polymers with numerous hydrogen-bonding groups, e.g., cross-linked polyacrylic acids, sodium carboxymethyl cellulose, sodium alginate, carrageenan, Carbopol 934P, or thiolated polycarbophil.

In some embodiments, the dosage form for the microbiome regulator compositions described herein is an expanding delivery system that rapidly increases in size in the stomach, which slows its passage through the pylorus. Such systems include systems that unfold in the stomach. For example, geometric shapes such as tetrahedrons, rings, disks, etc. can be packed into a gelatin capsule. When the capsule dissolves, the shape unfolds. The systems can be composed of one or more erodible polymer (e.g., hydroxypropyl cellulose), one or more nonerodible polymer (e.g., polyolefins, polyamides, polyurethanes). The microbiome regulator may then be dispersed within the polymer matrix. The retention times can be fine-tuned by the polymer blend. Alternatively, devices made out of elastic polymers that are stable in the acidic pH of the stomach but dissolve in the neutral/alkaline conditions further along the GI tract can be used. Such polymer formulations can prevent intestinal obstruction when the device exits the stomach. Supramolecular polymer gels crosslinked by hydrogen bonds between carboxyl groups may also be used, e.g. composed of poly(acryloyl 6-aminocaproic acid) (PA6ACA) and poly(methacrylic acid-co-ethyl acrylate) (EUDRAGIT L 100-55). Other systems include swellable excipients, such as collagen sponges. For example, a hydrogel matrix (e.g. a swellable core: polyvinyl pyrrolidone XL, Carbopol 934P, calcium carbonate) swells 2-50 times in the stomach. Superporous hydrogel composites swell to hundreds of times their original volume in a few minutes. Some systems exploit gas generation to achieve expansion, e.g. carbon dioxide-generating, expandable systems that are surrounded by a hydrophilic membrane.

In some embodiments, the dosage form for the microbiome regulator compositions described herein is a density-controlled delivery system. These systems are designed to either float or sink in gastric fluids, which delays their emptying from the stomach. For example, high-density systems enable the device to settle to the bottom of the stomach, below the pylorus, and thus avoid stomach emptying. Other systems are low-density/floating systems. Such devices may, e.g., comprise entrapped air in hollow chambers or may incorporate low-density materials like fats, oils, or foam powder. Low density may be achieved through swelling, e.g. hydrocolloid containing capsules dissolve upon contacting gastric fluid and the hydrocolloids swell to form a mucous body. Alternative polymers include: chitosans, sodium alginate, and glycerol monooleate matrix. Low density may be achieved through gas generation. For example, tablets loaded with carbonate and optionally citric acid generate carbon dioxide after contact with acidic aqueous media. The carbon dioxide generated is entrapped within the gelling hydrocolloid causing the system to float. Hydrocolloids include hydroxypropyl methylcellulose and carbopol 934P.

In some embodiments, the dosage form for the microbiome regulator compositions described herein employs a design to retain a device in the small or large intestine. The location-specific nature of the device is provided by a specific triggering method, e.g. pH, enzyme, etc. These include systems designed for mucoadhesion and also microneedle pills. Microneedle pills comprise a drug reservoir spiked with microneedles that is encapsulated in a pH-responsive coating. When the pill reaches the desired location in the GI tract and the coating dissolves, the microneedles enable the pill to become stuck to the lining of the GI tract. In other embodiments, the microneedle pills comprise a capsule that consists of two chemical compartments filled with citric acid and sodium bicarbonate, respectively. As the pill dissolves in the digestive system, barriers between the two substances erode, allowing them to mix and create a chemical reaction that pushes micro-needles of saccharides through the outer layer of the capsule and into the lining of the small intestine. The saccharide needles can be filled with drugs that are delivered into nearby blood vessels as the saccharide is absorbed.

In some embodiments, the dosage form for the microbiome regulator compositions described herein employs a pH sensitive polymer coating. For example, pH-dependent polymers (bi- or tri-phasic) can be insoluble at low pH levels (e.g. acid resistance in the stomach, pH 1-2) and become increasingly soluble as pH rises, e.g. to about 5.5 - 6.2 in the duodenum, to about pH 5.7 in the ascending colon, to about pH 6.4 in the cecum, to about pH 6.6 in the transverse colon, to about pH 7.0 in the descending colon, to about 7.2 - 7.5 in the ileum, or to about pH 7.5 in the distal small intestine. In one example, TARGIT^{™} technology may be used for site-specific delivery of the microbiome regulator compositions in the gastrointestinal (GI) tract. The system employs pH-sensitive coatings onto injection-moulded starch capsules to target the terminal ileum and colon.

In some embodiments, the dosage form for the microbiome regulator compositions described herein is a delayed release system or time controlled release system. Such systems usually employ enteric coatings that may be combined with pH sensitive and time release functions. For example, ETP (enteric coated time-release press coated) tablets may be used that are composed of three components: a microbiome regulator-containing core tablet (rapid release function), a press-coated, swellable hydrophobic polymer layer (e.g. hydroxypropyl cellulose layer (HPC), and a time release function. The duration of lag phase can be controlled either by weight or composition of polymer layer and an enteric coating layer (acid resistance function).

In some embodiments, the dosage form for the microbiome regulator compositions described herein employs Eudragit^{®} enteric coatings of tablets and capsules. Other suitable synthetic polymers include: Shellac, ethyl cellulose, cellulose acetate phthalate, hydroxypropylmethyl cellulose, polyvinyl acetate phthalate and poly glutamic acid coatings, such as poly-γ-glutamic acid (γ-PGA). These coatings combine both mucoadhesive and pH-dependent release strategies. To enhance colon targeted delivery Eudragits^{®} are methacrylic copolymers with varying side group compositions that alter the pH at which they are soluble. For example, for Eudragit^{®}-coated systems no significant drug release occurs in the stomach (e.g. at pH 1.4) and in the small intestine (e.g. at pH 6.3), while significant drug release can be seen at pH 7.8 in the ileocaecal region.

In some embodiments, the dosage form for the microbiome regulator compositions described herein is a microbial-triggered system, such as a polysaccharide based delivery system. Polysaccharide based delivery systems contain biodegradable and mucoadhesive polymer coatings, including coatings of chitosan and pectin. Other suitable natural polymers include, e.g., guar gum, inulin, cyclodextrin, dextran, amylase, chondrotin sulphate, and locust bean gum. These delivery systems can be used to target the microbiome regulator preparations to the small intestine. Coatings with naturally occurring polysaccharides like guar gum, xanthan gum, chitosan, alginates, etc. are degraded by colonic gut microbiota, e.g. enzymes such as, xylosidase, arabinosidase, galactosidase etc. For example, CODES^{™} technology may be used to deliver the microbiome regulator compositions. This system combines the polysaccharide coating with a pH-sensitive coating. In some embodiments, the system consists of a core tablet coated with three layers of polymer coatings: The outer coating is composed of Eudragit L. This coating gets dissolved in the duodenum and exposes the next coating. The next coating is composed of Eudragit E. This layer allows the release of lactulose present in the inner core. The lactulose gets metabolized into short chain fatty acids that lower the surrounding pH where the Eudragit E layer dissolves. The dissolving of Eudragit E results in the exposure of the microbiome regulator. The bacteria present in the colon are responsible for the degradation of polysaccharides that are released from the core tablet. The degradation of polysaccharides may result in organic acids formation that lowers the pH of the contents surrounding the tablet.

In some embodiments, the dosage form for the microbiome regulator compositions described herein is a pressure-controlled delivery system. The system employs the fact that higher pressures are encountered in the colon than in the small intestine. For example, for ethylcellulose systems that are insoluble in water, the release of microbiome regulators occurs following disintegration of a water-insoluble polymer capsule as a result of pressure in the lumen of the colon. The release profile may be adjusted by varying the thickness of the ethylcellulose, the capsule size and/or density of the capsule.

In some embodiments, the dosage form for the microbiome regulator compositions described herein is a pulsatile colon targeted delivery system. For example, the system can be a pulsincap system. The capsule which is employed comprises a plug that is placed in the capsule that controls the release of the microbiome regulator. A swellable hydrogel (e.g. hydroxyl propyl methyl cellulose (HPMC), poly methyl methacrylate or polyvinyl acetate) seals the drug content. When the capsule gets in contact with a fluid the plug is pushed off from the capsule and the microbiome regulator is released. The release profile can be controlled by varying the length and/or point of intersection of the plug with the capsule body. Another system is a port system. The capsule body is enclosed in a semi-permeable membrane. The insoluble plug consists of an osmotically active agent and the microbiome regulator. When the capsule gets in contact with a fluid the semi-permeable membrane permits inflow of the fluid which increases pressure in the capsule body. This leads to an expelling of the plug and release of the microbiome regulator.

In some embodiments, the dosage form for the microbiome regulator compositions described herein is an osmotically controlled colon targeted delivery system. An exemplary system, OROS-CT, consists of osmotic units (up to 5 or 6 push pull units) encapsulated in a hard gelatin capsule. The push pull units are bilayered with outer enteric impermeable membrane and inner semi-permeable membrane. The internal, central part of the push pull consists of the drug layer and push layer. The microbiome regulator is released through the semi-permeable membrane. The capsule body enclosing the push pull units is dissolved immediately after administration. In the GI tract the enteric impermeable membrane prevents water absorption. The enteric coating is dissolved in small intestine (higher pH, >7), water enters the unit through the semi-permeable membrane causing push layer to swell and force out the microbiome regulator preparation.

In some embodiments, the dosage form for the microbiome regulator compositions described herein is "smart pill" which can be used to release the microbiome regulator preparation just before reaching the ileocecal valve.

In some embodiments, the dosage form for the microbiome regulator compositions described herein is a rectally administered formulation. For example, enemas introduce a microbiome regulator composition in liquid formulation into the rectum. The volume administered is typically less than 10 mL. Suppositories introduce a microbiome regulator composition into the rectum. Suppositories are solid dosage forms that melt or dissolve when inserted into the rectum, releasing the microbiome regulators. Typical excipients for suppository formulations include cocoa butter, polyethylene glycols, and agar.

### Kits

Also disclosed are kits containing a course of treatment for a gastrointestinal disorder or condition. For example, a kit can comprise unit dosage forms of the pharmaceutical microbiome regulator composition, and a package insert containing instructions for use of the microbiome regulator composition in treatment of a gastrointestinal disorder or condition. The kits may include a microbiome regulator composition in suitable packaging for use by a subject in need thereof. Any of the compositions described herein can be packaged in the form of a kit. A kit can contain an amount of a microbiome regulator composition (optionally comprising a prebiotic substance, a probiotic bacterium, and/or a therapeutic agent) sufficient for an entire course of treatment, or for a portion of a course of treatment. Doses of a microbiome regulator composition can be individually packaged, or the microbiome regulator composition can be provided in bulk, or combinations thereof. Thus, in one embodiment, a kit provides, in suitable packaging, individual doses of a microbiome regulator composition that correspond to dosing points in a treatment regimen, wherein the doses are packaged in one or more packets.

In one embodiment, the microbiome regulator composition can be provided in bulk in a single container, or in two, three, four, five, or more than five containers. For example, \each container may contain enough of a microbiome regulator composition for a particular week of a treatment program that runs for a month. If more than one bulk container is provided, the bulk containers can be suitably packaged together to provide sufficient microbiome regulator composition for all or a portion of a treatment period. The container or containers can be labeled with a label indicating information useful to the subject in need thereof or the physician performing the treatment protocol, such as, e.g. dosing schedules.

The microbiome regulator may be packaged with other suitable substances, such as probiotic bacteria, prebiotic substances or other substances, as described herein. The other substance or substances can be packaged separately from the microbiome regulator composition, or mixed with the microbiome regulator composition, or combinations thereof. Thus, in one embodiment, kits include a dosage form containing all the ingredients intended to be used in a course of treatment or a portion of a course of treatment, e.g., a microbiome regulator composition and optionally buffers, excipients, etc., a probiotic, prebiotic or a therapeutic agent. In one embodiment, a microbiome regulator composition is packaged in one package or set of packages, and additional components, such as probiotic bacteria, prebiotics, and therapeutic agents are packaged separately from the microbiome regulator composition.

Kits can further include written materials, such as instructions, expected results, testimonials, explanations, warnings, clinical data, information for health professionals, and the like. In one embodiment, the kits contain a label or other information indicating that the kit is only for use under the direction of a health professional. The container can further include scoops, syringes, bottles, cups, applicators or other measuring or serving devices.

### Administration of Microbiome Regulators

For any microbiome regulator compound or composition used in a method described herein, a therapeutically effective dose can be estimated initially from laboratory animal models well known to those of skill in the art. Such information can be used to more accurately determine useful doses in humans. Initial dosages can also be estimated from in vitro or in vivo data. Initial dosages can also be formulated by comparing the effectiveness of the compounds used in the methods described herein in model assays with the effectiveness of known compounds. For instance, initial dosages can be formulated by comparing the effectiveness of the microbiome regulator preparations in model assays with the effectiveness of other compounds that have shown efficacy in treating the present conditions. In this method, an initial dosage can be obtained by multiplying the ratio of effective concentrations obtained in the model assay for the microbiome regulator preparations used in methods described herein and the control compound by the effective dosage of the control compound. For example, if a preparation useful in a present method is twice as effective in a model assay as a known compound (e.g., the EC₅₀ of the microbiome regulator preparation is equal to one-half the EC₅₀ of the known compound in the same assay), an initial effective dosage of the microbiome regulator preparation would be one-half the known dosage for the known compound. Using these initial guidelines an effective dosage in subjects, such as humans, can readily be determined by one of ordinary skill. Dosage amount and interval may be adjusted individually to provide levels of the microbiome regulator compound which are sufficient to maintain therapeutic effect. One of skill in the art will be able to optimize therapeutically effective local dosages without undue experimentation.

Depending upon the disorder and subject to be treated and the route of administration, the compositions may be administered at varying doses. In one embodiment, the smallest effective amount or dose of microbiome regulator is used. In some embodiments, the microbiome regulator is administered in a dose from about 0.01mg/kg to about 10,000 mg/kg, from about 0.1mg/kg to about 1,000 mg/kg, from about 1mg/kg to about 100 mg/kg, 0.05 mg/kg to about 5,000 mg/kg, from about 0.5 mg/kg to about 5,000 mg/kg, from about 5 mg/kg to about 500 mg/kg. This dose may be given as mg/kg/day and may be administered as an initial dose or may be increased or decreased over time (e.g., days or week) to reach a final dose.

In some embodiments, a symptom of a gastrointestinal disease, disorder or condition in a subject exhibiting the symptoms is decreased or eliminated by administering to the subject increasing, decreasing or constant amounts (or doses) of a microbiome regulator composition for a period of time (e.g. a treatment period).

In one embodiment, the composition contains beneficial, commensal and/or probiotic bacterial strains in an amount comprised from 1x10⁷ to 1x10¹³ CFU/dose and bacterial strain, or from 1x10⁹ to 1x10¹¹ CFU/dose and bacterial strain.

In some embodiments, the pharmaceutical composition is administered one, two, or three times a day. In some embodiments, the pharmaceutical composition is administered twice a day. In some embodiments, the pharmaceutical composition is administered each day for a predetermined number of days (the treatment period). In some embodiments, the treatment period is 1, 2, 3, 4, 5, 6, 7, 8, 9, 10, 11, 12, 13, 14, 21, 28, 35, 42, 49, 56, 63, 70, 100, 200, 300 or 365 days. In some embodiments, the treatment period is 1, 2, 3, 4, 5, 6, 7, 8, 9, 10, 11, or 12 months. In some embodiments, the treatment period is 1, 2, 3, 4, 5, 6, 7, 8, 9, 10, 11, 12 years, or life-long.

In one embodiment, the total duration of treatment periods for a gastrointestinal disease, disorder or condition can be from about one day to 10 years, one day to 1 year, 1 day to 6 months, 1 day to 3 months, 1 day to 1 months, one day to one week, one day to five days, one day to 10 days, one week to about 12 weeks, or about four weeks to about ten weeks, or about four weeks to about eight weeks, or about six weeks. The subject may undergo a suitable number of treatment periods, such as, e.g. 1, 2, 3, 4, 5, 6, 7, 8, 9, 10 or more than 10 treatment periods. During a treatment period, the subject takes a microbiome regulator composition described herein, optionally along with ingestion of prebiotic and/or probiotic containing food products. In one embodiment a microbiome regulator composition can also be administered in combination with another substance (such as a probiotic or commensal beneficial bacteria, a prebiotic substance or a therapeutic agent), as described herein.

In some embodiments, the microbiome regulator composition may also be combined with an antibiotic that disrupts normal gastrointestinal microbiota growth. Typically durations for antibiotic treatments are 1-14 days, or 2-10 days, or 5-7 days. In some embodiments, a microbiome regulator described herein is administered to a subject in need thereof immediately after one or more antibiotic treatment(s) has ended. During a course of antibiotic treatment, the microbiome regulator composition may be provided at the initiation of antibiotic treatment; shortly following antibiotic treatment, e.g. 1, 2, 3, 4, 5, 6, 7, or more days following treatment; or may be administered upon diagnosis of undesirable pathogen growth.

In some embodiments, the pharmaceutical microbiome regulator composition may also be combined with a dysbiosis-causing drug, e.g. a drug that disrupts normal gastrointestinal microbiota growth, e.g. a chemotherapeutic drug, an anti-diabetic drug, an immune-suppressive drug, an antimicrobial drug, an anti-psychotic drug, a proton pump inhibitor drug, or a non-steroid anti-inflammatory drug (NSAID). The pharmaceutical microbiome regulator composition, in some embodiments, reduces the drug- or treatment-induced symptoms in a human subject. The symptoms include digestive abnormalities, such as, e.g., weight-gain, constipation, heartburn, upset stomach, gas, bloating, flatulence, diarrhea, abdominal pain, cramping, nausea, and vomiting. In some embodiments, a microbiome regulator is administered to a subject in need thereof immediately after one or more drug treatment(s) has ended (e.g. 1 hour, 6 hours, 12 hours, 24 hours, 36 hours, 48 hours, 3 days, 4 days, 5 days, 6 days, 7 days, 2 weeks, 3 weeks or 4 weeks after the antibiotic treatment has ended). During a course of drug treatment, the pharmaceutical microbiome regulator composition may be provided prior to the initiation of drug treatment (e.g. 1, 2, 3, 4, 5, 6, 7 days prior); at the day of initiation of drug treatment; or shortly following antibiotic treatment, e.g. 1, 2, 3, 4, 5, 6, 7, or more days following treatment, and may optionally be provided only initially (e.g. for a short period) or throughout the duration of the drug-treatment, and may even be continued for a desired period after the drug treatment period has ended (e.g. for 1-7days, 1-14 days, or 1-21 days thereafter). In some embodiments, administration of the pharmaceutical microbiome regulator composition is initiated or continued when one or more adverse effects occur and/or are diagnosed (e.g. digestive abnormalities or pathogen growth) in conjunction with the drug treatment. In some embodiments, the treatment agent causing a dysbiosis is not a drug but radiation treatment or surgery and the pharmaceutical microbiome regulator composition may also be administered as described herein.

In some embodiments, the total number and duration of treatment periods is based on a subject's response to the treatment. For example, an individual can experience a reduction in symptoms after 1, 2, 3, 4, 5, 6, 7, 8, 9, 10, 11, 12, 13, or 14 days of treatment with a microbiome regulator composition. In another example, an individual can experience a reduction in symptoms after 1, 2, 3, 4, 5, 6, 7, 8, 9, 10, 11, 12 months of treatment with a microbiome regulator composition. Thus, the duration of treatment is determined by an individual subject's response to a microbiome regulator composition and the onset of relief from one or more symptoms. Thus, a subject can experience symptoms at a given dose of a microbiome regulator composition and can require that the subject stay at that dose, or a lower dose, until symptoms subside. Thus, in one embodiment, the duration of the treatment is not determined at the outset, but continues until the maximum dose of a microbiome regulator composition is achieved per day, or until the desired level of reduction in symptoms is achieved. In one embodiment, the treatment is continuous.

In one embodiment, a subject can be given one dose for the first treatment period during a treatment regimen and a second dose during a second treatment period. For example, a subject can be administered one dose of microbiome regulator composition for a one week period and a second dose for a subsequent one week period.

A subject may self-administer a microbiome regulator composition and the microbiome regulator composition is supplied or recommended (or prescribed) by a health professional, e.g., a physician or other qualified health professional and optionally test results (e.g. obtained for biomarkers from samples taken from the subject) and/or health changes and treatment endpoints are monitored by a health professional. In some embodiments, the microbiome regulator composition is administered by a health professional.

In one embodiment, a subject in need thereof can undergo repeated courses of treatment with a microbiome regulator composition. The course of treatment can be repeated when symptoms reappear or increase to an undesirable level. Alternatively, the course of treatment can be repeated at regular or predetermined intervals. Thus, treatment can be repeated after about one month, two months, three months, four months, six months, eight months, ten months, one year, 18 months, two years, three years, four years, five years, or more than five years, or any combination thereof (e.g., treatment can be repeated after one year, then every two to five years thereafter). The treatment can be repeated in the same form (e.g., duration, dosage, timing of dosage, additional substances, etc.) as used in the first treatment or it can be modified. For example, treatment duration can be shortened or lengthened, dosage can be increased or decreased. Optionally, treatment with the microbiome regulator can occur in combination with a different number or compositions of agents, e.g., containing more or less of other substances, or fewer or more substances (such as, e.g., a prebiotic substance, a probiotic bacterium or a therapeutic agent) in addition to the microbiome regulator.

Additional substances can be given in conjunction with a microbiome regulator composition. These substances can enhance the action of the doses of microbiome regulator by, e.g., encouraging the growth of bacteria in the GI tract that alleviate symptoms of the gastrointestinal disease, disorder or condition, increasing adhesion of probiotic or beneficial commensal bacteria in the niche or in the gut. These substances can be given prior to treatment with microbiome regulator, during treatment with microbiome regulator, after treatment with microbiome regulator, or any combination thereof. If administered during microbiome regulator treatment, they can be administered with the dose of microbiome regulator being given, or before or after the dose of microbiome regulator, or any combination thereof. In one embodiment substances of use in conjunction with a microbiome regulator composition include a probiotic microbe(s), prebiotics, therapeutic agents, or buffers/carriers/excipients. One or more of these substances can be used in combination with microbiome regulator composition at any suitable time before, during, after treatment, or some combination thereof.

### Identification of microbial (e.g. bacterial) constituents

In some embodiments, the microbiome regulator described herein is administered to a subject to increase the growth of beneficial bacteria and/or to decrease the growth of pathogens in the GI tract. In some embodiments, the microbial community is shifted by the microbiome regulator toward that of a healthy state. The microbial changes occurring in the GI tract can be analyzed using any number of methods known in the art and described herein.

As one quantitative method for determining whether a microbiome regulator composition results in a shift of the population of bacteria in the GI tract, quantitative PCR (qPCR) can be performed. Genomic DNA can be extracted from samples using commercially-available kits, such as the Mo Bio Powersoil^{®}-htp 96 Well Soil DNA Isolation Kit (Mo Bio Laboratories, Carlsbad, CA), the Mo Bio Powersoil^{®} DNA Isolation Kit (Mo Bio Laboratories, Carlsbad, CA), or the QIAamp DNA Stool Mini Kit (QIAGEN, Valencia, CA) according to the manufacturer's instructions.

In some embodiments, qPCR can be conducted using HotMasterMix (5PRIME, Gaithersburg, MD) and primers specific for certain (e.g. beneficial or desired) bacteria and may be conducted on a MicroAmp^{®} Fast Optical 96-well Reaction Plate with Barcode (0.1mL) (Life Technologies, Grand Island, NY) and performed on a BioRad C1000^{™} Thermal Cycler equipped with a CFX96^{™} Real-Time System (BioRad, Hercules, CA), with fluorescent readings of the FAM and ROX channels. The Cq value for each well on the FAM channel is determined by the CFX Manager^{™} software version 2.1. The log₁₀(cfu/ml) of each experimental sample is calculated by inputting a given sample's Cq value into linear regression model generated from the standard curve comparing the Cq values of the standard curve wells to the known log₁₀(cfu/ml) of those samples. The skilled artisan may employ alternative qPCR modes.

In some embodiments, the microbial constituents are identified by characterizing the DNA sequence of microbial 16S small subunit ribosomal RNA gene (16S rRNA gene). 16S rRNA gene is approximately 1,500 nucleotides in length, and in general is highly conserved across organisms, but contain specific variable and hypervariable regions (V1-V9) that harbor sufficient nucleotide diversity to differentiate species- and strain-level taxa of most organisms. These regions in bacteria are defined by nucleotides 69-99, 137-242, 433-497, 576-682, 822-879, 986-1043, 1117-1173, 1243-1294 and 1435-1465 respectively using numbering based on the E. coli system of nomenclature. (See, e.g., Brosius et al., PNAS 75(10):4801-4805 (1978)).

Composition of a microbial community can be deduced by sequencing full 16S rRNA gene, or at least one of the V1, V2, V3, V4, V5, V6, V7, V8, and V9 regions of this gene or by sequencing of any combination of variable regions from this gene (e.g. V1-3 or V3-5). In one embodiment, the V1, V2, and V3 regions are used to characterize a microbiota. In another embodiment, the V3, V4, and V5 regions are used to characterize a microbiota. In another embodiment, the V4 region is used to characterize a microbiota.

Sequences that are at least 97% identical to each other are grouped into Operational Taxonomic Units (OTUs). OTUs that contain sequences with 97% similarity correspond to approximately species level taxa. At least one representative sequence from each OTU is chosen, and is used to obtain a taxonomic assignment for an OTU by comparison to a reference database of highly curated 16S rRNA gene sequences (such as Greengenes or SILVA databases). Relationship between OTUs in a microbial community could be deduces by constructing a phylogenetic tree from representative sequences from each OTU.

Using well known techniques, in order to determine the full 16S sequence or the sequence of any variable region of the 16S sequence, genomic DNA is extracted from a bacterial sample, the 16S rRNA (full region or specific variable regions) amplified using polymerase chain reaction (PCR), the PCR products are cleaned, and nucleotide sequences delineated to determine the genetic composition of 16S rRNA gene or a variable region of the gene. If full 16S sequencing is performed, the sequencing method used may be, but is not limited to, Sanger sequencing. If one or more variable regions is used, such as the V4 region, the sequencing can be, but is not limited to being performed using the Sanger method or using a next-generation sequencing method, such as an Illumina method. Primers designed to anneal to conserved regions of 16S rRNA genes could contain unique barcode sequences to allow characterizing multiple microbial communities simultaneously.

As another method to identify microbial composition is characterization of nucleotide markers or genes, in particular highly conserved genes (e.g., "house-keeping" genes), or a combination thereof, or whole genome shotgun sequence (WGS). Using well defined methods, DNA extracted from a bacterial sample will have specific genomic regions amplified using PCR and sequenced to determine the nucleotide sequence of the amplified products. In the WGS method, extracted DNA will be fragmented into pieces of various length (from 300 to about 40,000 nucleotides) and directly sequenced without amplification. Sequence data can be generated using any sequencing technology including, but not limited to Sanger, Illumina, 454 Life Sciences, Ion Torrent, ABI, Pacific Biosciences, and/or Oxford Nanopore.

In addition to the 16S rRNA gene, a selected set of genes that are known to be marker genes for a given species or taxonomic group is analyzed to assess the composition of a microbial community. These genes are alternatively assayed using a PCR-based screening strategy. For example, various strains of pathogenic *Escherichia coli* are distinguished using genes that encode heat-labile (LTI, LTIIa, and LTIIb) and heat-stable (STI and STII) toxins, verotoxin types 1, 2, and 2e (VT1, VT2, and VT2e, respectively), cytotoxic necrotizing factors (CNF1 and CNF2), attaching and effacing mechanisms *(eaeA),* enteroaggregative mechanisms (Eagg), and enteroinvasive mechanisms (Einv). The optimal genes to utilize to determine the taxonomic composition of a microbial community by use of marker genes are familiar to one with ordinary skill in the art of sequence based taxonomic identification.

Nucleic acid sequences are analyzed to define taxonomic assignments using sequence similarity and phylogenetic placement methods or a combination of the two strategies. A similar approach is used to annotate protein names, protein function, transcription factor names, and any other classification schema for nucleic acid sequences. Sequence similarity based methods include BLAST, BLASTx, tBLASTn, tBLASTx, RDP-classifier, DNAclust, RapSearch2, DIAMOND, and various implementations of these algorithms such as QIIME or Mothur. These methods map a sequence read to a reference database and select the best match. Common databases include KEGG, MetaCyc, NCBI non-redundant database, Greengenes, RDP, and Silva for taxonomic assignments. For functional assignments, reads are mapped to various functional databases such as COG, KEGG, BioCyc, MetaCyc, and the Carbohydrate-Active Enzymes (CAZy) database. Microbial clades are assigned using software including MetaPhlAn.

Preparations of microbiome regulators may also be selected based on their ability to increase the expression of microbial proteins associated with healthy states or to decrease the expression of microbial proteins associated with diseased states. Proteomic analysis of microbial populations can be performed following protocols known to one skilled in the art (e.g., Cordwell, Methods in Molecular Biology, 2004, 266:115). To identify differentially expressed proteins (for example, to identify changes in protein expression upon treatment of microbial populations with microbiome regulators), proteomic analysis can be performed as described, e.g., in Juste et al. (Gut, 2014, 63:1566). For example, the protein is isolated from the microbial lysates of two samples (for example, an untreated microbial population and a population that has been treated with microbiome regulators). Each protein sample is labeled and analyzed by two-dimensional differential gel electrophoresis (2D-DIGE). Gels are stained and protein spots identified as being significantly different between the two samples are excised, digested, and analyzed by liquid chromatography-tandem mass spectrometry (LC-MS/MS). X! TandemPipeline (http://pappso.inra.fr/bioinfo/xtandempipeline/) can be used to identify differentially expressed proteins.

### Screening of Microbiome Regulators and Compositions Thereof

In order to characterize the effects of the microbiome regulators described herein, provided is an in vitro microplate-based screening system that demonstrates the efficacy of the microbiome regulators compositions, including the ability to inhibit (or antagonize/suppress) the growth of certain microbial constituents and the ability to promote (or increase) the growth of other microbial constituents. These methods provide novel compositions of microbiome regulators that are able to improve the health of the gastrointestinal microbiome and/or promote health of the subject. Some suitable screening methods are described in the Examples.

In some embodiments, microbiome regulators are provided with the ability to exclude the growth of pathogenic bacteria, e.g. by promoting the growth of beneficial bacteria. Provided herein are microbiome regulators that promote the growth of bacterial strains that are able to significantly reduce the rate of pathogen growth and/or capable of partially or fully restoring a bacterial community that is associated with a healthy GI tract. The effect of the microbiome regulators on bacterial growth can be tested in in vitro assays and using laboratory animal models. The bacteria can be collected from samples taken from the niche of interest (e.g. a stool sample containing feces) and propagated by methods known in the art. Competitive in vitro growth assays may then be performed using conditions that are suitable for bacteria from the niche of interest, e.g. conditions that may mimic the natural environment of the niche, e.g. the GI tract or a subset thereof, such as the large and small intestine. Such conditions include, but are not limited to aerobic, anaerobic, low/high/neutral pH, ambient temperature, etc.

In some disclosures, in vivo assays are performed to detect the effect of the microbiome regulator on bacterial growth in the GI tract. In order to determine whether the microbiome regulator composition modulates the microbial populations in the GI tract of a subject, a laboratory animal model, such as a mouse model of human disease, can be used. The model can begin by evaluating the microbiota of the mice. Qualitative assessments can be accomplished using 16S profiling of the microbial community in the GI tract of normal mice. It can also be accomplished by full genome sequencing, whole genome shotgun sequencing (WGS), or traditional microbiological techniques. Quantitative assessments can be conducted using quantitative PCR (qPCR), described herein, or by using traditional microbiological techniques and counting colony formation. Optionally, the mice can receive an antibiotic treatment to mimic the condition of a disturbed gastrointestinal microbiota in which the GI microbiota exhibit a dysbiosis. It is known that antibiotic treatment can decrease the taxonomic richness, diversity, and evenness of gut communities, including a reduction of abundance of a significant number of bacterial taxa. (Dethlefsen et al., PLoS Biology 6(11):3280 (2008)).

In some disclosures, the screening method include: i) providing a plurality of preparations of microbiome regulators, ii) subjecting the preparation to one or more selection screen, iii) selecting a preparation of microbiome regulators based on the selection screens, and optionally iv) isolating the selected preparation of microbiome regulators. Suitable selection screens are known to one of ordinary skill and any necessary experimental parameters may be adjusted with only routine experimentation. In some embodiments, the selection screen is an in vitro assay in which one or more bacterial taxa are grown in a growth medium and the growth is monitored in the presence of the microbiome regulators and compared to growth in the absence of the microbiome regulators. Any practical number of bacterial taxa may be grown in the medium, such as, e.g., 1, 2, 3, 4, 5, 6, 7, 8, 9, 10, 15, 20, 30, 40, or 50 taxa. In some embodiments, a preparation of microbiome regulators is selected that is capable of modulating (e.g. increasing or decreasing) the growth of at least 1, 2, 3, 4, 5, 6, 7, 8, 9, 10, 15, or at least 20 bacterial taxa.

In some embodiments, the growth of the one or more bacterium is increased by at least 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 100%, 110%, 120%, 130%, 140%, 150%, 160%, 170%, 180%, 190%, 200%, 250%, 300%, 350%, 400%, 450%, 500%, 600%, 700%, 800%, 900%, or by at least 1000% after 1 hour, 6 hours, 12 hours, 18 hours or 24 hours of contacting.

In other embodiments, the growth of the one or more bacterium is decreased by at least 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 96%, 97%, 98%, 99%, or by at least 99.9% after 1 hour, 6 hours, 12 hours, 18 hours or 24 hours of contacting.

In some embodiments, the microbiome regulator also modulates the concentration of one or more microbial metabolite selected from the group consisting of the metabolites listed in Table 2. In some embodiments, the metabolite concentration is increased by at least 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 100%, 110%, 120%, 130%, 140%, 150%, 160%, 170%, 180%, 190%, 200%, 250%, 300%, 350%, 400%, 450%, 500%, 600%, 700%, 800%, 900%, or by at least 1000% after 1 hour, 6 hours, 12 hours, 18 hours or 24 hours of contacting. In other embodiments, the metabolite concentration is decreased by at least 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 96%, 97%, 98%, 99%, or by at least 99.9% after 1 hour, 6 hours, 12 hours, 18 hours or 24 hours of contacting.

In some disclosures, the screening methods are carried out using a suitable laboratory animal model. For example, a preparation of microbiome regulators may be administered to a laboratory animal and after a period of time a sample is taken from the laboratory animal's GI tract and analyzed for growth of bacterial taxa. The laboratory animal may, if desired, be contacted with pathogens or other bacteria to facilitate colonization of the animal prior to or concurrent with administration of the microbiome regulator. In some disclosures, a preparation of microbiome regulators is selected that is capable of modulating (e.g. increasing or decreasing) the growth of at least 1, 2, 3, 4, 5, 6, 7, 8, 9, 10, 15, or at least 20 bacterial taxa in the laboratory animal.

### Proteomic Analysis of Microbial Populations

Preparations of microbiome regulators may be selected based on their ability to increase the expression of microbial proteins associated with healthy states or to decrease the expression of microbial proteins associated with diseased states. Proteomic analysis of microbial populations can be performed following protocols known to one skilled in the art (e.g., Cordwell, Methods in Molecular Biology, 2004, 266:115). To identify differentially expressed proteins (for example, to identify changes in protein expression upon treatment of microbial populations with a microbiome regulator), proteomic analysis can be performed as described, e.g., in Juste et al. (Gut, 2014, 63:1566). For example, the protein is isolated from the microbial lysates of two samples (for example, an untreated microbial population and a population that has been treated with a microbiome regulator). Each protein sample is labeled (e.g., with a fluorescent dye, e.g., Cy3 or Cy5 CyDye DIGE Fluor minimal dye, GE Healthcare) and analyzed by two-dimensional differential gel electrophoresis (2D-DIGE). Gels are stained and protein spots identified as being significantly different between the two samples are excised, digested, and analyzed by liquid chromatography-tandem mass spectrometry (LC-MS/MS). X!TandemPipeline (http://pappso.inra.fr/bioinfo/xtandempipeline/) can be used to identify differentially expressed proteins.

Preparations of microbiome regulators may also be selected for administration to a human subject based on their effect on the presence of microbial fermentation products. For example, preparations of microbiome regulators can be selected for their ability to induce or promote growth of bacteria that produce short chain fatty acids such as propionate (propionic acid), acetate, and/or butyrate (butyric acid). Similarly, preparations of a microbiome regulator can be selected for their ability to induce or promote growth of bacteria that produce lactic acid, which can modulate the growth of other bacteria by producing an acidic environment. Such analysis may also be used to pair probiotic bacteria with microbiome regulators such that the microbiome regulator is a substrate for the production of the desired fermentation products.

The metabolites that are present in fresh or spent culture media or in biological samples collected from humans may be determined using methods described herein. Unbiased methods that may be used to determine the relative concentration of metabolites in a sample and are known to one skilled in the art, such as gas or liquid chromatography combined with mass spectrometry or ¹H-NMR. These measurements may be validated by running metabolite standards through the same analytical systems.

In the case of gas chromatography-mass spectrometry (GC-MS) or liquid-chromatography-mass spectrometry (LC-MS) analysis, polar metabolites and fatty acids could be extracted using monophasic or biphasic systems of organic solvents and an aqueous sample and derivatized (Fendt et al., Nat Commun, 2013, 4:2236; Fendt et al., Cancer Res, 2013, 73:4429; Metallo et al., Nature, 2011, 481:380). An exemplary protocol for derivatization of polar metabolites involves formation of methoxime-tBDMS derivatives through incubation of the metabolites with 2% methoxylamine hydrochloride in pyridine followed by addition of N-tertbutyldimethylsilyl-N-methyltrifluoroacetamide (MTBSTFA) with 1% tert-butyldimethylchlorosilane (t-BDMCS). Non-polar fractions, including triacylglycerides and phospholipids, may be saponified to free fatty acids and esterified to form fatty acid methyl esters, for example, either by incubation with 2% H₂SO₄ in methanol or by using Methyl-8 reagent (Thermo Scientific). Derivatized samples may then be analyzed by GC-MS using standard LC-MS methods, for example, a DB-35MS column (30 m x 0.25 mm i.d. x 0.25 µm, Agilent J&W Scientific) installed on a gas chromatograph (GC) interfaced with an mass spectrometer (MS). Mass isotopomer distributions may be determined by integrating metabolite ion fragments and corrected for natural abundance using standard algorithms, such as those adapted from Fernandez et al. (Fernandez et al., J Mass Spectrom, 1996, 31:255). In the case of liquid chromatography-mass spectrometry (LC-MS), polar metabolites may be analyzed using a standard benchtop LC-MS/MS equipped with a column, such as a SeQuant ZIC-pHILIC Polymeric column (2.1 x 150 mm; EMD Millipore). Exemplary mobile phases used for separation could include buffers and organic solvents adjusted to a specific pH value.

In combination or in the alternative, extracted samples may be analyzed by ¹H-nuclear magnetic resonance (¹H-NMR). Samples may be combined with isotopically enriched solvents such as D2O, optionally in the presence of a buffered solution (e.g., Na₂HPO₄, NaH₂PO₄ in D₂O, pH 7.4). Samples may also be supplemented with a reference standard for calibration and chemical shift determination (e.g., 5 mM 2,2-dimethyl-2-silapentane-5-sulfonate sodium salt (DSS-d₆, Isotec, USA)). Prior to analysis, the solution may be filtered or centrifuged to remove any sediment or precipitates, and then transferred to a suitable NMR tube or vessel for analysis (e.g., a 5 mm NMR tube). ¹H-NMR spectra may be acquired on a standard NMR spectrometer, such as an Avance II + 500 Bruker spectrometer (500 MHz) (Bruker, DE), equipped with a 5 mm QXI-Z C/N/P probe-head) and analyzed with spectra integration software (such as Chenomx NMR Suite 7.1; Chenomx Inc., Edmonton, AB). (Duarte et al., 1H-NMR protocol for exometabolome analysis of cultured mammalian cells, Methods Mol Biol, 2014:237-47). Alternatively, ¹H-NMR may be performed following other published protocols known in the art (Chassaing et al., Lack of soluble fiber drives diet-induced adiposity in mice, Am J Physiol Gastrointest Liver Physiol, 2015; Bai et al., Comparison of Storage Conditions for Human Vaginal Microbiome Studies, PLoS ONE, 2012:e36934).

### EXAMPLES

The invention is further illustrated by the following examples. The examples are provided for illustrative purposes only, and are not to be construed as limiting the scope or content of the invention in any way. The practice of the present invention will employ, unless otherwise indicated, conventional methods of protein chemistry, biochemistry, recombinant DNA techniques and pharmacology, within the skill of the art. Such techniques are explained fully in the literature. See, e.g., T.E. Creighton, Proteins: Structures and Molecular Properties (W.H. Freeman and Company, 1993); Green & Sambrook et al., Molecular Cloning: A Laboratory Manual, 4th Edition (Cold Spring Harbor Laboratory Press, 2012); Colowick & Kaplan, Methods In Enzymology (Academic Press); Remington: The Science and Practice of Pharmacy, 22nd Edition (Pharmaceutical Press, 2012); Sundberg & Carey, Advanced Organic Chemistry: Parts A and B, 5th Edition (Springer, 2007).

### Example 1: Effect of glycans on microbial populations in vitro/ex-vivo

To determine the desired composition of microbime regulators, bacterial cultures are grown in the presence of candidate microbime regulators and assayed for their growth, community composition (e.g., by 16S rRNA gene sequencing), production of metabolites, and phenotypic or transcriptomic properties). Desired microbime regulators are selected based on their ability to elicit desired properties within the bacterial culture. Bacterial cultures include monocultures, mixed cultures, cultures isolated from humans or laboratory animal models, cultures isolated from a human or laboratory animal model and spiked with an isolate or collection of isolates, or cultures isolated from a human or laboratory animal model and depleted of a collection of species (for example, by application of an antibiotic). These assays can be performed in the presence of antibiotics or other test compounds. The results obtained from the *in vitro* assays are compared with those obtained after treating humans with microbime regulators or administering the microbime regulators to a laboratory animal establishing the *in vitro - in vivo* correlation of results.

### Example 2: Effect of glycans on commensal bacteria in vitro

An *in vitro* assay was performed to assess the ability of various bacterial strains, including commensals of the gastrointestinal tract, to utilize different sugars as growth substrates. This assay was designed to assess the ability of selected natural monosaccharides, disaccharides and synthetic sugars to promote the growth of microbiota associated with a healthy state. Bacterial strains were handled at all steps in an anaerobic chamber (AS-580, Anaerobe Systems) featuring a palladium catalyst. The chamber was initially made anaerobic by purging with an anaerobic gas mixture of 5% hydrogen, 5% carbon dioxide and 90% nitrogen and subsequently maintained in an anaerobic state using this same anaerobic gas mixture. Anaerobicity of the chamber was confirmed daily using Oxoid anaerobic indicator strips that change color in the presence of oxygen. All culture media, assay plates, other reagents and plastic consumables were pre-reduced in the anaerobic chamber for 24-48 hours prior to contact with bacteria. Sugars were prepared at 5% w/v in water, filter-sterilized and added to Costar 3370 assay plates for a final concentration of 0.5% w/v in the assay, with each microbiome regulator assayed in two non-adjacent wells and dextrose and water supplied as positive and negative controls.

Bacterial isolates were obtained from the American Type Culture Collection (ATCC) and Leibniz Institute DSMZ-German Institute of Microorganisms and Cell Cultures (DSMZ). Cultures of the Bacteroidetes *Bacteroides caccae* ATCC 43185 "BCA.26", *Bacteroides thetaiotaomicron* ATCC 29741 "BTH.8", *Bacteroides cellulosilyticus* DSM 14838 "BCE.71", *Parabacteroides distasonis* ATCC 8503 "PDI.6" and *Prevotella copri* DSM 18205 "PCO.72"; the Clostridiales *Clostridium scindens* ATCC 35704 "CSC.32", *Dorea formicigenerans* ATCC 27755 "DFO.36" and *Ruminococcus obeum* ATCC 29714 "ROB.74"; and the Bifidobacteria *Bifidobacterium longum* ATCC 15707 "BLO.16" and *Bifidobacterium longum* DSM 20088 "BLO.83", were grown anaerobically in Chopped Meat Glucose broth (CMG, Anaerobe Systems), a pre-reduced enriched medium including lean ground beef, enzymatic digest of casein, yeast extract, potassium phosphate, dextrose, cysteine, hemin and Vitamin K1, for 18-48 hours at 37° C. Inocula were prepared by determining the optical density of each culture at 600 nM (OD₆₀₀) in a Costar 3370 polystyrene 96-well flat-bottom assay plate using a Biotek Synergy 2 plate reader with Gen5 2.0 All-In-One Microplate Reader Software according to manufacturer's protocol, and diluting the cells to OD₆₀₀ 0.01 final in defined and semi-defined media that were prepared without sugars. *D. formicigenerans, P. distasonis* and *B. longum* isolates were tested in 900 mg/L sodium chloride, 26 mg/L calcium chloride dihydrate, 20 mg/L magnesium chloride hexahydrate, 10 mg/L manganese chloride tetrahydrate, 40 mg/L ammonium sulfate, 4 mg/L iron sulfate heptahydrate, 1 mg/L cobalt chloride hexahydrate, 300 mg/L potassium phosphate dibasic, 1.5 g/L sodium phosphate dibasic, 5 g/L sodium bicarbonate, 0.125 mg/L biotin, 1 mg/L pyridoxine, 1 m/L pantothenate, 75 mg/L histidine, 75 mg/L glycine, 75 mg/L tryptophan, 150 mg/L arginine, 150 mg/L methionine, 150 mg/L threonine, 225 mg/L valine, 225 mg/L isoleucine, 300 mg/L leucine, 400 mg/L cysteine, and 450 mg/L proline (Theriot CM et al. Nat Commun. 2014; 5:3114), supplemented with 0-3.5% (v/v) CMG. *B. thetaiotaomicron*, *B. caccae* and *B. cellulosyliticus* were tested in 100 mM potassium phosphate buffer (pH 7.2), 15 mM sodium chloride, 8.5 mM ammonium sulfate, 4 mM L-cysteine, 1.9 µM hematin, 200 µM L-histidine, 100 µM magnesium chloride, 1.4 µM iron sulfate heptahydrate, 50 µM calcium chloride, 1 µg/mL vitamin K3 and 5 ng/mL vitamin B12 (Martens EC et al. Cell Host & Microbe 2008; 4, 447-457). *C. scindens, P. copri* and *R. obeum* were tested in 10 g/L tryptone peptone, 5 g/L yeast extract, 0.5 g/L L-cysteine hydrochloride, 0.1 M potassium phosphate buffer pH 7.2, 1 µg/mL vitamin K3, 0.08% w/v calcium chloride, 0.4 µg/mL iron sulfate heptahydrate, 1 µg/mL resazurin, 1.2 µg/mL hematin, 0.2 mM histidine, 0.05% Tween 80, 0.5% meat extract (Sigma), 1% trace mineral supplement (ATCC), 1% vitamin supplement (ATCC), 0.017% v/v acetic acid, 0.001% v/v isovaleric acid, 0.2% v/v propionic acid and 0.2% v/v N-butyric acid (Romano KA et al. mBio 2015; 6(2):e02481-14). Bacteria were exposed to natural monosaccharides dextrose, D-fructose, D-galactose, L-arabinose, D-mannose, D-xylose, D (-) arabinose, ribose, L-fucose and L-rhamnose; natural disaccharides lactose, D (+) maltose and sucrose; and synthetic sugars lactulose, D-sorbitol, D-mannitol and sucralose at a final concentration of 0.5% w/v in 96-well microplates, 200 µL final volume per well, at 37° C for 18-24 hours, anaerobically. OD₆₀₀ measurements for each isolate at the end of the incubation period were obtained using a Biotek Synergy2 reader with Gen5 2.0 software according to manufacturer's specifications. Measurements were normalized by dividing the OD₆₀₀ readings of the isolate on test microbiome regulators by the average OD₆₀₀ of the isolate in medium supplemented with 0.5% w/v dextrose to facilitate comparison of microbiome regulator utilization by strains that grow within significantly different OD₆₀₀ ranges. **Table 7** summarizes the results obtained.

**Table 7. Sugar-supported growth of commensal bacteria**

| | Commensals, Average Normalized Growth | | | | | | | | | |
|---|---|---|---|---|---|---|---|---|---|---|
| | BCA. 26 | BCE. 71 | BTH. 8 | PDI. 6 | ROB. 74 | PCO. 72 | CSC. 32 | DFO. 36 | BLO. 16 | BLO. 83 |
| Monomers | | | | | | | | | | |
| D-fructose | ++ | +++ | ++ | +++ | ++ | ++ | +++ | +++ | +++ | +++ |
| D-galactose | ++ | +++ | +++ | +++ | + | +++ | ++ | + | - | +++ |
| L-arabinose | +++ | ++ | +++ | - | +* | ++ | - | +++ | ++ | - |
| D-mannose | +++ | +++ | +++ | +++ | ++ | - | + | - | - | -* |
| D-xylose | +++ | ++ | +++ | + | - | ++ | - | - | ++ | ND |
| D (-) arabinose | ++ | + | ++ | - | ++ | - | ++ | - | - | ND |
| ribose | - | + | - | +++ | - | - | +++ | +++ | - | ND |
| L-fucose | +++ | + | + | - | ++ | - | - | - | - | -* |
| L-rhamnose | - | ND | + | - | ++* | + | - | - | - | ND |

| Dimers | | | | | | | | | | |
|---|---|---|---|---|---|---|---|---|---|---|
| lactose | +++ | +++ | +++ | +++ | ++ | +++ | +++ | +++ | ++ | +++ |
| D (+) maltose | + | ++ | +++ | ++ | +++* | +++ | - | +++ | +++ | ND |
| sucrose | +++* | +++* | ++* | +* | ND | +++* | -* | -* | +* | ND |

| Synthetic | | | | | | | | | | |
|---|---|---|---|---|---|---|---|---|---|---|
| lactulose | ++ | ++ | - | + | -* | ++ | - | -* | ND | ++* |
| D-sorbitol | -* | -* | -* | -* | ND | -* | ++* | -* | -* | ND |
| D-mannitol | - | - | - | - | -* | - | + | -* | - | -* |
| sucralose | -* | -* | -* | -* | ND | +* | -* | -* | -* | ND |

| **Key** | |
|---|---|
| **symbol** | **NGV** |
| - | <0.2 |
| + | 0.2-0.6 |
| ++ | 0.6-1 |
| +++ | >1 |
| * | not statistically significant |

In the assay, most of the natural monosaccharides and natural disaccharides supported growth of most of the tested commensals, with an Average Normalized Growth value of at least 0.2. In the assay, the natural monosaccharides D-fructose, D-galactose and L-arabinose and the natural disaccharides lactose, D (+) maltose and sucrose supported some commensals from the taxa Bacteroidetes, Firmicutes, including Lachnospiraceae, and Bifidobacteria. In the assay, D-xylose supported some commensals from the taxa Bacteroidetes and Bifidobacteria, and D-mannose, D (+) arabinose, ribose and L-fucose supported some commensals from the taxa Bacteroidetes and Firmicutes. The synthetic sugars varied in the number of strains for which they supported growth in the assay. Lactulose supported growth of some strains from the taxa Bacteroidetes and Bifidobacteria in the assay, and D-sorbitol and D-mannitol each supported growth of CSC.32 alone among the panel of 10 commensals in the assay. Sucralose supported growth of PCO.72 alone among the panel of 10 commensals in the assay.

### Example 3. Synergistic effects of microbiome regulator compositions comprising sugars, short chain fatty acids, amino acids and vitamins on bacterial growth

An *in vitro* growth assay was performed to determine whether natural monomeric sugars such as, e.g., glucose, fructose or xylose exert synergistic effects on the growth of bacterial gut commensals when supplemented with the amino acids, such as, e.g., cysteine, histidine or leucine; short chain fatty acids, such as, e.g., acetate, butyrate or propionate; or vitamin, e.g., pantothenate. In each assay, a single sugar was tested in combination with a single amino acid, vitamin or short chain fatty acid. Strains were handled under strict anaerobic conditions with pre-reduced reagents and materials. The gut commensals *Parabacteroides distasonis* ATCC 8503 "PDI.6", *Bacteroides vulgatus* ATCC 8482 "BVU.10" and *Bifidobacterium longum* ATCC 15707 "BLO.16" were grown under anaerobic conditions in Chopped Meat Glucose broth (CMG, Anaerobe Systems), a pre-reduced enriched medium including lean ground beef, enzymatic digest of casein, yeast extract, potassium phosphate, dextrose, cysteine, hemin and Vitamin K1, for 18-24 hours at 37° C. Strains were diluted to OD₆₀₀ 0.01 final in a minimal medium including 900 mg/L sodium chloride, 26 mg/L calcium chloride dihydrate, 20 mg/L magnesium chloride hexahydrate, 10 mg/L manganese chloride tetrahydrate, 40 mg/L ammonium sulfate, 4 mg/L iron sulfate heptahydrate, 1 mg/L cobalt chloride hexahydrate, 300 mg/L potassium phosphate dibasic, 1.5 g/L sodium phosphate dibasic, 5 g/L sodium bicarbonate, 0.125 mg/L biotin, 1 mg/L pyridoxine, 75 mg/L glycine, 75 mg/L tryptophan, 150 mg/L arginine, 150 mg/L methionine, 150 mg/L threonine, 225 mg/L valine, 225 mg/L isoleucine, and 450 mg/L proline (Theriot CM et al. Nat Commun. 2014; 5:3114), supplemented with 0% -3.5% (v/v) CMG; either fructose, glucose or xylose, as described below; pantothenate, histidine, cysteine and leucine as described below; and short chain fatty acids as described below. Fructose was tested at 0.06-0.13% (w/v) with BVU.10, 0.03%-0.13% with BLO.16, and 0.016%-0.06% with PDI.6. Glucose was tested at 0.016%-0.06% with BVU.10 and PDI.6, and 0.03%-0.13% with BLO.16. Xylose was tested at 0.03%-0.13% with BVU.10, 0.06%-0.25% with BLO.16, and 0.016%-0.06% with PDI.6. Each supplement (pantothenate, histidine, cysteine, leucine, acetate, butyrate or propionate) was assessed separately across a range of 8 concentrations created by 2-fold serial dilutions from its highest concentration, while the other amino acids or and/or vitamin were held constant. For each strain, pantothenate was tested at 0-2 mg/L, histidine was tested at 0-150 mg/L, cysteine was tested at 0-800 mg/L, leucine was tested at 0-600 mg/L, and acetate, butyrate and propionate were tested at 0-0.75 ml/L. When not being tested in a dose response, pantothenate was included at 1 mg/L, histidine was included at 75 mg/L, leucine was included at 300 mg/L, and cysteine was included at 400 mg/L. Short chain fatty acids acetate, butyrate and propionate were only included in the assay when assessed in a dose response. The assay was performed in triplicate in 96-well polystyrene plates with 200 uL volume per well and incubated at 37° C for 18-24 hours. OD₆₀₀ measurements were obtained following the incubation period with a Biotek Synergy microplate reader. A total of 1456 conditions were tested in triplicate to assess potential synergy in 63 different combinations of strains, sugars and supplements.

OD₆₀₀ shifts were obtained by subtracting the OD₆₀₀ of the corresponding supplement-free control. The average OD600 shift was calculated for each set of 3 replicates. To assess whether a particular combination of sugar and supplement had a synergistic effect in promoting the growth of a given strain in the assay, a Growth Promotion Index (GPI) was calculated for the lowest concentration of a supplement that produced an OD₆₀₀ shift of at least 0.07. The GPI was calculated by dividing the average OD600 shift obtained with a given combination of sugar and supplement by the sum of the average OD600 shift obtained with half the sugar concentration and the same supplement concentration plus the average OD600 shift obtained with the same sugar concentration and half the supplement concentration: GPI = OD₆₀₀ shift at sugar [X], supplement [Y] / [(OD600 shift at sugar [X/2], supplement [Y]) + (OD600 shift at sugar [X], supplement [Y/2])]. Since synergy exists when the effect is greater than sum of the parts, e.g., when 0.5+0.5 > 1, a GPI > 1 indicates synergy. Results are summarized in **Table 8.**

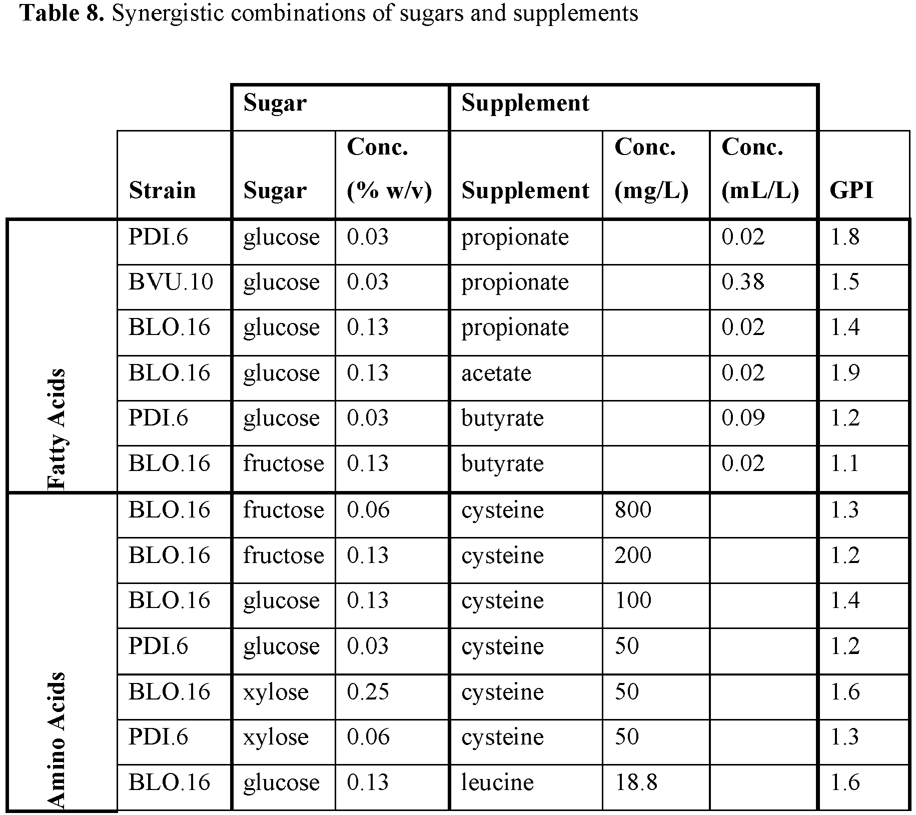

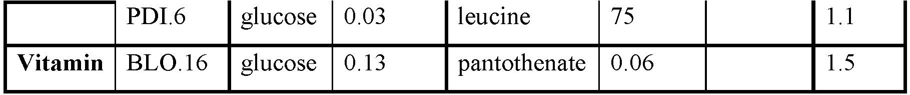

In the assay, 15 conditions (about 1% of conditions tested) produced GPIs greater than 1, consistent with synergistic effects of the tested sugar and short chain fatty acid, amino acid or vitamin on the growth of the tested strain. Synergy was not detected with 48 of the 63 tested combinations of sugars, supplements and strains. Short chain fatty acids varied with regard to the sugars and strains with which synergy was observed in the assay: propionate and glucose had synergistic effects on PDI.6, BVU.10 and BLO.16; acetate and glucose had synergistic effects on BLO.16; butyrate and glucose had synergistic effects on PDI.6; and butyrate and fructose had synergistic effects on BLO.16. Amino acids varied with regard to the sugars and strains with which synergy was observed in the assay. In the assay, cysteine was synergistic in combination with fructose, glucose and xylose for BLO. 16, and cysteine was also synergistic in combination with glucose and xylose for PDI.6. Leucine and glucose had synergistic effects on BLO.16 and PDI.6 in the assay. The vitamin pantothenate had synergistic effects with glucose on BLO.16 in the assay. 9 of the 15 synergistic interactions in the assay were observed with BLO.16, 5 were observed with PDI.6, and 1 was observed with BVU.10. 6 of the 15 synergistic interactions in the assay were observed with cysteine, 3 of the synergistic interaction were observed with propionate.

### Example 4: Collection of fecal samples

Fecal samples were collected by providing subjects with the Fisherbrand Commode Specimen Collection System (Fisher Scientific) and associated instructions for use. Collected samples were stored with ice packs or at -80° C until processing (McInnes & Cutting, Manual of Procedures for Human Microbiome Project: Core Microbiome Sampling Protocol A, v12.0, 2010, hmpdacc.org/doc/HMP MOP Version12 0 072910.pdf). Alternative collection devices may also be used. For example, samples may be collected into the Globe Scientific Screw Cap Container with Spoon (Fisher Scientific) or the OMNIgene-GUT collection system (DNA Genotek, Inc.), which stabilizes microbial DNA for downstream nucleic acid extraction and analysis. Aliquots of fecal samples were stored at -20 °C and -80 °C following standard protocols known to one skilled in the art.

### Example 5: Determining the titer of microbial samples collected from feces and culturing samples

To determine the titer of common bacteria of the gastrointestinal tract, fecal samples were collected as described in Example 4 and prepared as a 10% weight/volume suspensions in sterile phosphate buffered saline (PBS). Ten-fold serial dilutions were prepared in sterile PBS and plated (100 µL per dilution) to Brucella Blood Agar (Anaerobe Systems; incubated anaerobically to non-selectively titer common member of the gut microbiota, including Bacteroides, or incubated aerobically to non-selectively titer facultative anaerobes such as Proteobacteria). Bacteroides Bile Esculin Agar (Anaerobe Systems; cultured anaerobically to titer Bacteroides fragilis group), Cycloserine-Cefoxitin Fructose Agar (Anaerobe Systems; cultured anaerobically to titer *Clostridium difficile*), Lactobacillus-MRS Agar (Anaerobe Systems; cultured anaerobically to titer Lactobacillus), Eosin Methylene Blue Agar (Teknova; cultured aerobically to titer *Escherichia coli* and other Gram-negative enteric bacteria), Bile Esculin Agar (BD; cultured aerobically to titer Enterococcus species), Bifidobacterium Selective Agar (Anaerobe Systems; to titer Bifidobacterium species), or MacConkey Agar (Fisher Scientific; to titer *E. coli* and other Gram-negative enteric bacteria) may also be used. Plates were incubated at 37°C under aerobic or anaerobic conditions as appropriate for the target species. After 24-48 hours, colonies were counted and used to back-calculate the concentration of viable cells in the original sample.

To non-selectively culture samples containing bacteria collected from a human or laboratory animal model, rich media or agar such as Brucella Blood Agar (Anaerobe Systems), Brain Heart Infusion Broth (Teknova), or Chopped Meat Glucose Broth (Anaerobe Systems) were used. A minimal media formulation such as M9 (Life Technologies) supplemented with amino acids, carbon sources, or other nutrients as needed were used to non-selectively culture bacteria during in vitro assays testing the effects of microbiome regulators or other compounds on bacterial populations. Alternatively, other minimal media formulations known to one skilled in the art were used, for example, as reported in Martens et al. (Mucosal Glycan Foraging Enhances Fitness and Transmission of a Saccharolytic Human Gut Bacterial Symbiont, 2008, Cell Host & Microbe, 4:447-457). Alternatively, fecal slurries at a concentration of 0.1%-10% weight/volume in PBS were used in the presence or absence of additional media elements for in vitro assays testing the effects of microbiome regulators or other compounds on bacterial populations.

### Example 6: Effects of microbiome regulators on ex vivo human fecal microbial communities

The *ex vivo* assay was designed to determine if natural monosaccharides, such as glucose can modulate a complex human fecal microbial community. Fecal samples and slurries were handled in an anaerobic chamber (AS-580, Anaerobe Systems) featuring a palladium catalyst. Glucose and a commercially available control, FOS (Nutraflora FOS; NOW Foods, Bloomingdale IL), were prepared at 5% w/v in water, filter-sterilized and added to 96- deep well assay plates for a final concentration of 0.5% w/v.

A human fecal sample donation was stored at -80 °C. To prepare working stocks the fecal sample was transferred into the anaerobic chamber and allowed to thaw. The fecal sample was prepared to 20% w/v in phosphate buffered saline (PBS) pH 7.4 (P0261, Teknova Inc., Hollister, CA), 15% glycerol, centrifuged at 2,000xg, the supernatant was removed, and the pellet was suspended in PBS pH 7.4 to 1% w/v fecal slurry. Prepared 1% w/v fecal slurry were contacted with microbiome regulators to 500 µL final volume per well, at 37° C for 18 hours, anaerobically. Genomic DNA was extracted from the fecal samples and variable region 4 of the 16S rRNA gene was amplified and sequenced (Earth Microbiome Project protocol; www.earthmicrobiome.org/emp-standard-protocols/16s/and Caporaso JG et al. 2012. ISME J.). Operational Taxonomic Units (OTUs) were generated by aligning 16S rRNA sequences at 97% identity. Microbial communities were compared to each other using UniFrac distance metric (Lozupone C. et al., Appl. Environ. Microbiol. December 2005 vol. 71 no. 12 8228-8235).

In the assay, glucose increased the relative abundance of Bifidobacteriales (**Fig. 1A**) and Bifidobacteria (**Fig. 1D**) in 1% w/v fecal slurry compared to control fecal slurry lacking added carbon source. It did so comparably to FOS. The relative abundance of Bacteroidales (**Fig. 1B**) and Clostridiales (**Fig. 1C**) decreased in FOS and glucose.

In addition, the presence of both glucose and FOS increased the relative abundance of Actinobacteria while the relative of Bacteroidetes and Firmicutes were decreased in 1% w/v fecal slurry, compared to control fecal slurry lacking added carbon source (**Fig. 1E**).

### Example 7. Effect of microbiome regulators on microbial SCFA metabolite production in vitro

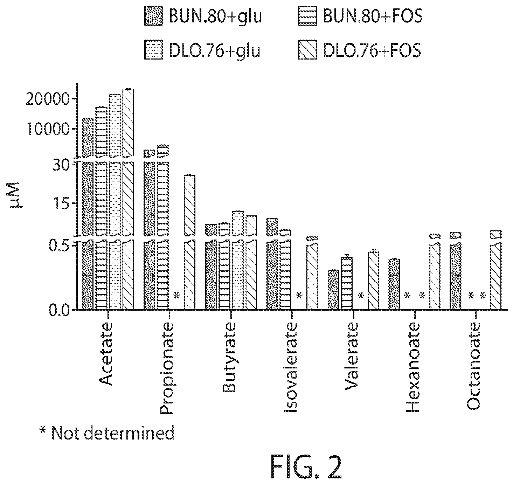

An *in vitro* assay was performed to assess the production of short chain fatty acids by gut commensal bacteria cultured with glucose or FOS as a carbon source. Strains were handled under strictly anaerobic conditions in an AS-580 anaerobic chamber (Anaerobe Systems) using pre-reduced reagents and materials. The Bacteroidete *Bacteroides uniformis* (ATCC 8492) "BUN.80" was tested in 100 mM potassium phosphate buffer (pH 7.2), 15 mM sodium chloride, 8.5 mM ammonium sulfate, 4 mM L-cysteine, 1.9 µM hematin, 200 µM L-histidine, 100 µM magnesium chloride, 1.4 µM iron sulfate heptahydrate, 50 µM calcium chloride, 1 µg/mL vitamin K3 and 5 ng/mL vitamin B12 (Martens EC et al. Cell Host & Microbe 2008; 4, 447-457). The Lachnospiracea *Dorea longicatena* (DSM 13814) "DLO.76" was tested in 900 mg/L sodium chloride, 26 mg/L calcium chloride dihydrate, 20 mg/L magnesium chloride hexahydrate, 10 mg/L manganese chloride tetrahydrate, 40 mg/L ammonium sulfate, 4 mg/L iron sulfate heptahydrate, 1 mg/L cobalt chloride hexahydrate, 300 mg/L potassium phosphate dibasic, 1.5 g/L sodium phosphate dibasic, 5 g/L sodium bicarbonate, 0.125 mg/L biotin, 1 mg/L pyridoxine, 1 m/L pantothenate, 75 mg/L histidine, 75 mg/L glycine, 75 mg/L tryptophan, 150 mg/L arginine, 150 mg/L methionine, 150 mg/L threonine, 225 mg/L valine, 225 mg/L isoleucine, 300 mg/L leucine, 400 mg/L cysteine, and 450 mg/L proline (Theriot CM et al. Nat Commun. 2014; 5:3114), supplemented with 10% (v/v) Chopped Meat Glucose broth (Anaerobe Systems). Bacteria were exposed to either glucose (dextrose) or FOS at 0.5% (w/v) final and incubated at 37° C for 39-50 hours. Following incubation, cells were pelleted from 1.5 mL aliquots of cultures in duplicate by centrifugation at 18,000 x g for five minutes, the supernatant was sterilized through a 0.22 um polyethersulfone filter, and the supernatant was stored at -80° C or on dry ice until it was analyzed. Short chain fatty acid (SCFA) analysis was performed on the filtered culture supernatants using a cold extraction of short chain fatty acids, measured by EI-CGMS without derivatization. **Figure 2** summarizes the results obtained.

In the assay, cultures of Bacteroidete BUN.80 and Lachnospiracea DLO.76 grown with either glucose or FOS produced supernatants with total SCFA concentrations in excess of 15,000 µM. Acetate was the SCFA produced in the highest concentrations in the assay, and propionate was produced at the second-highest levels. Butyrate, isovalerate, valerate, hexanoate and octanoate were also detected in the in the assay.

### EQUIVALENTS AND SCOPE

This application refers to various issued patents, published patent applications, journal articles, and other publications. If there is a conflict between any of the incorporated references and the instant specification, the specification shall control. In addition, any particular embodiment of the present invention that falls within the prior art may be explicitly excluded from any one or more of the claims. Because such embodiments are deemed to be known to one of ordinary skill in the art, they may be excluded even if the exclusion is not set forth explicitly herein. Any particular embodiment of the invention can be excluded from any claim, for any reason, whether or not related to the existence of prior art.

Those skilled in the art will recognize or be able to ascertain using no more than routine experimentation many equivalents to the specific embodiments described herein. The scope of the present embodiments described herein is not intended to be limited to the above Description, Figures, or Examples but rather is as set forth in the appended claims.

## Claims

1. A dosage form comprising a composition for use in promoting growth of commensal bacteria, said composition comprising a microbiome regulator capable of modulating the microbiome of a subject, wherein the dosage form targets the release of the composition substantially in the large intestine,
wherein the microbiome regulator comprises a sugar or a sugar alcohol, wherein the sugar or sugar alcohol is selected from a monosaccharide and a disaccharide, wherein the sugar or sugar alcohol is selected from the group consisting of D-fructose, D-galactose, L-arabinose, D-mannose, D-xylose, D-arabinose, ribose, L-fucose, L-rhamnose, lactose, D-maltose, sucrose, and glucose;
and wherein the composition comprises more than about 50% (w/w) of the microbiome regulator.

2. The dosage form of claim 1, wherein the composition is formulated as a unit dosage form.

3. The dosage form of any one of claims 1-2, wherein the unit dosage form comprises a liquid dosage form or solid dosage form.

4. The dosage form of any one of claims 2-3, wherein the unit dosage form is formulated for oral, gastric or rectal delivery.

5. The dosage form of any one of claims 3, wherein the solid dosage form for oral administration comprises a pill, tablet, or capsule.

6. The dosage form of claim 5, wherein the solid dosage form for oral administration is enterically coated, coated for timed release, or coated for controlled release.

7. The dosage form of any one of claims 1-6, wherein the dosage form is targeted to the cecum, colon, or rectum.

8. The dosage form of any one of the preceding claims, wherein the sugar monosaccharide comprises L-arabinose.

9. The dosage form of any one of the preceding claims, wherein the sugar monosaccharide comprises glucose.

10. The dosage form of any one of the preceding claims, wherein the composition comprises more than about 80%, about 85%, about 90% (w/w), about 95%, about 96%, about 97%, about 98%, or more than about 99%, (w/w) of the microbiome regulator.

## Patentansprüche

1. Eine Darreichungsform, umfassend eine Zusammensetzung zur Verwendung bei der Förderung des Wachstums von Kommensalbakterien, wobei die Zusammensetzung einen Mikrobiomregulator umfasst, der das Mikrobiom eines Individuums modulieren kann, wobei die Darreichungsform auf die Freisetzung der Zusammensetzung im Wesentlichen im Dickdarm zielt,
wobei der Mikrobiomregulator einen Zucker oder einen Zuckeralkohol umfasst, wobei der Zucker oder Zuckeralkohol aus einem Monosaccharid und einem Disaccharid ausgewählt ist, wobei der Zucker oder Zuckeralkohol aus der Gruppe bestehend aus D-Fructose, D-Galactose, L-Arabinose, D-Mannose, D-Xylose, D-Arabinose, Ribose, L-Fucose, L-Rhamnose, Lactose, D-Maltose, Saccharose und Glucose ausgewählt ist;
und wobei die Zusammensetzung mehr als etwa 50 Gew.-% des Mikrobiomregulators umfasst.

2. Die Darreichungsform nach Anspruch 1, wobei die Zusammensetzung als Unit-Dose-Arzneiform formuliert ist.

3. Die Darreichungsform nach einem der Ansprüche 1-2, wobei die Unit-Dose-Arzneiform eine flüssige Darreichungsform oder feste Darreichungsform umfasst.

4. Die Darreichungsform nach einem der Ansprüche 2-3, wobei die Unit-Dose-Arzneiform zur oralen, gastrischen oder rektalen Verabreichung formuliert ist.

5. Die Darreichungsform nach Anspruch 3, wobei die feste Darreichungsform zur oralen Verabreichung eine Pille, Tablette oder Kapsel umfasst.

6. Die Darreichungsform nach Anspruch 5, wobei die feste Darreichungsform zur oralen Verabreichung einen magensaftresistenten Überzug oder einen Überzug für Retard (Timed Release oder Controlled Release) aufweist.

7. Die Darreichungsform nach einem der Ansprüche 1-6, wobei die Darreichungsform auf den Blinddarm, Kolon oder Mastdarm ausgerichtet ist.

8. Die Darreichungsform nach einem der vorhergehenden Ansprüche, wobei das Zuckermonosaccharid L-Arabinose umfasst.

9. Die Darreichungsform nach einem der vorhergehenden Ansprüche, wobei das Zuckermonosaccharid Glucose umfasst.

10. Die Darreichungsform nach einem der vorhergehenden Ansprüche, wobei die Zusammensetzung mehr als etwa 80 Gew.-%, etwa 85 Gew.-%, etwa 90 Gew.-%, etwa 95 Gew.-%, etwa 96 Gew.-%, etwa 97 Gew.-%, etwa 98 Gew.-% oder mehr als etwa 99 Gew.-% des Mikrobiom-Regulators umfasst.

## Revendications

1. Une forme posologique comprenant une composition destinée à être utilisée pour favoriser la croissance de bactéries commensales, ladite composition comprenant un régulateur de microbiome capable de moduler le microbiome d'un sujet, dans laquelle la forme posologique cible la libération de la composition sensiblement dans le gros intestin,
dans laquelle le régulateur de microbiome comprend un sucre ou un alcool de sucre, le sucre ou l'alcool de sucre étant choisi parmi un monosaccharide et un disaccharide, le sucre ou l'alcool de sucre étant choisi dans le groupe constitué par le D-fructose, le D-galactose, le L-arabinose, le D-mannose, le D-xylose, D-arabinose, le ribose, le L-fucose, le L-rhamnose, le lactose, le D-maltose, le saccharose, et le glucose ;
et dans laquelle la composition comprend plus d'environ 50 % (p/p) du régulateur de microbiome.

2. La forme posologique selon la revendication 1, dans laquelle la composition est formulée sous la forme d'une forme posologique unitaire.

3. La forme posologique selon l'une quelconque des revendications 1 à 2, dans laquelle la forme posologique unitaire comprend une forme posologique liquide ou une forme posologique solide.

4. La forme posologique selon l'une quelconque des revendications 2 à 3, dans laquelle la forme posologique unitaire est formulée pour une administration orale, gastrique ou rectale.

5. La forme posologique selon l'une quelconque des revendications 3, dans laquelle la forme posologique solide pour administration orale comprenant une pilule, un comprimé, ou une capsule.

6. La forme posologique selon la revendication 5, dans laquelle la forme posologique solide pour administration orale est enrobée de façon entérique, enrobée pour une libération programmée, ou enrobée pour une libération contrôlée.

7. La forme posologique selon l'une quelconque des revendications 1 à 6, dans laquelle la forme posologique est ciblée sur le caecum, le côlon ou le rectum.

8. La forme posologique selon l'une quelconque des revendications précédentes, dans laquelle le monosaccharide de sucre comprend du L-arabinose.

9. La forme posologique selon l'une quelconque des revendications précédentes, dans laquelle le monosaccharide de sucre comprend du glucose.

10. La forme posologique selon l'une quelconque des revendications précédentes, la composition comprenant plus d'environ 80 %, environ 85 %, environ 90 % (p/p), environ 95 %, environ 96 %, environ 97 %, environ 98 %, ou plus d'environ 99 %, (p/p) du régulateur de microbiome.
